(19) Europäisches Patentamt
European Patent Office
Office européen des brevets

(11) EP 4 118 697 B1

# (12) EUROPEAN PATENT SPECIFICATION

(45) Date of publication and mention of the grant of the patent:
**24.01.2024 Bulletin 2024/04**

(21) Application number: **21711556.7**

(22) Date of filing: **12.03.2021**

(51) International Patent Classification (IPC):
***H10K 85/60*** *(2023.01)*

(52) Cooperative Patent Classification (CPC):
**H10K 85/622; H10K 85/615; H10K 85/626;**
H10K 50/11; H10K 2101/90; Y02E 10/549;
Y02P 70/50

(86) International application number:
**PCT/EP2021/056390**

(87) International publication number:
**WO 2021/180950 (16.09.2021 Gazette 2021/37)**

## (54) MATERIALS FOR ORGANIC ELECTROLUMINESCENT DEVICES

MATERIALIEN FÜR ORGANISCHE ELEKTROLUMINESZENZVORRICHTUNGEN

MATÉRIAUX POUR DISPOSITIFS ÉLECTROLUMINESCENTS ORGANIQUES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **13.03.2020 EP 20163090**

(43) Date of publication of application:
**18.01.2023 Bulletin 2023/03**

(73) Proprietor: **Merck Patent GmbH**
**64293 Darmstadt (DE)**

(72) Inventors:
- **LINGE, Rouven**
  **64293 Darmstadt (DE)**
- **ENGEL, Miriam**
  **64293 Darmstadt (DE)**
- **STOLZ, Sebastian**
  **64293 Darmstadt (DE)**
- **MEYER, Sebastian**
  **64293 Darmstadt (DE)**

(74) Representative: **Merck Patent Association**
**Merck Patent GmbH**
**64271 Darmstadt (DE)**

(56) References cited:
**US-A1- 2009 026 919 US-A1- 2010 187 505**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 4 118 697 B1

## Description

The present invention relates to a composition comprising a compound of formula (H1) and a compound of formula (H2). The present invention furthermore relates to a formulation comprising a composition comprising a compound of formula (H1) and a formula (H2) and a solvent. Finally, the present invention relates to an electronic device comprising such a composition.

The development of functional compounds for use in electronic devices is currently the subject of intensive research. The aim is, in particular, the development of compounds with which improved properties of electronic devices in one or more relevant points can be achieved, such as, for example, power efficiency and lifetime of the device as well as colour coordinates of the emitted light.

In accordance with the present invention, the term electronic device is taken to mean, inter alia, organic integrated circuits (OICs), organic field-effect transistors (OFETs), organic thin-film transistors (OTFTs), organic light-emitting transistors (OLETs), organic solar cells (OSCs), organic optical detectors, organic photoreceptors, organic field-quench devices (OFQDs), organic light-emitting electrochemical cells (OLECs), organic laser diodes (O-lasers) and organic electroluminescent devices (OLEDs).

Of particular interest is the provision of compounds for use in the last-mentioned electronic devices called OLEDs. The general structure and the functional principle of OLEDs are known to the person skilled in the art and are described, for example, in US 4539507.

Further improvements are still necessary with respect to the performance data of OLEDs, in particular with a view to broad commercial use, for example in display devices or as light sources. Of particular importance in this connection are the lifetime, the efficiency and the operating voltage of the OLEDs and as well as the colour values achieved. In particular, in case of blue-emitting OLEDs, there is potential for improvement with respect to the efficiency, lifetime and operating voltage of the devices.

An important starting point for achieving the said improvements is the choice of the emitter compound and of the host compound. Indeed, the emitter compound is generally employed in the emitting layer in combination with a second compound, which serves as matrix compound or host compound. An emitter compound here is taken to mean a compound which emits light during operation of the electronic device. A host compound in this case is taken to mean a compound which is present in the mixture in a greater proportion than the emitter compound. The term matrix compound and the term host compound can be used synonymously. The host compound preferably does not emit light. Even if a plurality of different host compounds are present in the mixture of the emitting layer, their individual proportions are typically greater than the proportion of the emitter compounds, or the proportions of the individual emitter compounds if a plurality of emitter compounds are present in the mixture of the emitting layer.

Such embodiments have been described for fluorescent emitting layers for example in US 4769292.

If a mixture of a plurality of compounds is present in the emitting layer, the emitter compound is typically the component present in smaller amount, i.e. in a smaller proportion than the other compounds present in the mixture of the emitting layer. In this case, the emitter compound is also referred to as dopant.

Hosts compounds for fluorescent emitters that are known from the prior art are a multiplicity of compounds. The emitting layer may comprise one host compounds or more.

Host compounds comprising phenanthrene groups have been disclosed in the prior art (for example in WO 2009/100925 or US 2009/026919 A1). Host compounds comprising benzanthracene groups have also been disclosed in the prior art (for example in WO 2015/158409).

However, there is still a need for further host materials for fluorescent emitters, which may be employed in OLEDs and lead to OLEDs having very good properties in terms of lifetime, color emission and efficiency. More particularly, there is a need for host materials for fluorescent emitters combining very high efficiencies, very good lifetime and very good thermal stability.

Furthermore, it is known that an OLED may comprise different layers, which may be applied either by vapour deposition in a vacuum chamber or by processing from a solution. The processes based on vapour deposition lead to very good results, but they might be complex and expensive. Therefore, there is also a need for compositions comprising OLED materials that can be easily and reliably processed from a solution. More particularly, there is a need for compositions comprising OLED materials that can be deposited as homogeneous films during the fabrication of OLEDs when processed from a formulation, more particularly from a solution like an ink. In this case, the materials should have good solubility properties in the solution that comprises them and the deposited films comprising OLED materials should be as smooth as possible after the drying step leading to the removing of the solvent. It is important that the deposited layer form a smooth and homogenous film as layer thickness inhomogeneities cause uneven luminance distributions with areas of thinner film thickness showing increased luminance and thicker areas with reduced luminance, which leads to a decrease of the OLED's quality. At the same time, the OLEDs comprising the films processed form a solution should exhibit good performances, for example in terms of lifetime, operating voltage and efficiency.

There is furthermore still a need for processes, which lead to stable OLED materials, which are easily purified

and easily processed. There is a need for processes, which are economically and qualitatively interesting by providing OLED materials in acceptable purity and with a high yield.

The present invention is thus based on the technical object of providing compositions comprising OLED materials, which are suitable for use in electronic devices, such as OLEDs, more particularly as a matrix component for fluorescent emitters. The present invention is also based on the technical object of providing compositions comprising OLED materials, which are particularly suitable for solution processing. The present invention is also based on the technical object of providing processes.

In investigations on novel compositions for use in electronic devices, it has now been found, that the compositions comprising a compound of formula (H1) and a compound of formula (H2) as defined below are eminently suitable for use in electronic devices. In particular, they achieve one or more, preferably all, of the above-mentioned technical objects.

The present application thus relates to a composition comprising a compound of formula (H1) and a compound of formula (H2),

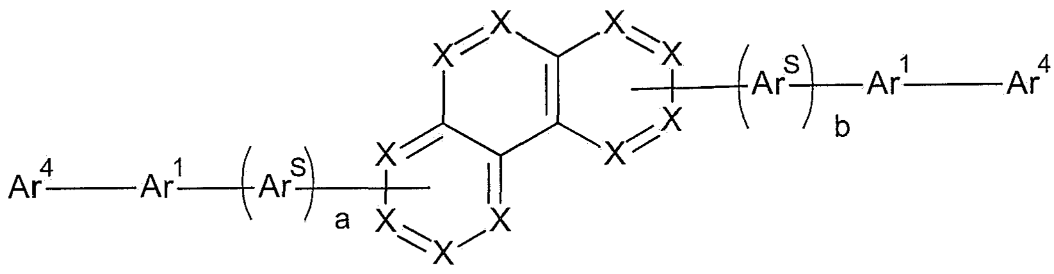

formula (H1)

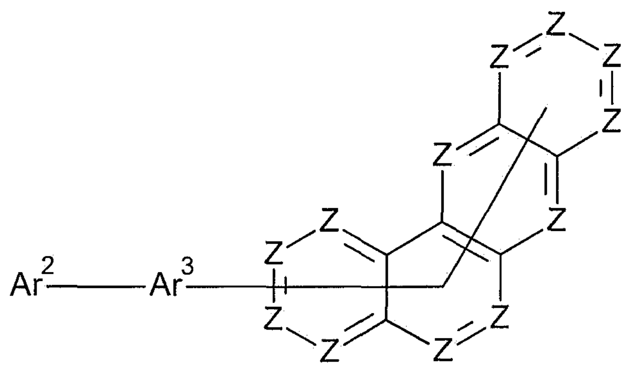

formula (H2)

where the following applies to the symbols and indices used:

X stands on each occurrence, identically or differently, for $CR^X$ or N; or X is C if X is bonded to a group $Ar^1$ or $Ar^S$;

Z stands on each occurrence, identically or differently, for $CR^Z$ or N; or Z is C if Z is bonded to a group $Ar^3$;

$Ar^1$ is, on each occurrence, identically or differently, an aryl or heteroaryl group having 10 to 60 aromatic ring atoms, which may in each case also be substituted by one or more radicals $R^V$;

$Ar^3$ is, on each occurrence, identically or differently, an aryl or heteroaryl group having 10 to 60 aromatic ring atoms, which may in each case also be substituted by one or more radicals $R^Y$;

$Ar^2$, $Ar^4$, $Ar^S$ are, on each occurrence, identically or differently, an aromatic or heteroaromatic ring system having 5 to 60 aromatic ring atoms, which may in each case also be substituted by one or more radicals R;

$R^V$, $R^X$, $R^Y$, $R^Z$ stand on each occurrence, identically or differently, for H, D, F, Cl, Br, I, CHO, CN, C(=O)Ar,

$P(=O)(Ar)_2$, S(=O)Ar, $S(=O)_2Ar$, $N(R)_2$, $N(Ar)_2$, $NO_2$, $Si(R)_3$, $B(OR)_2$, $OSO_2R$, a straight-chain alkyl, alkoxy or thioalkyl group having 1 to 40 C atoms or branched or a cyclic alkyl, alkoxy or thioalkyl group having 3 to 40 C atoms, each of which may be substituted by one or more radicals R, where in each case one or more non-adjacent $CH_2$ groups may be replaced by RC=CR, C≡C, $Si(R)_2$, $Ge(R)_2$, $Sn(R)_2$, C=O, C=S, C=Se, P(=O)(R), SO, $SO_2$, O, S or CONR and where one or more H atoms may be replaced by D, F, Cl, Br, I, CN or $NO_2$, an aromatic or heteroaromatic ring system having 5 to 60 aromatic ring atoms, which may in each case be substituted by one or more radicals R, or an aryloxy group having 5 to 60 aromatic ring atoms, which may be substituted by one or more radicals R; where two adjacent radicals $R^V$, two adjacent radicals $R^X$, two adjacent radicals $R^Y$, two adjacent radicals $R^Z$ may form an aliphatic, aromatic or heteroaromatic ring system together, which may be substituted by one or more radicals R;

R stands on each occurrence, identically or differently, for H, D, F, Cl, Br, I, CHO, CN, C(=O)Ar, $P(=O)(Ar)_2$, S(=O)Ar, $S(=O)_2Ar$, $N(R')_2$, $N(Ar)_2$, $NO_2$, $Si(R')_3$, $B(OR')_2$, $OSO_2R'$, a straight-chain alkyl, alkoxy or thioalkyl group having 1 to 40 C atoms or branched or a cyclic alkyl, alkoxy or thioalkyl group having 3 to 40 C atoms, each of which may be substituted by one or more radicals R , where in each case one or more non-adjacent $CH_2$ groups may be replaced by R'C=CR', C=C, $Si(R')_2$, $Ge(R')_2$, $Sn(R')_2$, C=O, C=S, C=Se, P(=O)(R'), SO, $SO_2$, O, S or CONR' and where one or more H atoms may be replaced by D, F, Cl, Br, I, CN or $NO_2$, an aromatic or heteroaromatic ring system having 5 to 60 aromatic ring atoms, which may in each case be substituted by one or more radicals R', or an aryloxy group having 5 to 60 aromatic ring atoms, which may be substituted by one or more radicals R ; where two adjacent substituents R may form an aliphatic or aromatic ring system together, which may be substituted by one or more radicals R';

Ar is, on each occurrence, identically or differently, an aromatic or heteroaromatic ring system having 5 to 60 aromatic ring atoms, which may in each case also be substituted by one or more radicals R';

R' stands on each occurrence, identically or differently, for H, D, F, Cl, Br, I, CN, a straight-chain alkyl, alkoxy or thioalkyl group having 1 to 20 C atoms or branched or cyclic alkyl, alkoxy or thioalkyl group having 3 to 20 C atoms, where in each case one or more non-adjacent $CH_2$ groups may be replaced by SO, $SO_2$, O, S and where one or more H atoms may be replaced by D, F, Cl, Br or I, or an aromatic or heteroaromatic ring system having 5 to 24 aromatic ring atoms; and

a, b are on each occurrence, identically or differently, 0 or 1; wherein when a or b is 0, then the corresponding $Ar^S$ is absent and the group $Ar^1$ is directly bonded to a group X.

Adjacent substituents in the sense of the present invention are substituents which are bonded to atoms which are linked directly to one another or which are bonded to the same atom.

Furthermore, the following definitions of chemical groups apply for the purposes of the present application:
An aryl group in the sense of this invention contains 6 to 60 aromatic ring atoms, preferably 6 to 40 aromatic ring atoms, more preferably 6 to 20 aromatic ring atoms; a heteroaryl group in the sense of this invention contains 5 to 60 aromatic ring atoms, preferably 5 to 40 aromatic ring atoms, more preferably 5 to 20 aromatic ring atoms, at least one of which is a heteroatom. The heteroatoms are preferably selected from N, O and S. This represents the basic definition. If other preferences are indicated in the description of the present invention, for example with respect to the number of aromatic ring atoms or the heteroatoms present, these apply.

An aryl group or heteroaryl group here is taken to mean either a simple aromatic ring, i.e. benzene, or a simple heteroaromatic ring, for example pyridine, pyrimidine or thiophene, or a condensed (annellated) aromatic or heteroaromatic polycycle, for example naphthalene, phenanthrene, quinoline or carbazole. A condensed (annellated) aromatic or heteroaromatic polycycle in the sense of the present application consists of two or more simple aromatic or heteroaromatic rings condensed with one another.

An aryl or heteroaryl group, which may in each case be substituted by the above-mentioned radicals and which may be linked to the aromatic or heteroaromatic ring system via any desired positions, is taken to mean, in particular, groups derived from benzene, naphthalene, anthracene, phenanthrene, pyrene, dihydropyrene, chrysene, perylene, fluoranthene, benzanthracene, benzophenanthrene, tetracene, pentacene, benzopyrene, furan, benzofuran, isobenzofuran, dibenzofuran, thiophene, benzothiophene, isobenzothiophene, dibenzothiophene, pyrrole, indole, isoindole, carbazole, pyridine, quinoline, isoquinoline, acridine, phenanthridine, benzo-5,6-quinoline, benzo-6,7-quinoline, benzo-7,8-quinoline, phenothiazine, phenoxazine, pyrazole, indazole, imidazole, benzimidazole, naphthimidazole, phenanthrimidazole, pyridimidazole, pyrazinimidazole, quinoxalinimidazole, oxazole, benzoxazole, naphthoxazole, anthroxazole, phenanthroxazole, isoxazole, 1,2-thiazole, 1,3-thiazole, benzothiazole, pyridazine, benzopyridazine, pyrimidine, benzpyrimidine, quinoxaline, pyrazine, phenazine, naphthyridine, azacarbazole, benzocarboline, phenanthroline, 1,2,3-triazole, 1,2,4-triazole, benzotriazole, 1,2,3-oxadiazole, 1,2,4-oxadiazole, 1,2,5-oxadiazole, 1,3,4-oxadiazole, 1,2,3-thiadiazole, 1,2,4-thiadiazole, 1,2,5-thiadiazole, 1,3,4-thiadiazole, 1,3,5-triazine, 1,2,4-triazine, 1,2,3-triazine, tetrazole, 1,2,4,5-tetrazine, 1,2,3,4-tetrazine, 1,2,3,5-tetrazine, purine, pteridine, indolizine and benzothiadiazole.

An aryloxy group in accordance with the definition of the present invention is taken to mean an aryl group, as defined above, which is bonded via an oxygen atom. An analogous definition applies to heteroaryloxy groups.

An aromatic ring system in the sense of this invention contains 6 to 60 C atoms in the ring system, preferably 6 to 40 C atoms, more preferably 6 to 20 C atoms. A heteroaromatic ring system in the sense of this invention contains 5 to 60 aromatic ring atoms, preferably 5 to 40 aromatic ring atoms, more preferably 5 to 20 aromatic ring atoms, at least one of which is a heteroatom. The heteroatoms are preferably selected from N, O and/or S. An aromatic or heteroaromatic ring system in the sense of this invention is intended to be taken to mean a system which does not necessarily contain only aryl or heteroaryl groups, but instead in which, in addition, a plurality of aryl or heteroaryl groups may be connected by a non-aromatic unit (preferably less than 10% of the atoms other than H), such as, for example, an $sp^3$-hybridised C, Si, N or O atom, an $sp^2$-hybridised C or N atom or an sp-hybridised C atom. Thus, for example, systems such as 9,9'-spirobifluorene, 9,9'-diarylfluorene, triarylamine, diaryl ether, stilbene, etc., are also intended to be taken to be aromatic ring systems in the sense of this invention, as are systems in which two or more aryl groups are connected, for example, by a linear or cyclic alkyl, alkenyl or alkynyl group or by a silyl group. Furthermore, systems in which two or more aryl or heteroaryl groups are linked to one another via single bonds are also taken to be aromatic or heteroaromatic ring systems in the sense of this invention, such as, for example, systems such as biphenyl, terphenyl or diphenyltriazine.

An aromatic or heteroaromatic ring system having 5-60 aromatic ring atoms, which may in each case also be substituted by radicals as defined above and which may be linked to the aromatic or heteroaromatic group via any desired positions, is taken to mean, in particular, groups derived from benzene, naphthalene, anthracene, benzanthracene, phenanthrene, benzophenanthrene, pyrene, chrysene, perylene, fluoranthene, naphthacene, pentacene, benzopyrene, biphenyl, biphenylene, terphenyl, terphenylene, quaterphenyl, fluorene, spirobifluorene, dihydrophenanthrene, dihydropyrene, tetrahydropyrene, cis- or trans-indenofluorene, truxene, isotruxene, spirotruxene, spiroisotruxene, furan, benzofuran, isobenzofuran, dibenzofuran, thiophene, benzothiophene, isobenzothiophene, dibenzothiophene, pyrrole, indole, isoindole, carbazole, indolocarbazole, indenocarbazole, pyridine, quinoline, isoquinoline, acridine, phenanthridine, benzo-5,6-quinoline, benzo-6,7-quinoline, benzo-7,8-quinoline, phenothiazine, phenoxazine, pyrazole, indazole, imidazole, benzimidazole, naphthimidazole, phenanthrimidazole, pyridimidazole, pyrazinimidazole, quinoxalinimidazole, oxazole, benzoxazole, naphthoxazole, anthroxazole, phenanthroxazole, isoxazole, 1,2-thiazole, 1,3-thiazole, benzothiazole, pyridazine, benzopyridazine, pyrimidine, benzopyrimidine, quinoxaline, 1,5-diazaanthracene, 2,7-diazapyrene, 2,3-diazapyrene, 1,6-diazapyrene, 1,8-diazapyrene, 4,5-diazapyrene, 4,5,9,10-tetraazaperylene, pyrazine, phenazine, phenoxazine, phenothiazine, fluorubin, naphthyridine, azacarbazole, benzocarboline, phenanthroline, 1,2,3-triazole, 1,2,4-triazole, benzotriazole, 1,2,3-oxadiazole, 1,2,4-oxadiazole, 1,2,5-oxadiazole, 1,3,4-oxadiazole, 1,2,3-thiadiazole, 1,2,4-thiadiazole, 1,2,5-thiadiazole, 1,3,4-thiadiazole, 1,3,5-triazine, 1,2,4-triazine, 1,2,3-triazine, tetrazole, 1,2,4,5-tetrazine, 1,2,3,4-tetrazine, 1,2,3,5-tetrazine, purine, pteridine, indolizine and benzothiadiazole, or combinations of these groups.

For the purposes of the present invention, a straight-chain alkyl group having 1 to 40 C atoms or a branched or cyclic alkyl group having 3 to 40 C atoms or an alkenyl or alkynyl group having 2 to 40 C atoms, in which, in addition, individual H atoms or $CH_2$ groups may be substituted by the groups mentioned above under the definition of the radicals, is preferably taken to mean the radicals methyl, ethyl, n-propyl, i-propyl, n-butyl, i-butyl, s-butyl, t-butyl, 2-methylbutyl, n-pentyl, s-pentyl, cyclopentyl, neopentyl, n-hexyl, cyclohexyl, neohexyl, n-heptyl, cycloheptyl, n-octyl, cyclooctyl, 2-ethylhexyl, trifluoromethyl, pentafluoroethyl, 2,2,2-trifluoroethyl, ethenyl, propenyl, butenyl, pentenyl, cyclopentenyl, hexenyl, cyclohexenyl, heptenyl, cycloheptenyl, octenyl, cyclooctenyl, ethynyl, propynyl, butynyl, pentynyl, hexynyl or octynyl. An alkoxy or thioalkyl group having 1 to 40 C atoms is preferably taken to mean methoxy, trifluoromethoxy, ethoxy, n-propoxy, i-propoxy, n-butoxy, i-butoxy, s-butoxy, t-butoxy, n-pentoxy, s-pentoxy, 2-methylbutoxy, n-hexoxy, cyclohexyloxy, n-heptoxy, cycloheptyloxy, n-octyloxy, cyclooctyloxy, 2-ethylhexyloxy, pentafluoroethoxy, 2,2,2-trifluoroethoxy, methylthio, ethylthio, n-propylthio, i-propylthio, n-butylthio, i-butylthio, s-butylthio, t-butylthio, n-pentylthio, s-pentylthio, n-hexylthio, cyclohexylthio, n-heptylthio, cycloheptylthio, n-octylthio, cyclooctylthio, 2-ethylhexylthio, trifluoromethylthio, pentafluoroethylthio, 2,2,2-trifluoroethylthio, ethenylthio, propenylthio, butenylthio, pentenylthio, cyclopentenylthio, hexenylthio, cyclohexenylthio, heptenylthio, cycloheptenylthio, octenylthio, cyclooctenylthio, ethynylthio, propynylthio, butynylthio, pentynylthio, hexynylthio, heptynylthio or octynylthio.

The formulation that two or more radicals may form a ring with one another is, for the purposes of the present application, intended to be taken to mean, inter alia, that the two radicals are linked to one another by a chemical bond. This is illustrated by the following schemes:

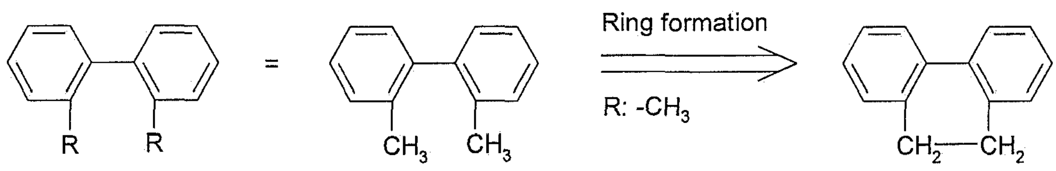


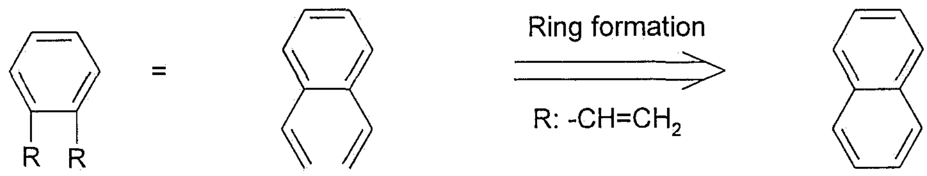


Furthermore, however, the above-mentioned formulation is also intended to be taken to mean that, in the case where one of the two radicals represents hydrogen, the second radical is bonded at the position to which the hydrogen atom was bonded, with formation of a ring. This is illustrated by the following scheme:

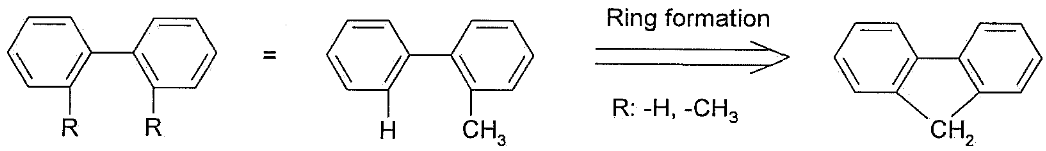


Preferably, the groups $Ar^1$ and $Ar^3$ stand on each occurrence, identically or differently, for an anthracene, phenanthrene, pyrene, chrysene, perylene, fluoranthene, benzanthracene, benzophenanthrene, tetracene or pentacene, each of which may in each case be substituted by one or more radicals $R^V$ at any free positions for $Ar^1$ or by one or more radicals $R^Y$ at any free positions for $Ar^3$

Preferably, the groups $Ar^1$, $Ar^3$ stand on each occurrence, identically or differently, for a condensed aryl group having 10 to 18 aromatic ring atoms. More preferably, the groups $Ar^1$, $Ar^3$ stand on each occurrence, identically or differently, for an anthracene, naphthalene, phenanthrene, tetracene, chrysene, benzanthracene, benzophenanthracene, pyrene, perylene, triphenylene, benzopyrene or fluoranthene, each of which may be substituted by one or more radicals $R^V$ in the case of $Ar^1$ or $R^Y$ in the case of $Ar^3$ at any free positions. Very preferably, the groups $Ar^1$, $Ar^3$ stand for an anthracene group, which may be substituted by one or more radicals $R^V$ at any free positions for $Ar^1$ or by one or more radicals $R^Y$ at any free positions for $Ar^3$.

Examples of suitable groups $Ar^1$ and $Ar^3$ are the groups of formulae (Ar1-1) to (Ar1-11) as represented in the table below:

| | | | |
|---|---|---|---|
| 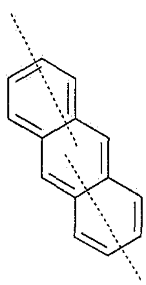 | 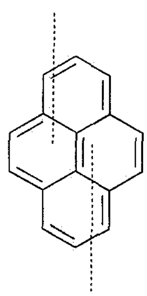 | 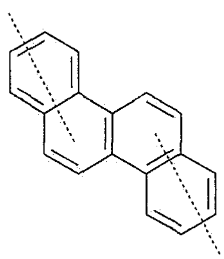 | 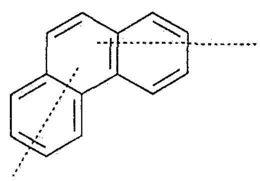 |
| (Ar1-1) | (Ar1-2) | (Ar1-3) | (Ar4-4) |

(continued)

| | | | |
|---|---|---|---|
| 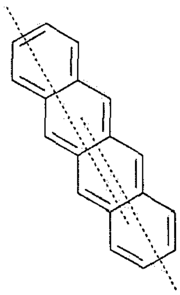 | 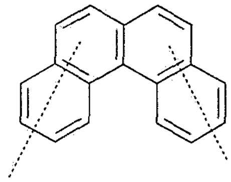 | 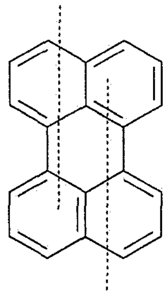 | 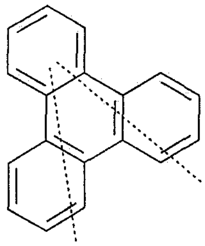 |
| (Ar1-5) | (Ar1-6) | (Ar1-7) | (Ar4-8) |
| 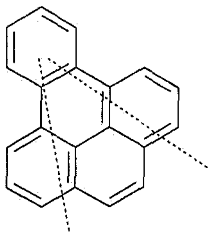 | 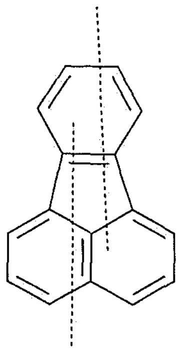 | 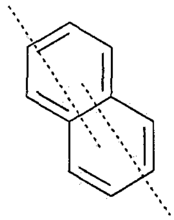 | |
| (Ar1-9) | (Ar1-10) | (Ar1-11) | |

where
the dashed bonds indicate the bonding to the adjacent groups; and where the groups of formulae (Ar1-1) to (Ar1-11) may be substituted at each free position by a group $R^V$ in the case of $Ar^1$ or by a group $R^Y$ in the case of $Ar^3$, where $R^V$ and $R^Y$ have the same meaning as above.

Among the groups of formulae (Ar1-1) to (Ar1-11), the group of formula (Ar1-1) is preferred.

Examples of very suitable groups $Ar^1$ and $Ar^3$ are the groups of formulae (Ar1-1-1) to (Ar1-12-1) as represented in the table below:

| | | |
|---|---|---|
| 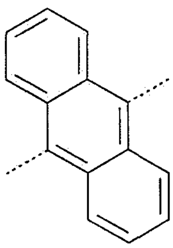 | 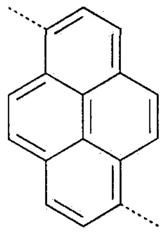 | 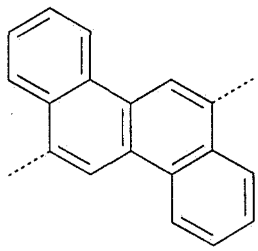 |
| (Ar1-1-1) | (Ar1-2-1) | (Ar1-3-1) |
| 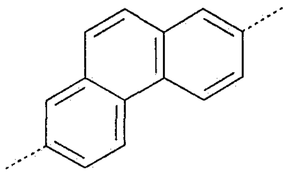 | 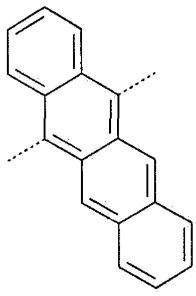 | 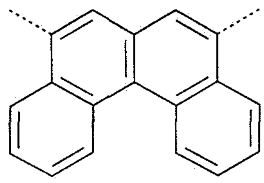 |
| (Ar1-4-1) | (Ar1-5-1) | (Ar1-6-1) |

(continued)

| | | |
|---|---|---|
| 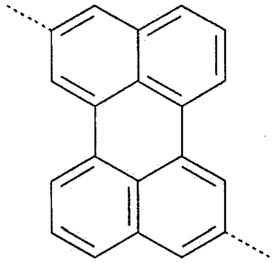 | 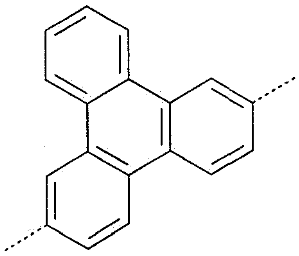 | 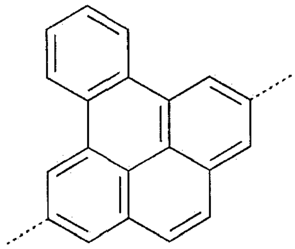 |
| (Ar1-7-1) | (Ar1-8-1) | (Ar1-9-1) |
| 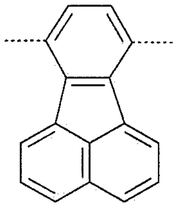 | 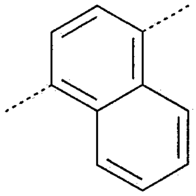 | 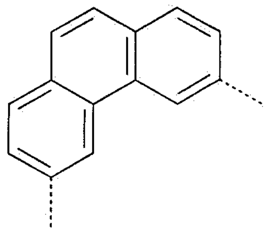 |
| (Art-10-1) | (Ar1-11-1) | (Ar1-12-1) |

where
the dashed bonds indicate the bonding to the adjacent groups; and where the groups of formulae (Ar1-1-1) to (Ar1-12-1) may be substituted at each free position by a group $R^V$ in the case of $Ar^1$ or by a group $R^Y$ in the case of $Ar^3$, where $R^V$ and $R^Y$ have the same meaning as above.

Among the groups of formulae (Ar1-1-1) to (Ar1-12-1), the group of formula (Ar1-1-1) is preferred.

Preferably, the compound of formula (H2) is selected from the compounds of formula (H2-1),

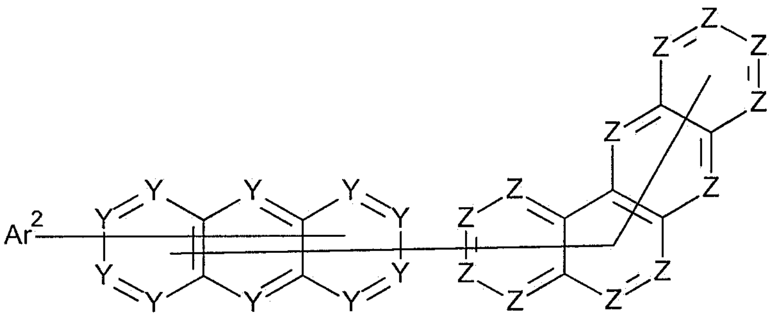

formula (H2-1)

where the symbol $Ar^2$ and Z have the same meaning as above; and where Y is $CR^Y$ or N; or Y is C if bonded to $Ar^2$ or a group Z; where $R^Y$ has the same meaning as above.

More preferably, the compound of formula (H2) is selected from the compounds of formula (H2-2),

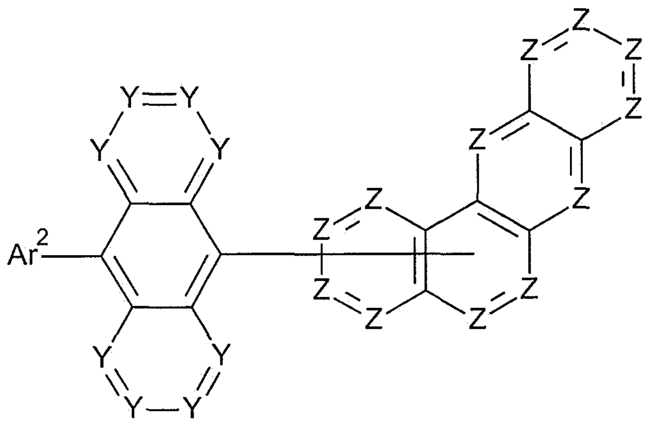

formula (H2-2)

where $Ar^2$, Y, Z have the same meaning as above.

Even more preferably, the compound of formula (H2) is selected from the compounds of formula (H2-3),

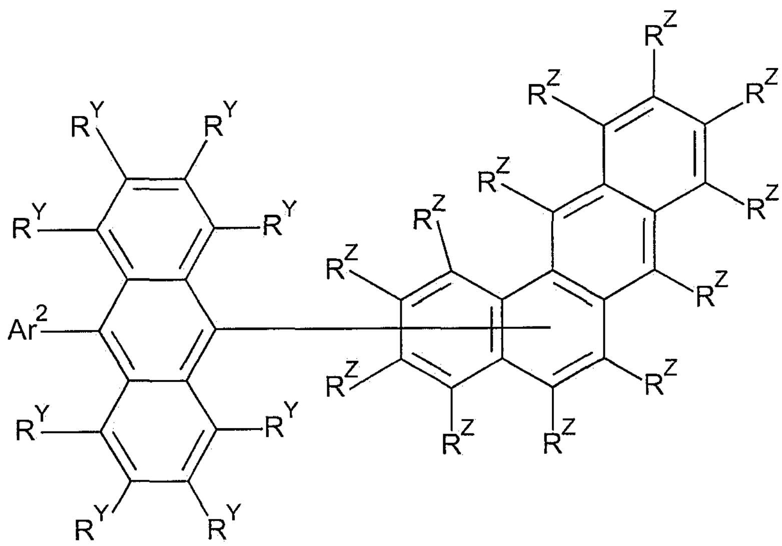

formula (H2-3)

where the symbols have the same meaning as above and with the proviso that the group $CR^Z$ correspond to a group C at the bonding position of the adjacent anthracene.

Particularly preferably, the compound of formula (H2) is selected from the compounds of formula (H2-4),

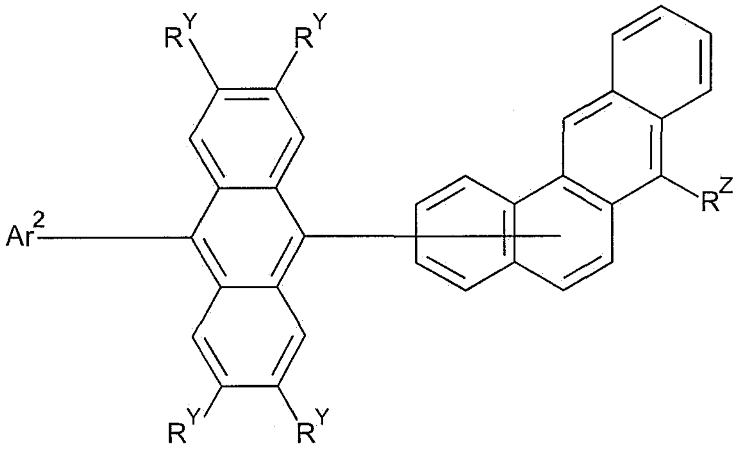

formula (H2-4)

where the symbols have the same meaning as above.

Very particularly preferably, the compound of formula (H2) is selected from the compounds of formula (H2-5),

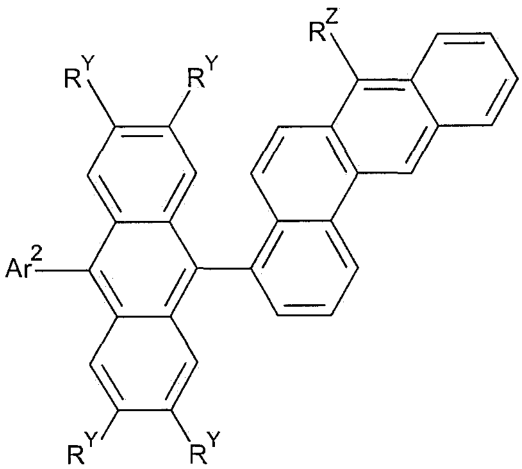

formula (H2-5)

where the symbols have the same meaning as above.

Examples of very suitable compounds of formula (H2-5) are the compounds (H2-5-1) to (H2-5-4),

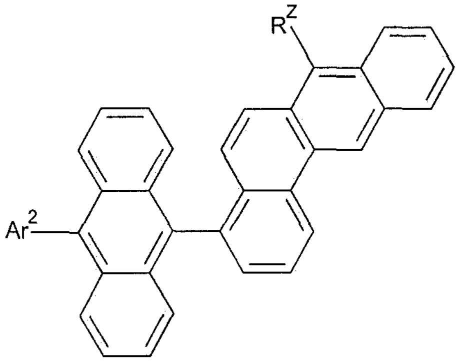

formula (H2-5-1)

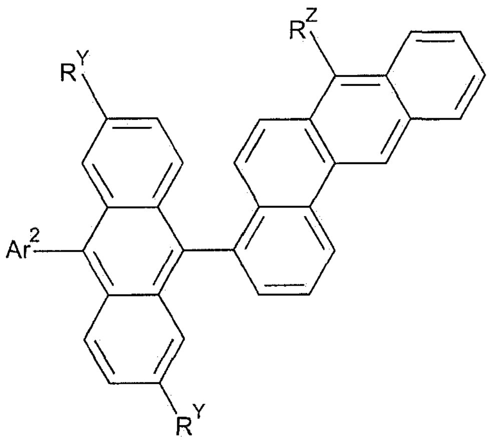

formula (H2-5-2)

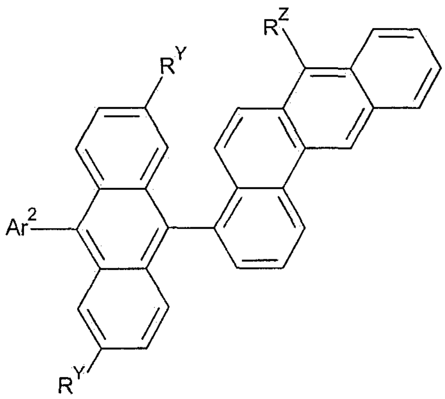

formula (H2-5-3)

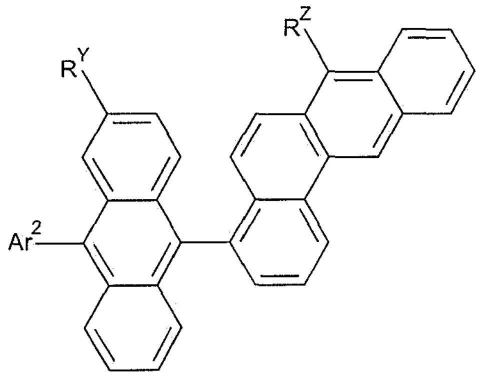

formula (H2-5-4)

where the symbols have the same meaning as above.

Preferably, $R^Y$, $R^Z$ stand on each occurrence, identically or differently, for H, D, F, a straight-chain alkyl, alkoxy or thioalkyl group having 1 to 40, preferably 1 to 20, more preferably 1 to 10 C atoms or branched or a cyclic alkyl, alkoxy or thioalkyl group having 3 to 40, preferably 3 to 20, more preferably 3 to 10 C atoms, each of which may be substituted by one or more radicals R, where in each case one or more non-adjacent $CH_2$ groups may be replaced by RC=CR, C=C, O or S and where one or more H atoms may be replaced by D or F, an aromatic or heteroaromatic ring system having 5 to 60, preferably 5 to 40, more preferably 5 to 30, particularly preferably 5 to 18 aromatic ring atoms, which may in each case be substituted by one or more radicals R. More preferably, $R^Y$, $R^Z$ stand on each occurrence, identically or differently, for H, D, F, a straight-chain alkyl group having 1 to 20, preferably 1 to 10, more preferably 1 to 6 C atoms or branched or a cyclic alkyl group having 3 to 20, preferably 3 to 10, more preferably 3 to 6 C atoms, each of which may be substituted by one or more radicals R, an aromatic or heteroaromatic ring system having 5 to 40, preferably 5 to 30, more preferably 5 to 18 aromatic ring atoms, which may in each case be substituted by one or more radicals R.

Particularly preferably, $R^Y$ stands for H.

Particularly preferably, $R^Z$ stands on each occurrence, identically or differently, for a straight-chain alkyl group having 1 to 10, more preferably 1 to 6 C atoms or branched or a cyclic alkyl group having 3 to 10, more preferably 3 to 6 C atoms, each of which may be substituted by one or more radicals R, an aromatic or heteroaromatic ring system having 5 to 40, preferably 5 to 30, more preferably 5 to 18 aromatic ring atoms, which may in each case be substituted by one or more radicals R.

Preferably, the compound of formula (H1) is selected from the compounds of formula (H1-1),

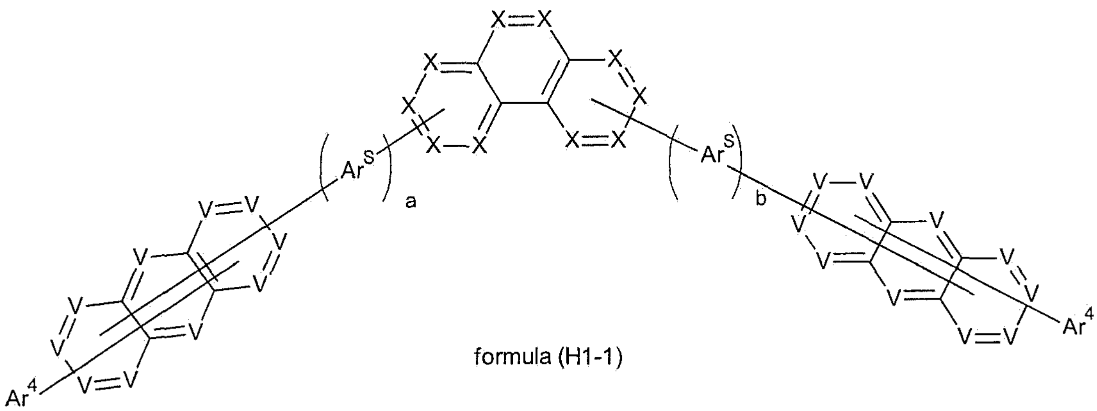

formula (H1-1)

where the symbols X, $Ar^S$, $Ar^4$ and the indices a and b have the same meaning as above; and
V is $CR^V$ or N; or V is C if bonded to $Ar^4$, $Ar^S$ or a group X; where $R^V$ has the same meaning as above.

Preferably, the indices a and b are equal to 0, so that the group $Ar^S$ is absent and the anthracene moiety is directly bonded to the phenanthrene moiety.

Preferably, the group $Ar^S$ stands on each occurrence, identically or differently, for phenyl, biphenyl, fluorene, spirobifluorene, naphthalene, phenanthrene, anthracene, dibenzofuran, dibenzothiophene, carbazole, pyridine, pyrimidine, pyrazine, pyridazine, triazine, benzopyridine, benzopyridazine, benzopyrimidine and quinazoline, each of which may be substituted by one or more radicals R.

Examples of suitable groups $Ar^S$ are the groups of formulae (ArS-1) to (ArS-26) as represented in the table below:

| | | |
|---|---|---|
| 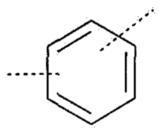 | 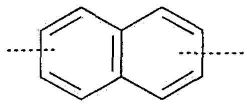 | 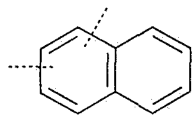 |
| (ArS-1) | (ArS-2) | (ArS-3) |
| 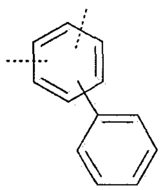 | 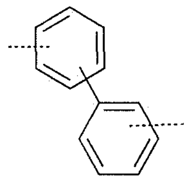 | 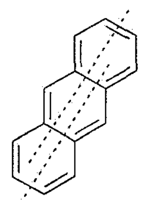 |
| (ArS-4) | (ArS-5) | (ArS-6) |
| 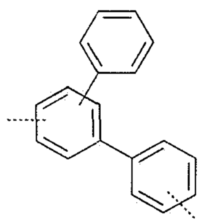 | 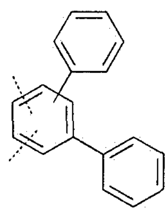 | 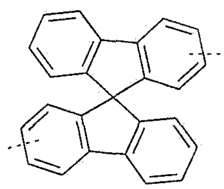 |
| (ArS-7) | (ArS-8) | (ArS-9) |
| 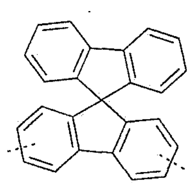 | 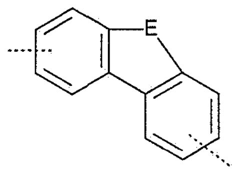 | 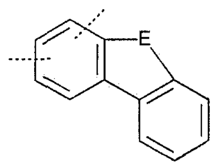 |
| (ArS-10) | (ArS-11) | (ArS-12) |

(continued)

| | | |
|---|---|---|
| 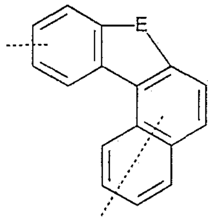 | 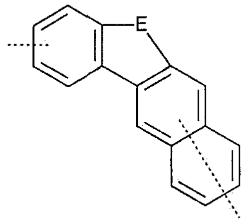 | 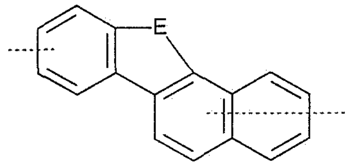 |
| (ArS-13) | (ArS-14) | (ArS-15) |
| 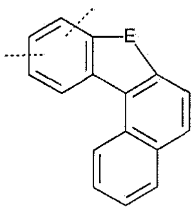 | 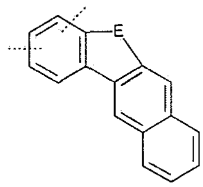 | 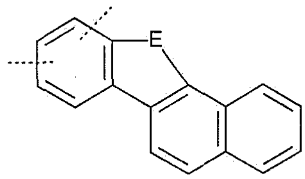 |
| (ArS-16) | (ArS-17) | (ArS-18) |
| 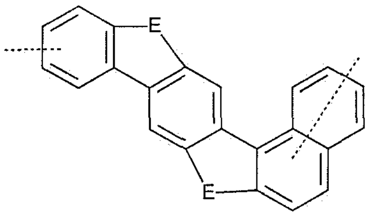 | 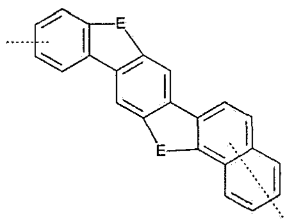 | 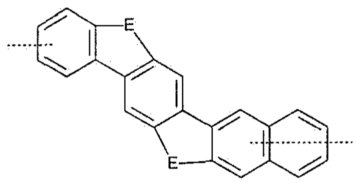 |
| (ArS-19) | (ArS-20) | (ArS-21) |
| 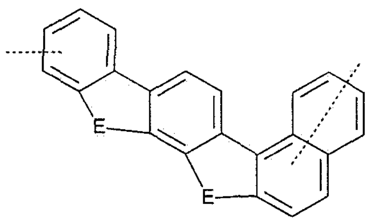 | 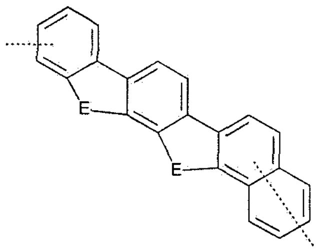 | 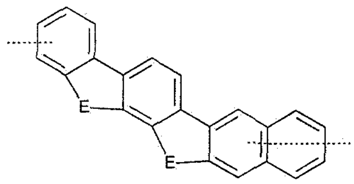 |
| (ArS-22) | (ArS-23) | (ArS-24) |
| 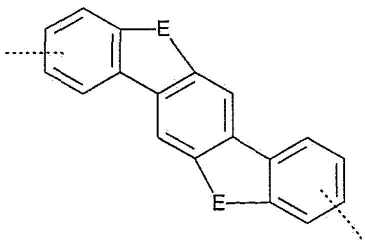 | 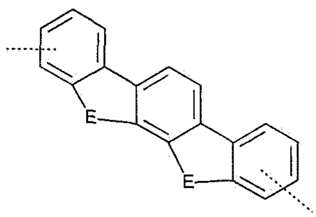 | |
| (ArS-25) | (ArS-26) | |

where the dashed bonds indicate the bonding to the adjacent groups in formula (1);
where the groups of formulae (ArS-1) to (ArS-26) may be substituted at each free position by a group R, which has the same meaning as defined above; and
where the group E is on each occurrence, identically or differently, selected from $-BR^0-$, $-C(R^0)_2-$, $-Si(R^0)_2-$, -C(=O)-, -O-, -S-, -S(=O)-, $-SO_2-$, $-N(R^0)-$, and $-P(R^0)-$,
where $R^0$ stands on each occurrence, identically or differently, for H, D, F, a straight-chain alkyl group having 1 to 20 , preferably 1 to 10 C atoms or branched or a cyclic alkyl group having 3 to 20, preferably 3 to 10 C atoms, each

of which may be substituted by one or more radicals R, where in each case one or more non-adjacent $CH_2$ groups may be replaced by O or S and where one or more H atoms may be replaced by D or F, or an aromatic or heteroaromatic ring systems having 5 to 40, preferably 5 to 30, more preferably 6 to 18 aromatic ring atoms, which may in each case be substituted by one or more radicals R, where two adjacent radicals $R^0$, may form an aliphatic or aromatic ring system together, which may be substituted by one or more radicals R.

Among the groups of formulae (ArS-1) to (ArS-26), the groups of formulae (ArS-1), (ArS-2), (ArS-3), (ArS-11) and (ArS-12) are preferred. The groups of formula (ArS-1), (ArS-2), (ArS-3) are very preferred.

More preferably, the compound of formula (H1) is selected from the compounds of formula (H1-2),

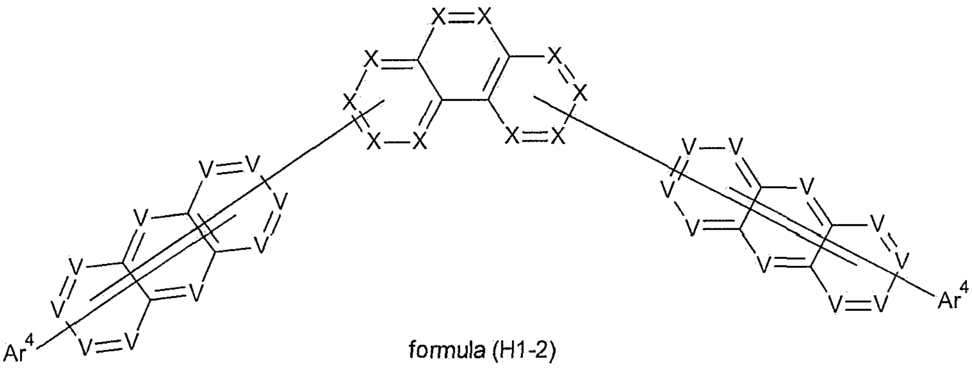

formula (H1-2)

where X, $Ar^4$ and V have the same meaning as above.

Even more preferably, the compound of formula (H1) is selected from the compounds of formula (H1-3),

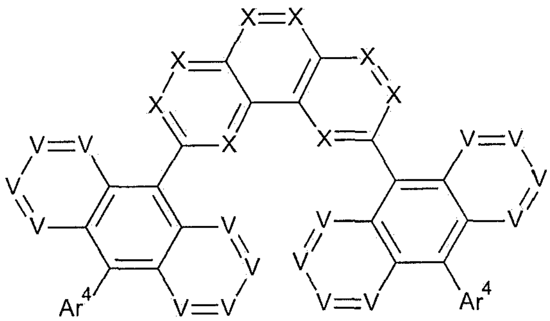

formula (H1-3)

where the symbols have the same meaning as above.

Particularly preferably, the compound of formula (H1) is selected from the compounds of formula (H1-4),

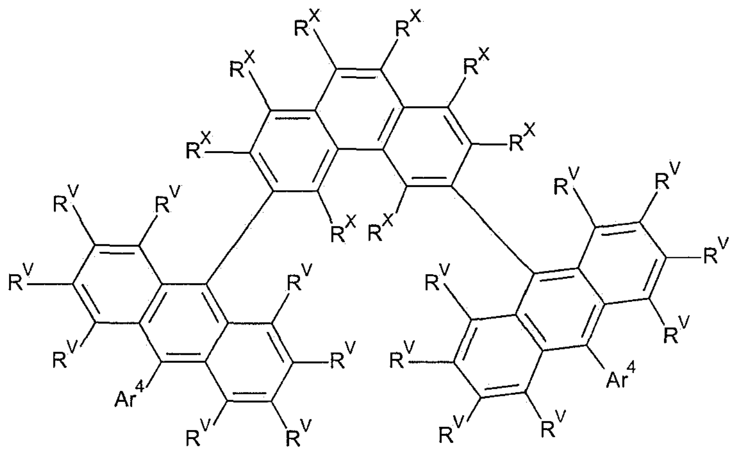

formula (H1-4)

where the symbols have the same meaning as above.

Very particularly preferably, the compound of formula (H1) is selected from the compound of formula (H1-5),

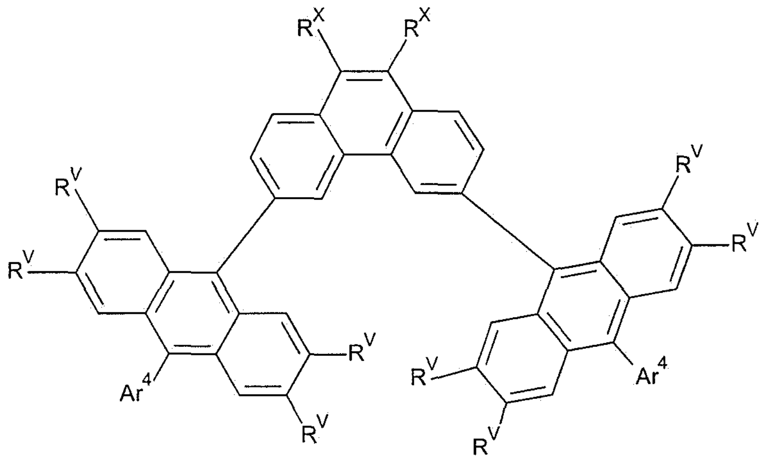

formula (H1-5)

where the symbols have the same meaning as in claim 1.

Examples of very suitable compounds of formula (H1-5) are the compounds (H1-5-1) to (H1-5-4),

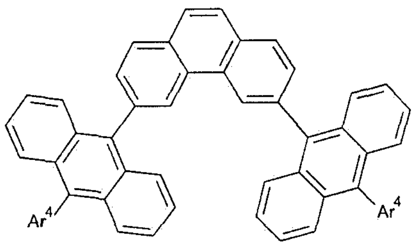

formula (H1-5-1)

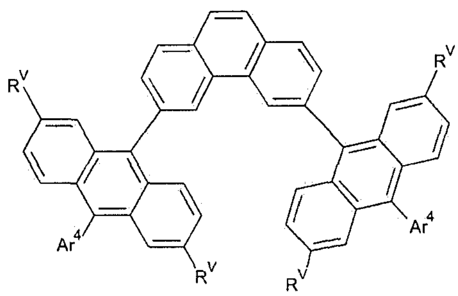

formula (H1-5-2)

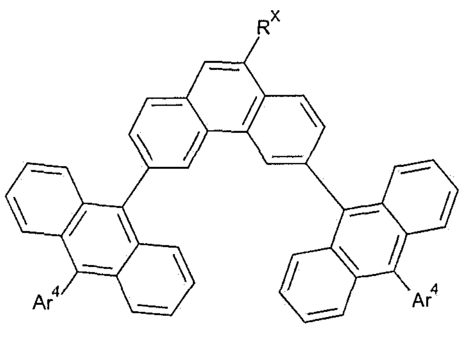

formula (H1-5-3)

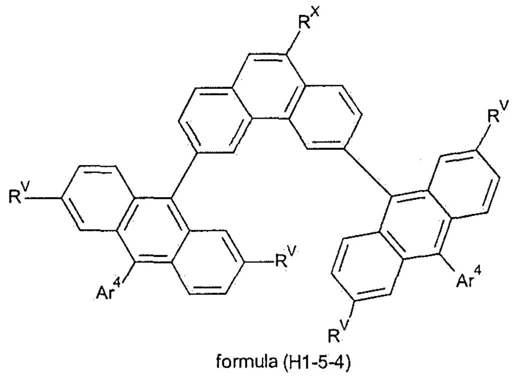

formula (H1-5-4)

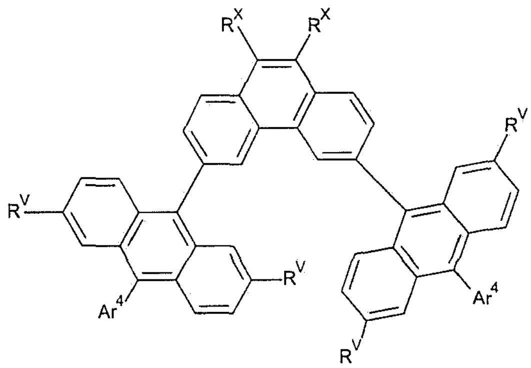

formula (H1-5-5)

where the symbols have the same meaning as above.

Preferably, $R^X$, $R^V$ stand on each occurrence, identically or differently, for H, D, F, a straight-chain alkyl, alkoxy or thioalkyl group having 1 to 40, preferably 1 to 20, more preferably 1 to 10 C atoms or branched or a cyclic alkyl, alkoxy or thioalkyl group having 3 to 40, preferably 3 to 20, more preferably 3 to 10 C atoms, each of which may be substituted by one or more radicals R, where in each case one or more non-adjacent $CH_2$ groups may be replaced by RC=CR, C=C, O or S and where one or more H atoms may be replaced by D or F, an aromatic or heteroaromatic ring system having 5 to 60, preferably 5 to 40, more preferably 5 to 30, particularly preferably 5 to 18 aromatic ring atoms, which may in each case be substituted by one or more radicals R. More preferably, $R^X$, $R^V$ stand on each occurrence, identically or differently, for H, D, F, a straight-chain alkyl group having 1 to 20, preferably 1 to 10, more preferably 1 to 6 C atoms or branched or a cyclic alkyl group having 3 to 20, preferably 3 to 10, more preferably 3 to 6 C atoms, each of which may be substituted by one or more radicals R, an aromatic or heteroaromatic ring system having 5 to 40, preferably 5 to 30, more preferably 5 to 18 aromatic ring atoms, which may in each case be substituted by one or more radicals R.

More preferably, $R^X$, $R^V$ stand on each occurrence, identically or differently, for H, a straight-chain alkyl group having 1 to 10, more preferably 1 to 6 C atoms or branched or a cyclic alkyl group having 3 to 10, more preferably 3 to 6 C atoms, each of which may be substituted by one or more radicals R, an aromatic or heteroaromatic ring system having 5 to 40, preferably 5 to 30, more preferably 6 to 18 aromatic ring atoms, which may in each case be substituted by one or more radicals R.

Preferably, the groups $Ar^2$, $Ar^4$ are on each occurrence, identically or differently, selected from aromatic or heteroaromatic ring systems having 5 to 30, preferably 5 to 25 aromatic ring atoms, which may in each case be substituted by one or more radicals R. More preferably, the group $Ar^2$, $Ar^4$ are selected from the group consisting of phenyl, biphenyl, terphenyl, quaterphenyl, fluorene, spirobifluorene, naphthalene, phenanthrene, anthracene, triphenylene, fluoranthene, tetracene, chrysene, benzanthracene, benzophenanthracene, pyrene, perylene, indole, benzofuran, benzothiophene, dibenzofuran, dibenzothiophene, carbazole, indenocarbazole, indolocarbazole, pyridine, pyrimidine, pyrazine, pyridazine, triazine, quinolone, benzopyridine, benzopyridazine, benzopyrimidine, benzimidazole and quinazoline, each of which may be substituted by one or more radicals R; where $Ar^2$, $Ar^4$ might also be a combination of two or more of the previously cited groups. Particularly preferably, the groups $Ar^2$, $Ar^4$ are selected from the group consisting of phenyl, biphenyl, terphenyl, quaterphenyl, fluorene, spirobifluorene, naphthalene, anthracene, phenanthrene, triphenylene, fluoranthene, tetracene, chrysene, benzanthracene, benzophenanthracene, pyrene or perylene, dibenzofuran, carbazole and dibenzothiophene, each of which may be substituted by one or more radicals R at any free positions; and where $Ar^2$, $Ar^4$ might also be a combination of two or more of the previously cited groups. Very partcualrly preferably, the groups

$Ar^2$, $Ar^4$ are selected from the group consisting of phenyl, biphenyl, terphenyl, quaterphenyl, fluorene, spirobifluorene, naphthalene, anthracene, phenanthrene, triphenylene, fluoranthene, dibenzofuran, carbazole and dibenzothiophene, each of which may be substituted by one or more radicals R at any free positions; and where $Ar^2$, $Ar^4$ might also be a combination of two or more of the previously cited groups.

Examples of suitable groups $Ar^2$ and $Ar^4$ are the groups of formulae (Ar2-1) to (Ar2-27) as depicted in the table below:

| | | |
|---|---|---|
| 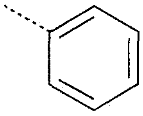 | 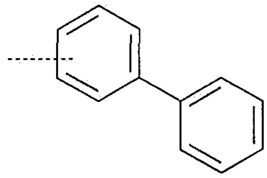 | 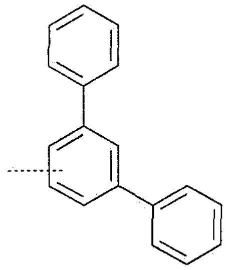 |
| (Ar2-1) | (Ar2-2) | (Ar2-3) |
| 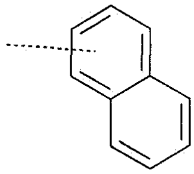 | 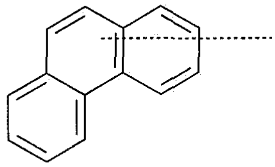 | 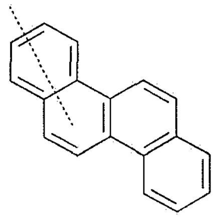 |
| (Ar2-4) | (Ar2-5) | (Ar2-6) |
| 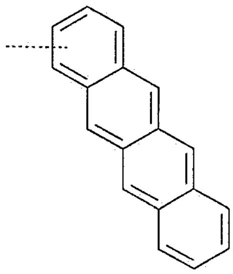 | 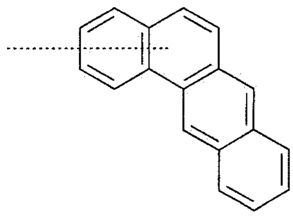 | 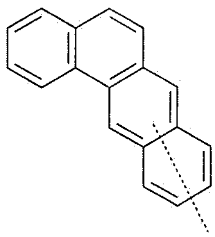 |
| (Ar2-7) | (Ar2-8) | (Ar2-9) |
| 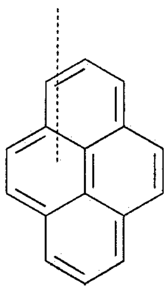 | 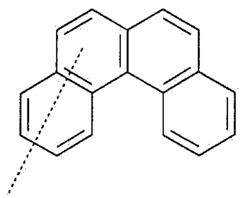 | 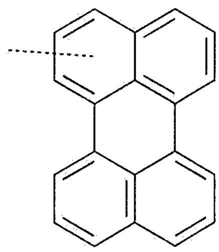 |
| (Ar2-10) | (Ar2-11) | (Ar2-12) |

(continued)

| | | |
|---|---|---|
| 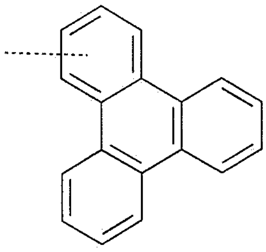 | 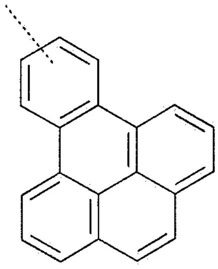 | 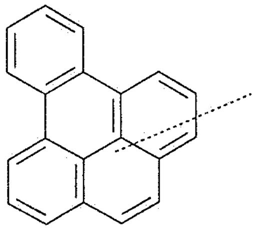 |
| (Ar2-13) | (Ar2-14) | (Ar2-15) |
| 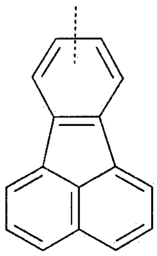 | 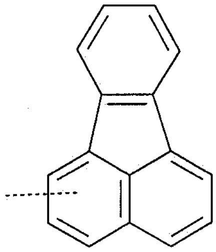 | 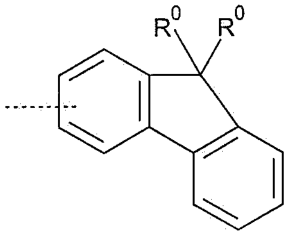 |
| (Ar2-16) | (Ar2-17) | (Ar2-18) |
| 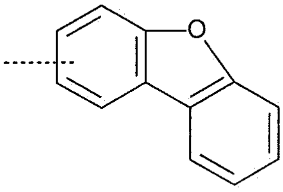 | 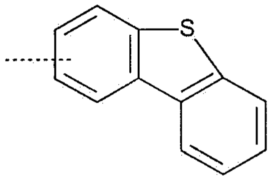 | 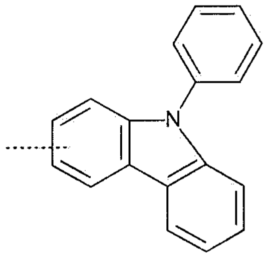 |
| (Ar2-19) | (Ar2-20) | (Ar2-21) |
| 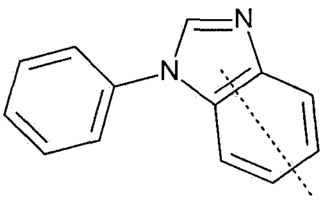 | 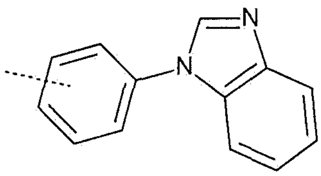 | 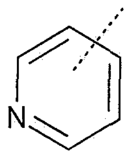 |
| (Ar2-22) | (Ar2-23) | (Ar2-24) |
| 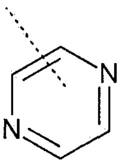 | 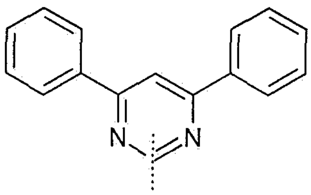 | 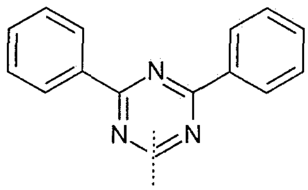 |
| (Ar2-25) | (Ar2-26) | (Ar2-27) |

where the dashed bond indicates the bonding to the adjacent group and where the group R° has the same meaning as above; and where the groups of formulae (Ar2-1) to (Ar2-27) may be substituted at each free position by a group R, which has the same meaning as above.

Among the groups of formulae (Ar2-1) to (Ar2-27), the groups of formulae (Ar2-1), (Ar2-2), (Ar2-3), (Ar2-4), (Ar2-5), (Ar2-8), (Ar2-18), (Ar2-19) are preferred. The groups of formula (Ar2-1), (Ar2-2), (Ar2-3), (Ar2-4), (Ar2-5) are

very preferred.

Preferably, R stands on each occurrence, identically or differently, for H, D, F, CN, $N(Ar)_2$, a straight-chain alkyl, alkoxy or thioalkyl groups having 1 to 40, preferably 1 to 20, more preferably 1 to 10 C atoms or branched or a cyclic alkyl, alkoxy or thioalkyl groups having 3 to 40, preferably 3 to 20, more preferably 3 to 10 C atoms, each of which may be substituted by one or more radicals R', where in each case one or more non-adjacent $CH_2$ groups may be replaced by R'C=CR', C=C, O or S and where one or more H atoms may be replaced by D or F, or an aromatic or heteroaromatic ring systems having 5 to 60, preferably 5 to 40, more preferably 5 to 30, particularly preferably 6 to 18 aromatic ring atoms, which may in each case be substituted by one or more radicals R'.

Preferably, R' stands on each occurrence, identically or differently, for H, D, F, Cl, Br, I, CN, a straight-chain alkyl group having 1 to 10 C atoms or branched or cyclic alkyl group having 3 to 10 C atoms, where in each case one or more H atoms may be replaced by D or F, or an aromatic or heteroaromatic ring system having 5 to 18 C atoms.

The following compounds are examples of compounds of formula (H1):

| | | | | | |
|---|---|---|---|---|---|
| 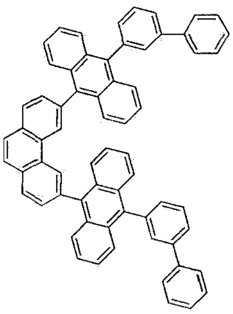 | 3 | 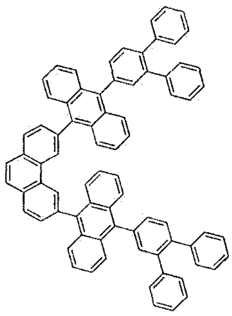 | 6 | 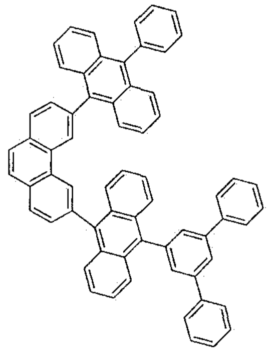 | 9 |
| 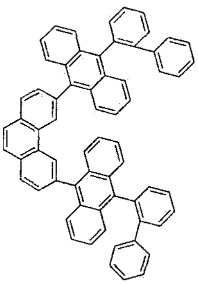 | 2 | 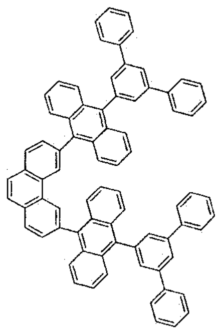 | 5 | 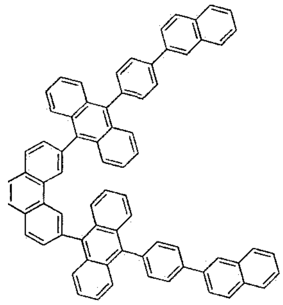 | 8 |
| 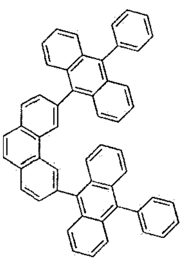 | 1 | 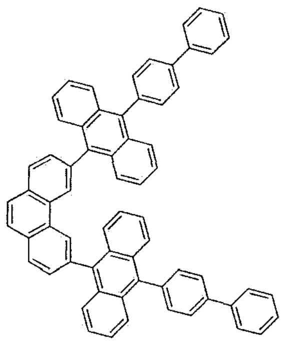 | 4 | 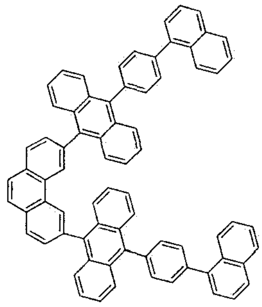 | 7 |

(continued)

| | | |
|---|---|---|
| 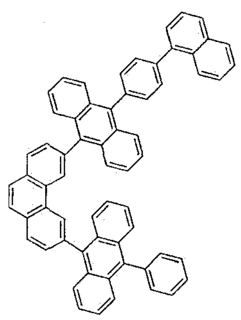 | 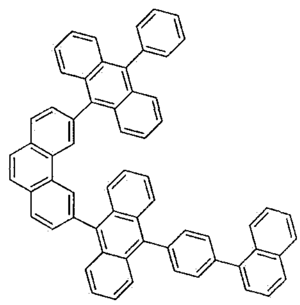 | 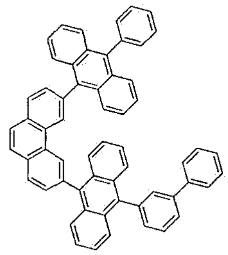 |
| 10 | 11 | 12 |
| 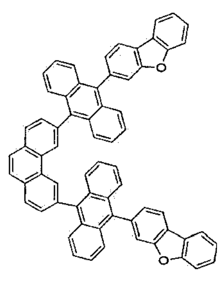 | 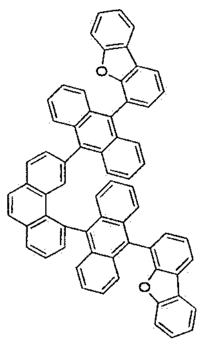 | 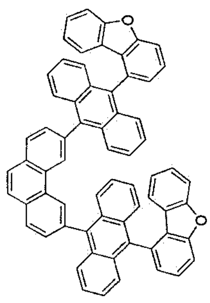 |
| 13 | 14 | 15 |
| 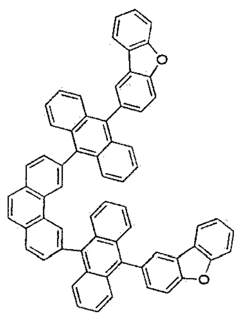 | 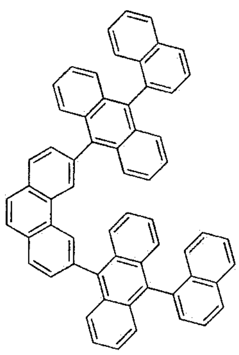 | 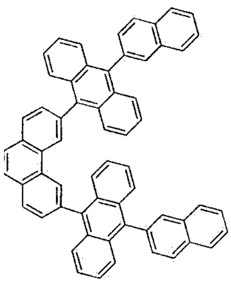 |
| 16 | 17 | 18 |

(continued)

| | | |
|---|---|---|
| 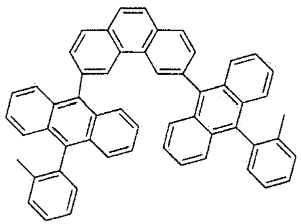 | 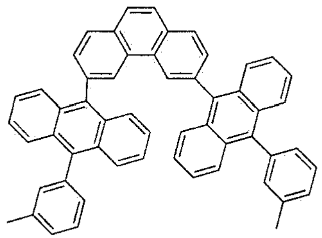 | 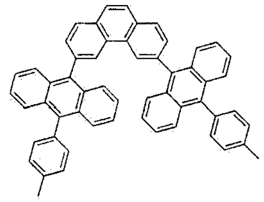 |
| 19 | 20 | 21 |
| 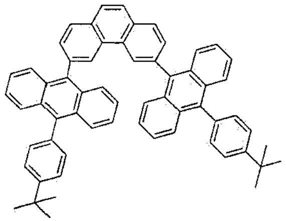 | 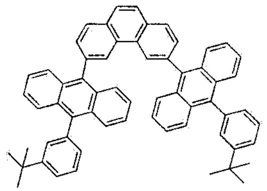 | 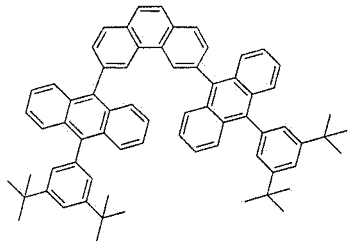 |
| 22 | 23 | 24 |
| 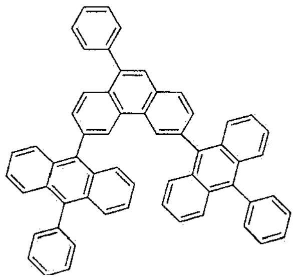 | 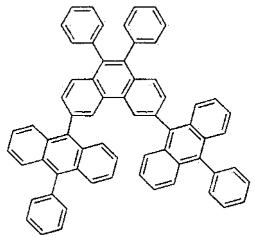 | 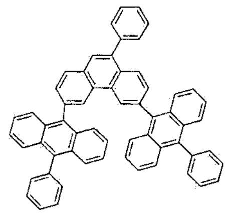 |
| 25 | 26 | 27 |

(continued)

| | | |
|---|---|---|
| 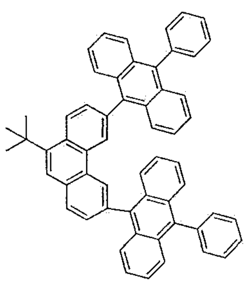 | 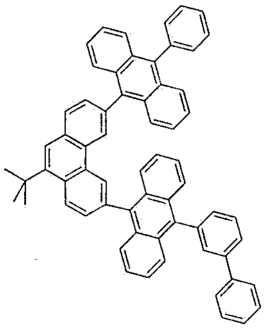 | 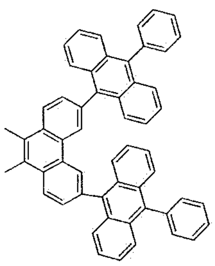 |
| 28 | 29 | 30 |
| 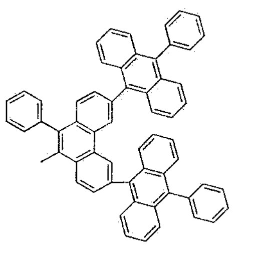 | 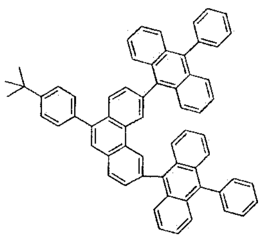 | 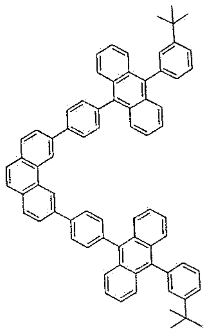 |
| 31 | 32 | 33 |
| 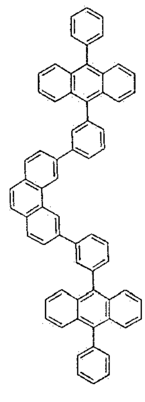 | 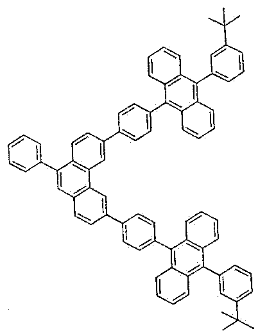 | 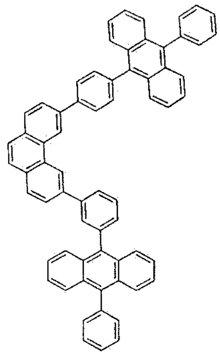 |
| 34 | 35 | 36 |

(continued)

| | | |
|---|---|---|
| 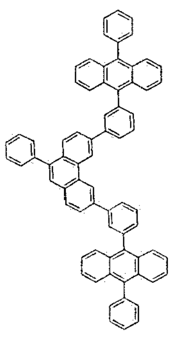 | 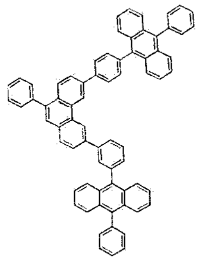 | 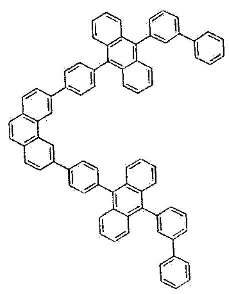 |
| 37 | 38 | 39 |
| 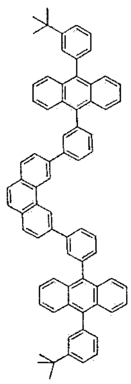 | 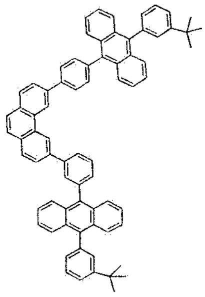 | 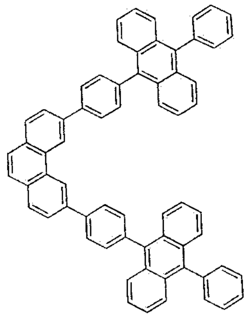 |
| 40 | 41 | 42 |
| 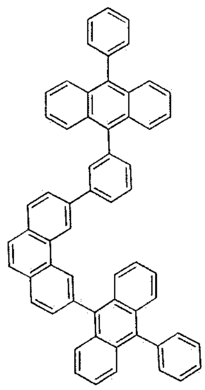 | 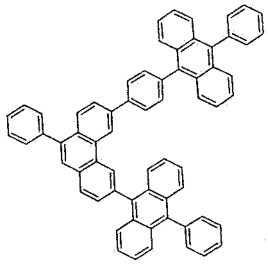 | 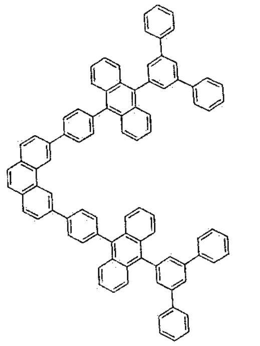 |
| 43 | 44 | 45 |
| 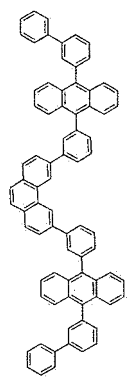 | 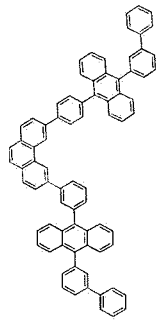 | 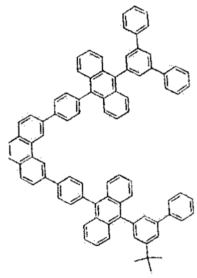 |

(continued)

| | | |
|---|---|---|
| 46 | 47 | 48 |
| 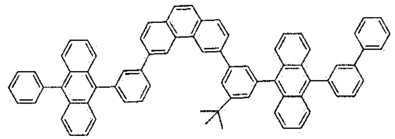 | 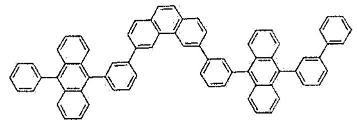 | 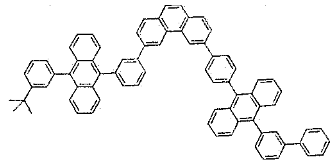 |
| 49 | 50 | 51 |
| 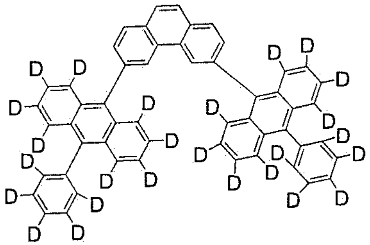 | 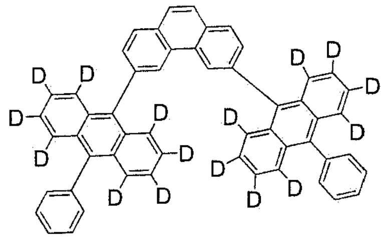 | 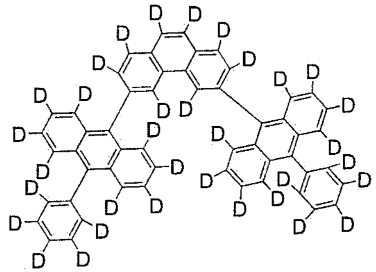 |
| 52 | 53 | 54 |
| 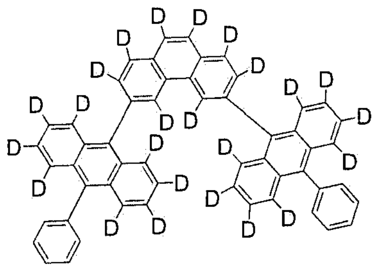 | 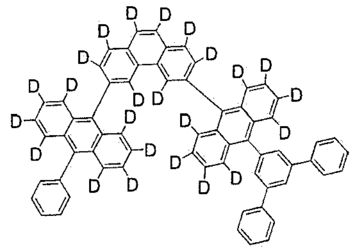 | 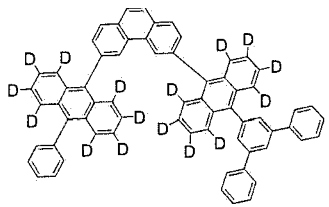 |
| 55 | 56 | 57 |

(continued)

| | | |
|---|---|---|
| 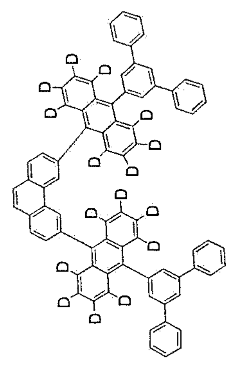 | 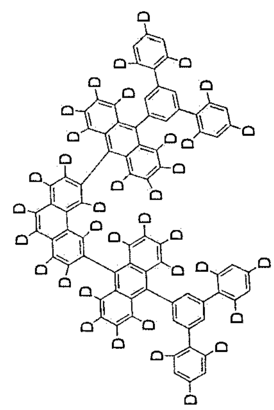 | 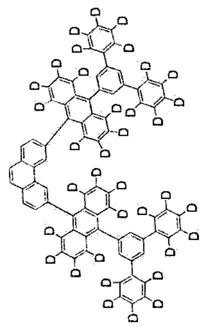 |
| 58 | 59 | 60 |
| 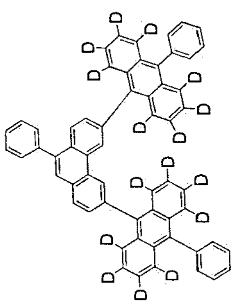 | 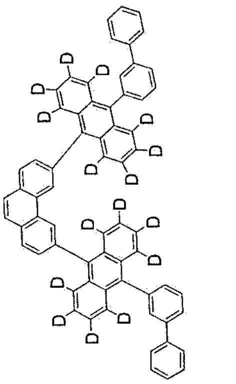 | 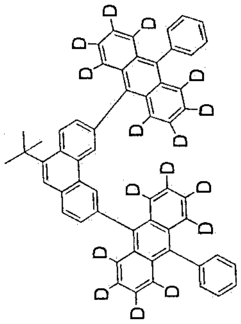 |
| 61 | 62 | 63 |
| 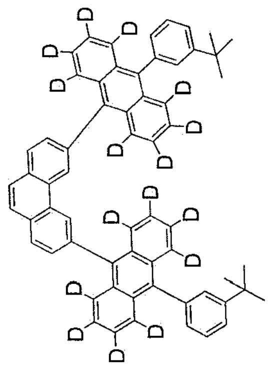 | 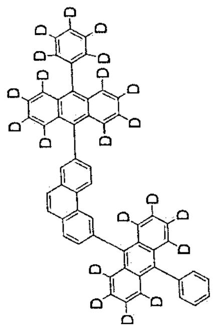 | |
| 64 | 65 | |

The following compounds are examples of compounds of formula (H2):

| | | | | | | | |
|---|---|---|---|---|---|---|---|
| 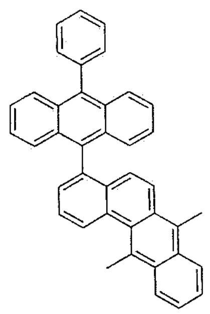 | 3 | | 6 | 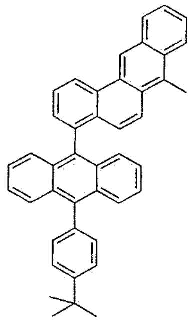 | 9 | 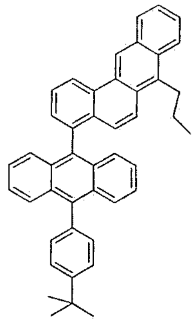 | 12 |
| 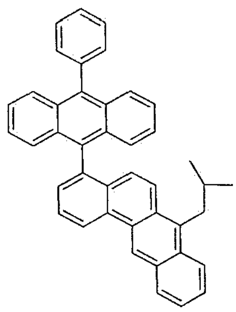 | 2 | 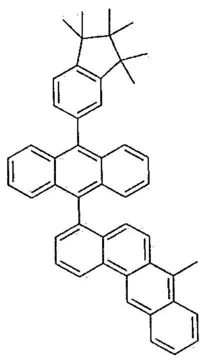 | 5 | 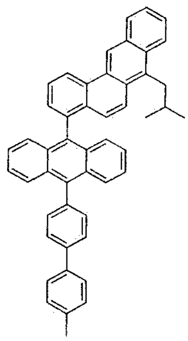 | 8 | 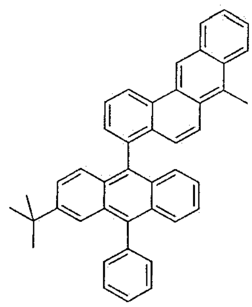 | 11 |
| 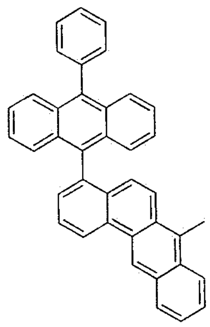 | 1 | 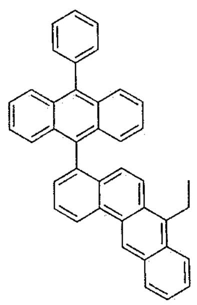 | 4 | 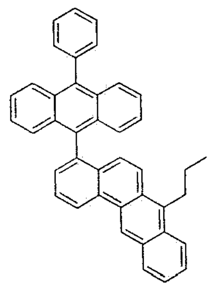 | 7 | 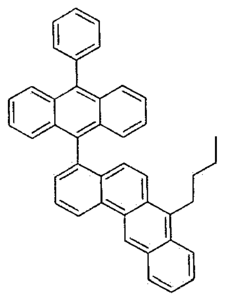 | 10 |

(continued)

| | | |
|---|---|---|
| 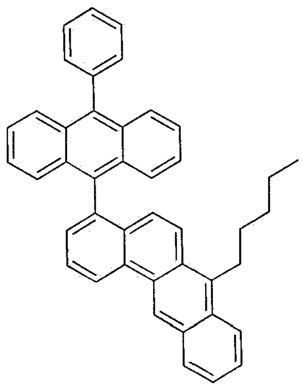 | 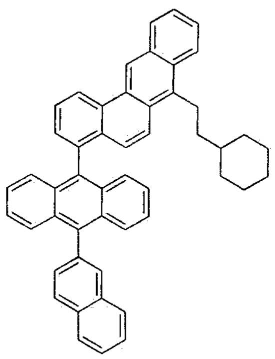 | 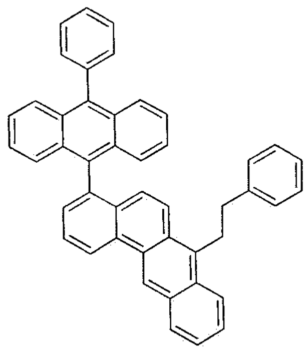 |
| 13 | 14 | 15 |
| 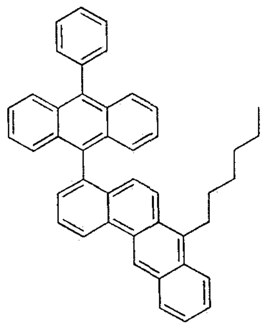 | 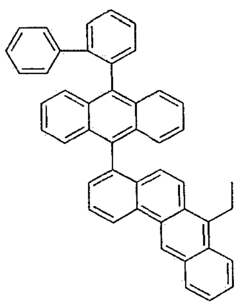 | 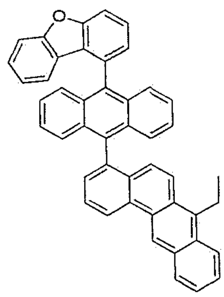 |
| 16 | 17 | 18 |
| 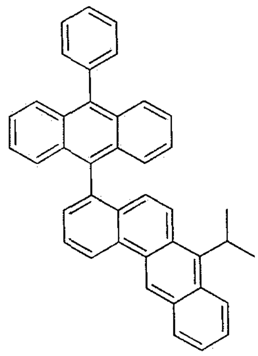 | 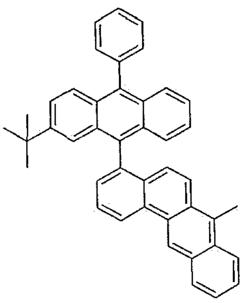 | 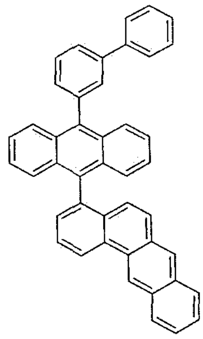 |
| 19 | 20 | 21 |

(continued)

| | | |
|---|---|---|
| 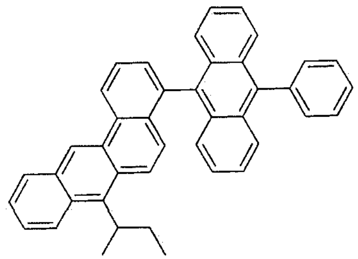 | 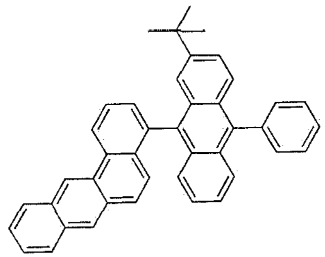 | 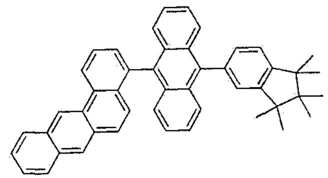 |
| 22 | 23 | 24 |
| 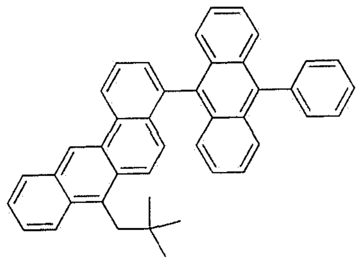 | 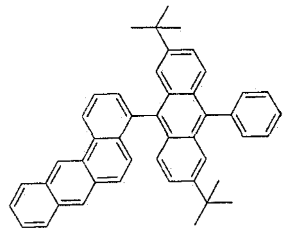 | 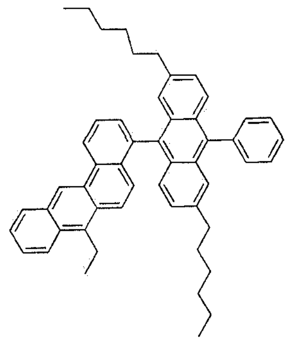 |
| 25 | 26 | 27 |
| 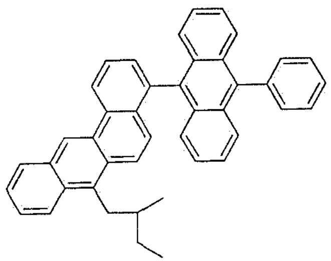 | 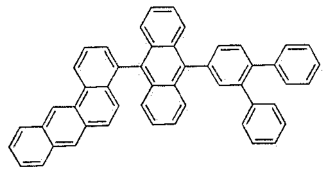 | 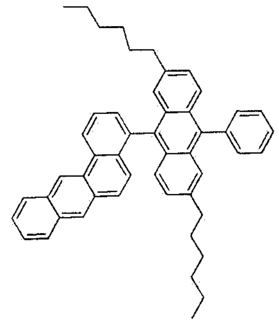 |
| 28 | 29 | 30 |

(continued)

| | | |
|---|---|---|
| 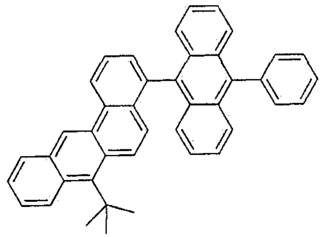 | 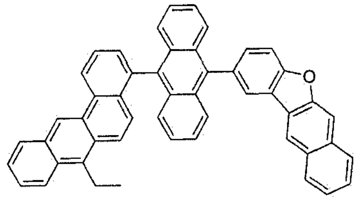 | 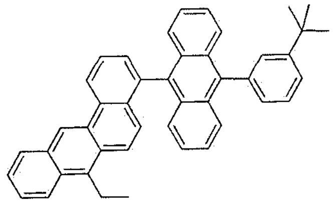 |
| 31 | 32 | 33 |
| 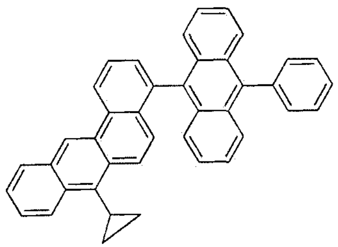 | 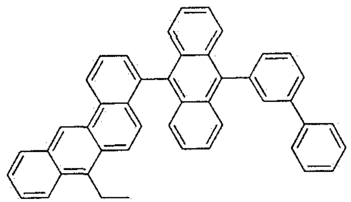 | 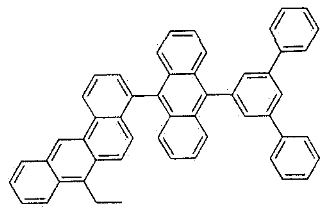 |
| 34 | 35 | 36 |
| 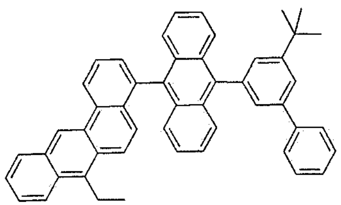 | 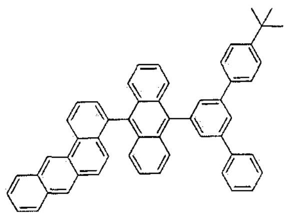 | 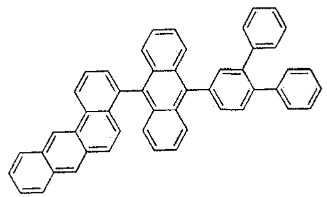 |
| 37 | 38 | 39 |

(continued)

| | | |
|---|---|---|
| 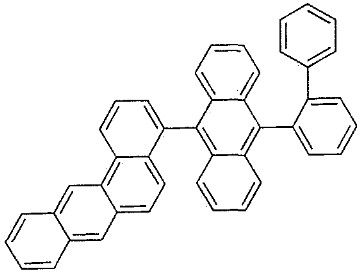 | 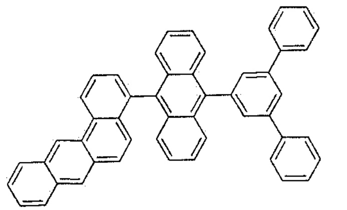 | 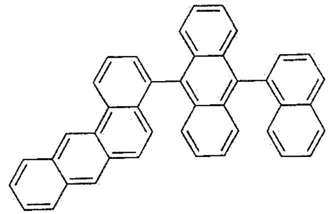 |
| 40 | 41 | 42 |
| 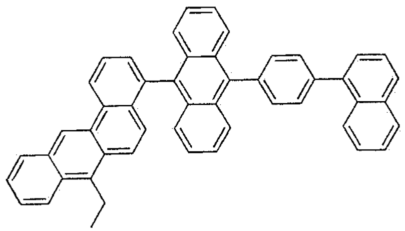 | 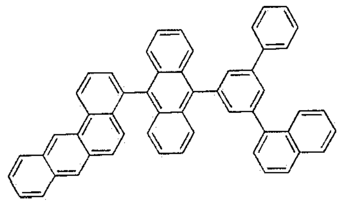 | 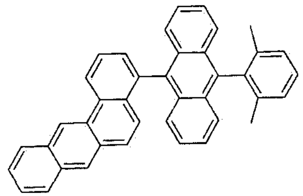 |
| 43 | 44 | 45 |
| 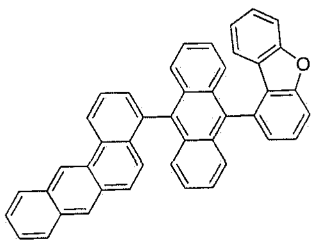 | 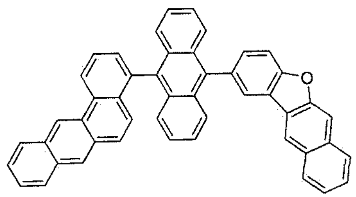 | 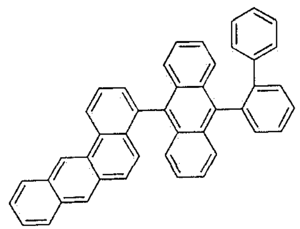 |
| 46 | 47 | 48 |
| 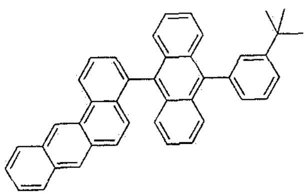 | 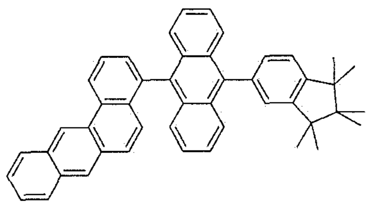 | 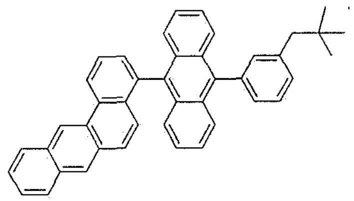 |

(continued)

| 49 | 50 | 51 |
|---|---|---|
| 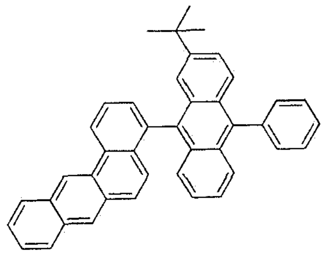 | 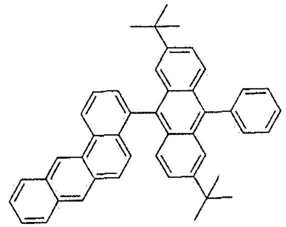 | 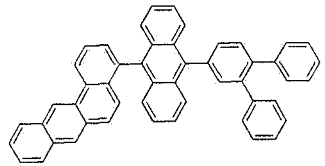 |
| 52 | 53 | 54 |
| 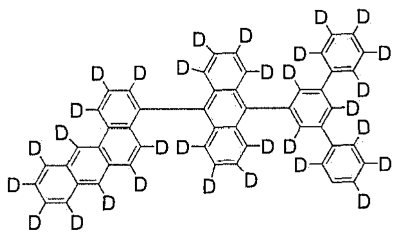 | 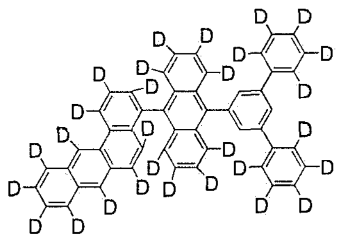 | 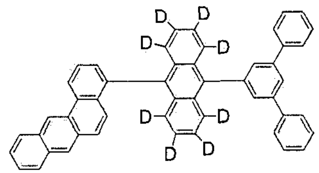 |
| 55 | 56 | 57 |
| 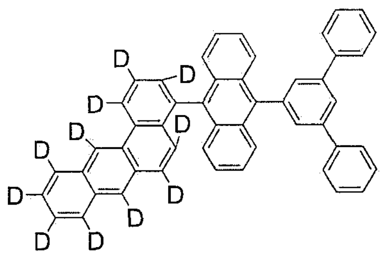 | 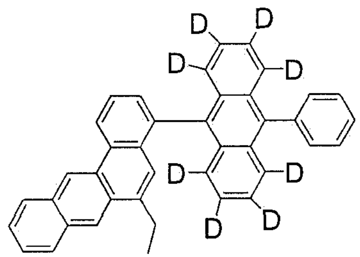 | 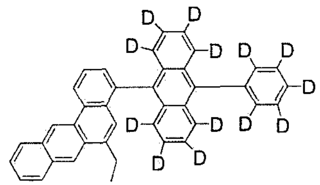 |
| 58 | 59 | 60 |

(continued)

| | | |
|---|---|---|
| 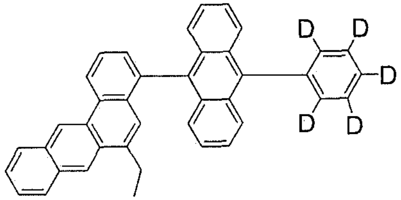 | 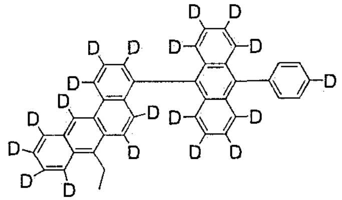 | 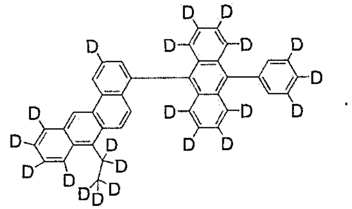 |
| 61 | 62 | 63 |
| 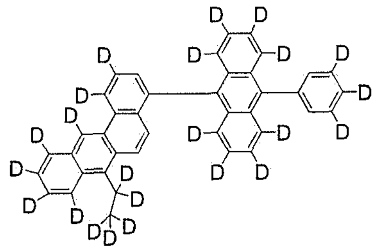 | 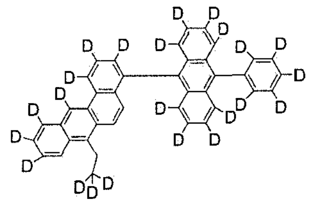 | 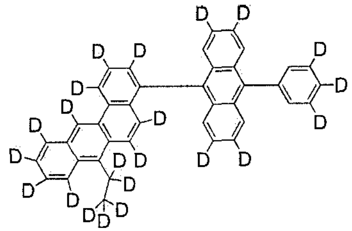 |
| 64 | 65 | 66 |
| 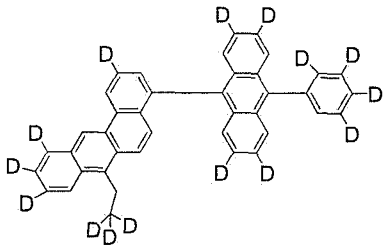 | 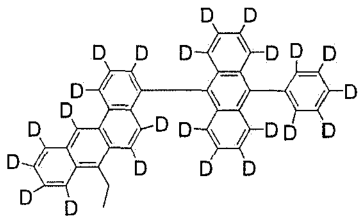 | 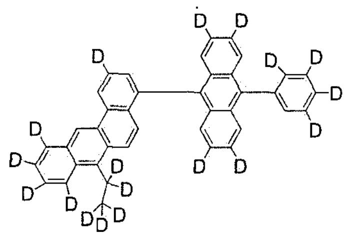 |
| 67 | 68 | 69 |
| 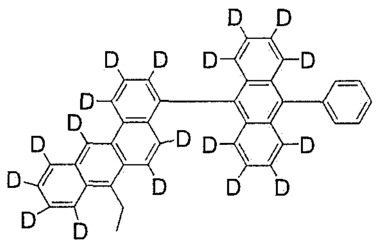 | 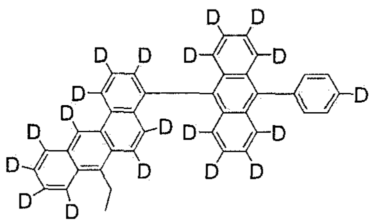 | 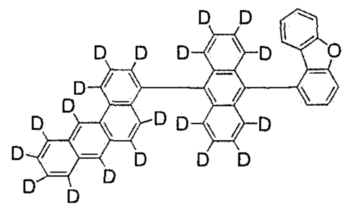 |

(continued)

| 70 | 71 | 72 |
|---|---|---|
| 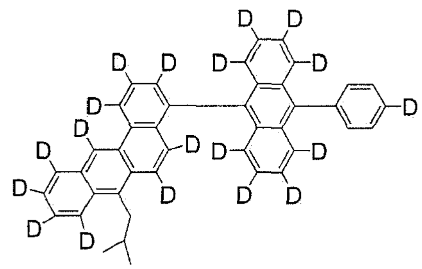 | 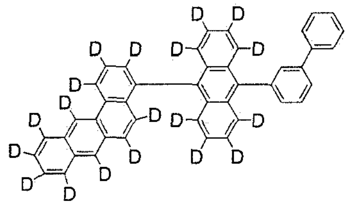 | 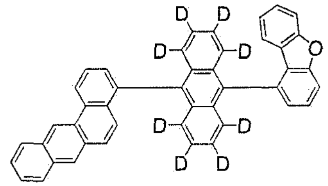 |
| 73 | 74 | 75 |
| 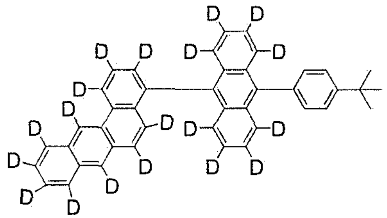 | 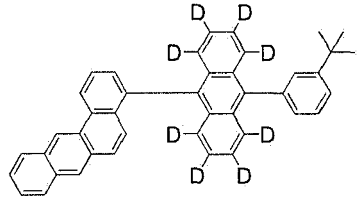 | 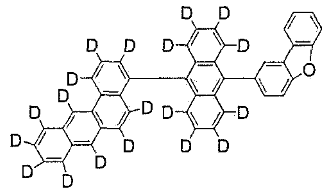 |
| 76 | 77 | 78 |
| 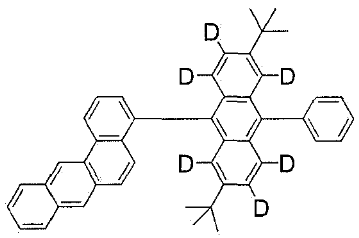 | 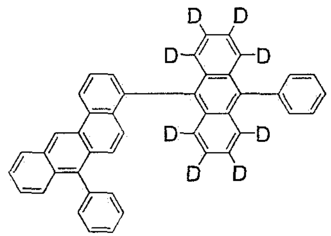 | 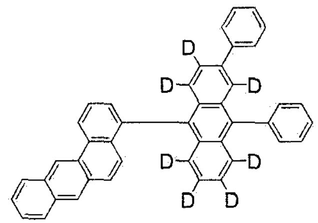 |
| 79 | 80 | 81 |

In accordance with a preferred embodiment, the composition comprises a compound of formula (H1), a compound of formula (H2) and at least one fluorescent emitter. The expression "at least one fluorescent emitter" means "one, two, three or more fluorescent emitters".

Preferably, the composition comprises at least one fluorescent emitter, which comprises at least one of the following group:

- an arylamine containing three substituted or unsubstituted aromatic or heteroaromatic ring systems bonded directly to the nitrogen;
- a condensed aromatic or heteroaromatic ring system having at least 14 aromatic ring atoms;
- an indenofluorene, indenofluorenamine or indenofluorenediamine;
- a benzoindonofluorene, benzoindenofluorenamine or benzoindenofluorenediamine;
- a dibenzoindenofluorene, dibenzoindenofluorenamine or dibenzo-indenofluorenediamine;
- an indenofluorene containing a condensed aryl group having at least 10 aromatic ring atoms;
- a bisindenoindenofluorene;
- an indenodibenzofuran; indenofluorenamine or indenofluorenediamine;
- a fluorene dimer;
- a phenoxazine; or
- a boron derivative.

More preferably, the composition comprises at least one fluorescent emitter of formula (E-1),

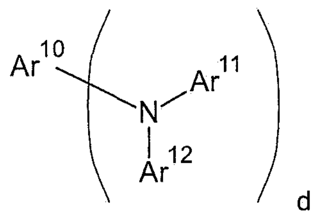

Formula (E-1)

where

$Ar^{10}$, $Ar^{11}$, $Ar^{12}$ are on each occurrence, identically or differently, an aromatic or heteroaromatic ring system having 6 to 60 aromatic ring atoms, which may in each case also be substituted by one or more radicals R; with the proviso that at least one group $Ar^{10}$, $Ar^{11}$, $Ar^{12}$ is an aromatic or heteroaromatic ring system having 10 to 40 aromatic ring atoms, containing at least one condensed aryl or heteroaryl group consisting of 2 to 4 aromatic rings condensed with one another, where the aromatic or heteroaromatic ring system may be substituted by one or more radicals R; R has the same definition as above ; and
d is 1, 2, 3 or 4; more preferably, d is 1;

or at least one fluorescent emitter of formula (E-2),

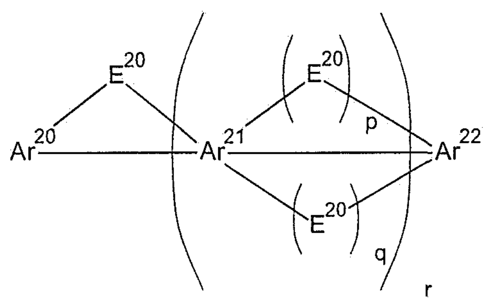

Formula (E-2)

where

$Ar^{20}$, $Ar^{21}$, $Ar^{22}$ are on each occurrence, identically or differently, an aryl or heteroaryl group having 6 to 30 aromatic ring atoms, which may in each case also be substituted by one or more radicals R;
$E^{20}$ is on each occurrence, identically or differently a group selected from BR, $C(R^0)_2$, $Si(R^0)_2$, C=O, $C=NR^0$, $C=C(R^0)_2$, O, S, S=O, $SO_2$, $NR^0$, $PR^0$, P(=O)R° or $P(=S)R^0$; wherein $Ar^{20}$, $Ar^{21}$ and $E^{20}$ together form a five-membered ring or a six-membered ring, and $Ar^{21}$, $Ar^{23}$ and $E^{20}$ together form a five-membered ring or a six-membered ring;
$R^0$ has the same definition as above;
p, q are on each occurrence, identically or differently, 0 or 1, with the proviso that p + q = 1;
r is 1, 2 oder 3;

or at least one fluorescent emitter of formula (E-3),

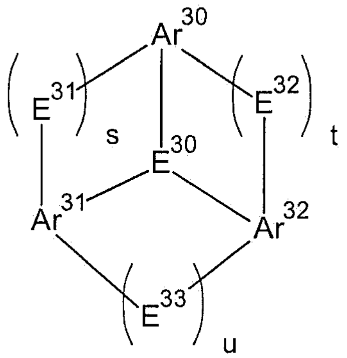

Formula (E-3)

where

$Ar^{30}$, $Ar^{31}$, $Ar^{32}$ stand on each occurrence, identically or differently, for a substituted or unsubstituted aryl or heteroaryl group having 5 to 22, preferably 5 to 18, more preferably 6 to 14 aromatic ring atoms;
$E^{30}$ stands for B or N;
$E^{31}$, $E^{32}$, $E^{33}$ stand on each occurrence, identically or differently, for O, S, $C(R^0)_2$, C=O, C=S, $C=NR^0$, $C=C(R^0)_2$, $Si(R^0)_2$, $BR^0$, $NR^0$, $PR^0$, $SO_2$, $SeO_2$ or a chemical bond, with the proviso that if $E^{30}$ is B, then at least one of the groups $E^{31}$, $E^{32}$, $E^{33}$ stands for $NR^0$ and if $E^{30}$ is N, then at least one of the groups $E^{31}$, $E^{32}$, $E^{33}$ stands for $BR^0$;
$R^0$ has the same definition as above;
s, t, u are on each occurrence, identically or differently, 0 or 1, with the proviso that $s + t + u \geq 1$.

Preferably, the fluorescent emitter of formula (E-1) comprises at least one group $Ar^{10}$, $Ar^{11}$ or $Ar^{12}$, preferably $Ar^{10}$, which is selected from the groups of formulae ($Ar^{10}$-1) to ($Ar^{10}$-24):

| | |
|---|---|
| 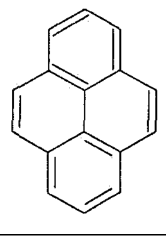 | 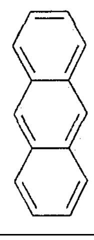 |
| $Ar^{10}$ -1 | $Ar^{10}$-2 |
| 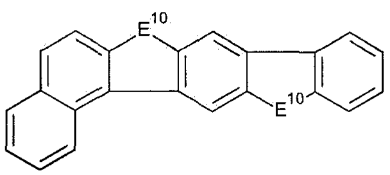 | 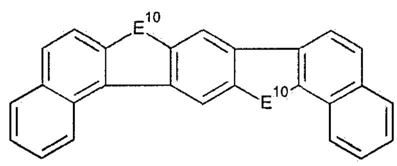 |
| $Ar^{10}$-3 | $Ar^{10}$-4 |

(continued)

| | |
|---|---|
| 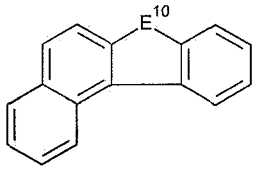 | 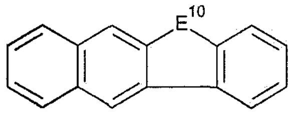 |
| $Ar^{10}$-5 | $Ar^{10}$-6 |
| 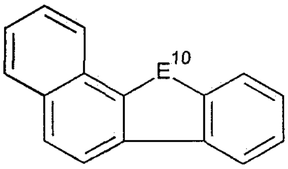 | 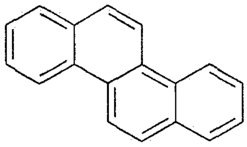 |
| $Ar^{10}$-7 | $Ar^{10}$-8 |
| 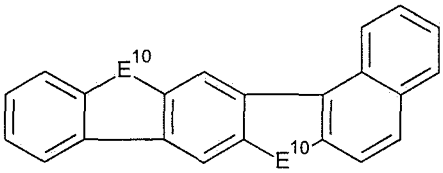 | 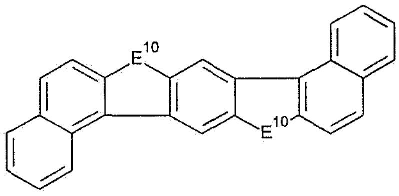 |
| $Ar^{10}$-9 | $Ar^{10}$-10 |
| 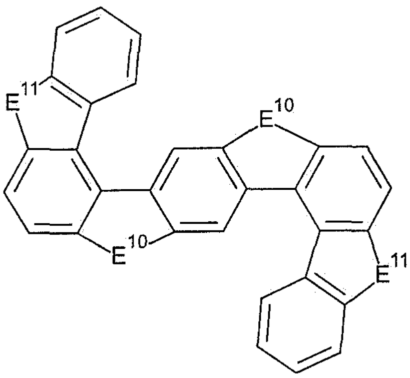 | 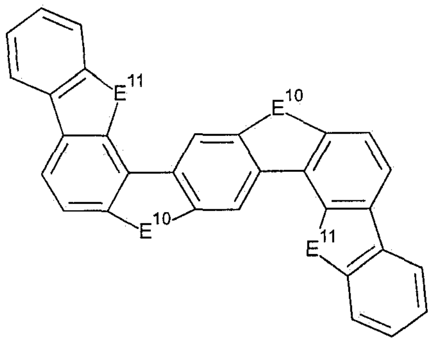 |
| $Ar^{10}$-11 | $Ar^{10}$-12 |
| 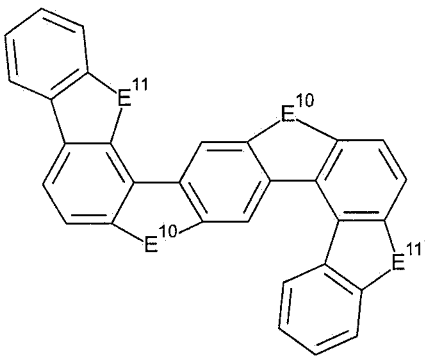 | 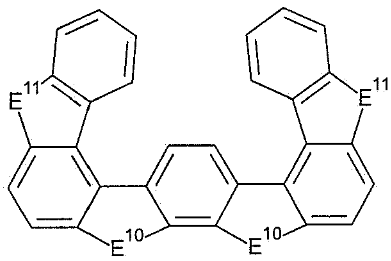 |
| $Ar^{10}$-13 | $Ar^{10}$-14 |

(continued)

| | |
|---|---|
| 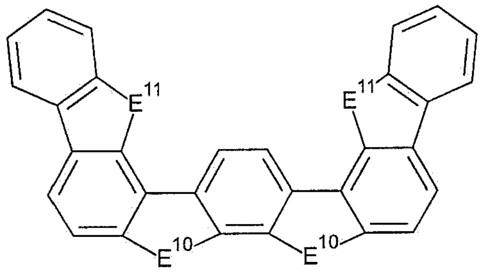 | 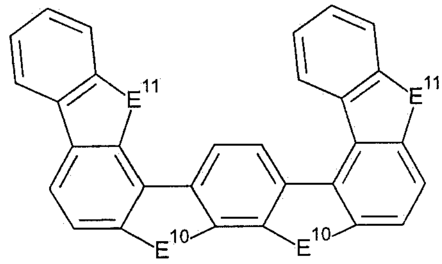 |
| $Ar^{10}$-15 | $Ar^{10}$-16 |
| 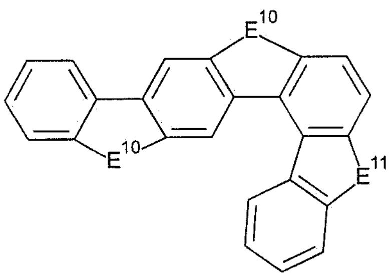 | 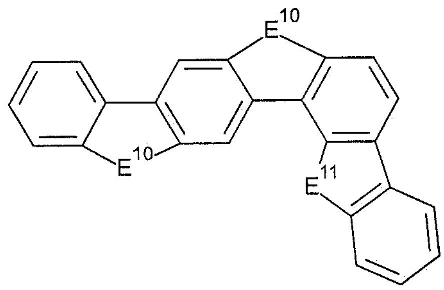 |
| $Ar^{10}$-17 | $Ar^{10}$-18 |
| 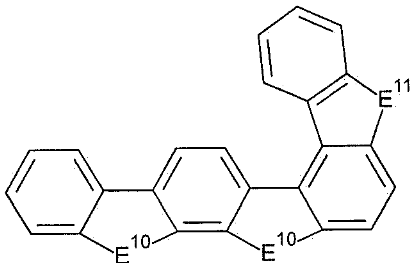 | 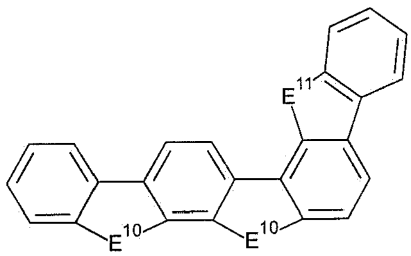 |
| $Ar^{10}$-19 | $Ar^{10}$-20 |
| 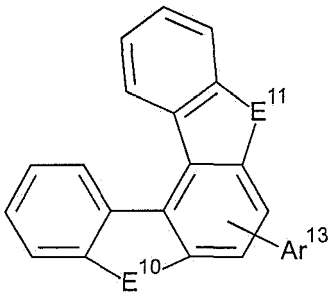 | 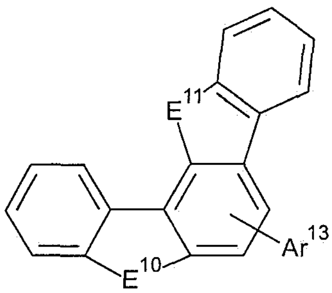 |
| $Ar^{10}$-21 | $Ar^{10}$-22 |
| 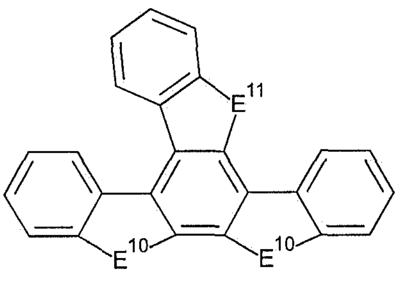 | 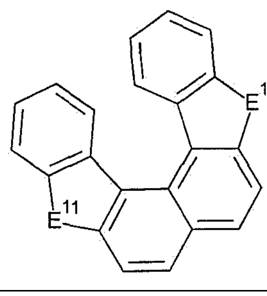 |
| $Ar^{10}$-23 | $Ar^{10}$-24 |

where the groups $Ar^{10}$-1 to $Ar^{10}$-24 may be substituted at all free positions by one or more radicals R; and where $E^{10}$ is on each occurrence, identically or differently a group selected from $BR^0$, $C(R^0)_2$, $Si(R^0)_2$, C=O, $C=NR^0$, $C=C(R^0)_2$, O, S, S=O, $SO_2$, $NR^0$, $PR^0$, P(=O)R° or P(=S)R°, preferably $E^{10}$ is $C(R^0)_2$;

where $R^0$ has the same definition as above;

$E^{11}$ is on each occurrence, identically or differently a group selected from C=O, O, S, S=O or $SO_2$, preferably O or S, more preferably O; and

$Ar^{13}$ is on each occurrence, identically or differently, an aromatic or heteroaromatic ring system having 5 to 60 aromatic ring atoms, which may in each case also be substituted by one or more radicals R.

In accordance with a preferred embodiment, the emitters of formula (E-1) comprise a group $Ar^{10}$ selected from the groups of formulae ($Ar^{10}$-15) to ($Ar^{10}$-22), wherein d is preferably equal to 1 and wherein preferably at least one group $Ar^{11}$, $Ar^{12}$ is selected from the groups of formulae ($Ar^{10}$-15) to ($Ar^{10}$-22).

In accordance with a very preferred embodiment, the fluorescent emitter of formula (E-1) is selected from the emitters of formulae (E-1-1) to (E-1-6),

| | |
|---|---|
| 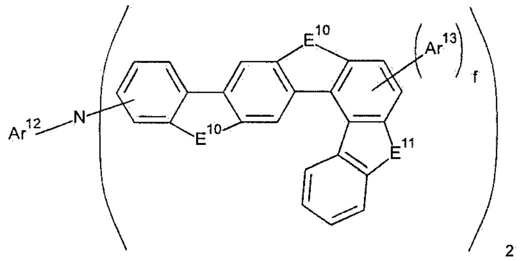 | 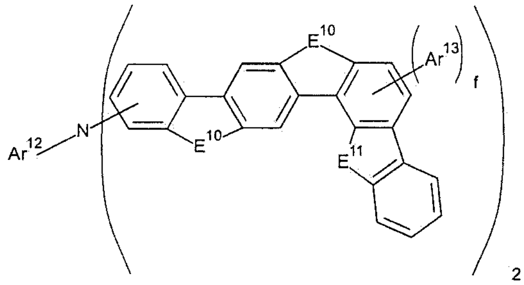 |
| (E-1-1) | (E-1-2) |
| 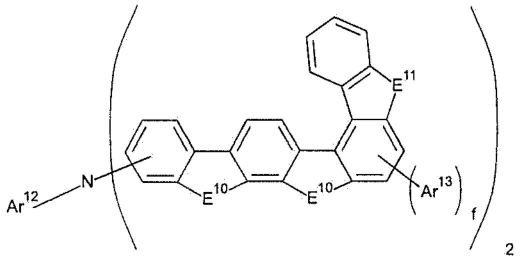 | 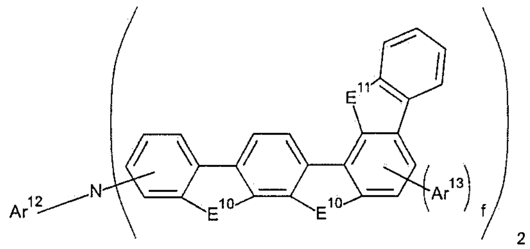 |
| (E-1-3) | (E-1-4) |
| 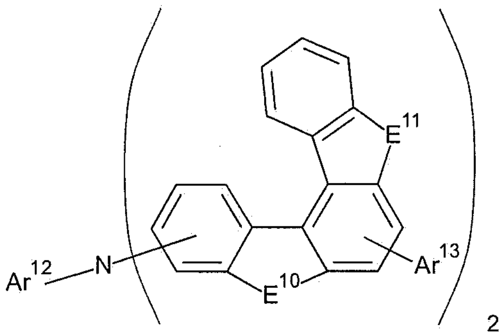 | 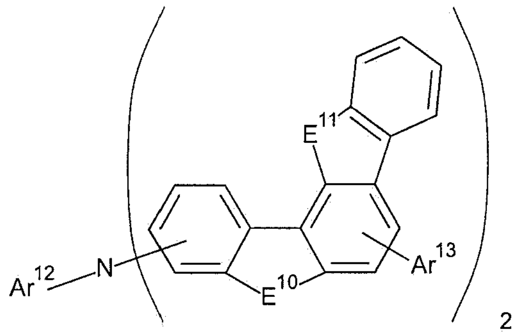 |
| (E-1-5) | (E-1-6) |

where the symbols have the same meaning as above and where:

f is 0, 1 or 2; and

the benzene rings represented above in the compounds of formulae (E-1-1) to (E-1-6) may be substituted at all free positions by one or more radicals R.

Particularly preferably, the fluorescent emitter of formula (E-1) is selected from the compounds of formulae (E-1-1-A) to (E-1-6-A),

| |
|---|
| 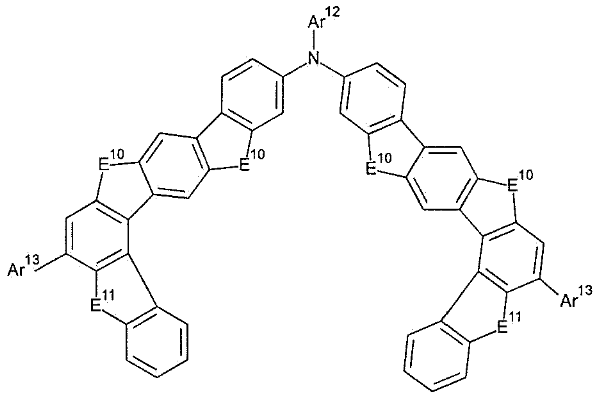 |
| formula (E-1-1-A) |
| 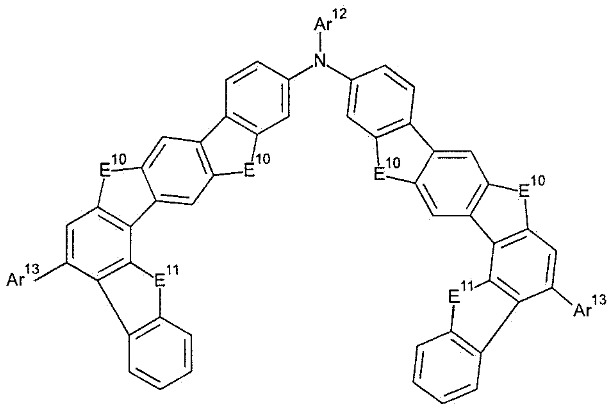 |
| formula (E-1-2-A) |
| 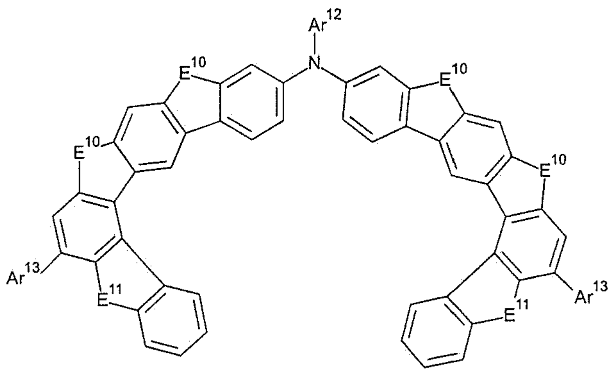 |
| formula (E-1-3-A) |

(continued)

| |
|---|
| 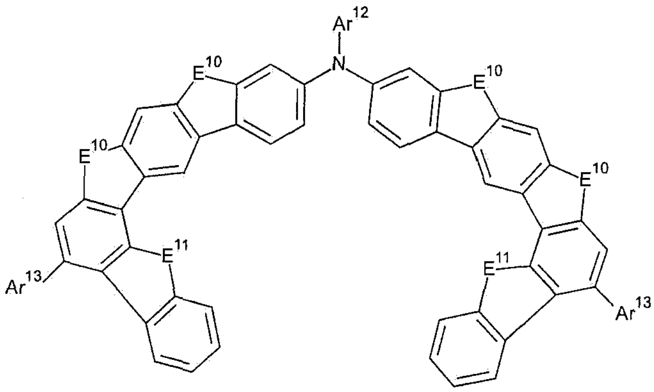 |
| formula (E-1-4-A) |
| 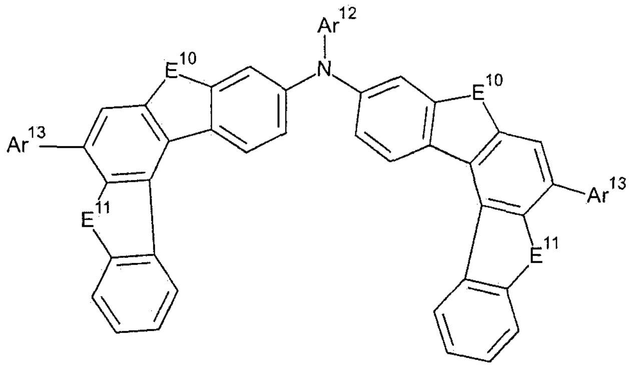 |
| formula (E-1-5-A) |
| 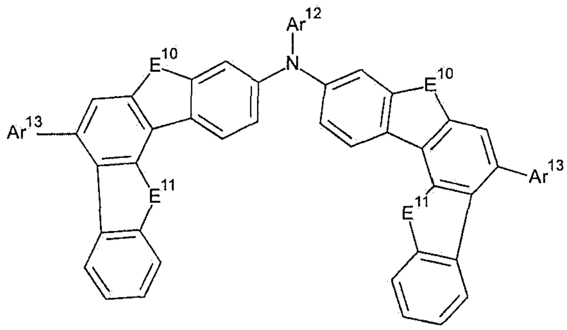 |
| formula (E-1-6-A) |

where the symbols and indices have the same meaning as above and where the benzene rings represented above in the compounds of formulae (E-1-1-A) to (E-1-6-A) may be substituted at all free positions by one or more radicals R.

Preferably, the fluorescent emitter of formula (E-2) is selected from fluorescent emitters of formula (E-2-1) to (E-2-43),

| | |
|---|---|
| 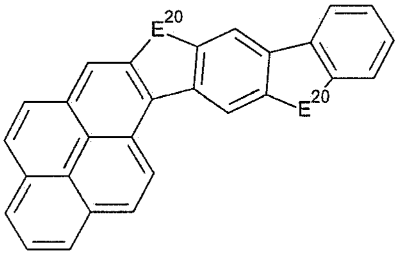 | 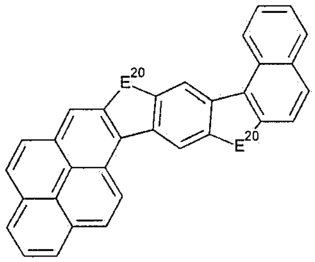 |
| (E-2-1) | (E-2-2) |
| 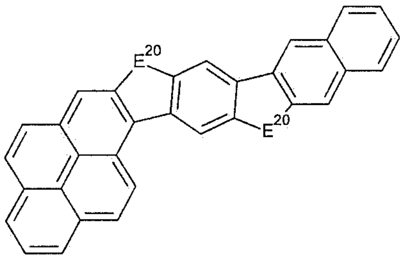 | 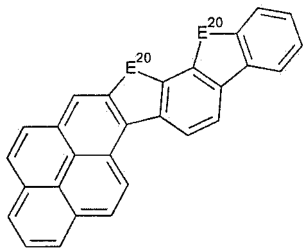 |
| (E-2-3) | (E-2-4) |
| 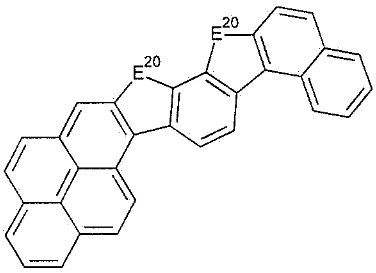 | 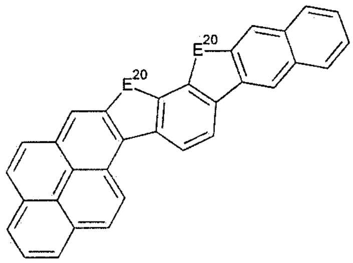 |
| (E-2-5) | (E-2-6) |
| 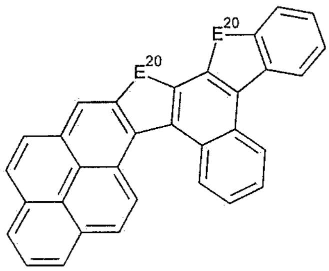 | 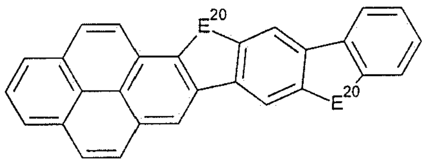 |
| (E-2-7) | (E-2-8) |
| 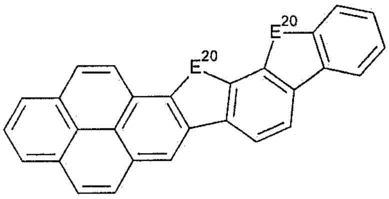 | 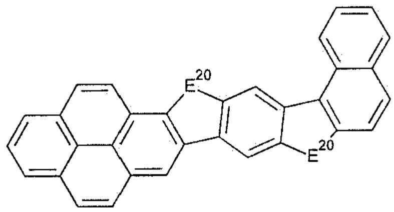 |
| (E-2-9) | (E-2-10) |

(continued)

| | |
|---|---|
| 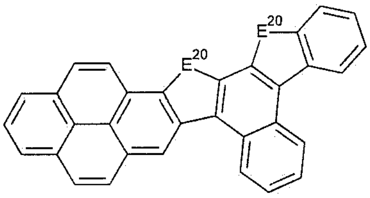 | 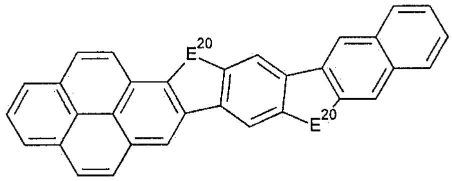 |
| (E-2-11) | (E-2-12) |
| 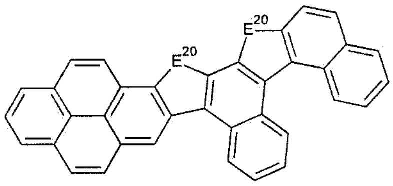 | 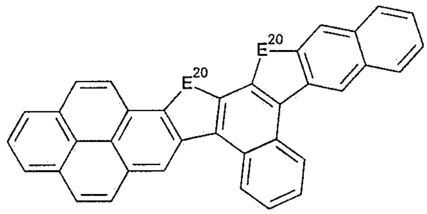 |
| (E-2-13) | (E-2-14) |
| 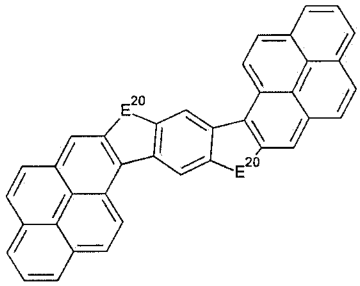 | 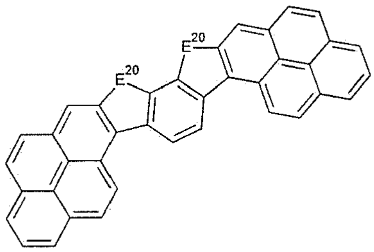 |
| (E-2-15) | (E-2-16) |
| 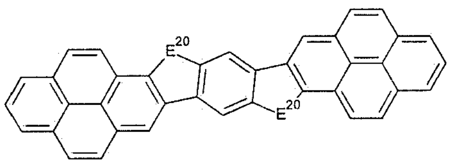 | 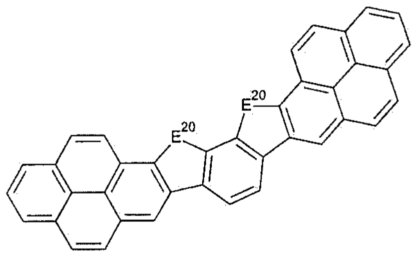 |
| (E-2-17) | (E-2-18) |
| 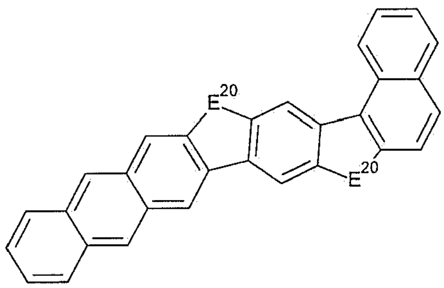 | 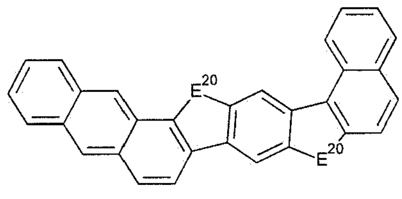 |
| (E-2-19) | (E-2-20) |

(continued)

| | |
|---|---|
| 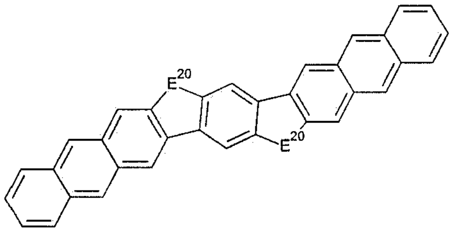 | 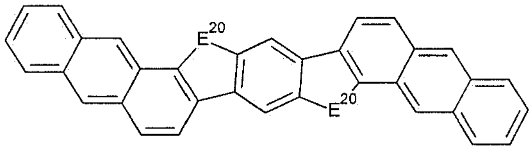 |
| (E-2-21) | (E-2-22) |
| 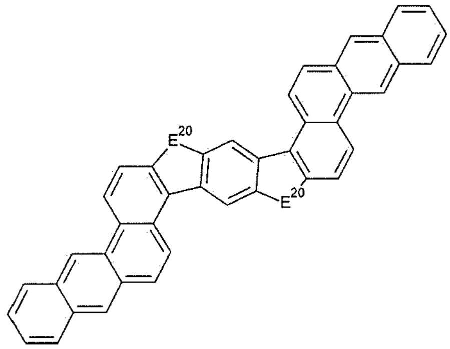 | 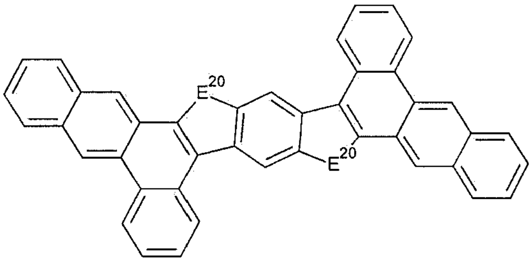 |
| (E-2-23) | (E-2-24) |
| 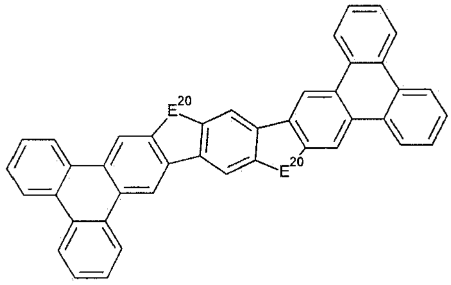 | 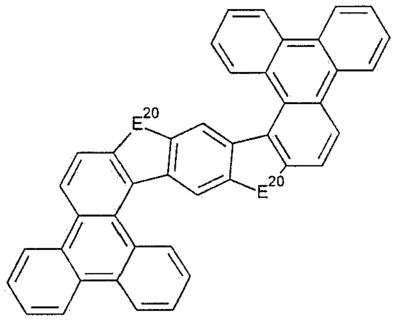 |
| (E-2-25) | (E-2-26) |
| 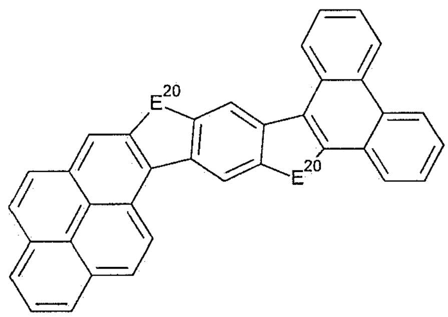 | 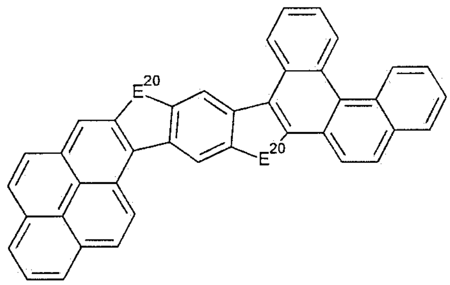 |
| (E-2-27) | (E-2-28) |

(continued)

| | |
|---|---|
| 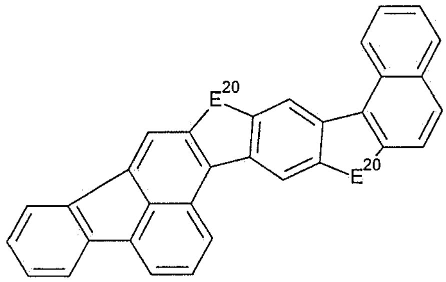 | 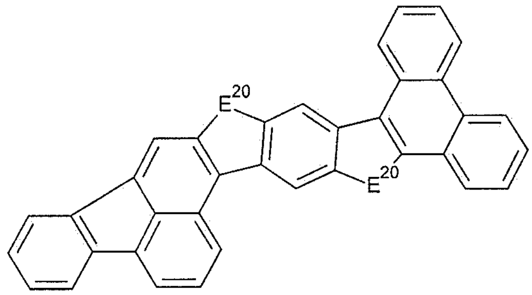 |
| (E-2-29) | (E-2-30) |
| 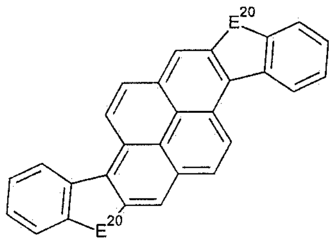 | |
| (E-2-31) | |
| 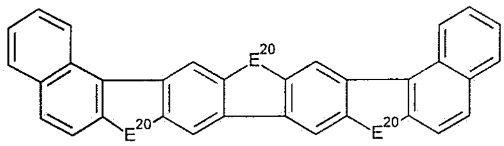 | |
| (E-2-32) | |
| 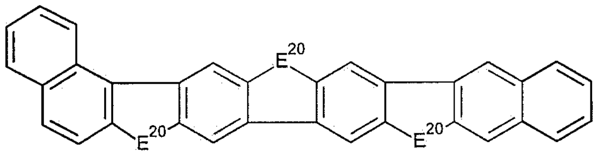 | |
| (E-2-33) | |
| 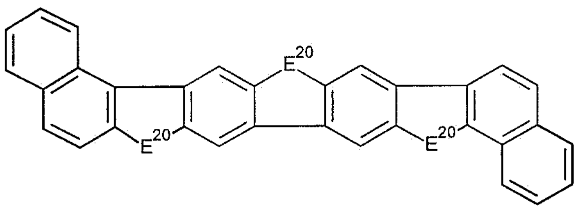 | |
| (E-2-34) | |
| 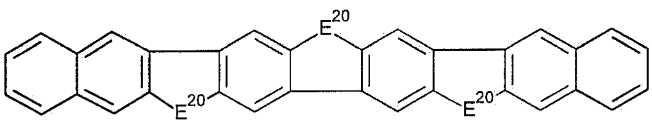 | |
| (E-2-35) | |

(continued)

| |
|---|
| 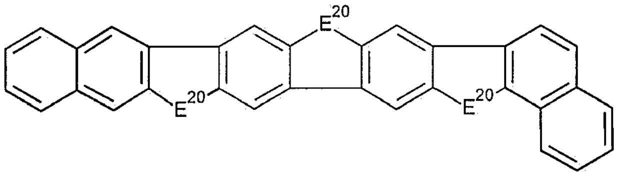 |
| (E-2-36) |
| 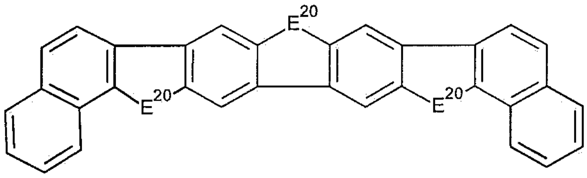 |
| (E-2-37) |
| 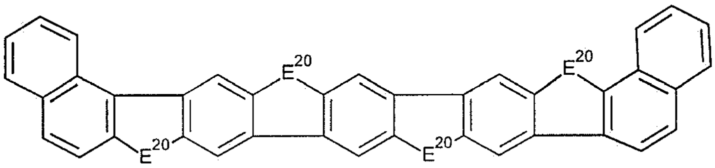 |
| (E-2-38) |
| 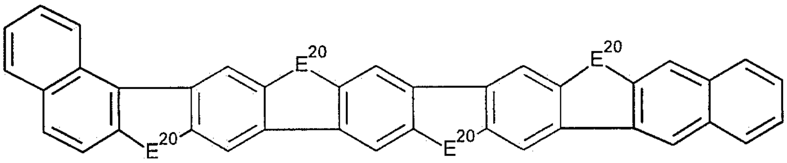 |
| (E-2-39) |
| 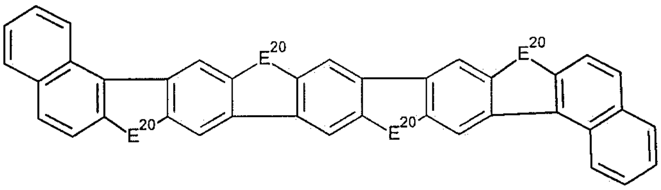 |
| (E-2-40) |
| 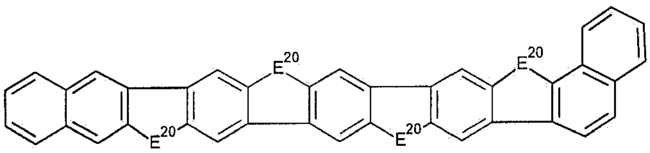 |
| (E-2-41) |
| 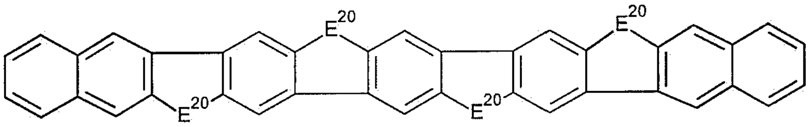 |
| (E-2-42) |

(continued)

| |
|---|
| 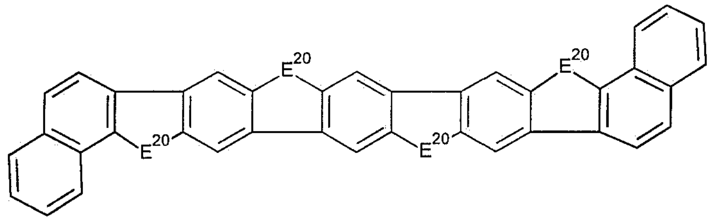 |
| (E-2-43) |

where the groups of formulae (E-2-1) to (E-2-43) may be substituted at all free positions by one or more radicals R; and where $E^{20}$ has the same definition as above. Preferably, $E^{20}$ is $C(R^0)_2$.

The fluorescent emitters of formula (E-2) are preferably selected from the compounds of formulae (E-2-32) to (E-2-43). More preferably, the compounds of formula (E-2) are selected from the compounds (E-2-32-A) to (E-2-43-A):

| |
|---|
| 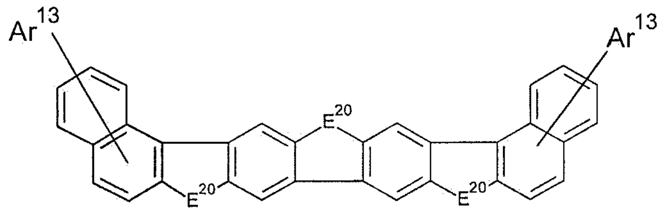 |
| (E-2-32-A) |
| 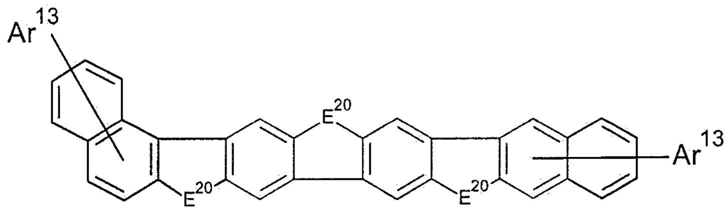 |
| (E-2-33-A) |
| 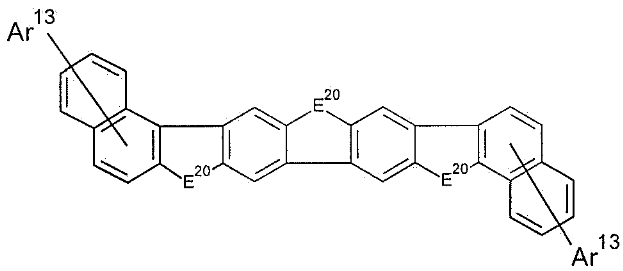 |
| (E-2-34-A) |
| 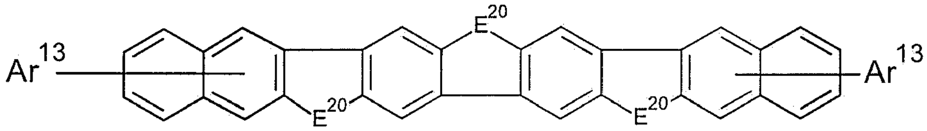 |
| (E-2-35-A) |

(continued)

| |
|---|
| 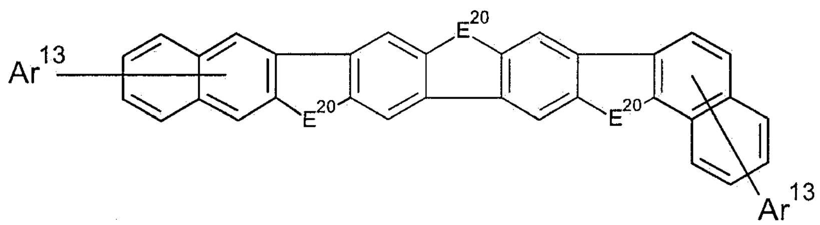 |
| (E-2-36-A) |
| 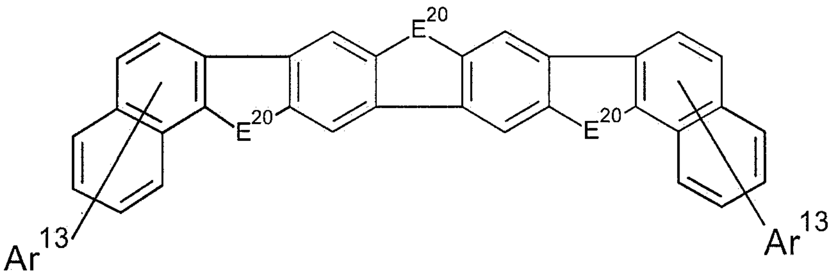 |
| (E-2-37-A) |
| 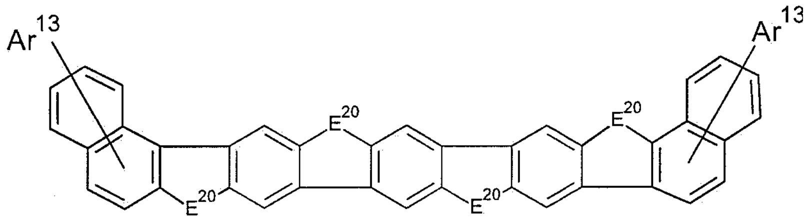 |
| (E-2-38-A) |
| 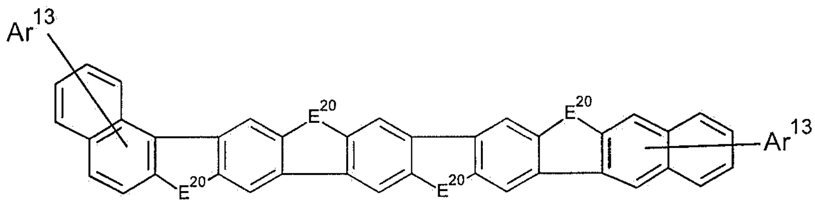 |
| (E-2-39-A) |
| 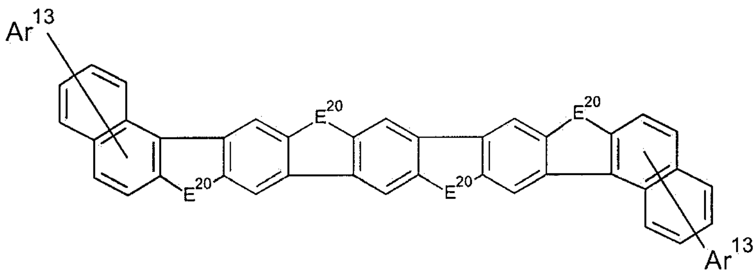 |
| (E-2-40-A) |
| 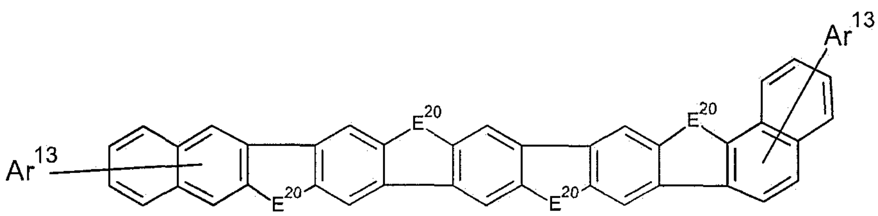 |

(continued)

| |
|---|
| (E-2-41-A) |
| 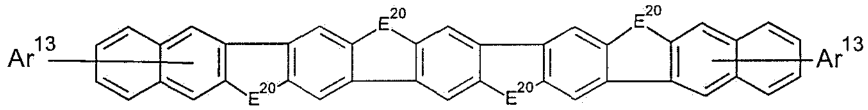 |
| (E-2-42-A) |
| 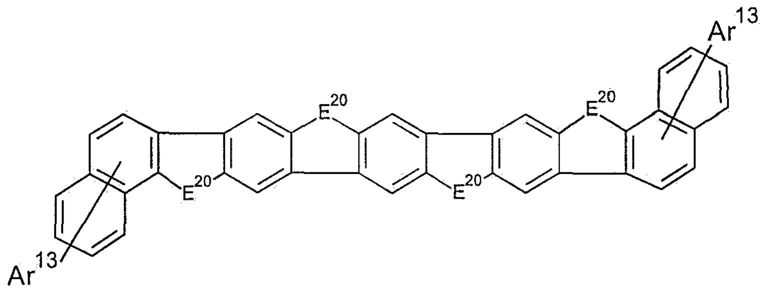 |
| (E-2-43-A) |

where the symbols have the same meaning as above and where the benzene and naphthalene rings represented above in the compounds of formulae (E-2-32-A) to (E-2-43-A) may be substituted at all free positions by one or more radicals R.

Preferably, the fluorescent emitter of formula (E-3) is selected from fluorescent emitters of formula (E-3-1),

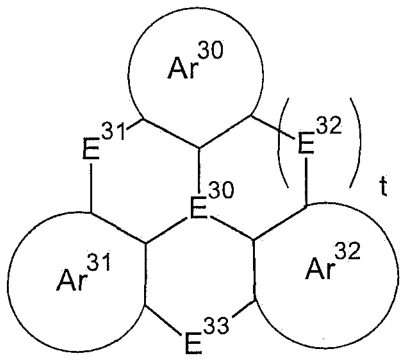

Formula (E-3-1)

where the symbols and indices have the same meaning as above.

More preferably, the fluorescent emitter of formula (E-3) is selected from fluorescent emitters of formula (E-3-2),

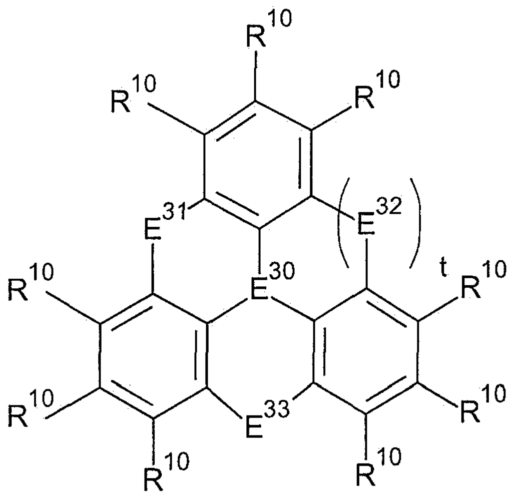

Formula (E-3-2)

where the symbols $E^{30}$ to $E^{33}$ have the same meaning as above; where t is 0 or 1, wherein when t is 0, the group $E^{32}$ is absent and radicals $R^{30}$ are present, which replace the bonds to $E^{32}$; and where

$R^{10}$ stands on each occurrence, identically or differently, for H, D, F, Cl, Br, I, CHO, CN, C(=O)Ar, $P(=O)(Ar)_2$, S(=O)Ar, $S(=O)_2Ar$, $N(R')_2$, $N(Ar)_2$, $NO_2$, $Si(R')_3$, $B(OR')_2$, $OSO_2R'$, a straight-chain alkyl, alkoxy or thioalkyl group having 1 to 40 C atoms or branched or a cyclic alkyl, alkoxy or thioalkyl group having 3 to 40 C atoms, each of which may be substituted by one or more radicals R', where in each case one or more non-adjacent $CH_2$ groups may be replaced by R'C=CR', C=C, $Si(R')_2$, $Ge(R')_2$, $Sn(R')_2$, C=O, C=S, C=Se, P(=O)(R'), SO, $SO_2$, O, S or CONR' and where one or more H atoms may be replaced by D, F, Cl, Br, I, CN or $NO_2$, an aromatic or heteroaromatic ring system having 5 to 60 aromatic ring atoms, which may in each case be substituted by one or more radicals R', or an aryloxy group having 5 to 60 aromatic ring atoms, which may be substituted by one or more radicals R'; where two adjacent substituents $R^{10}$ may form an aliphatic or aromatic ring system together, which may be substituted by one or more radicals R'; where R' has the same definition as above.

Even more preferably, the fluorescent emitter of formula (E-3) is selected from fluorescent emitters of formula (E-3-3) and (E-3-4),

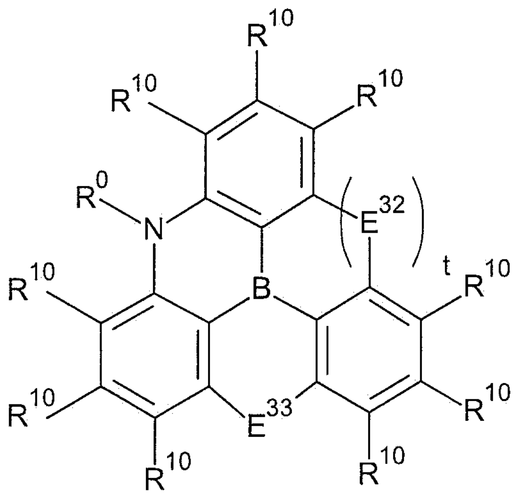

Formula (E-3-3)

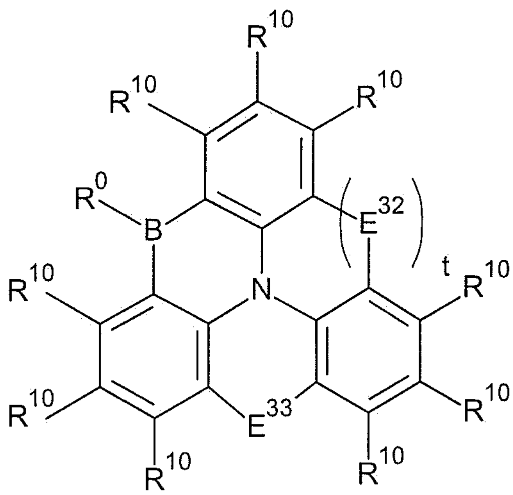

Formula (E-3-4)

where the symbols and indices have the same meaning as above.

In accordance with a preferred embodiment, the fluorescent emitter of formula (E-1), (E-2) or (E-3), comprises

a group RS, wherein the group RS is selected:

- from branched or cyclic alkyl groups represented by the general following formula a group of formula (RS-a),

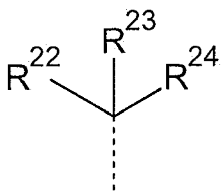

(RS-a)

wherein

$R^{22}$, $R^{23}$, $R^{24}$ are at each occurrence, identically or differently, selected from H, a straight-chain alkyl group having 1 to 10 carbon atoms, or a branched or cyclic alkyl group having 3 to 10 carbon atoms, where the above-mentioned groups may each be substituted by one or more radicals $R^{25}$, and where two of radicals $R^{22}$, $R^{23}$, $R^{24}$ or all radicals $R^{22}$, $R^{23}$, $R^{24}$ may be joined to form a (poly)cyclic alkyl group, which may be substituted by one or more radicals $R^{25}$;
$R^{25}$ is at each occurrence, identically or differently, selected from a straight-chain alkyl group having 1 to 10 carbon atoms, or a branched or cyclic alkyl group having 3 to 10 carbon atoms;
with the proviso that at each occurrence at least one of radicals $R^{22}$, $R^{23}$ and $R^{24}$ is other than H, with the proviso that at each occurrence all of radicals $R^{22}$, $R^{23}$ and $R^{24}$ together have at least 4 carbon atoms and with the proviso that at each occurrence, if two of radicals $R^{22}$, $R^{23}$, $R^{24}$ are H, the remaining radical is not a straight-chain; or

- from branched or cyclic alkoxy groups represented by the general following formula (RS-b)

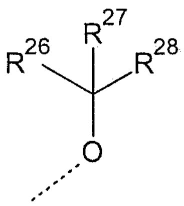

(RS-b)

wherein
$R^{26}$, $R^{27}$, $R^{28}$ are at each occurrence, identically or differently, selected from H, a straight-chain alkyl group having 1 to 10 carbon atoms, or a branched or cyclic alkyl group having 3 to 10 carbon atoms, where the above-mentioned groups may each be substituted by one or more radicals $R^{25}$ as defined above, and where two of radicals $R^{26}$, $R^{27}$, $R^{28}$ or all radicals $R^{26}$, $R^{27}$, $R^{28}$ may be joined to form a (poly)cyclic alkyl group, which may be substituted by one or more radicals $R^{25}$ as defined above; with the proviso that at each occurrence only one of radicals $R^{26}$, $R^{27}$ and $R^{28}$ may be H;

- from aralkyl groups represented by the general following formula (RS-c)

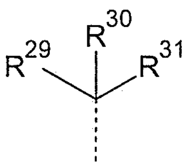

(RS-c)

wherein

$R^{29}$, $R^{30}$, $R^{31}$ are at each occurrence, identically or differently, selected from H, a straight-chain alkyl group having 1 to 10 carbon atoms, or a branched or cyclic alkyl group having 3 to 10 carbon atoms, where the above-mentioned groups may each be substituted by one or more radicals $R^{32}$, or an aromatic ring system having 6 to 30 aromatic ring atoms, which may in each case be substituted by one or more radicals $R^{32}$, and where two or all of radicals $R^{29}$, $R^{30}$, $R^{31}$ may be joined to form a (poly)cyclic alkyl group or an aromatic ring system, each of which may be substituted by one or more radicals $R^{32}$;

$R^{32}$ is at each occurrence, identically or differently, selected from a straight-chain alkyl group having 1 to 10 carbon atoms, or a branched or cyclic alkyl group having 3 to 10 carbon atoms, or an aromatic ring system having 6 to 24 aromatic ring atoms;

with the proviso that at each occurrence at least one of radicals $R^{29}$, $R^{30}$ and $R^{31}$ is other than H and that at each occurrence at least one of radicals $R^{29}$, $R^{30}$ and $R^{31}$ is or contains an aromatic ring system having at least 6 aromatic ring atoms;

- from aromatic ring systems represented by the general following formula (RS-d)

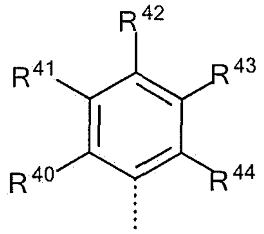

(RS-d)

wherein

$R^{40}$ to $R^{44}$ is at each occurrence, identically or differently, selected from H, a straight-chain alkyl group having 1 to 10 carbon atoms, or a branched or cyclic alkyl group having 3 to 10 carbon atoms, where the above-mentioned groups may each be substituted by one or more radicals $R^{32}$, or an aromatic ring system having 6 to 30 aromatic ring atoms, which may in each case be substituted by one or more radicals $R^{32}$, and where two or more of radicals $R^{40}$ to $R^{44}$ may be joined to form a (poly)cyclic alkyl group or an aromatic ring system, each of which may be substituted by one or more radicals $R^{32}$ as defined above; or

- from groups of formula (RS-e),

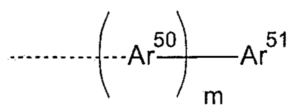

formula (RS-e)

where the dashed bond in formula (RS-e) indicates the bonding to the fluorescent emitter, where $Ar^{50}$, $Ar^{51}$ stand on each occurrence, identically or differently, for an aromatic or heteroaromatic ring systems having 5 to 60 aromatic ring atoms, which may in each case be substituted by one or more radicals R; and where m is an integer selected from 1 to 10.

Preferably, the index m in the group of formula (RS-e) is an integer selected from 1 to 6, very preferably from 1 to 4.

Preferably, where $Ar^{50}$, $Ar^{51}$ stand on each occurrence, identically or differently, for an aromatic or heteroaromatic ring systems having 5 to 40, preferably 5 to 30, more preferably 6 to 18 aromatic ring atoms, which may in each case be substituted by one or more radicals R. More preferably, $Ar^{50}$, $Ar^{51}$ are selected from phenyl, biphenyl, terphenyl, quaterphenyl, fluorene, spirobifluorene, naphthalene, anthracene, phenanthrene, triphenylene, fluoranthene, dibenzofuran, carbazole and dibenzothiophene, which may in each case be substituted by one or more radicals R. Very preferably, at least one group $Ar^{50}$ or $Ar^{51}$ is a fluorene, which may be substituted by one or more radicals R.

More particularly, it is preferred that at least one group $Ar^{50}$ stands for a group of formula (Ar50-2) and/or at

least one group $Ar^{51}$ stands for a group of formula (Ar51-2),

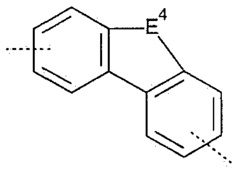
(Ar50-2)

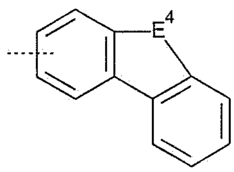
(Ar51-2)

where

the dashed bonds in formula (Ar50-2) indicate the bonding to the fluorescent emitter and to a group $Ar^{50}$ or $Ar^{51}$; and the dashed bond in formula (Ar51-2) indicates the bonding to $Ar^{50}$;

$E^4$ is selected from $-C(R^{0a})_2-$, $-Si(R^{0a})_2-$, -O-, -S- or $-N(R^{0a})-$, preferably - $C(R^{0a})2$;

$R^{0a}$ stands on each occurrence, identically or differently, for H, D, F, CN, a straight-chain alkyl group having 1 to 40, preferably 1 to 20, more preferably 1 to 10 C atoms or branched or cyclic alkyl group having 3 to 40, preferably 3 to 20, more preferably 3 to 10 C atoms, each of which may be substituted by one or more radicals R, an aromatic or heteroaromatic ring system having 5 to 60, preferably 5 to 40, more preferably 5 to 30, very preferably 5 to 18 aromatic ring atoms, which may in each case be substituted by one or more radicals R; where two adjacent substituents $R^{0a}$ may form a mono- or polycyclic, aliphatic ring system or aromatic ring system, which may be substituted by one or more radicals R, which has the same meaning as above; and

the groups of formulae (Ar50-2) and (Ar51-2) may be substituted at each free position by a group R, which has the same meaning as above.

The group RS is preferably located at a position, where it replaces R, $R^0$ or R'.

Examples of fluorescent emitters which may be employed in the composition comprising the compounds of formulae (H1) and (H2) are aromatic anthra-cenamines, aromatic anthracenediamines, aromatic pyrenamines, aromatic pyrenediamines, aromatic chrysenamines or aromatic chrysenediamines. An aromatic anthracenamine is taken to mean a compound in which one diarylamino group is bonded directly to an anthracene group, preferably in the 9-position. An aromatic anthracenediamine is taken to mean a compound in which two diarylamino groups are bonded directly to an anthracene group, preferably in the 9,10-position. Aromatic pyrenamines, pyrenediamines, chrysenamines and chrysenediamines are defined analogously thereto, where the diarylamino groups are preferably bonded to the pyrene in the 1-position or in the 1,6-position. Further preferred emitters are indenofluoren-amines or indenofluorenediamines, for example in accordance with WO 2006/108497 or WO 2006/122630, benzoindenofluorenamines or benzoindenofluorenediamines, for example in accordance with WO 2008/ 006449, and dibenzoindenofluorenamines or dibenzoindenofluorene-diamines, for example in accordance with WO 2007/140847, and the indenofluorene derivatives containing condensed aryl groups which are disclosed in WO 2010/012328. Still further preferred emitters are benzanthracene derivatives as disclosed in WO 2015/158409, anthracene derivatives as disclosed in WO 2017/036573, fluorene dimers connected via heteroaryl groups like in WO 2016/150544 or phenoxazine derivatives as disclosed in WO 2017/028940 and WO 2017/028941. Preference is likewise given to the pyrenarylamines disclosed in WO 2012/048780 and WO 2013/185871. Preference is likewise given to the benzoindenofluorenamines disclosed in WO 2014/037077, the benzofluorenamines disclosed in WO 2014/106522 and the indenofluorenes disclosed in WO 2014/111269 or WO 2017/036574, WO 2018/007421. Also preferred are the emitters comprising dibenzofuran or indenodibenzofuran moieties as disclosed in WO 2018/095888, WO 2018/095940, WO 2019/076789, WO 2019/170572 as well as in the unpublished applications PCT/EP2019/072697, PCT/EP2019/072670 and PCT/EP2019/072662. Preference is likewise given to boron derivatives as disclosed, for example, in WO 2015/102118, CN108409769, CN107266484, WO2017195669, US2018069182 as well as in the unpublished applications EP 19168728.4, EP 19199326.0 and EP 19208643.7.

In the case of the present invention, very suitable fluorescent emitters are the indenofluorene derivatives disclosed in WO 2018/007421 and the dibenzofuran derivatives disclosed in WO 2019/076789,

Examples of preferred fluorescent emitting compounds, which may be employed in the composition comprising the compounds of formulae (H1) and (H2) are depicted in the following table:

| | |
|---|---|
| 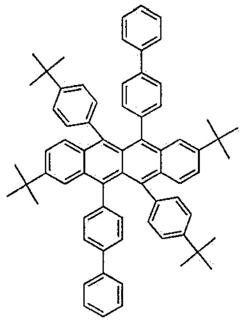 | 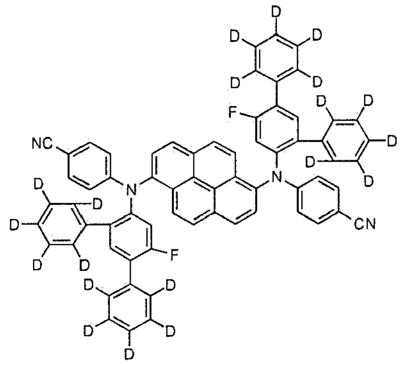 |

| | |
|---|---|
| 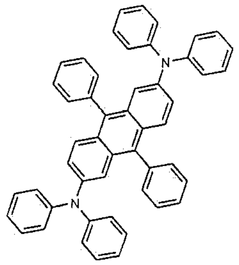 | 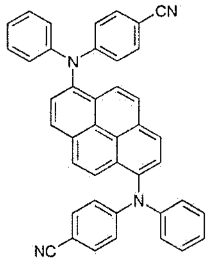 |
| 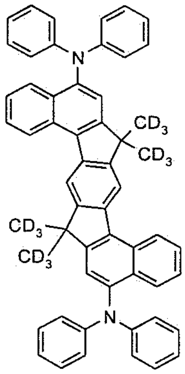 | 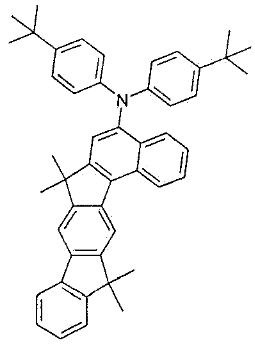 |
| 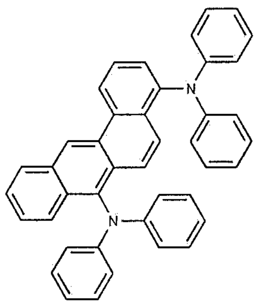 | 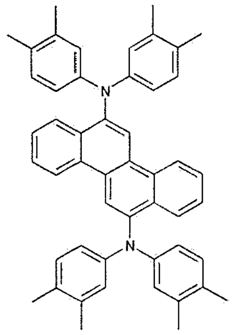 |
| 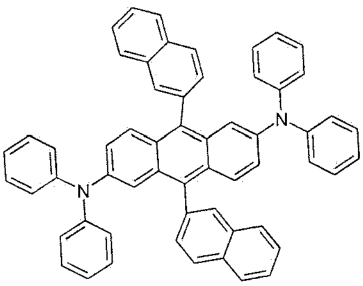 | 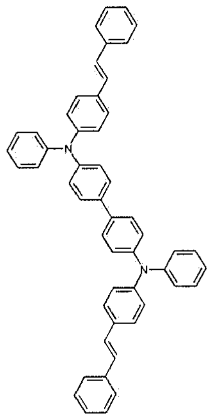 |

| | |
|---|---|
| 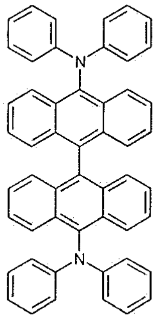 | 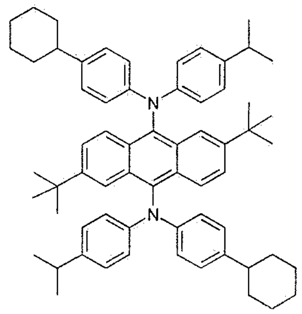 |
| 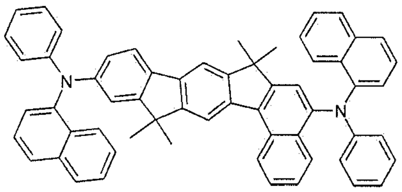 | 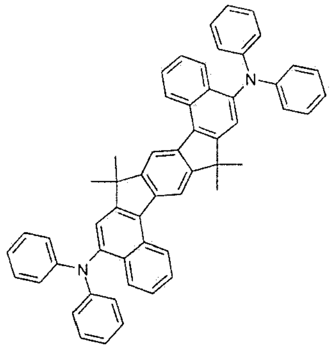 |
| 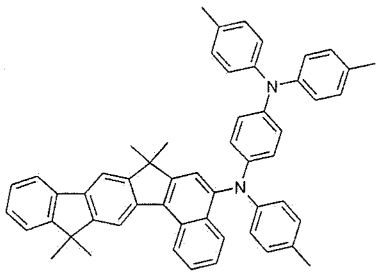 | 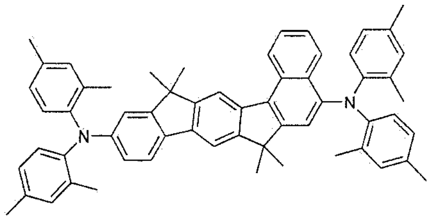 |
| 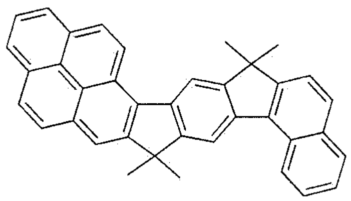 | 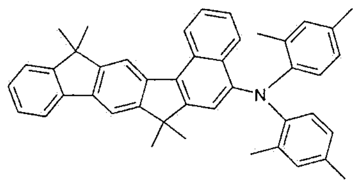 |

| | |
|---|---|
| 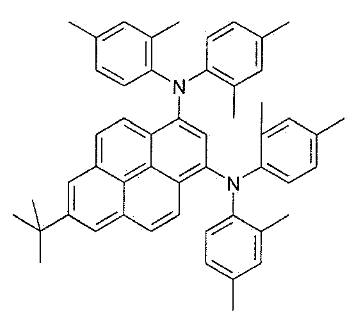 | 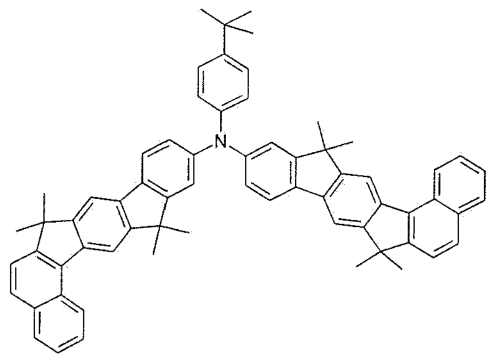 |
| 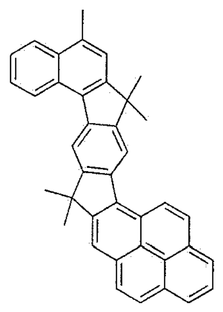 | 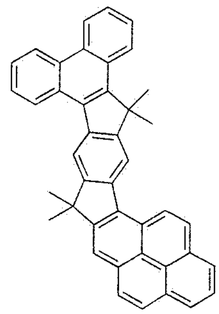 |
| 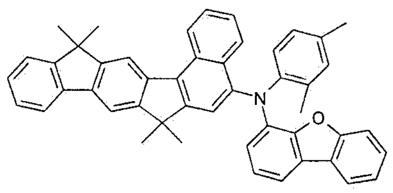 | 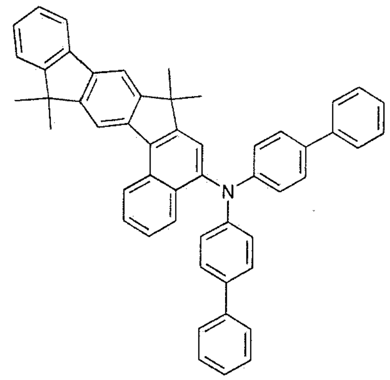 |
| 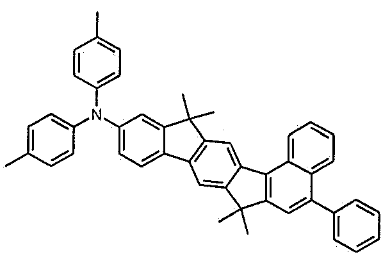 | 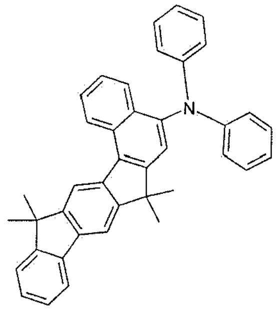 |

| | |
|---|---|
| 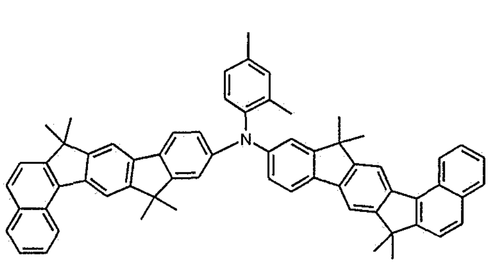 | 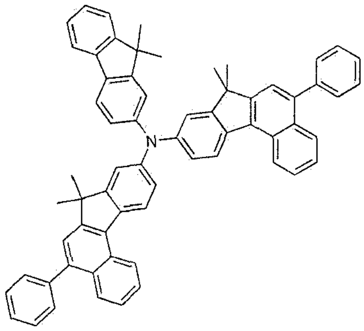 |
| 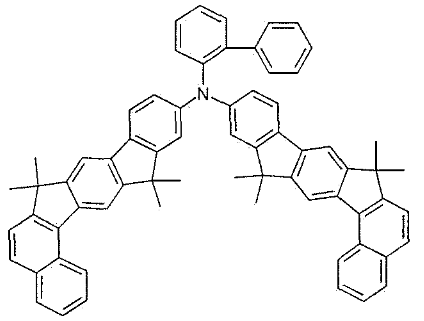 | 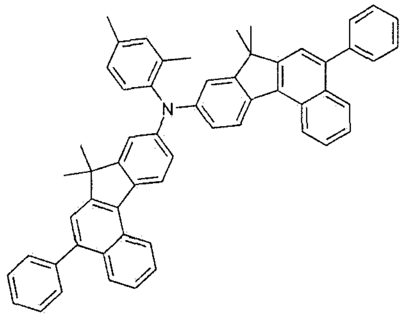 |
| 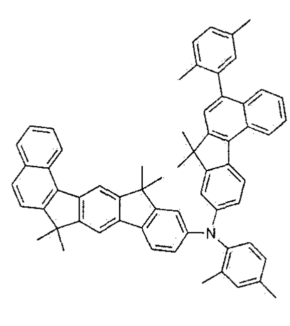 | 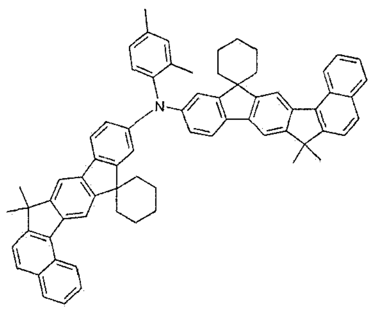 |
| 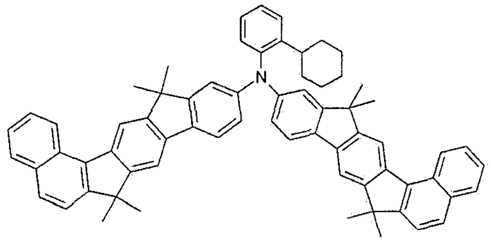 | 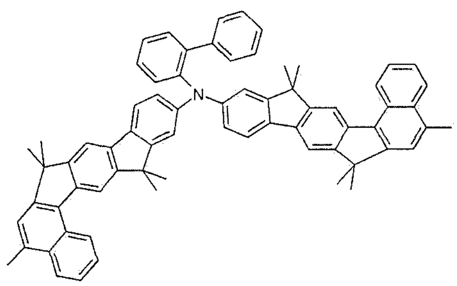 |

| | |
|---|---|
| 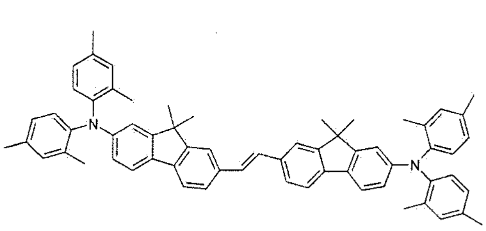 | 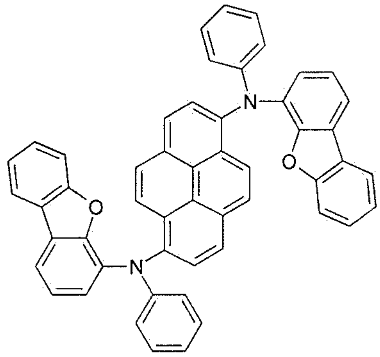 |
| 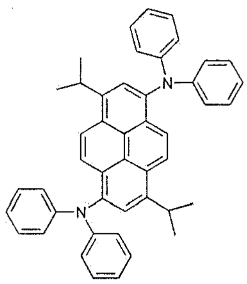 | 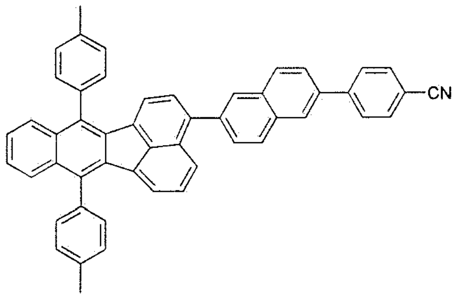 |
| 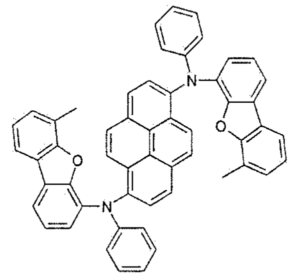 | 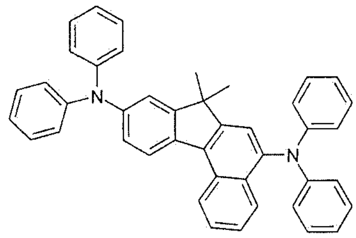 |
| 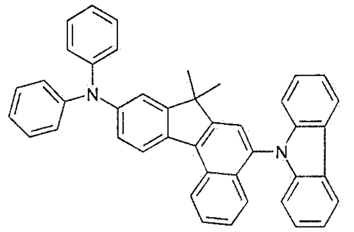 | 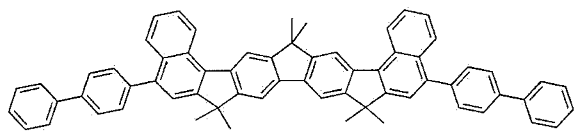 |
| 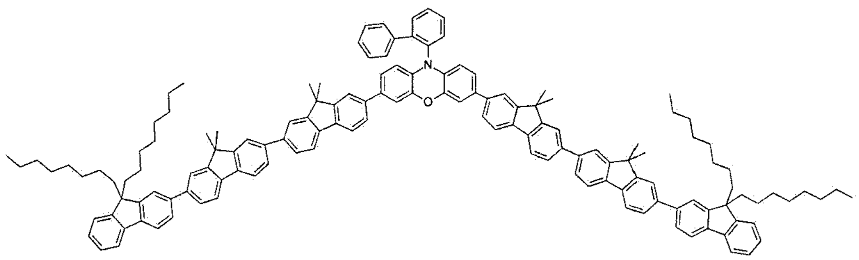 | |

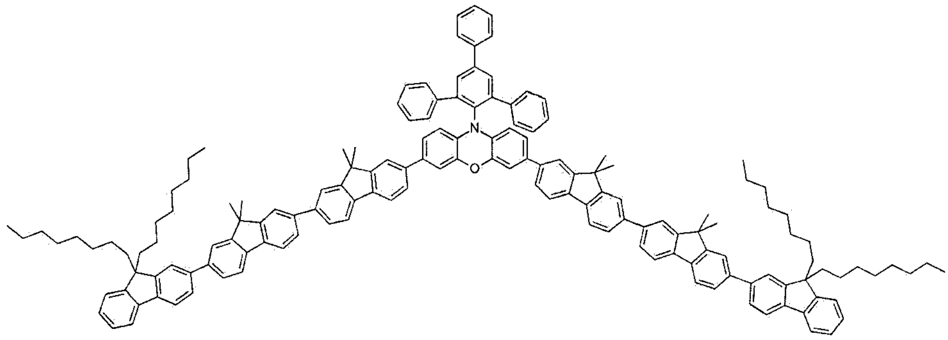

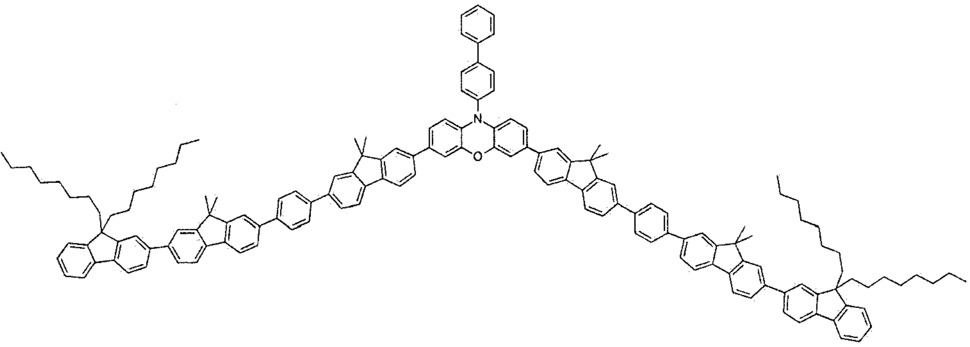

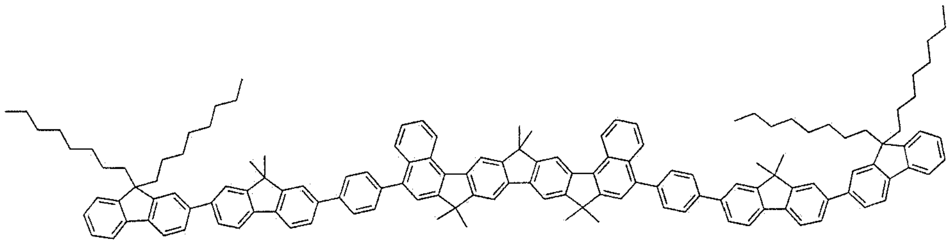

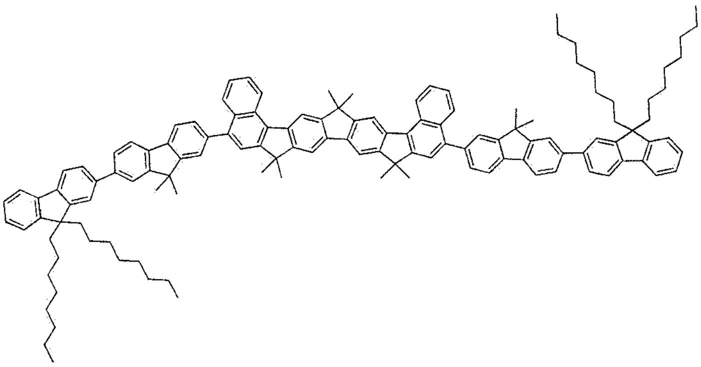

| | |
|---|---|
| 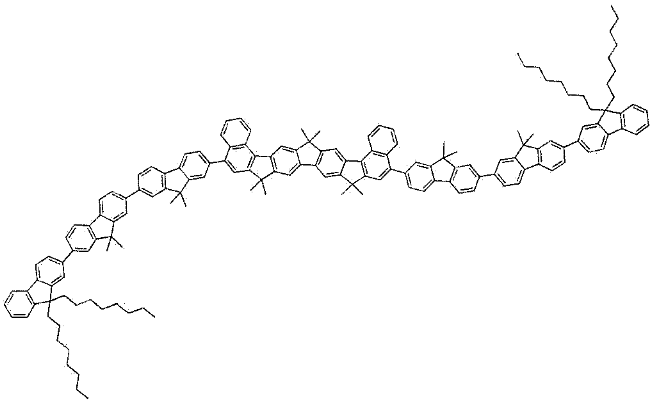 | |
| 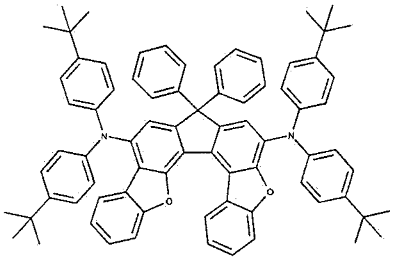 | 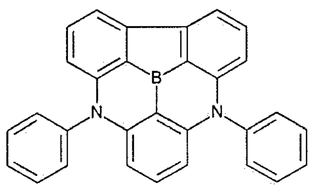 |
| 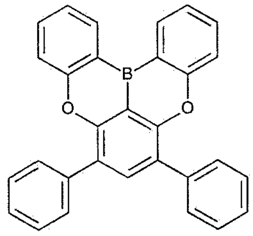 | 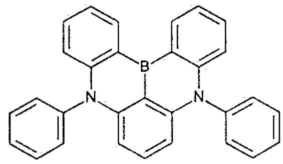 |
| 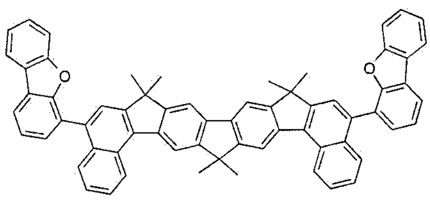 | 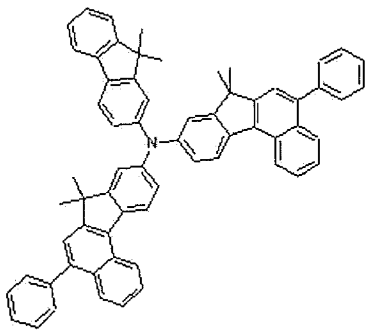 |

| | |
|---|---|
| 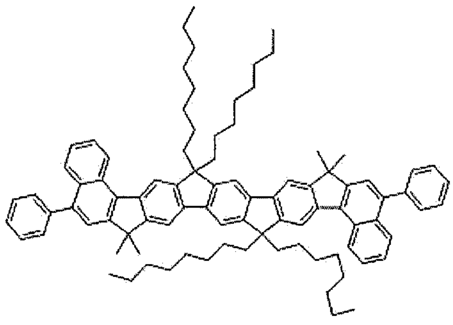 | 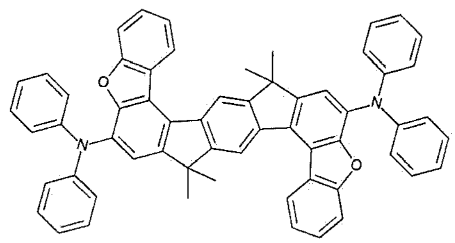 |
| 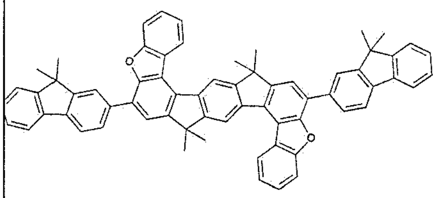 | 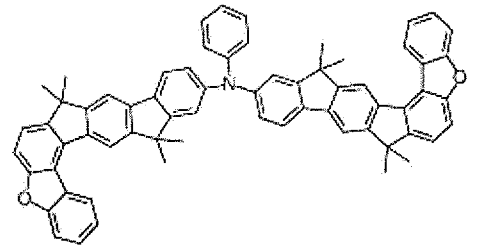 |
| 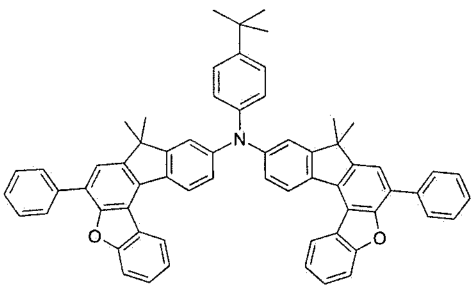 | 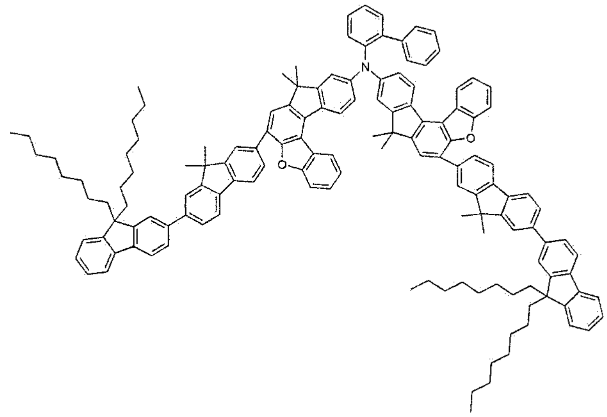 |
| 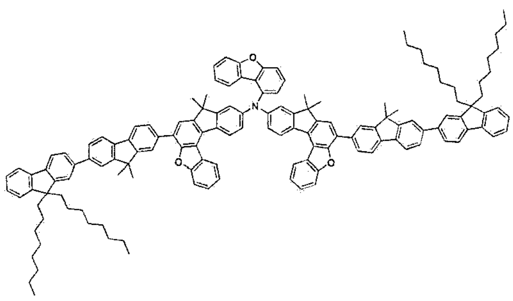 | 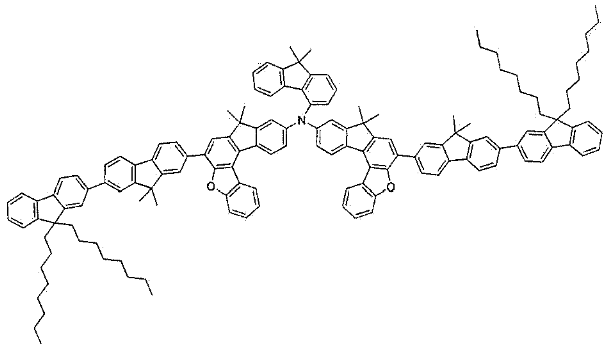 |

| | |
|---|---|
| 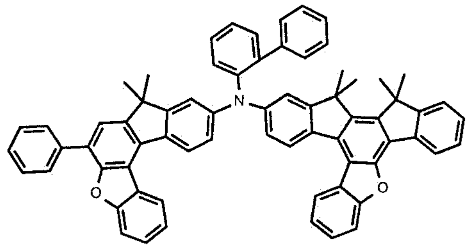 | 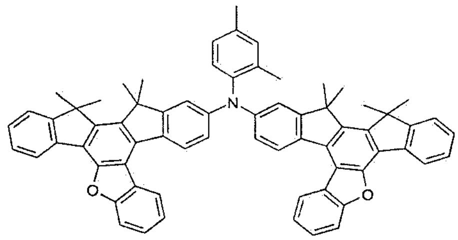 |
| 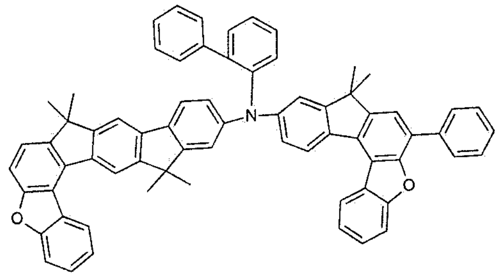 | 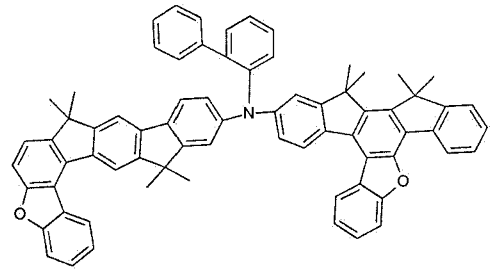 |
| 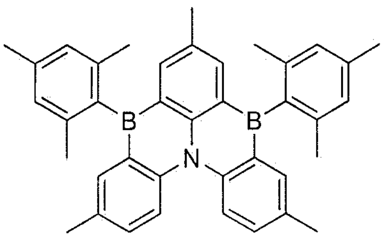 | 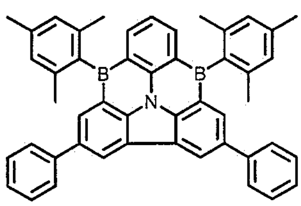 |
| 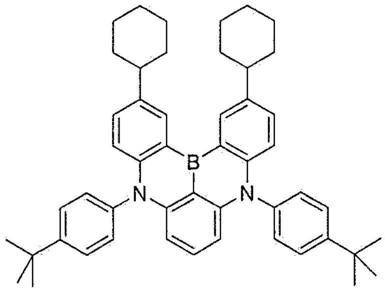 | 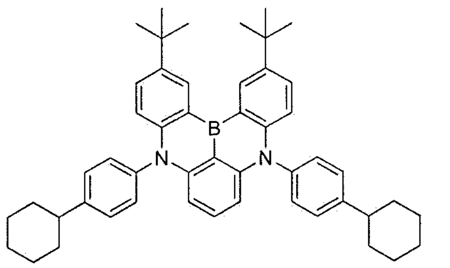 |
| 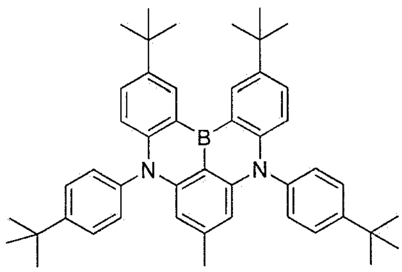 | 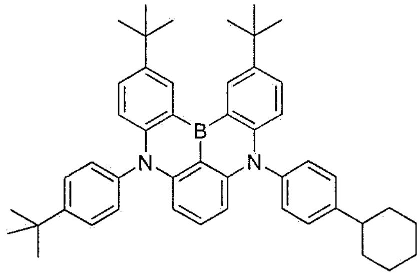 |

| | |
|---|---|
| 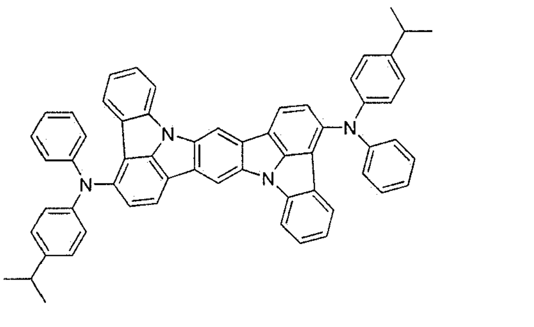 | 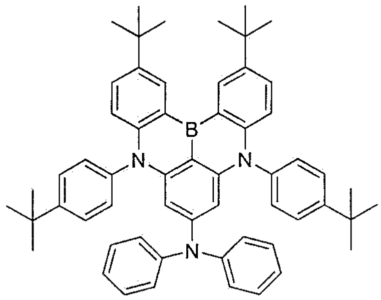 |
| 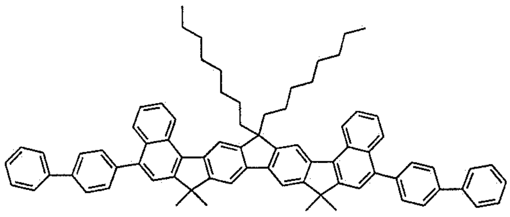 | |
| 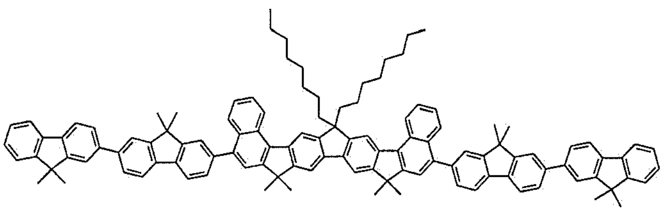 | |
| 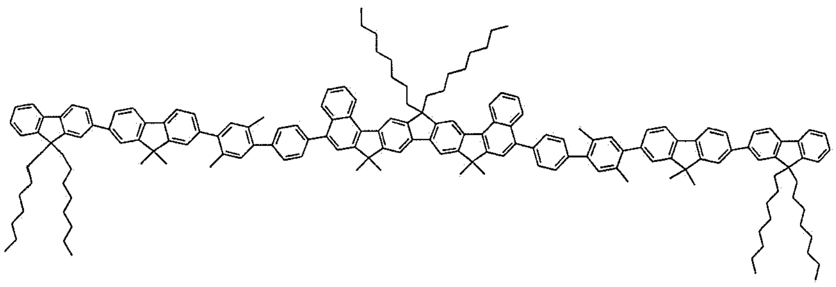 | |
| 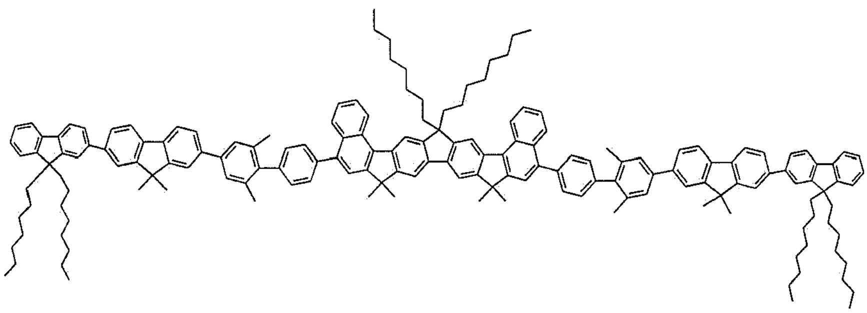 | |

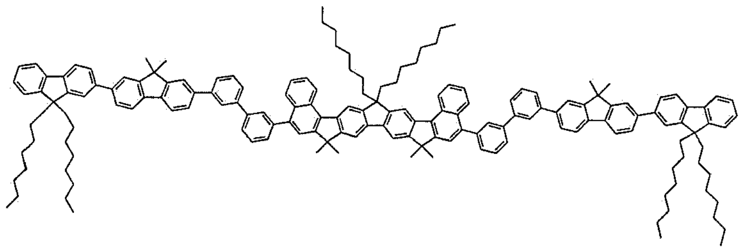

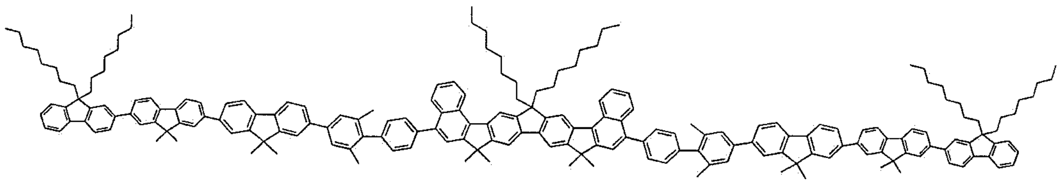

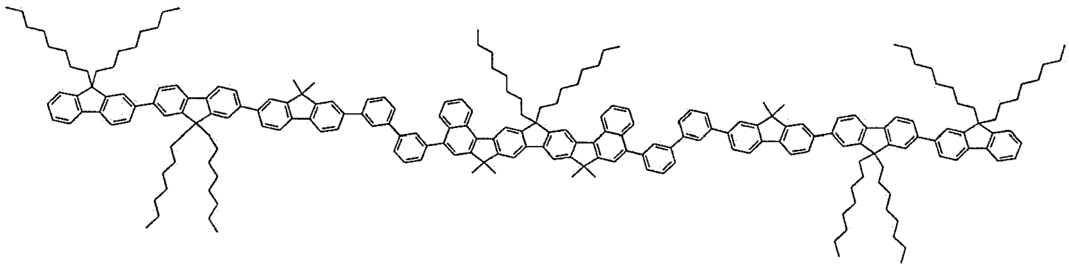

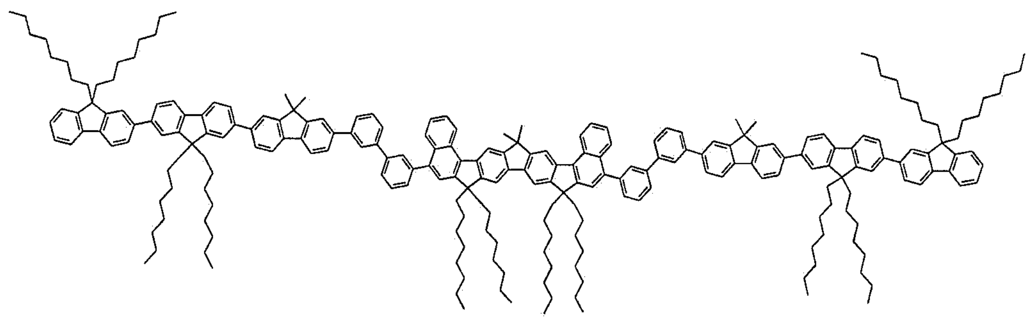

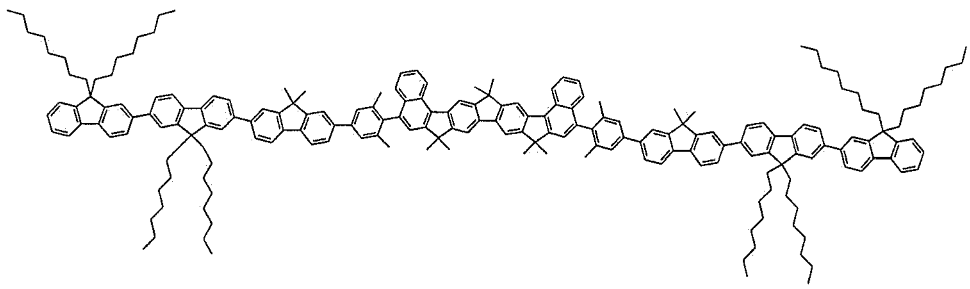

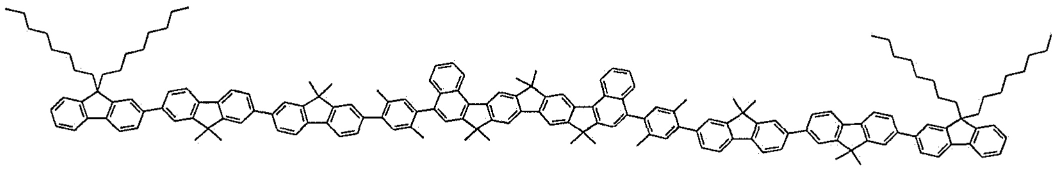

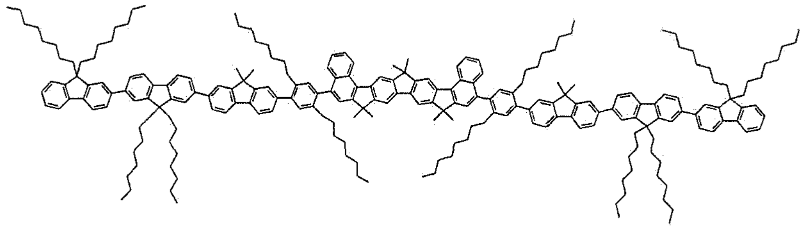

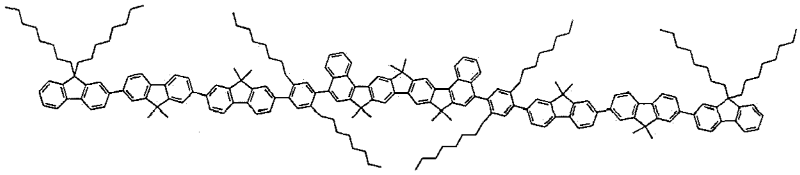

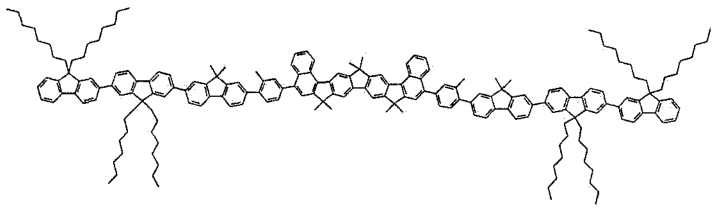

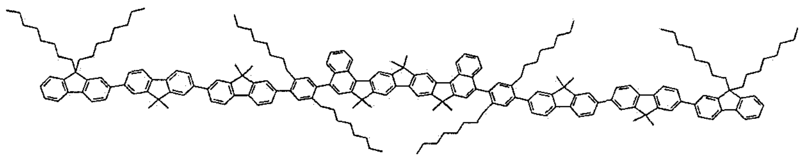

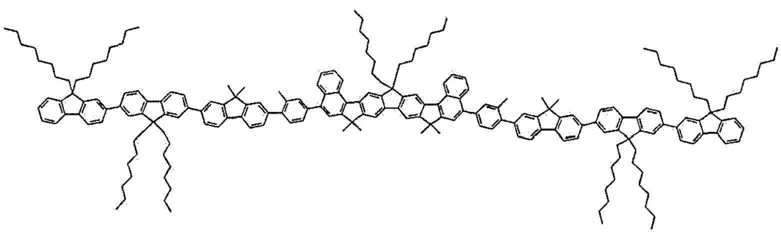

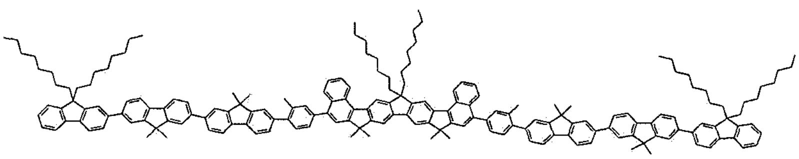

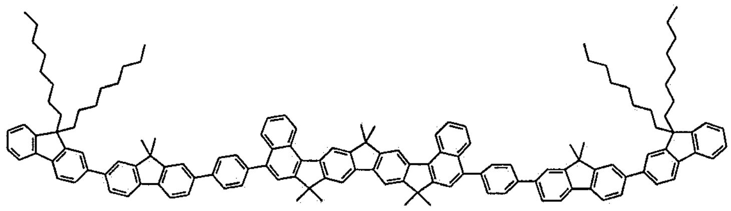

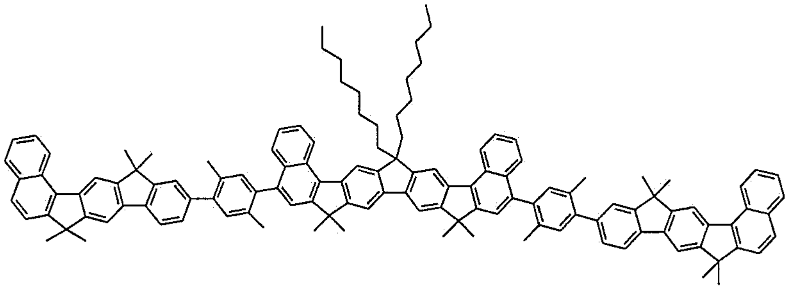

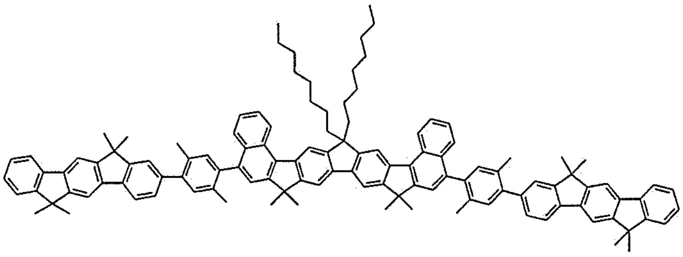

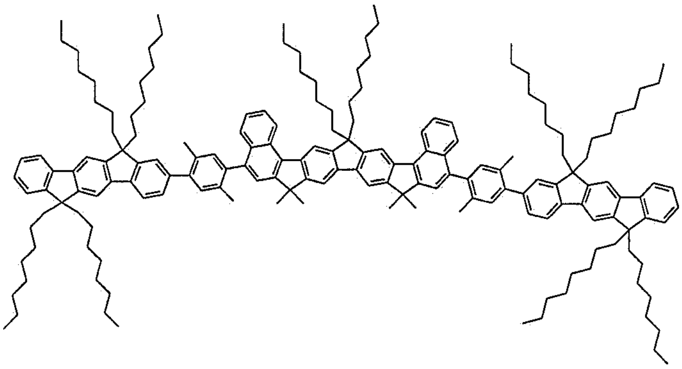

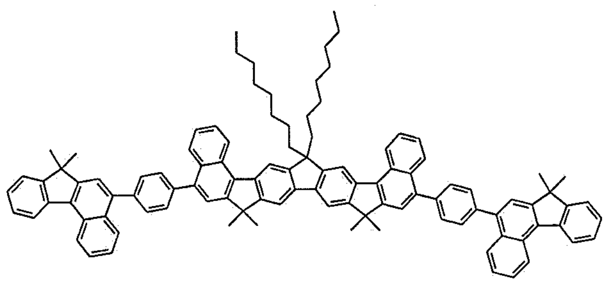

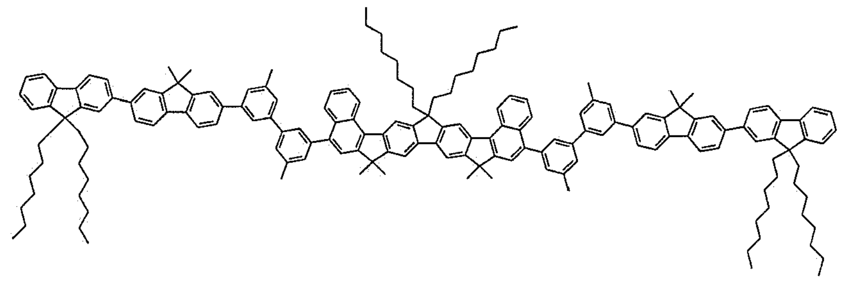

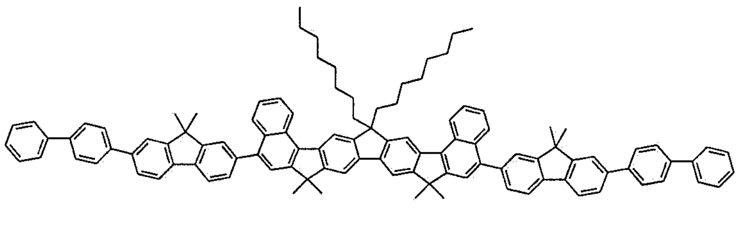

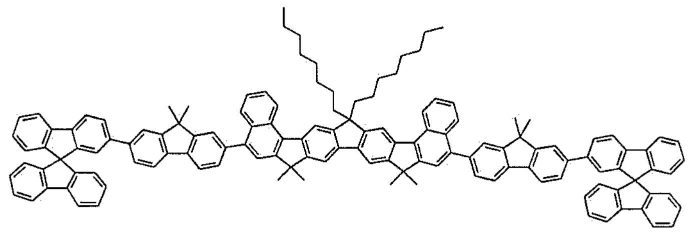

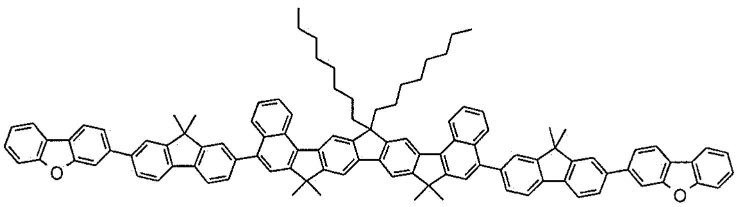

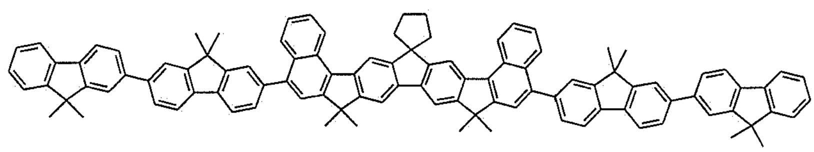

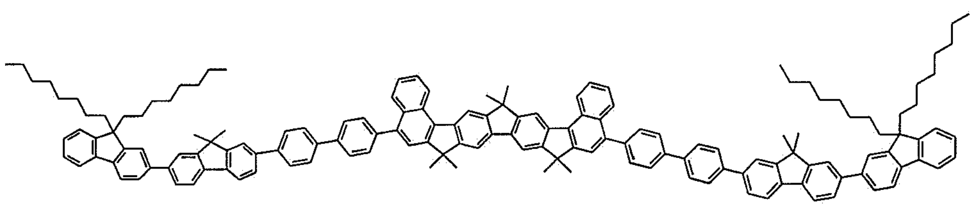

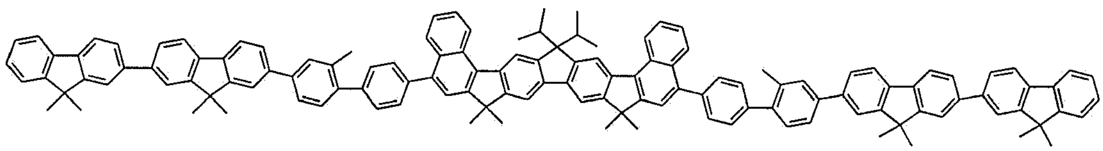

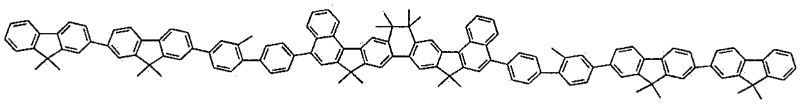

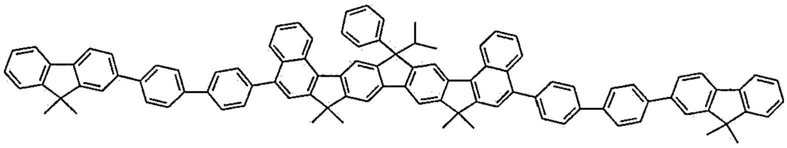

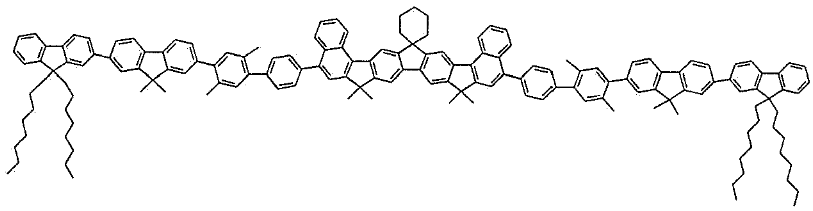

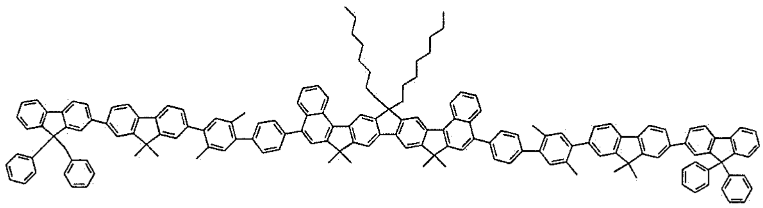

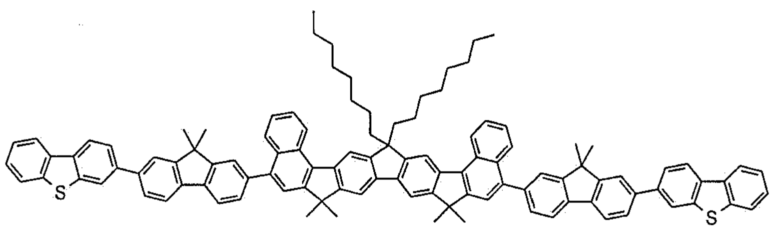

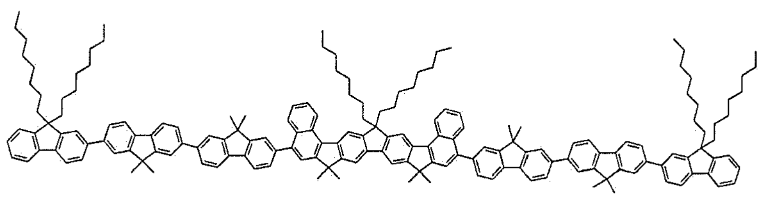

| |
|---|
| 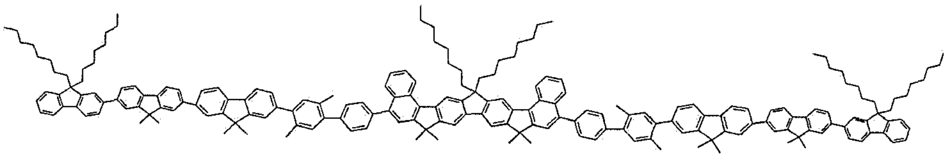 |
| 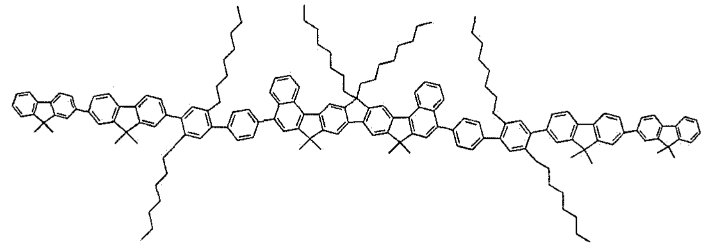 |
| 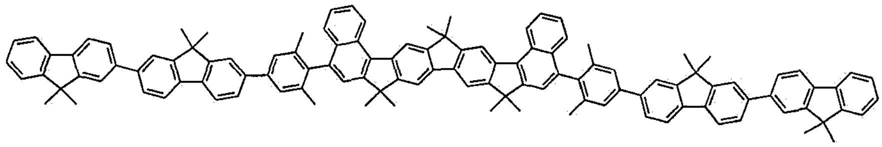 |
| 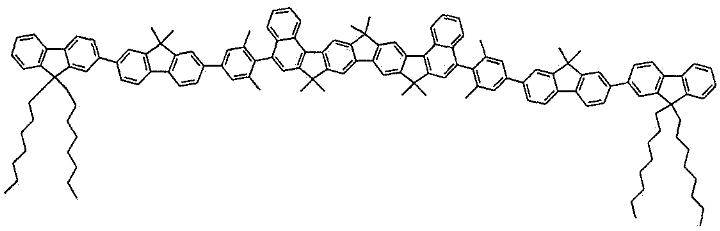 |
| 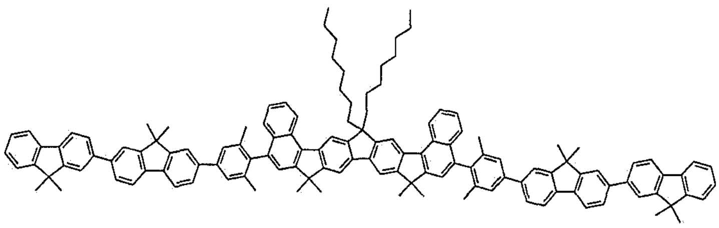 |
| 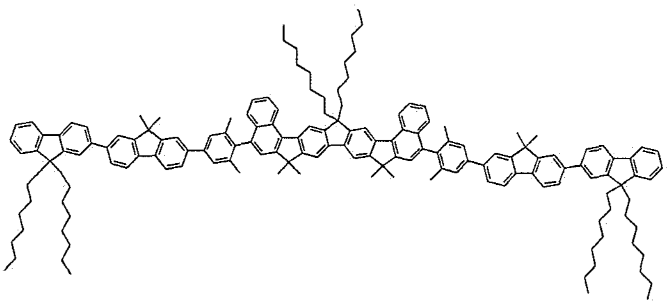 |

| |
|---|
| 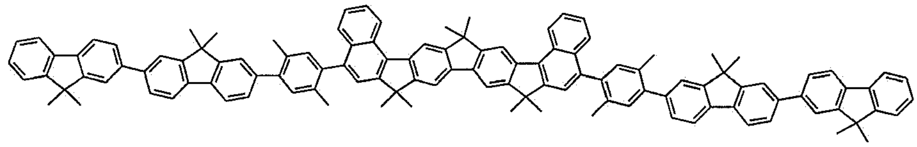 |
| 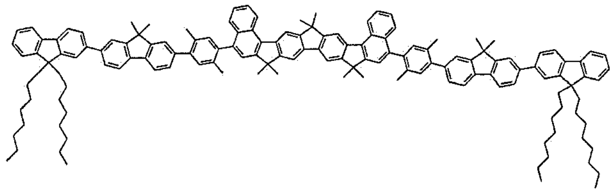 |
| 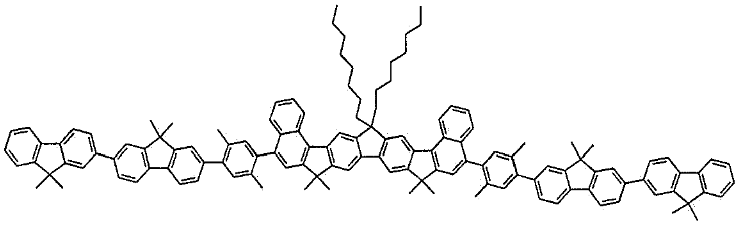 |
| 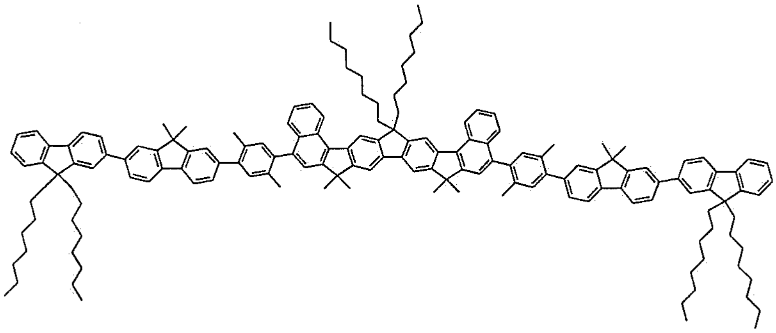 |
| 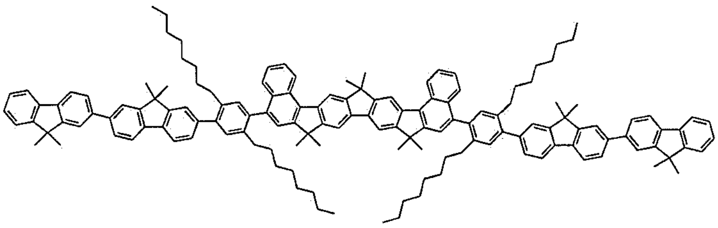 |
| 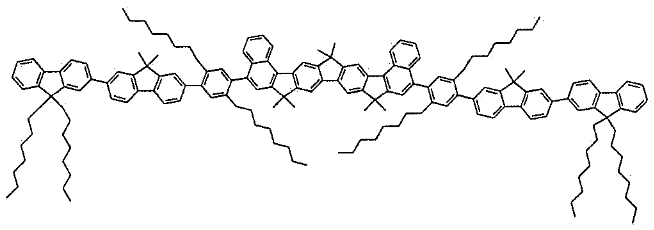 |

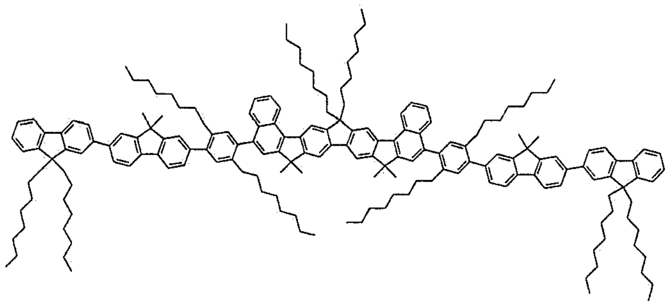

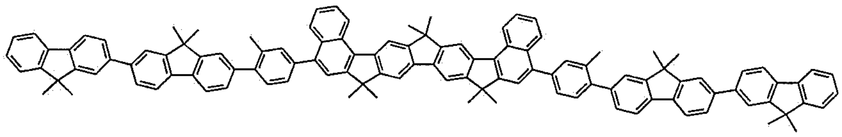

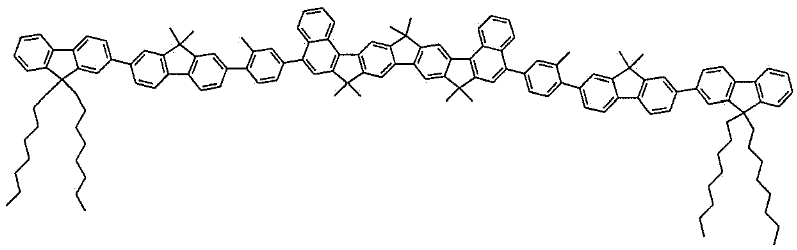

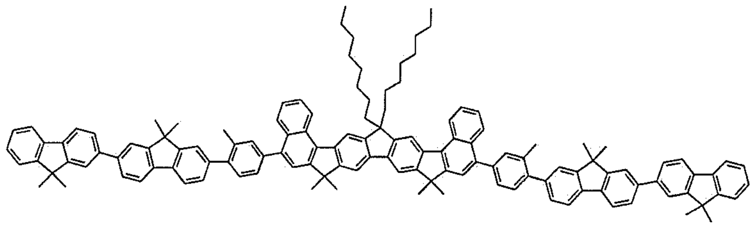

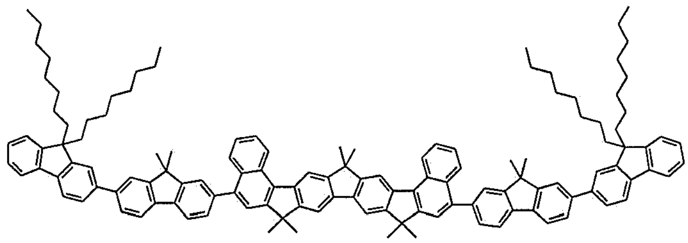

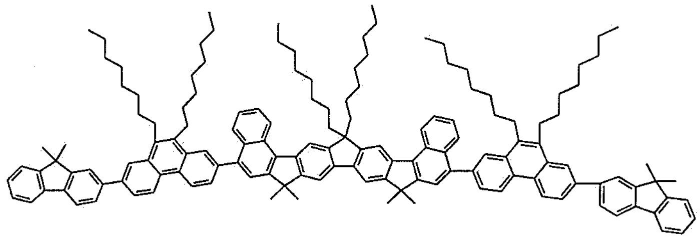

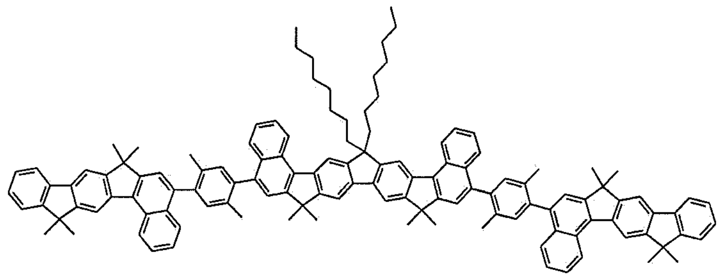

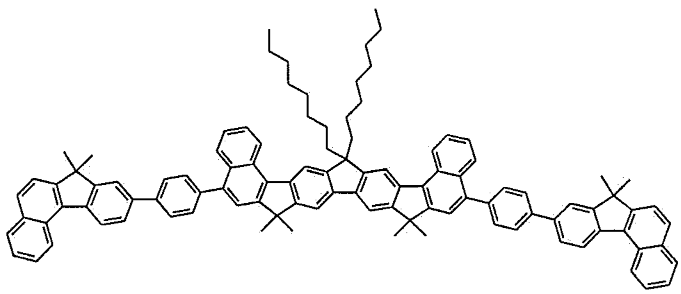

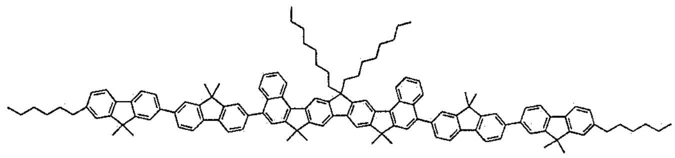

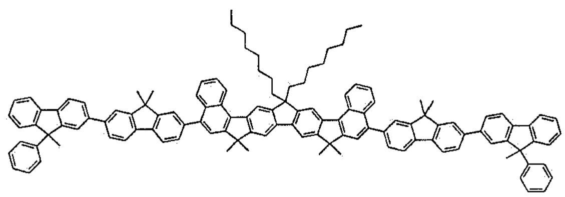

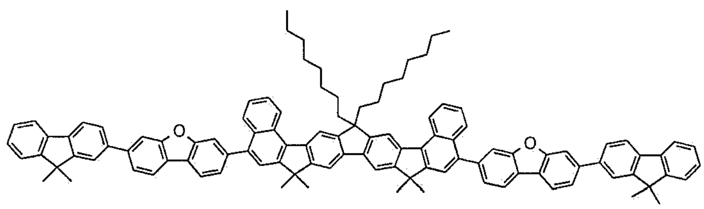

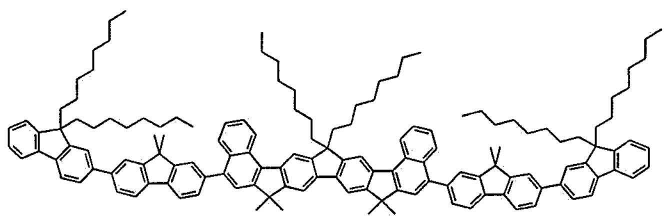

| |
|---|
| 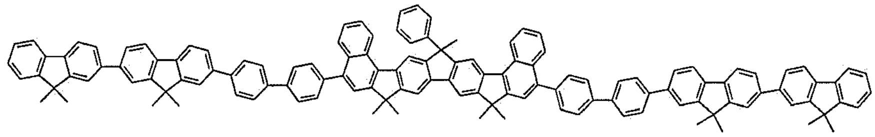 |
| 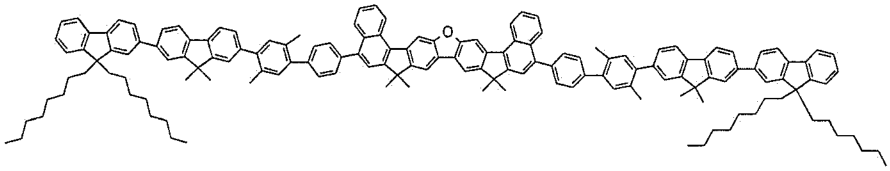 |
| 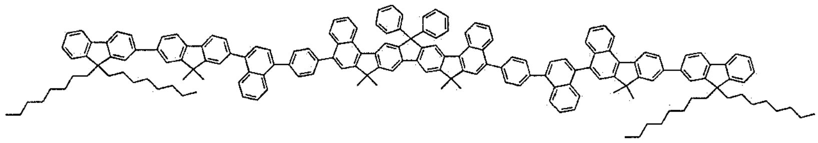 |
| 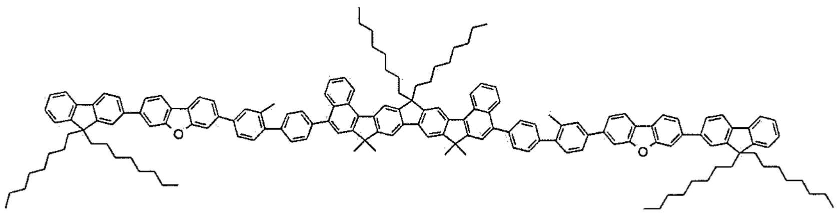 |
| 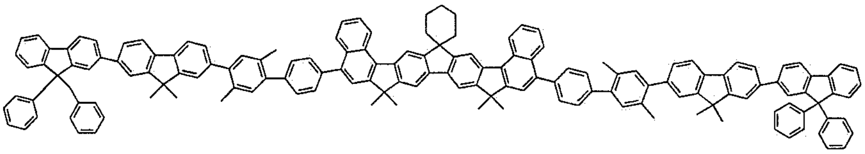 |
| 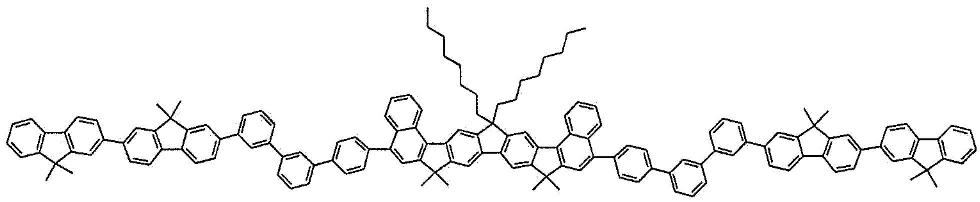 |
| 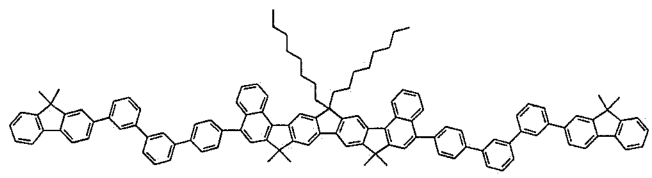 |

| |
|---|
| 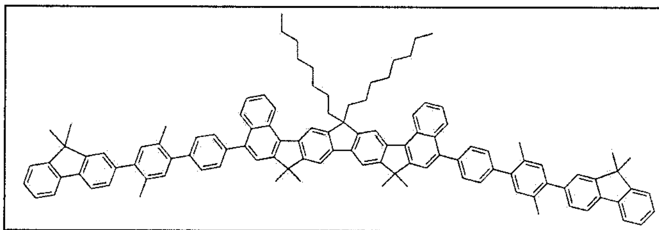 |
| 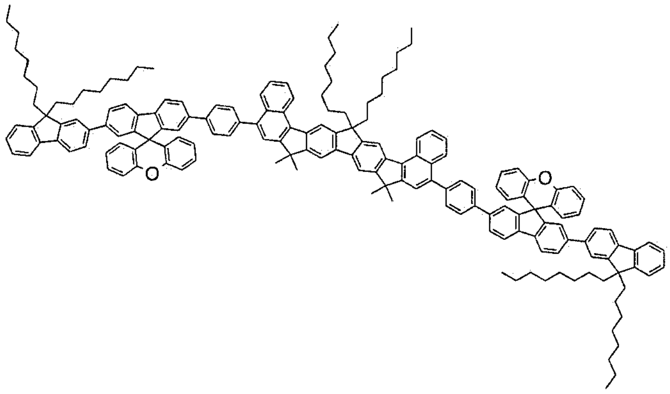 |
| 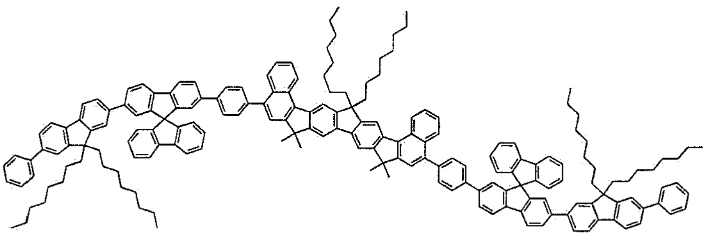 |
| 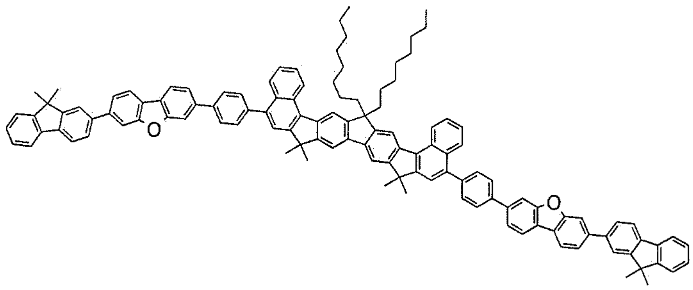 |
| 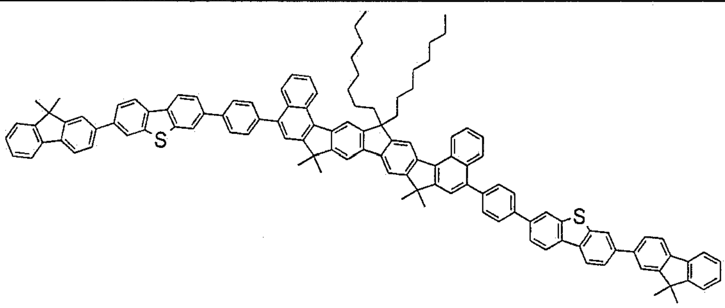 |

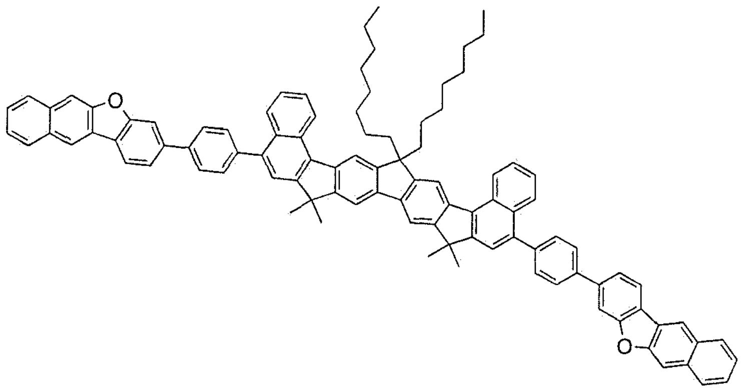

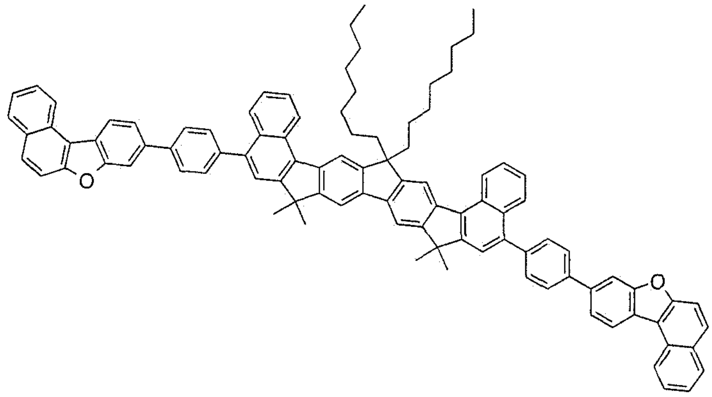

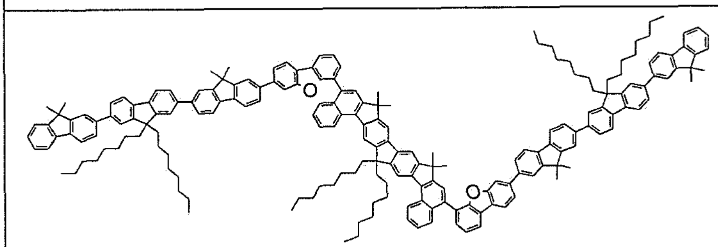

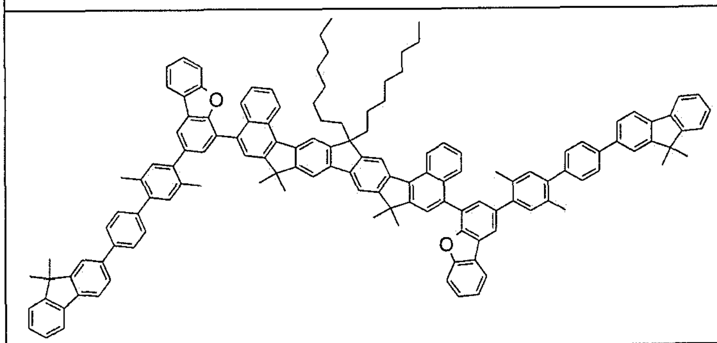

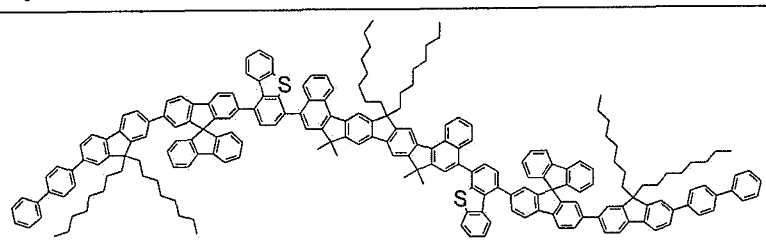

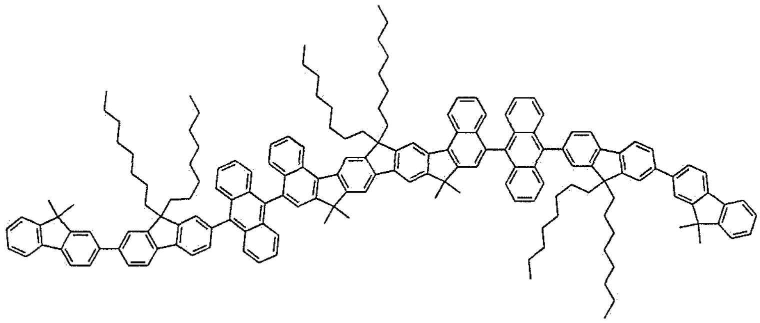

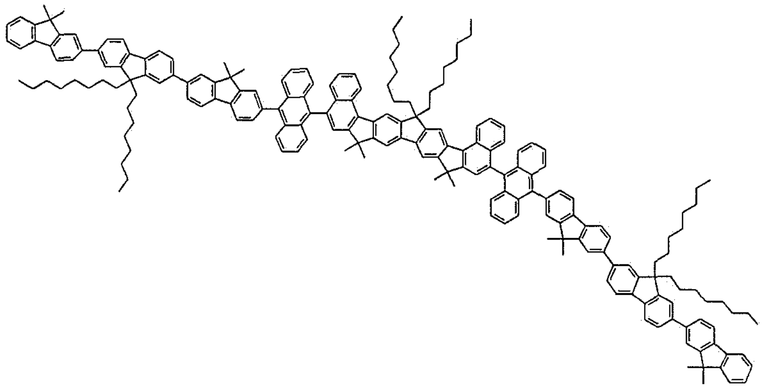

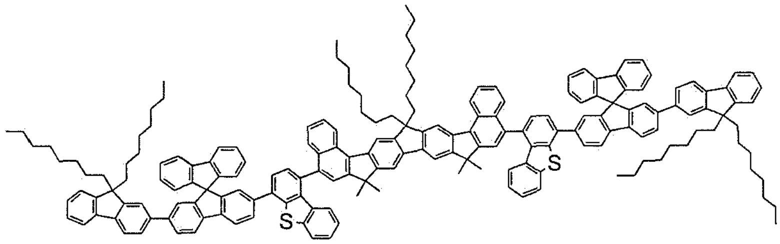

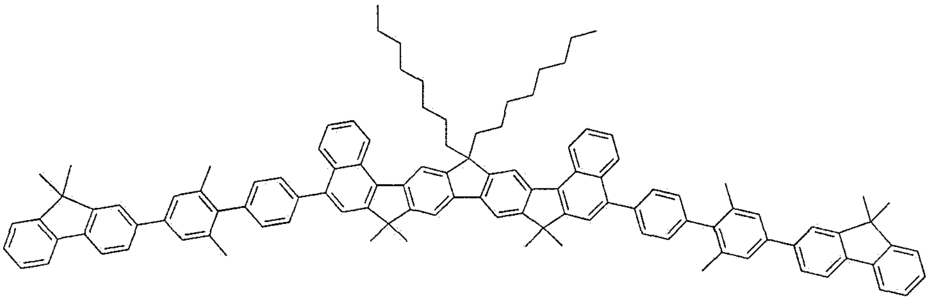

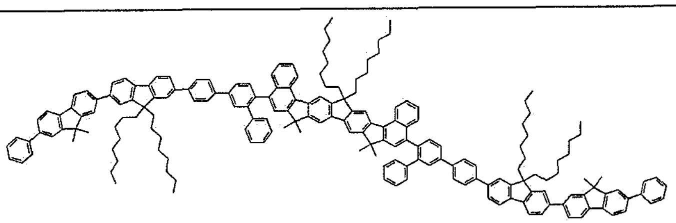

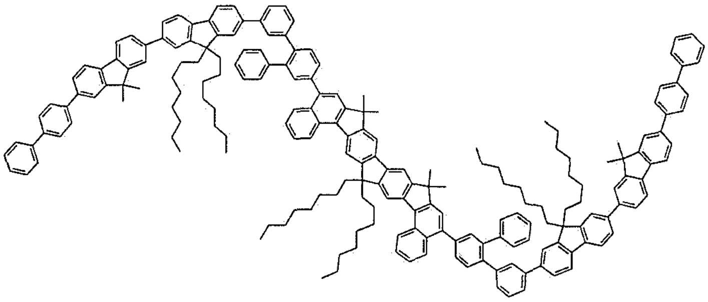

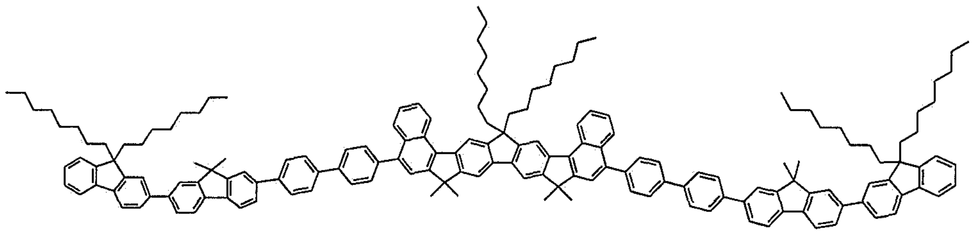

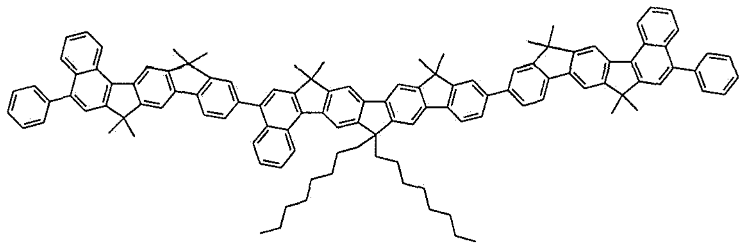

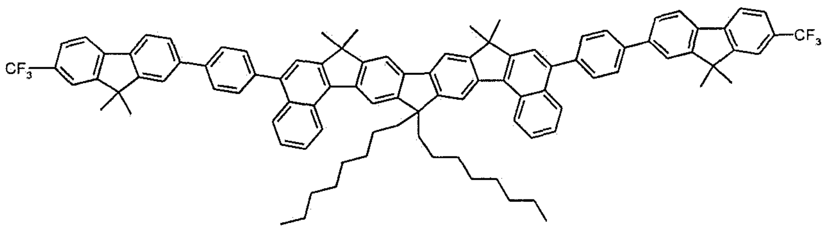

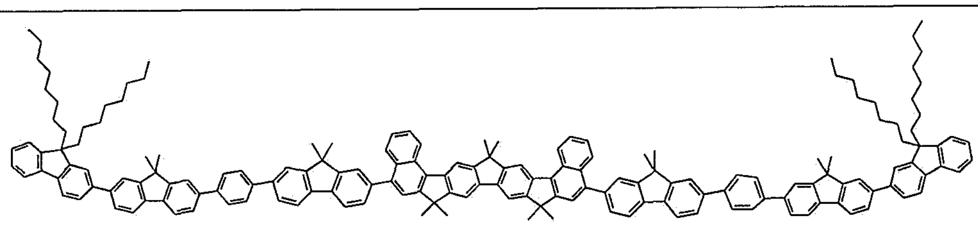

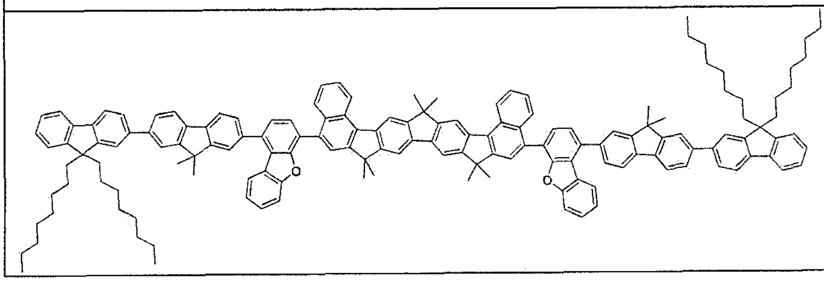

| |
|---|
| 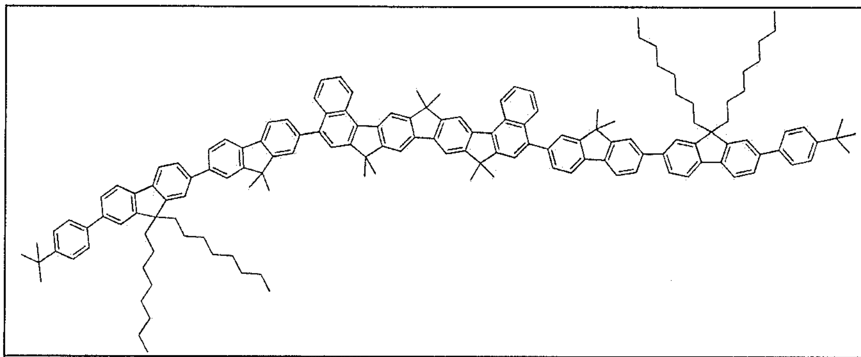 |
| 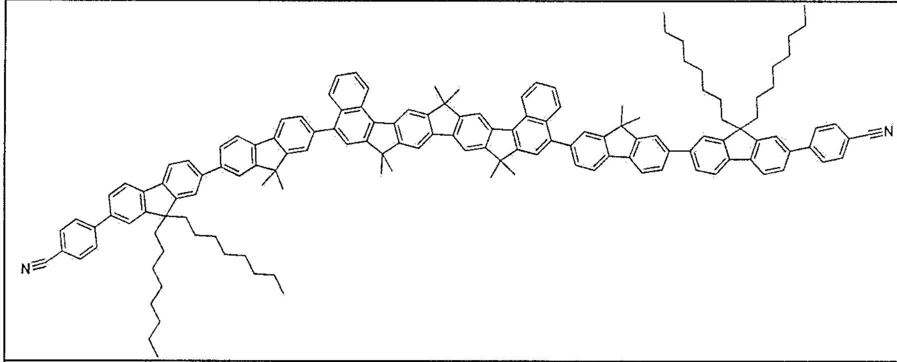 |
| 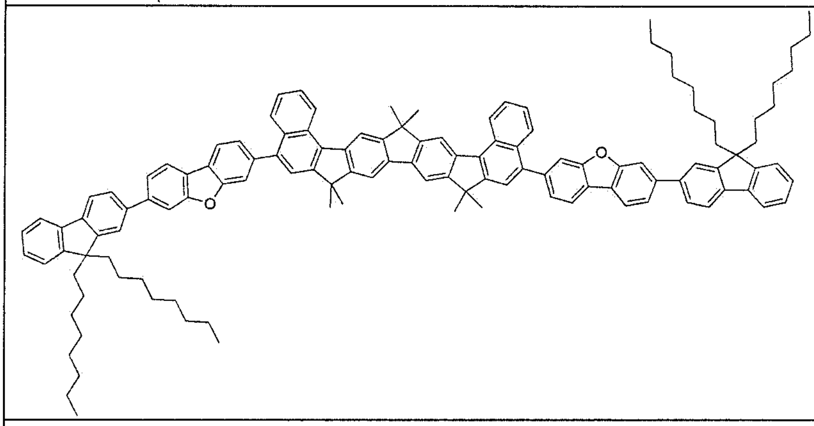 |
| 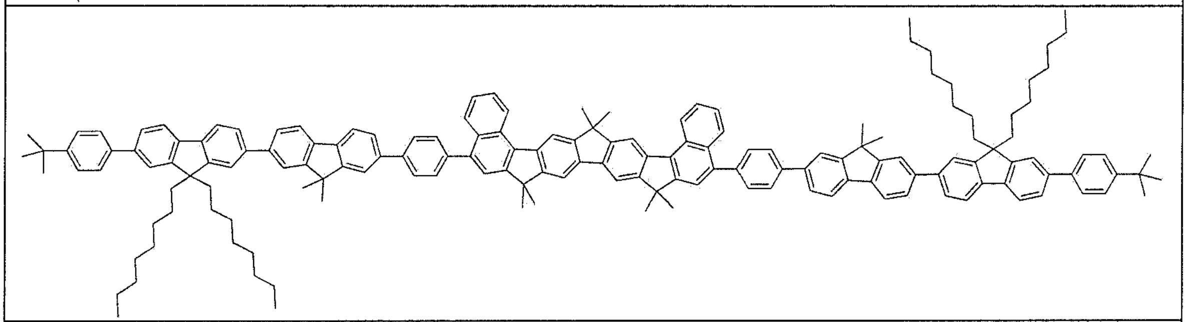 |
| 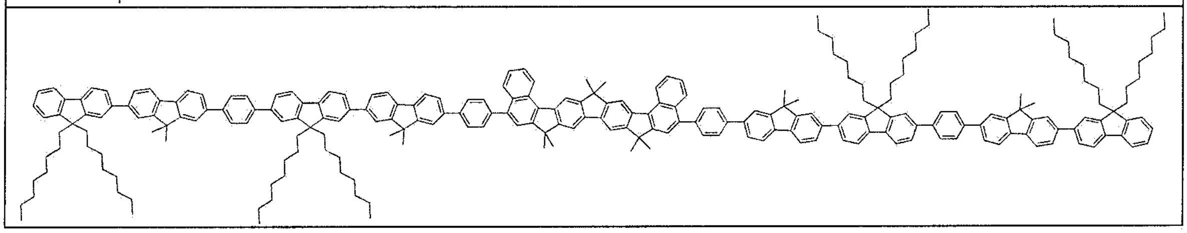 |

| |
|---|
| 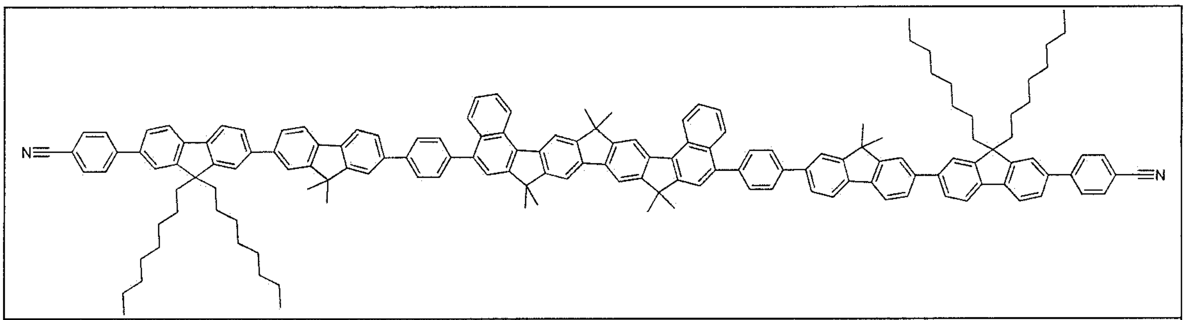 |
| 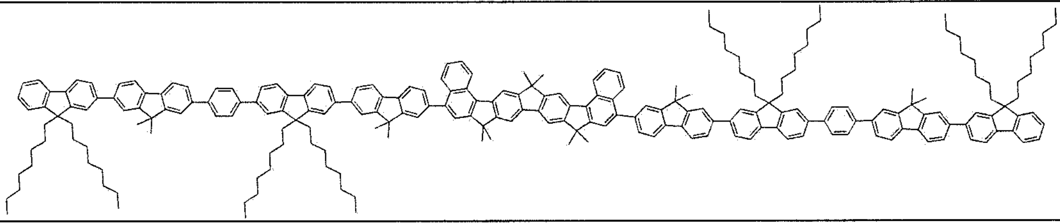 |
| 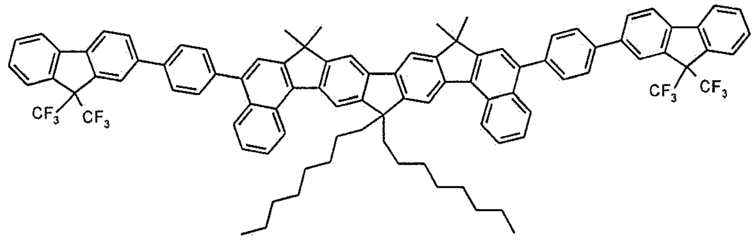 |
| 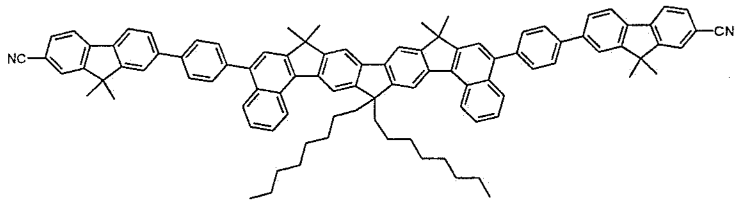 |
| 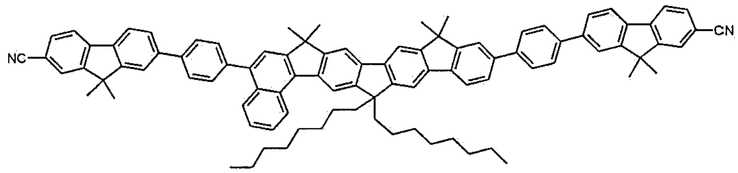 |
| 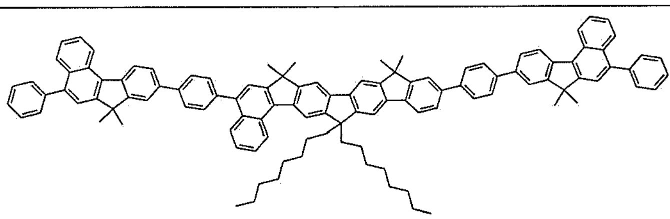 |

| | |
|---|---|
| 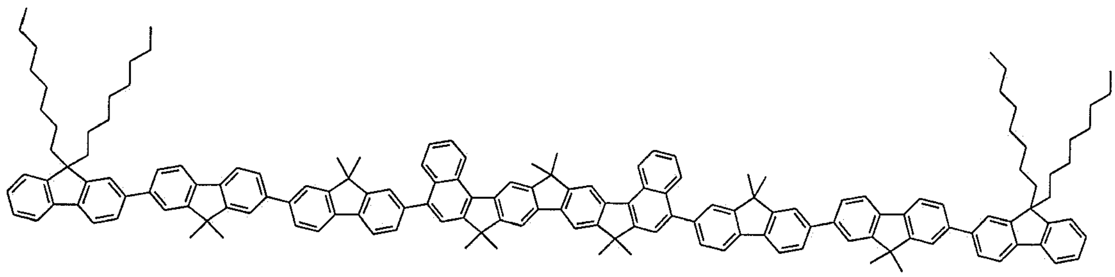 | |
| 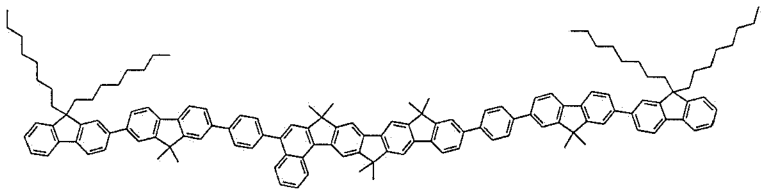 | |
| 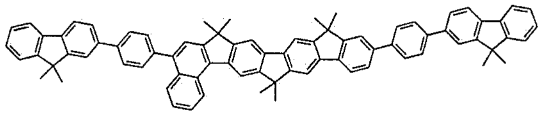 | |
| 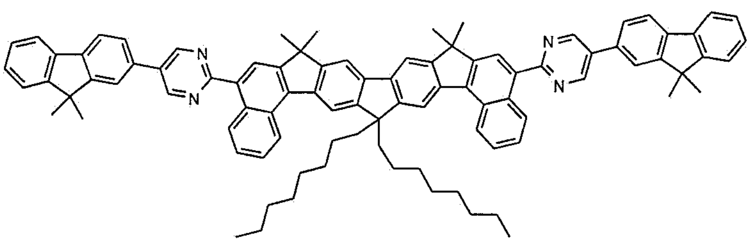 | |
| 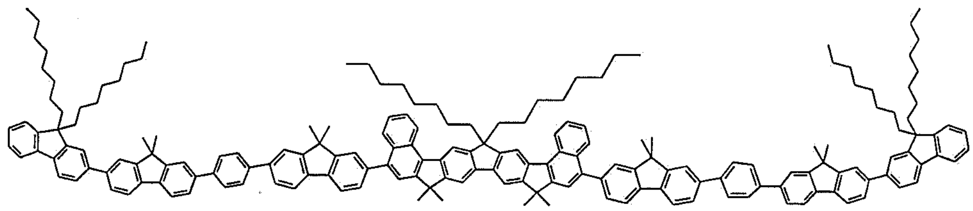 | |
| 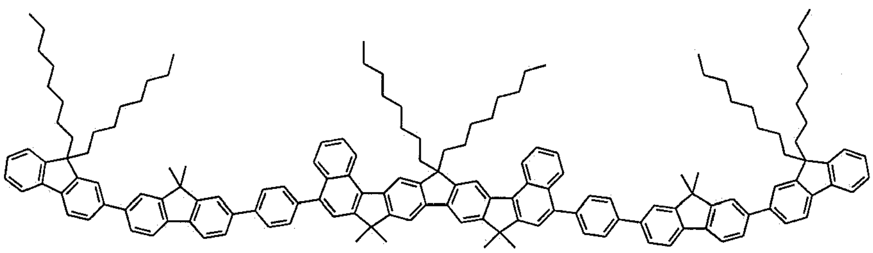 | |
| 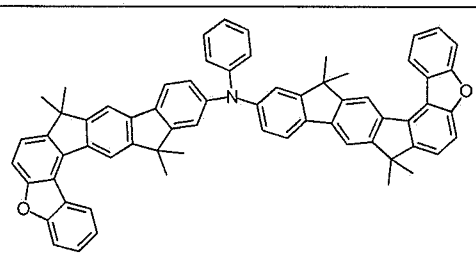 | 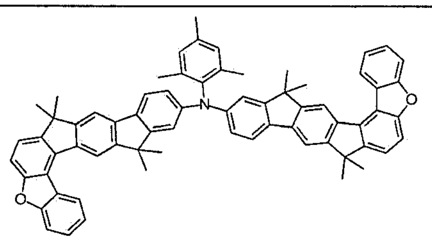 |

| | |
|---|---|
| 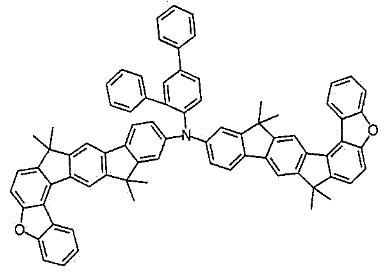 | 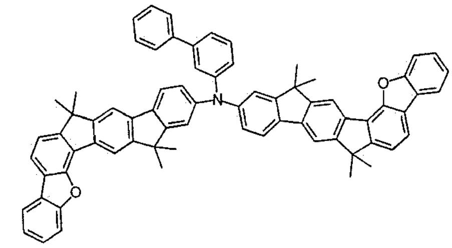 |
| 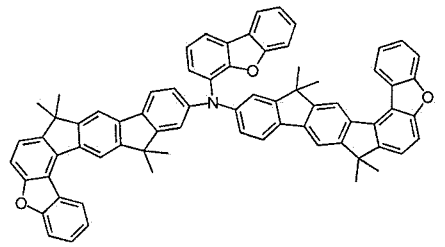 | 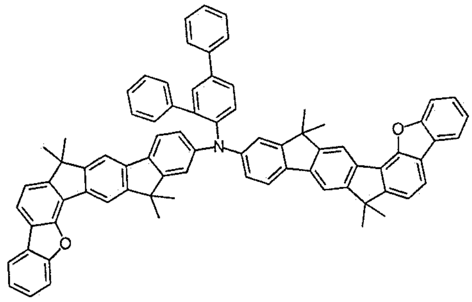 |
| 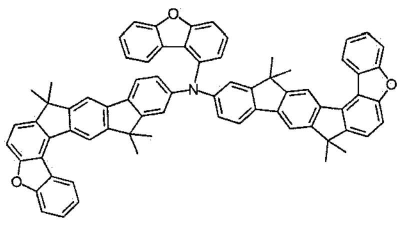 | 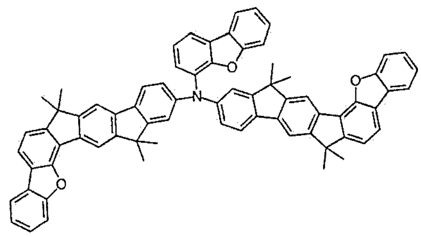 |
| 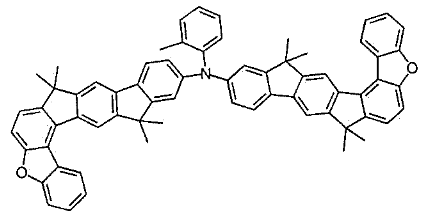 | 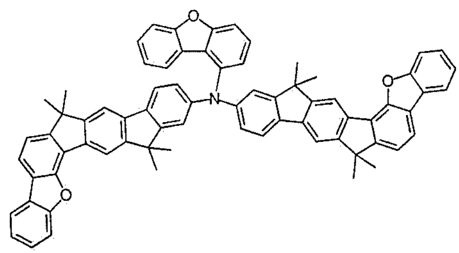 |
| 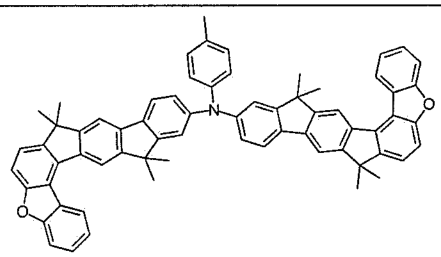 | 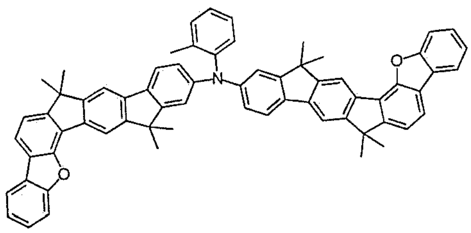 |
| 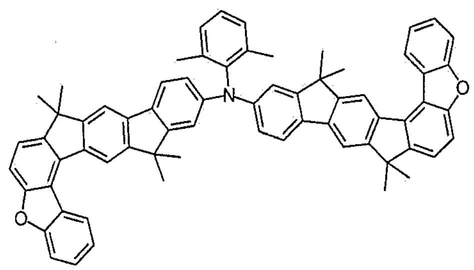 | 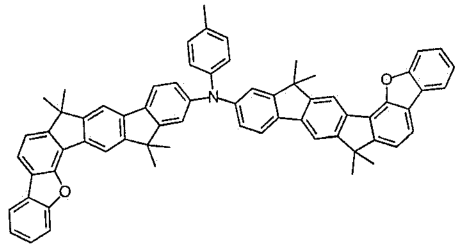 |

| | |
|---|---|
| 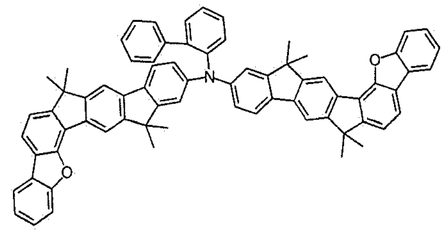 | 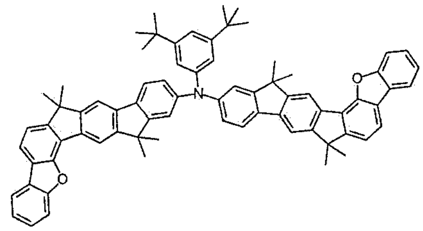 |
| 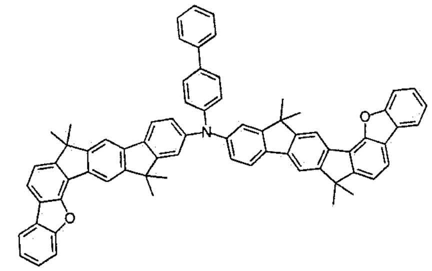 | 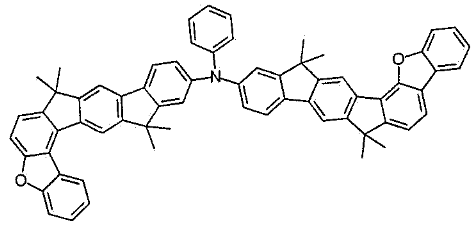 |
| 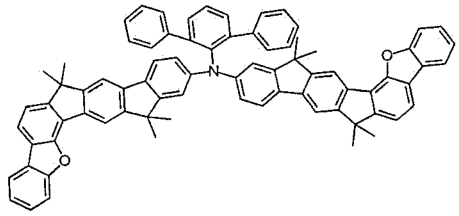 | 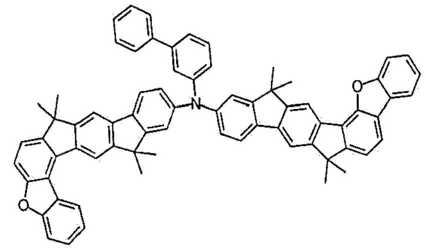 |
| 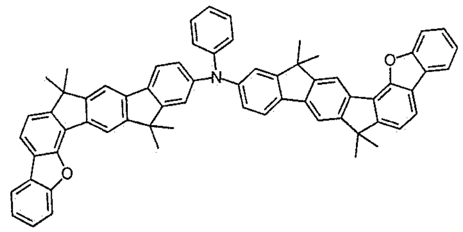 | 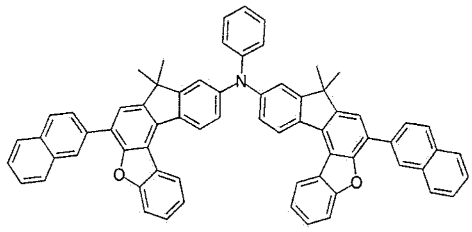 |
| 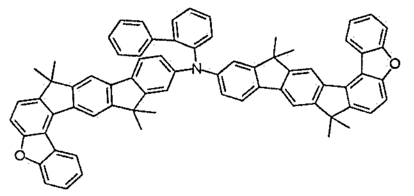 | 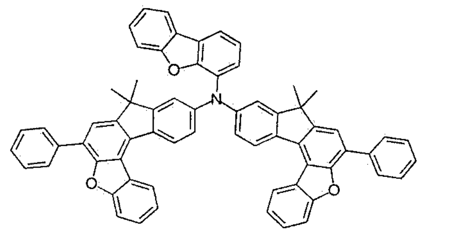 |
| 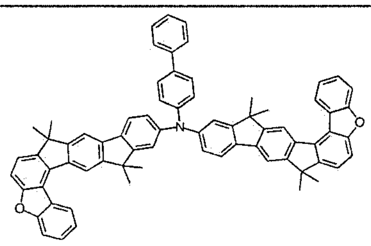 | 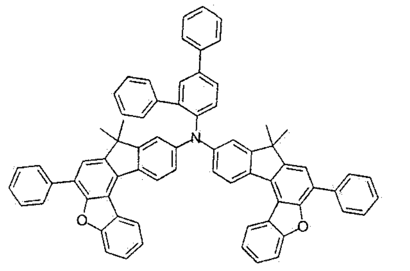 |

| | |
|---|---|
| 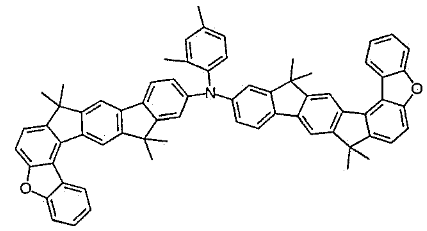 | 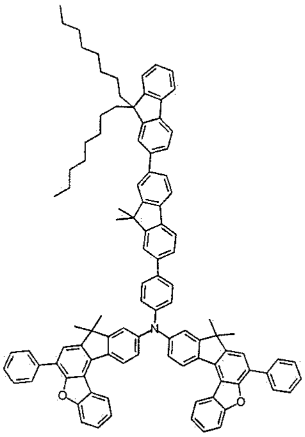 |
| 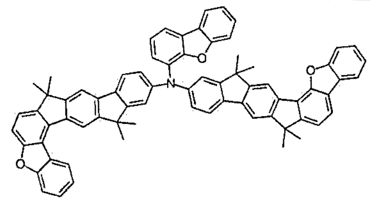 | 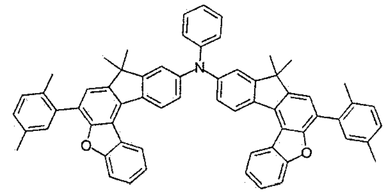 |
| 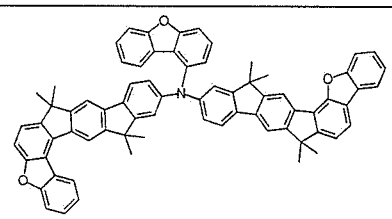 | 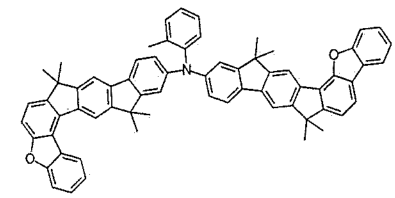 |
| 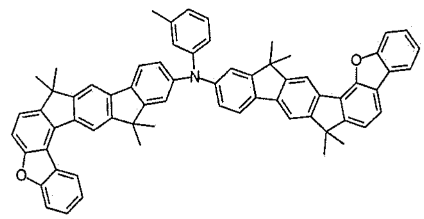 | 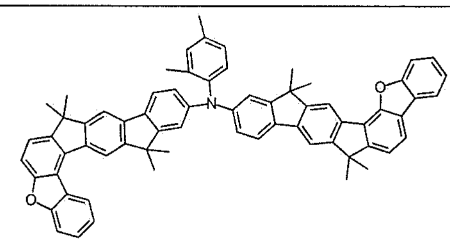 |
| 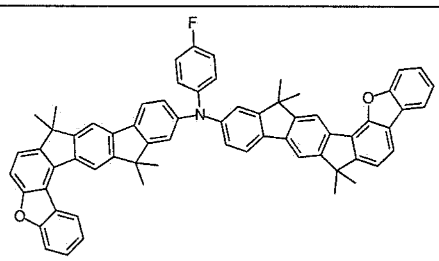 | 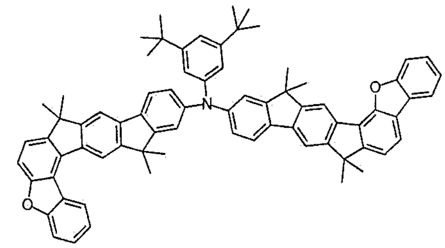 |
| 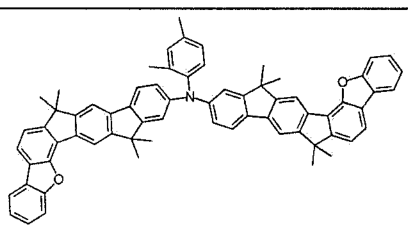 | 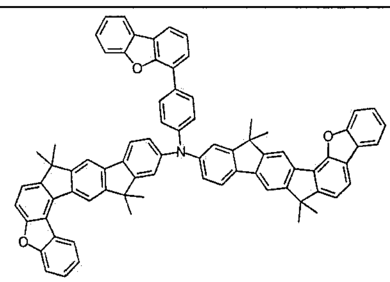 |

| | |
|---|---|
| 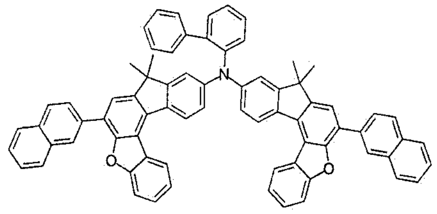 | 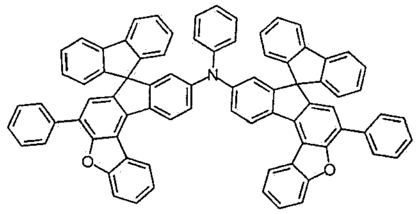 |
| 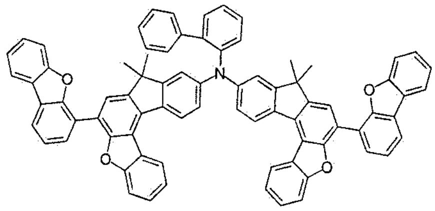 | 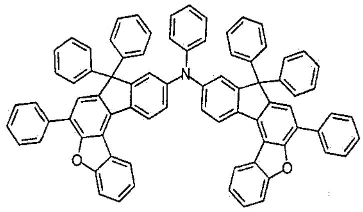 |
| 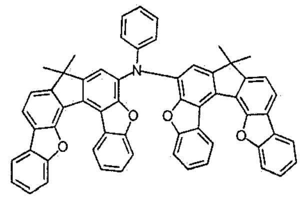 | 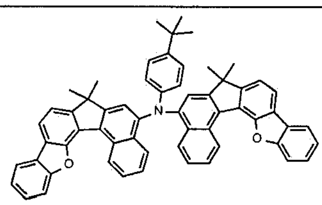 |
| 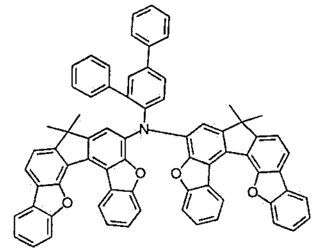 | 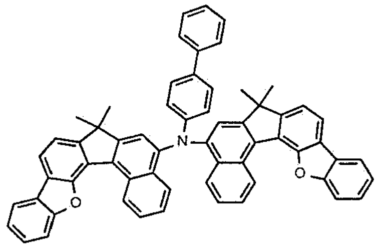 |
| 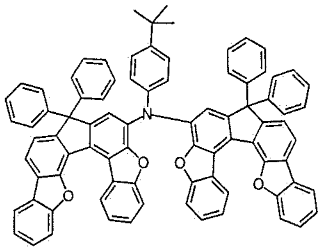 | 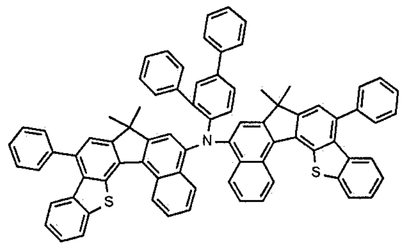 |
| 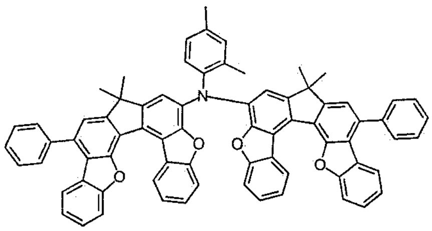 | 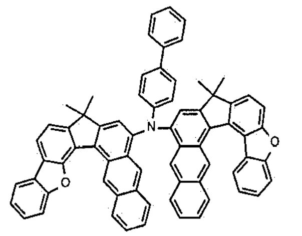 |
| 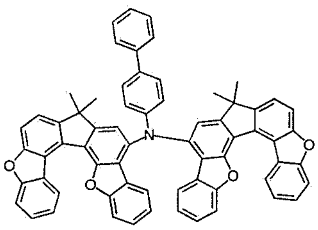 | 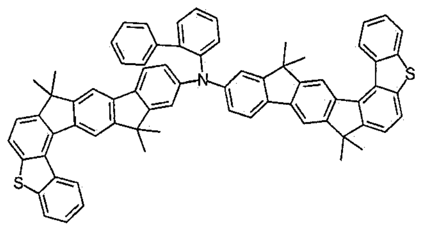 |

| | |
|---|---|
| 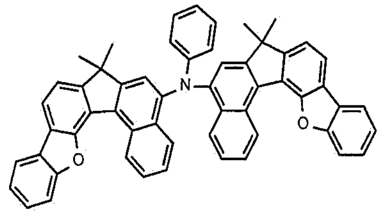 | 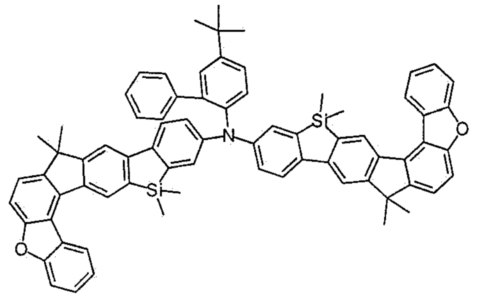 |
| 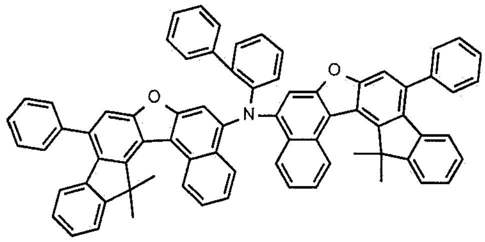 | 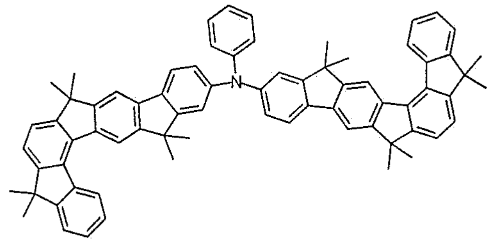 |
| 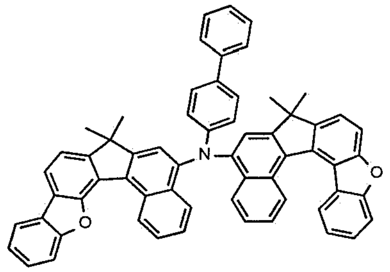 | 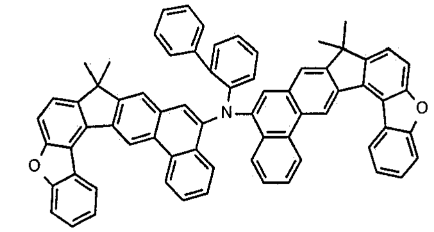 |
| 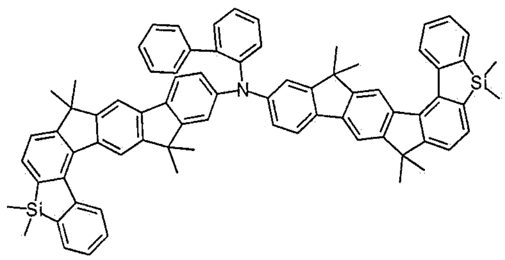 | 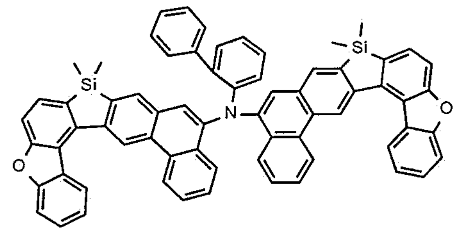 |
| 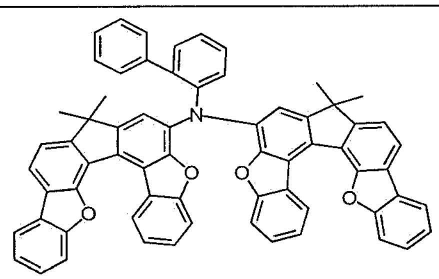 | 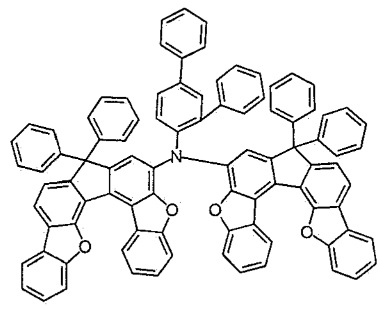 |

| | |
|---|---|
| 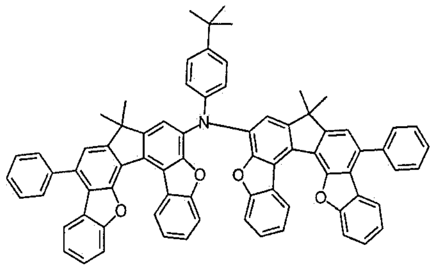 | 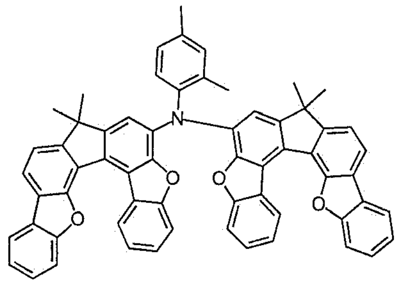 |
| 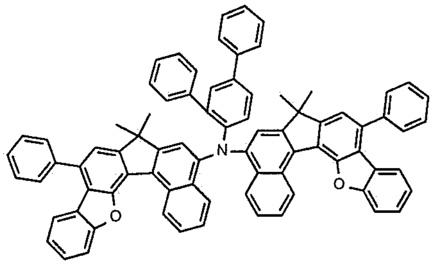 | 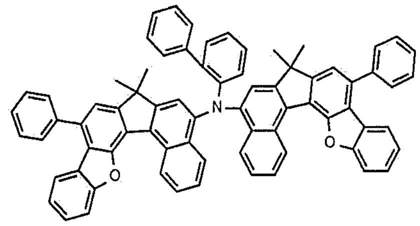 |
| 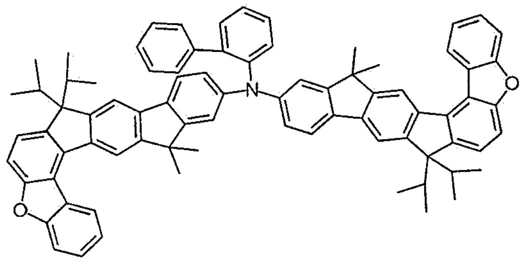 | 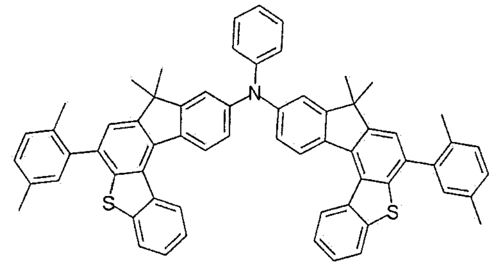 |
| 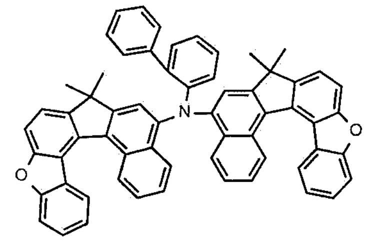 | 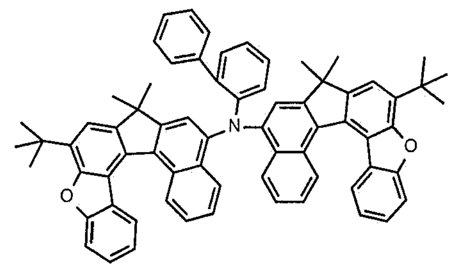 |
| 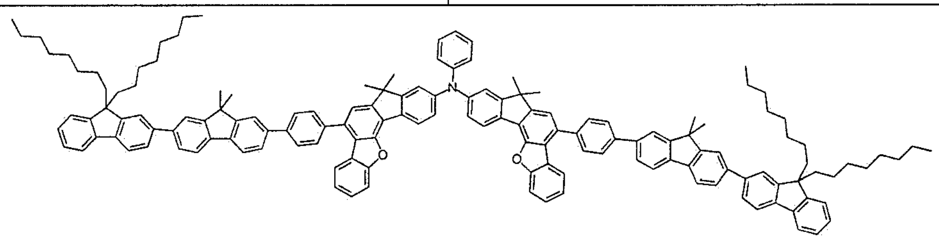 | |
| 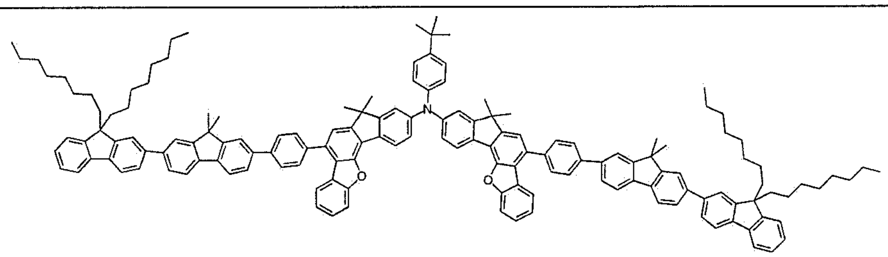 | |

| |
|---|
| 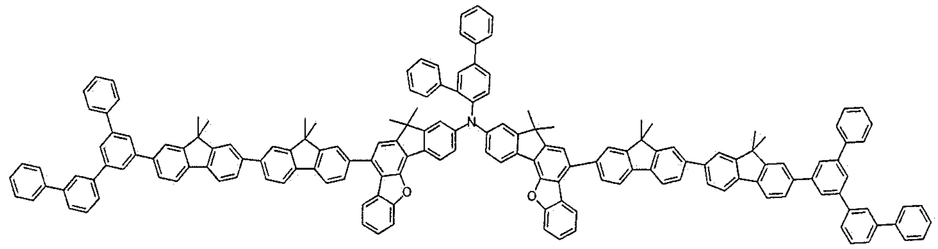 |
| 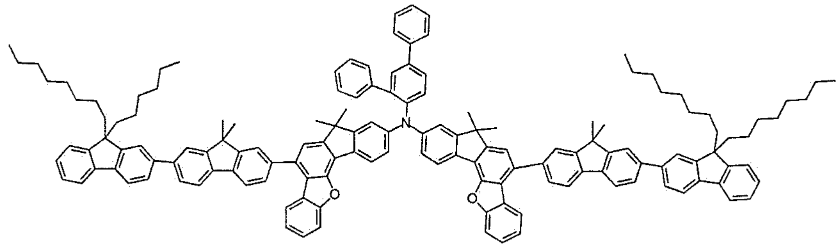 |
| 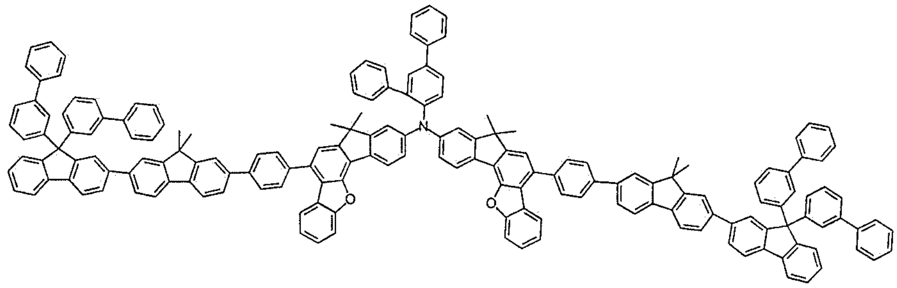 |
| 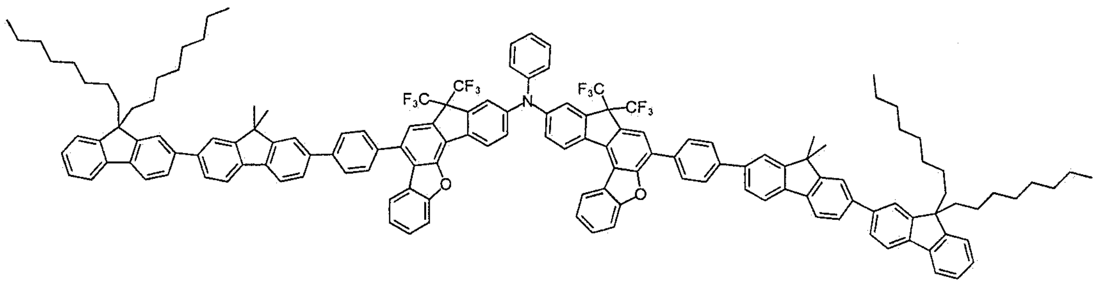 |
| 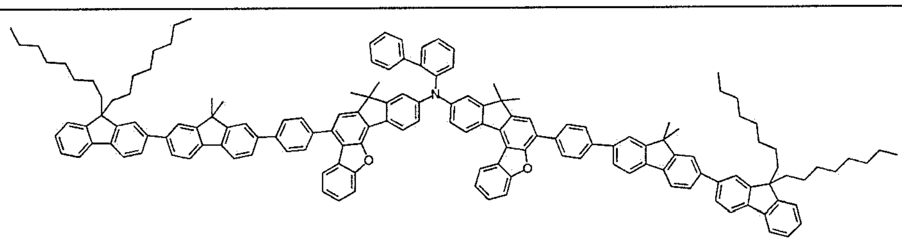 |
| 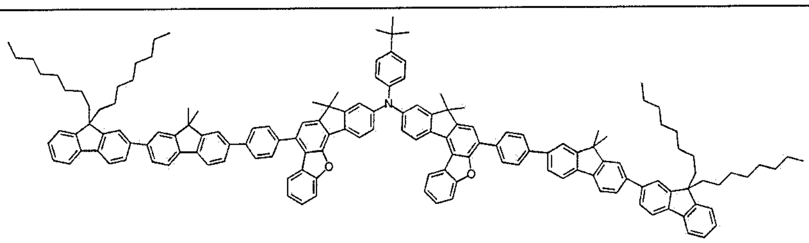 |

| |
|---|
| 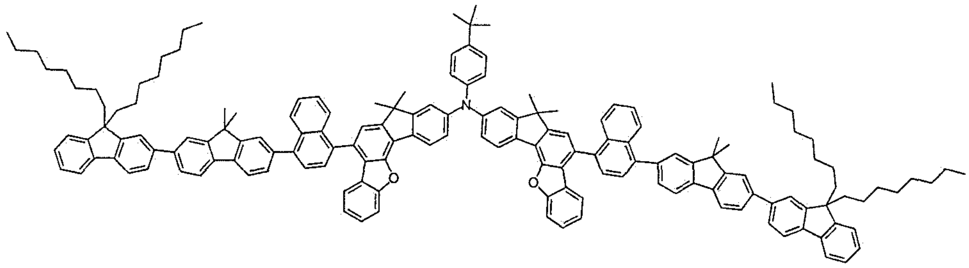 |
| 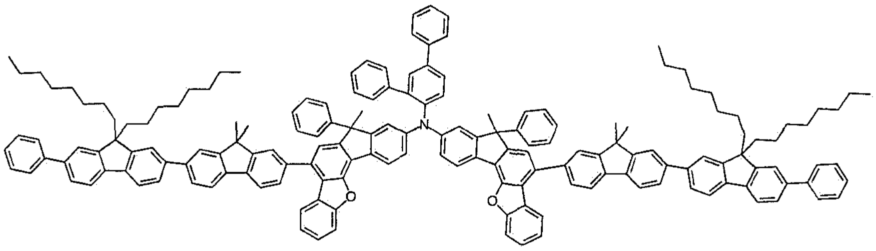 |
| 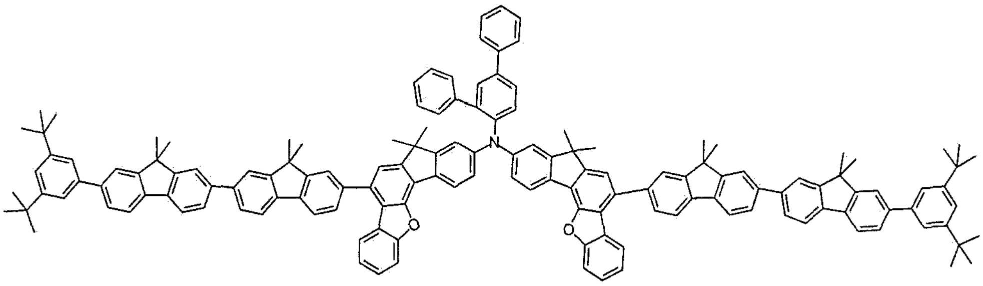 |
| 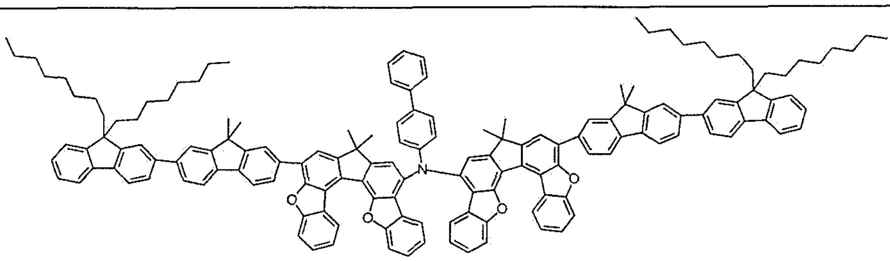 |
| 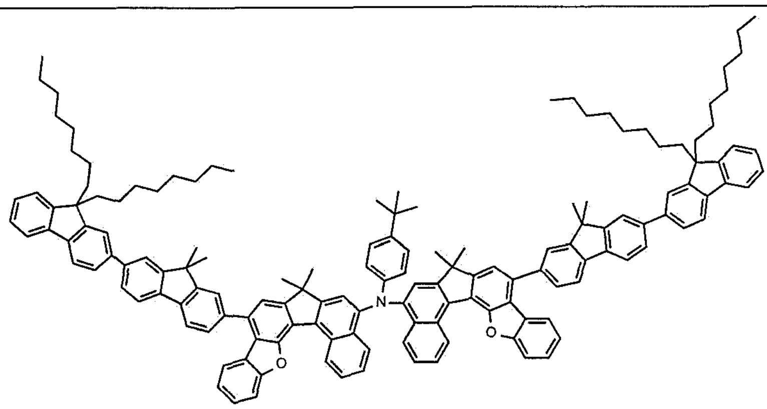 |

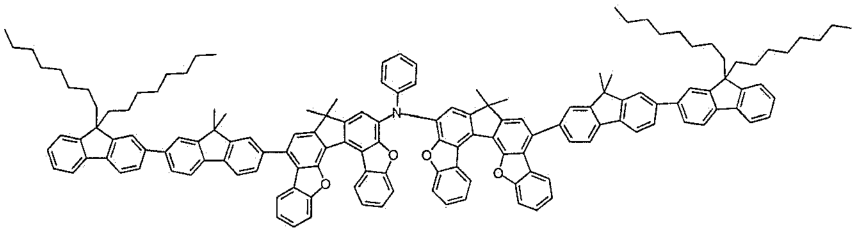

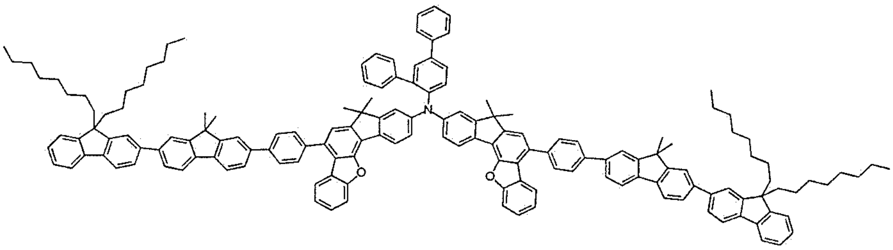

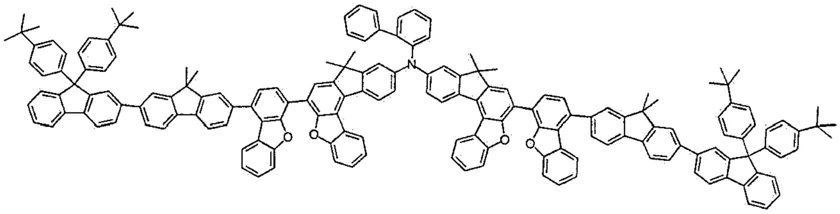

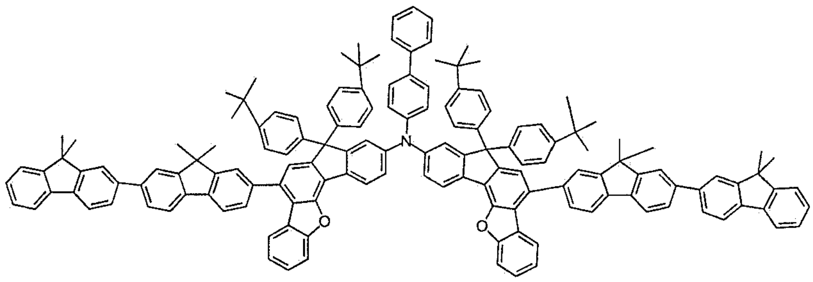

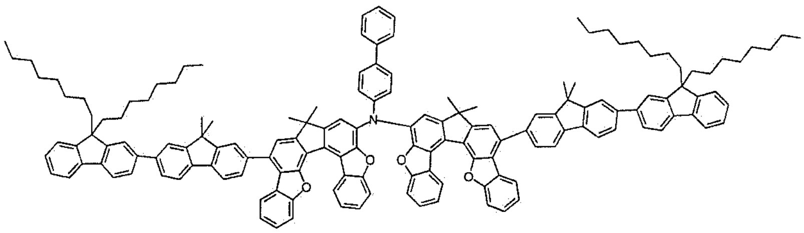

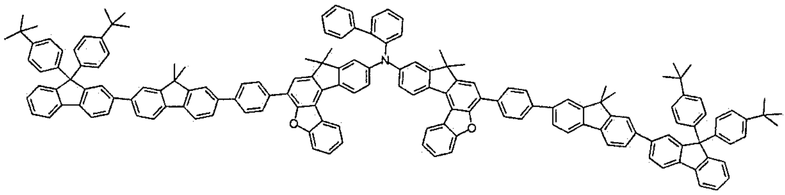

| |
|---|
| 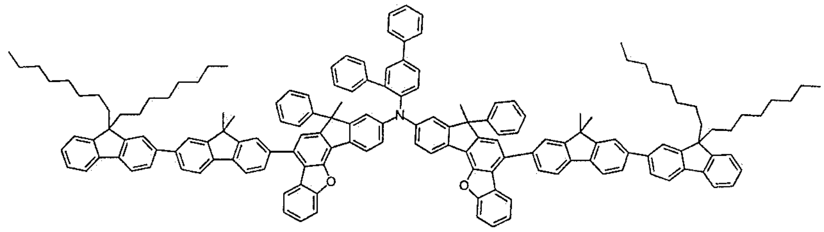 |
| 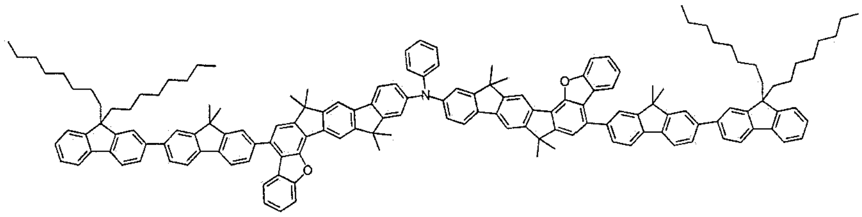 |
| 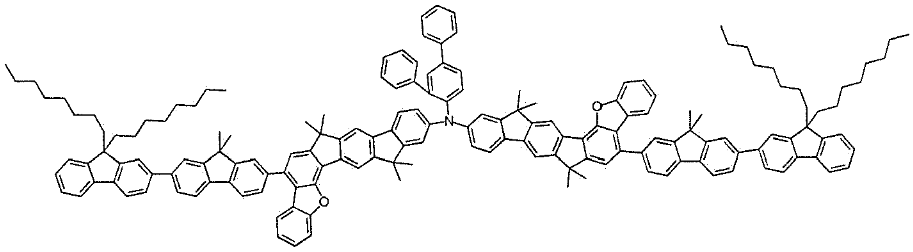 |
| 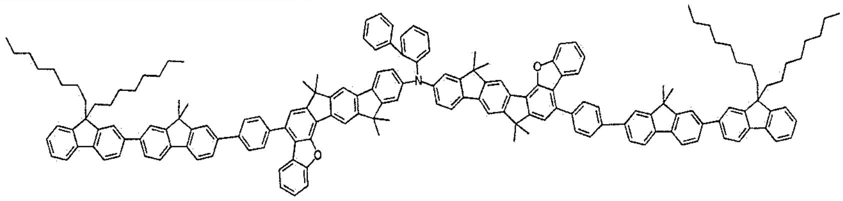 |
| 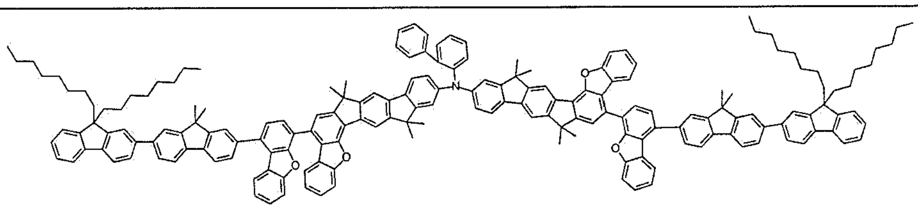 |
| 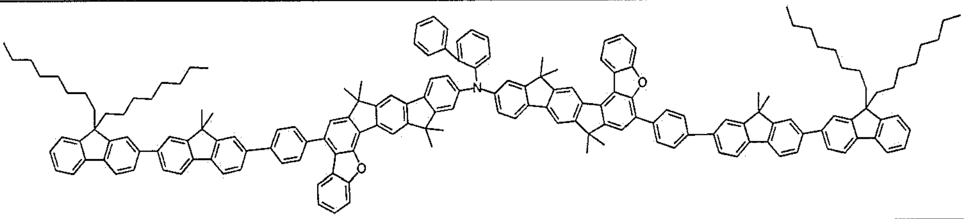 |
| 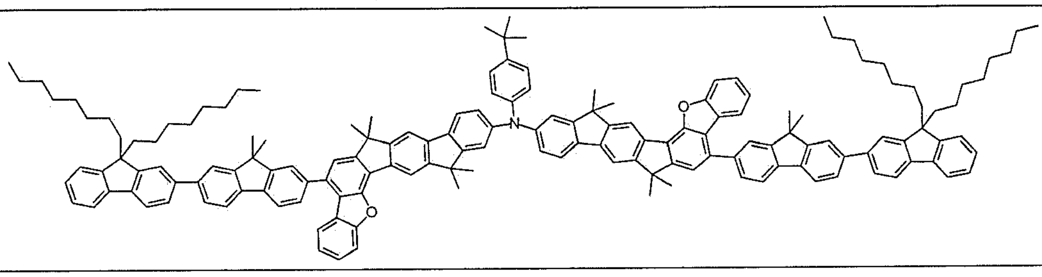 |

| |
|---|
| 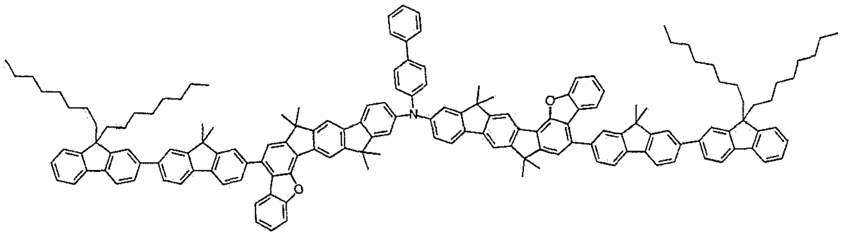 |
| 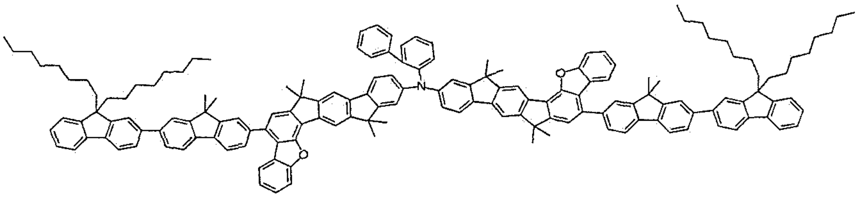 |
| 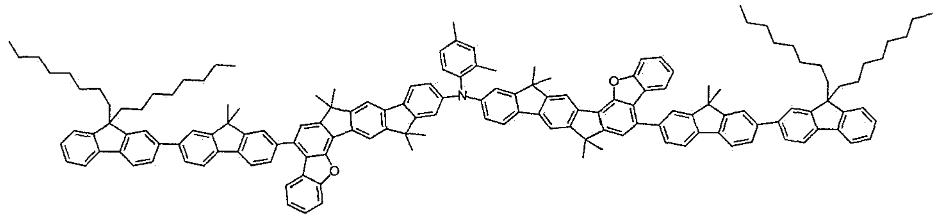 |
| 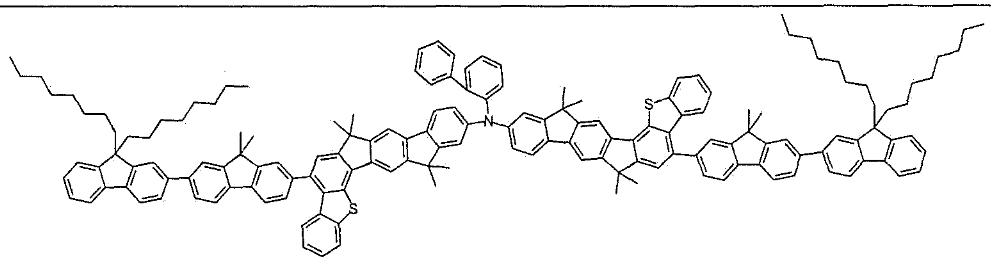 |
| 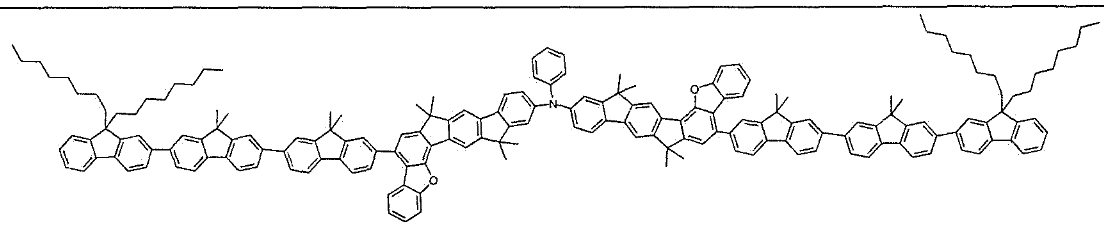 |
| 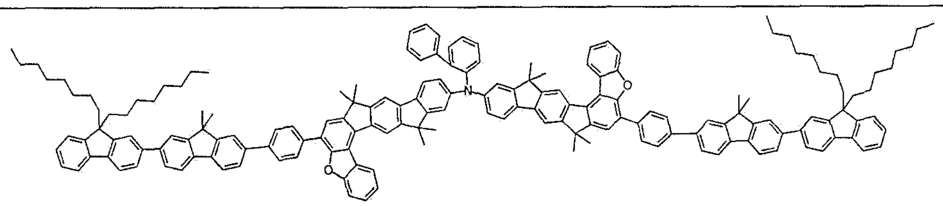 |
| 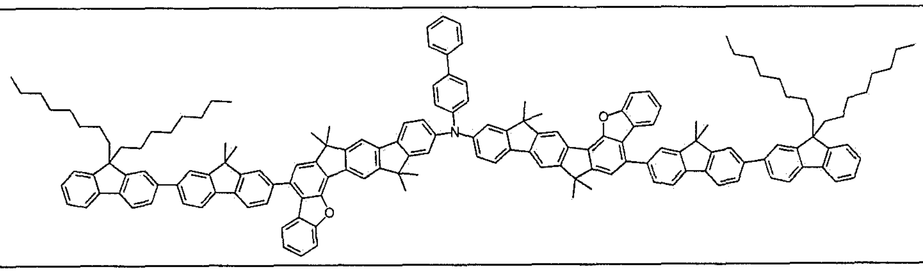 |

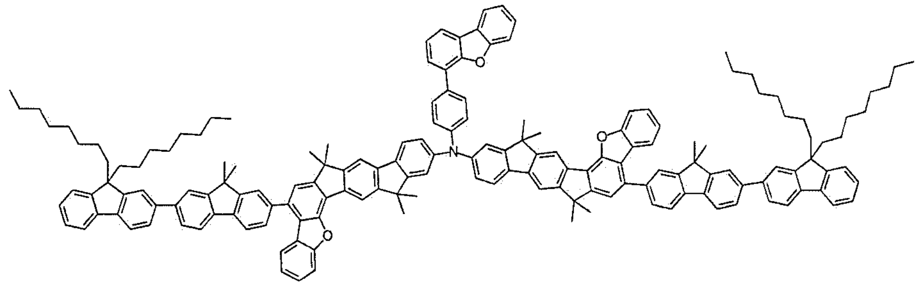

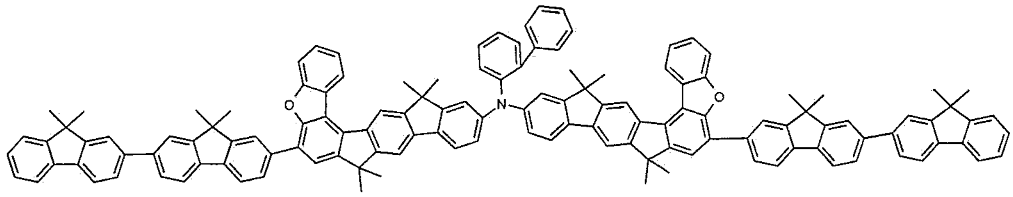

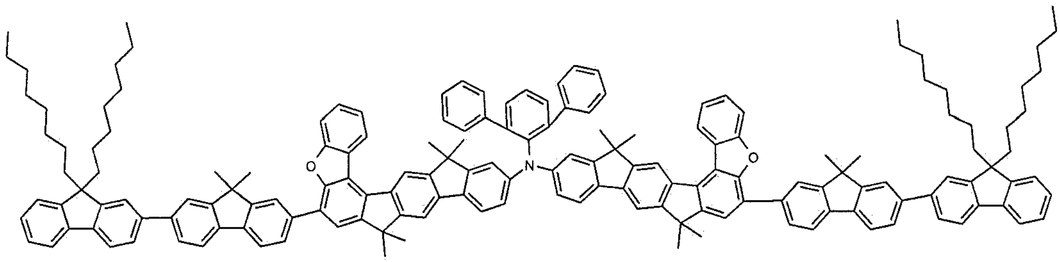

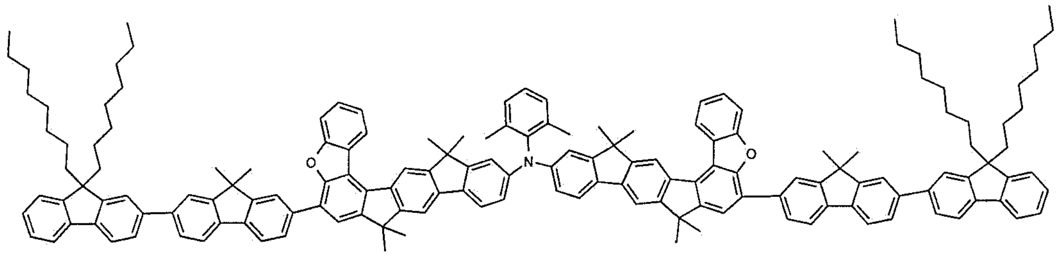

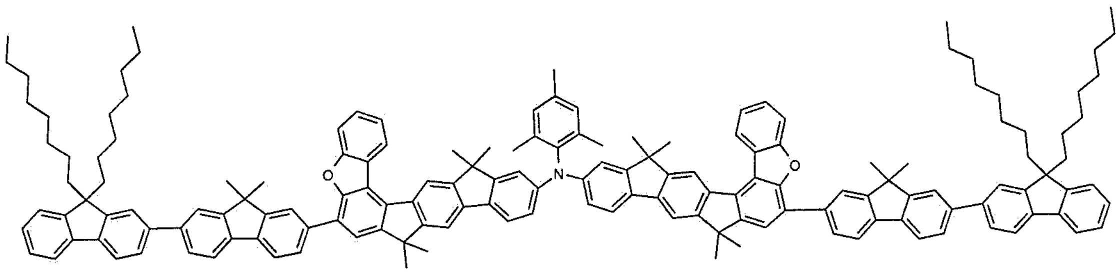

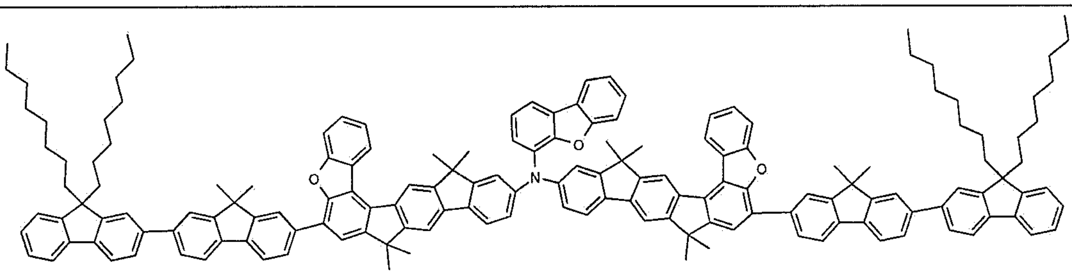

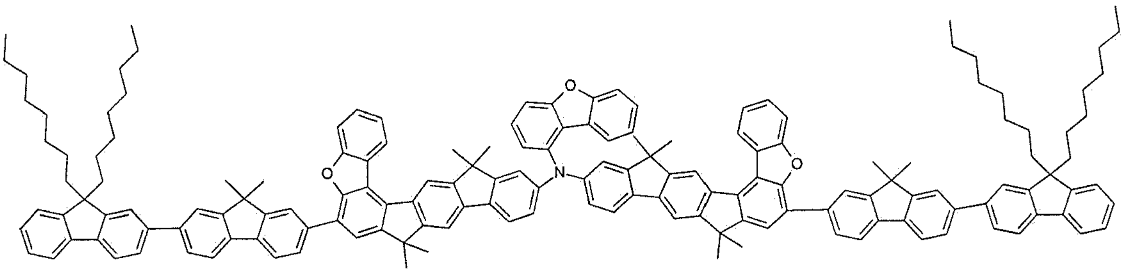

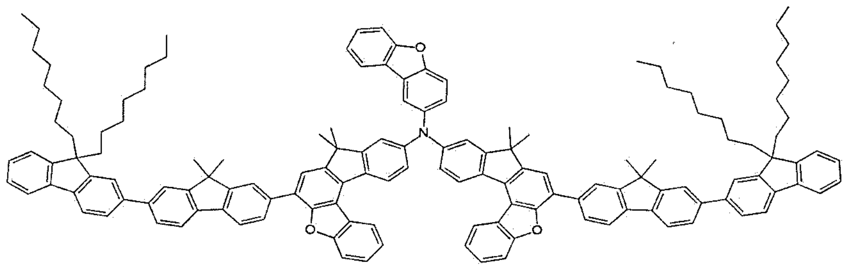

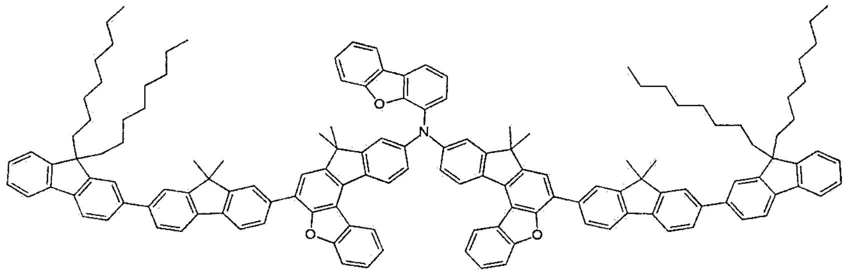

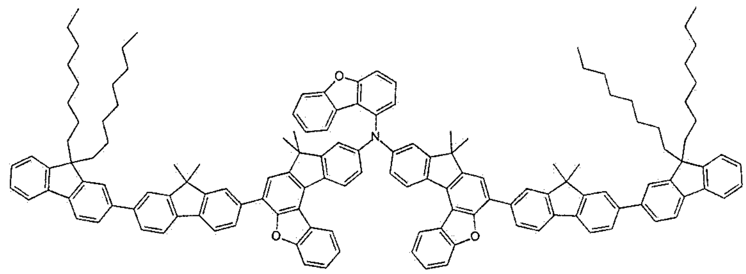

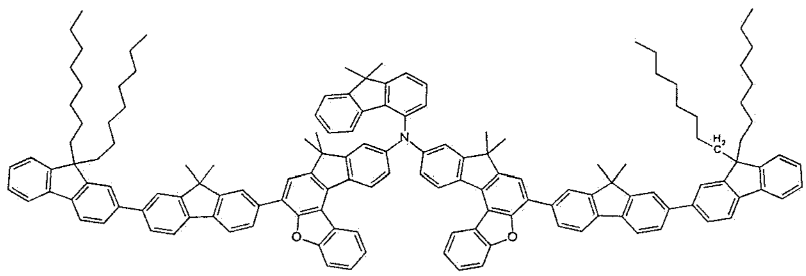

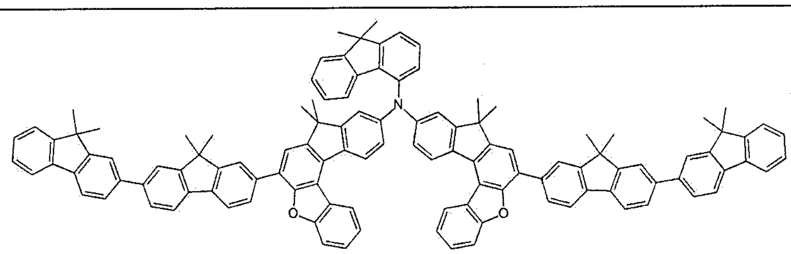

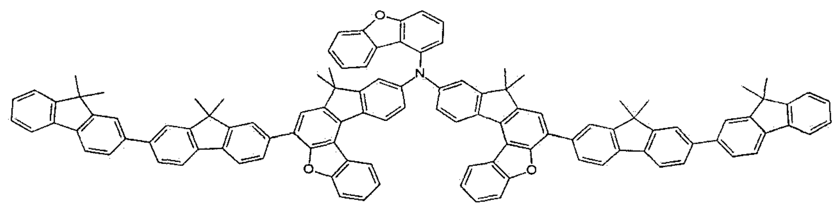

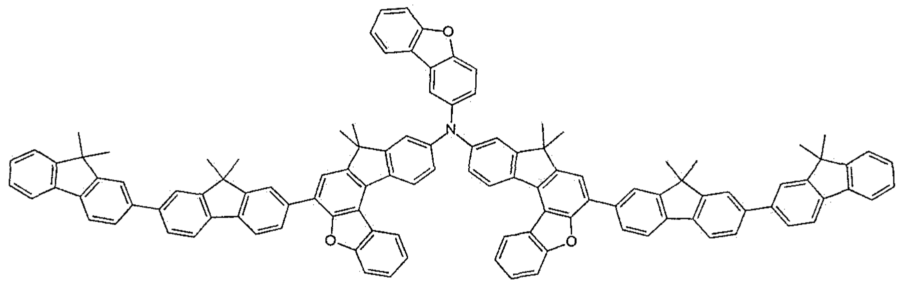

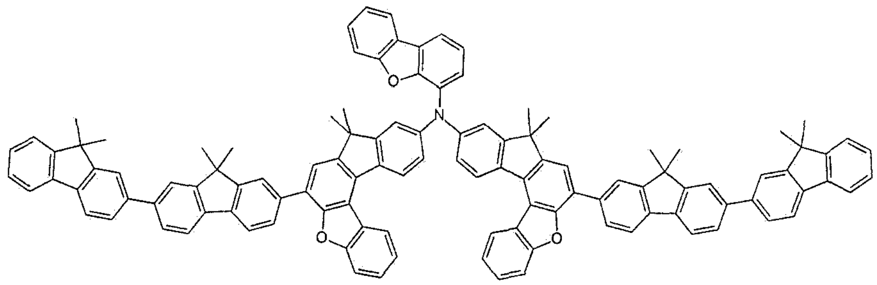

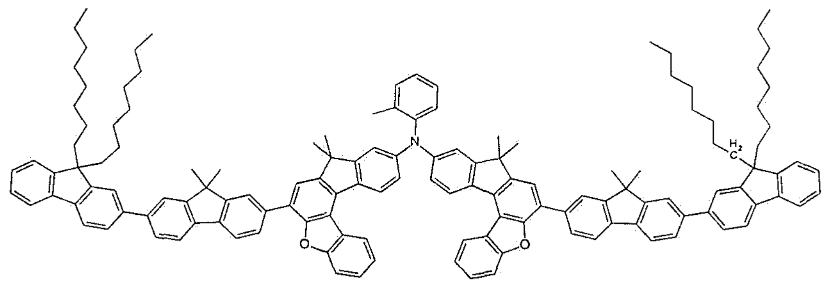

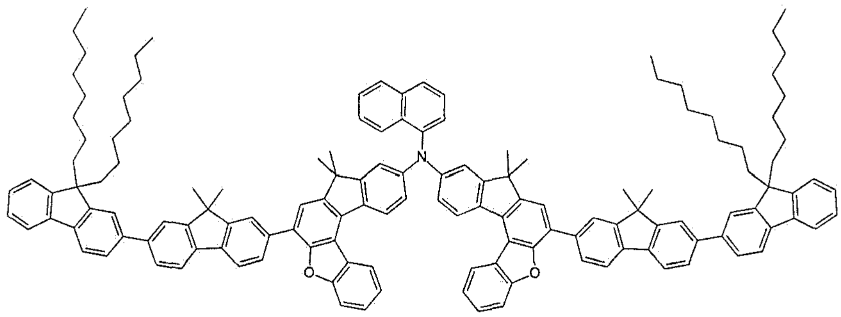

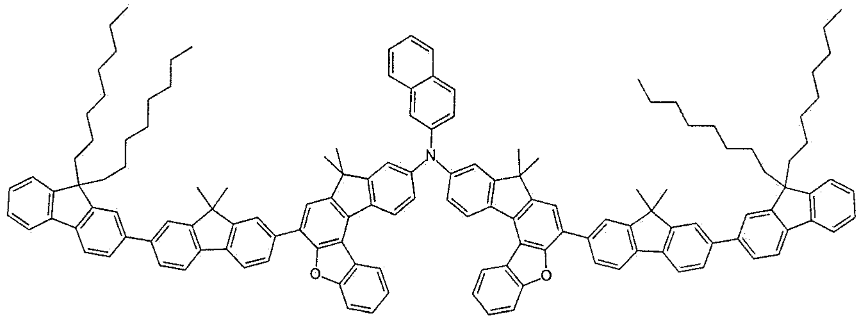

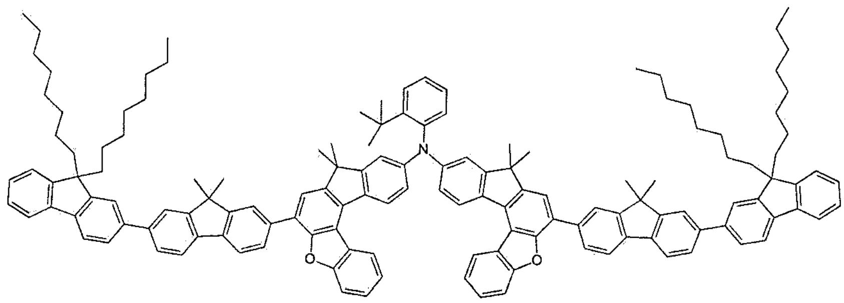

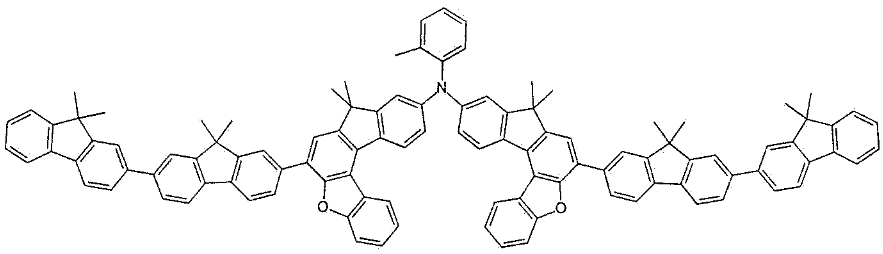

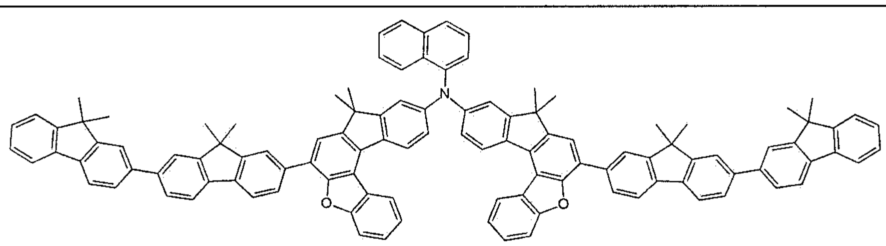

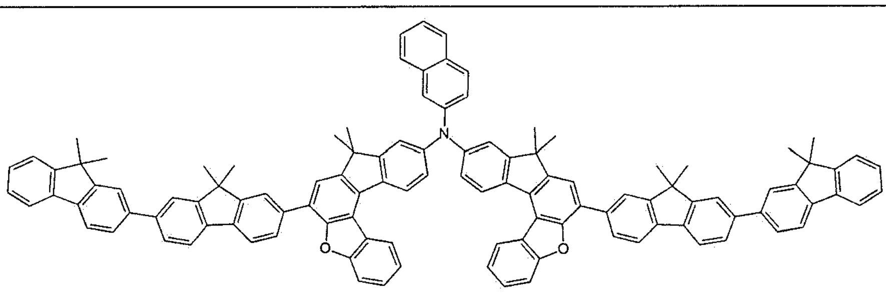

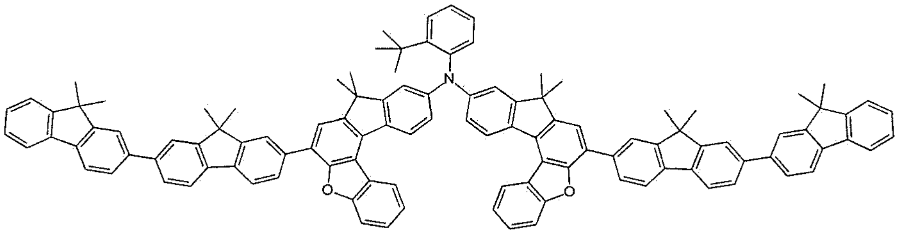

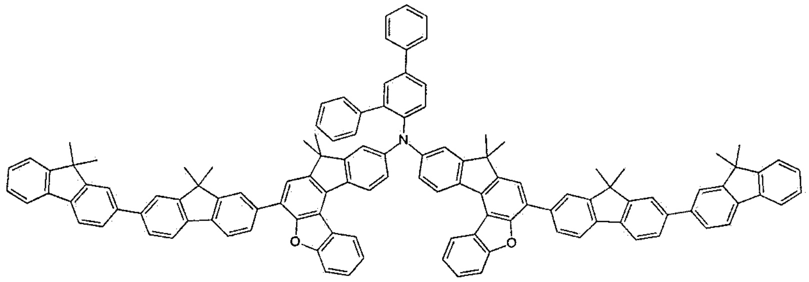

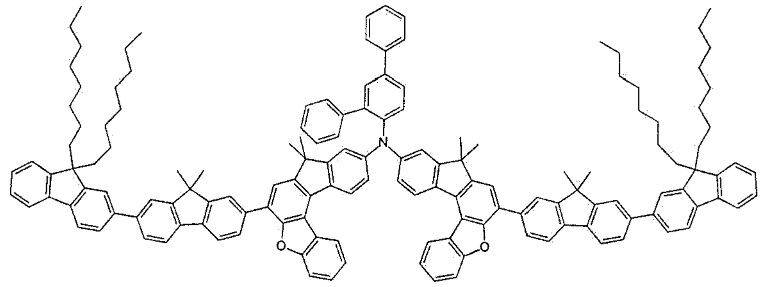

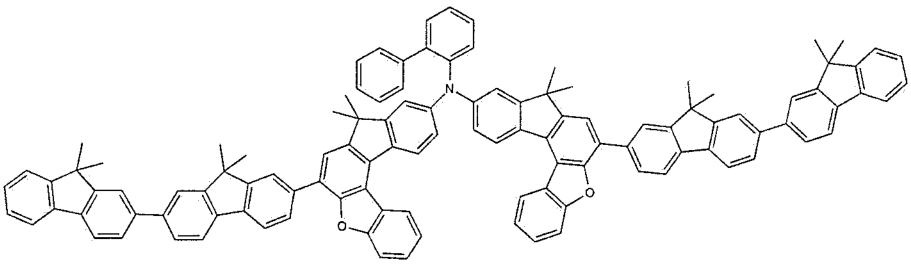

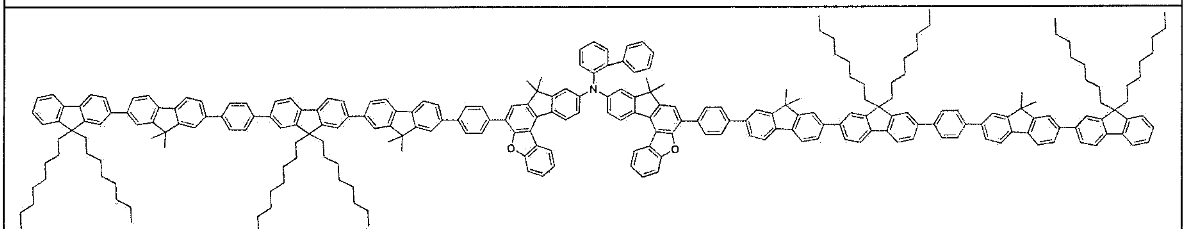

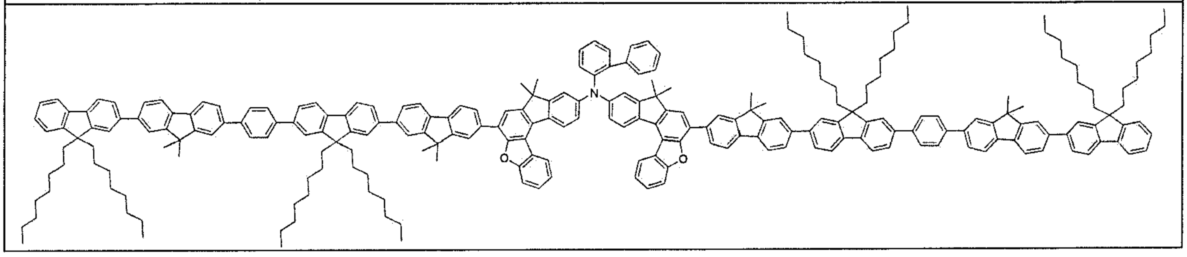

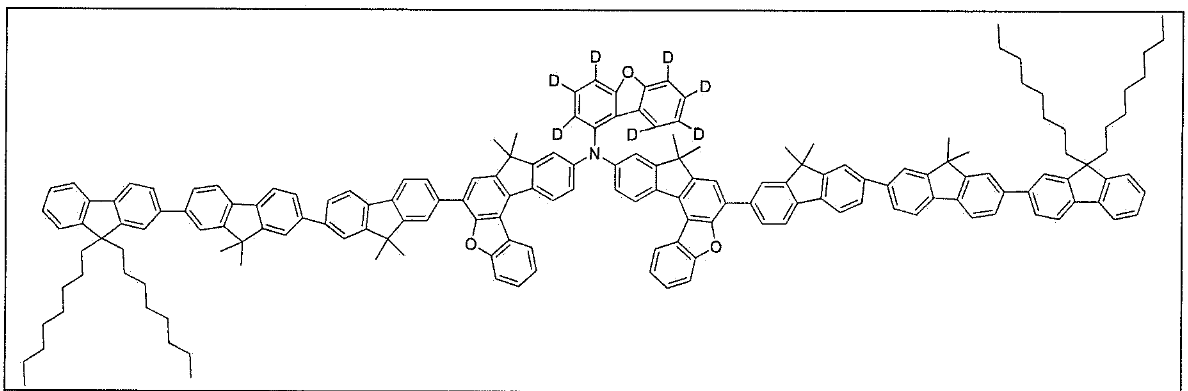

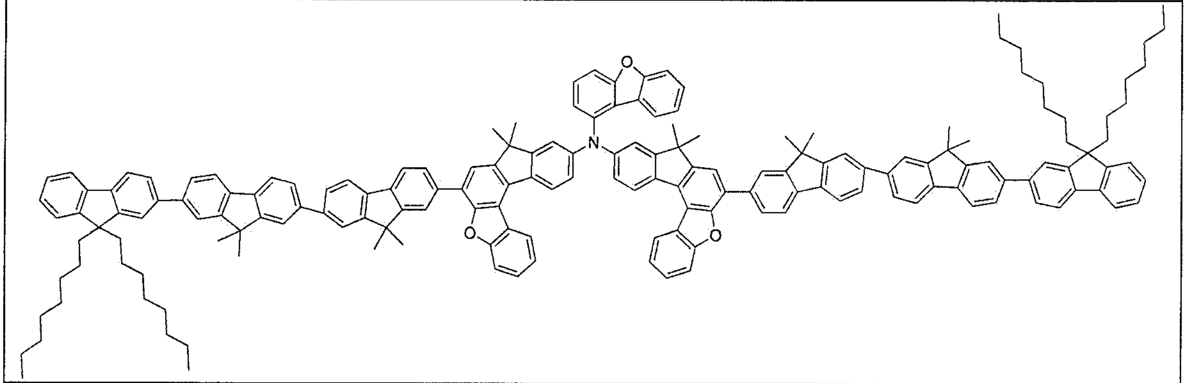

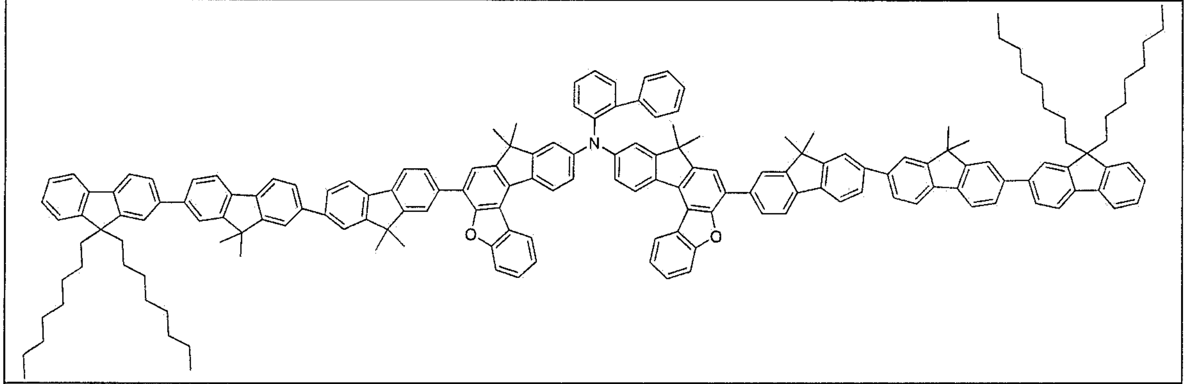

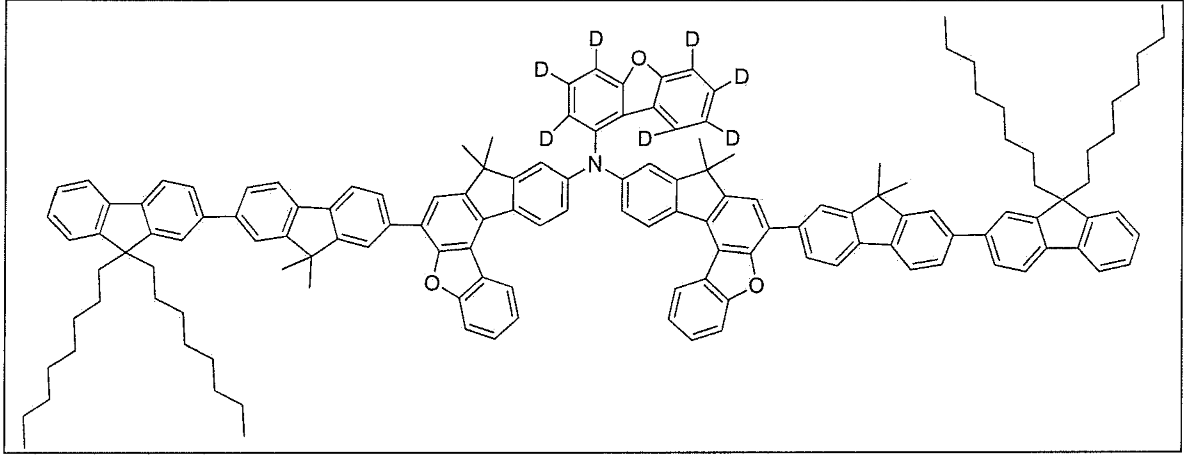

| | |
|---|---|
| 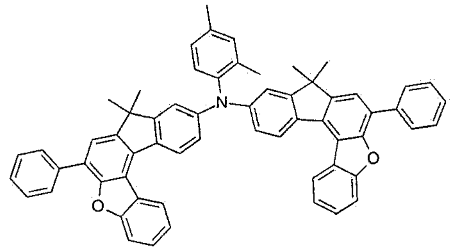 | 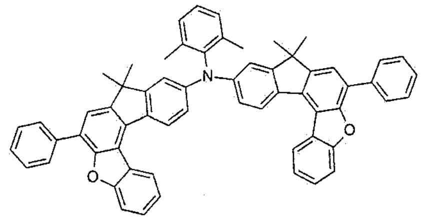 |
| 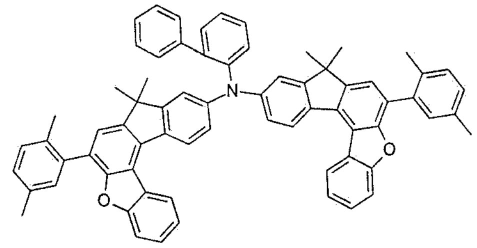 | 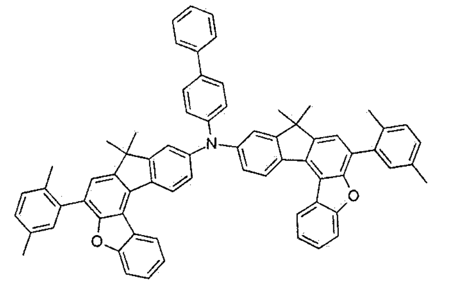 |
| 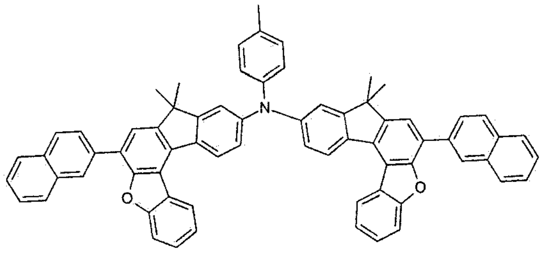 | 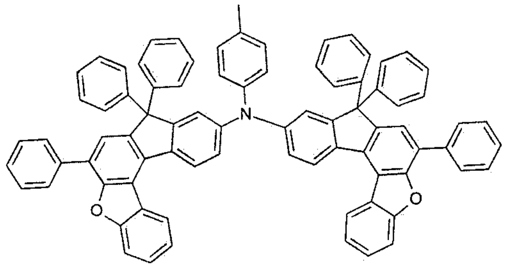 |
| 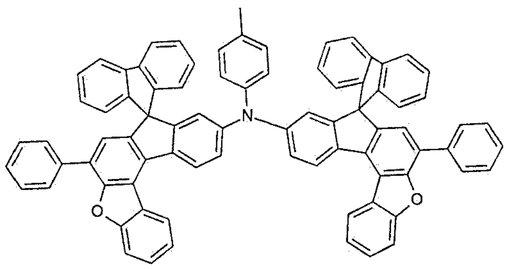 | 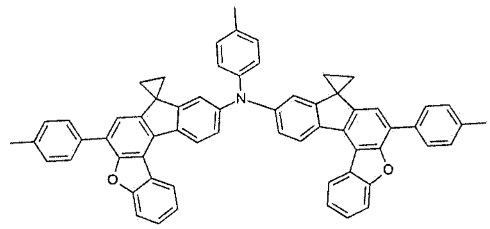 |
| 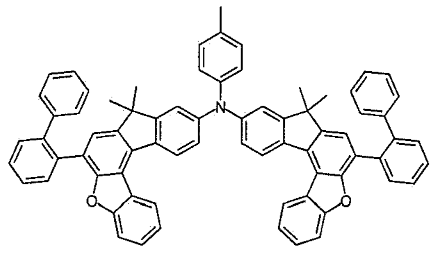 | 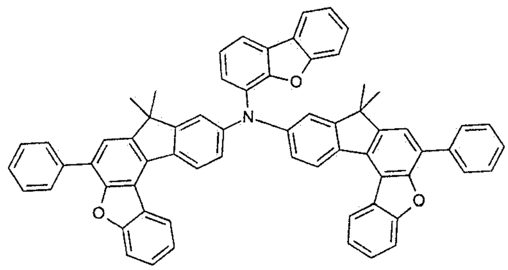 |
| 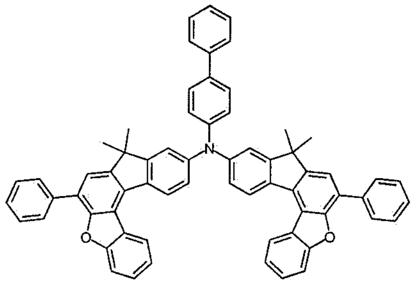 | 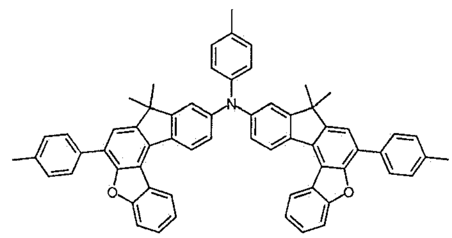 |

| | |
|---|---|
| 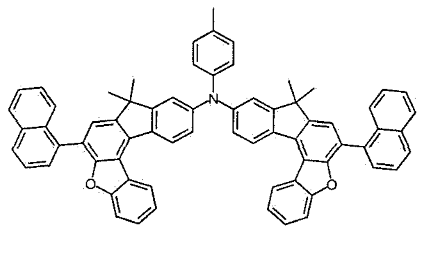 | 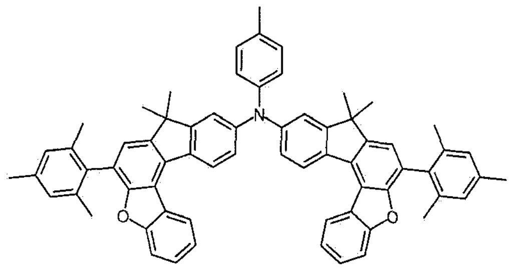 |
| 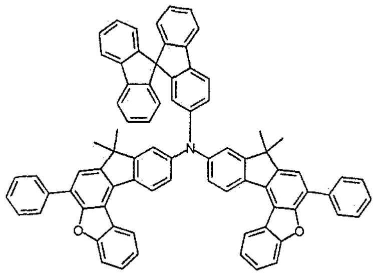 | 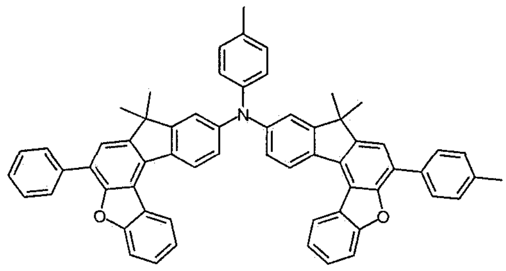 |
| 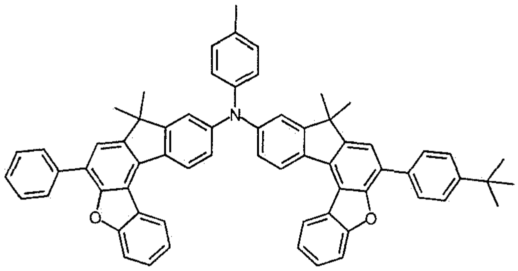 | 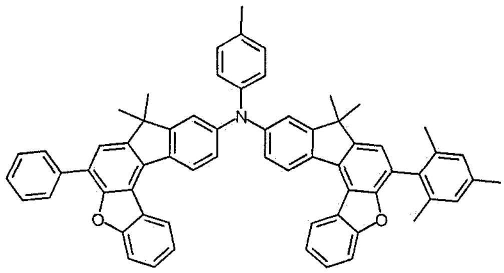 |
| 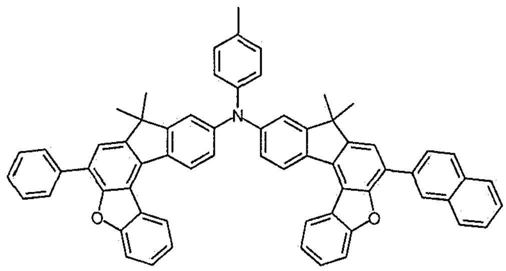 | 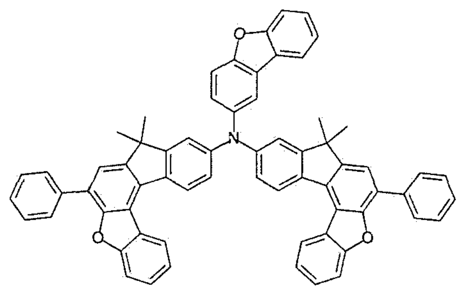 |
| 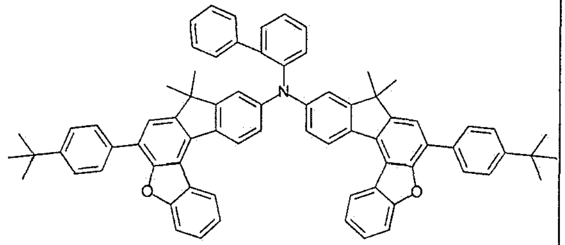 | 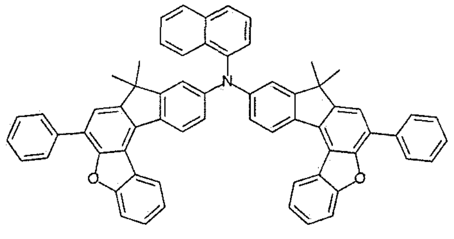 |

In accordance with the invention, the compound of formula (H1) and the compound of formula (H2) are present together in the composition, preferably in a homogeneous mixture.

Preferably, the compound of formula (H1) is present in the composition according to the invention in a proportion of 1 - 60 %, preferably 5 - 50 %, more preferably 10-50 %, particularly preferably 5 - 40 %, more particularly preferably 10 - 40 %, and very more particularly preferably 20 - 40 %.

Preferably, the compound of formula (H2) is present in the composition in a proportion of 30 - 99 %, preferably 50 - 95 %, more preferably 50 - 90 %, particularly preferably 60 - 95%, more particularly preferably 60 - 90 % and very more particularly preferably 60 - 80 %.

According to a preferred embodiment, the composition according to the invention further comprises at least one fluorescent emitter. In this case, it is preferred that the fluorescent emitter is present in the composition in a proportion of 0.1 and 50.0%, preferably between 0.5 and 20.0%, particularly preferably between 1.0 and 10.0%.

The specifications of the proportions in % are, for the purposes of the present application, taken to mean % by vol. if the compounds are applied from the gas phase and % by weight if the compounds are applied from solution.

For the processing of the compounds according to the invention from the liquid phase, for example by coating processes like spin coating or by printing processes, formulations of the compositions according to the invention are necessary. These formulations can be, for example, solutions, dispersions or emulsions. It may be preferred to use mixtures of two or more solvents for this purpose. The solvents are preferably selected from organic and inorganic solvents, more preferably organic solvents. The solvents are very preferably selected from hydrocarbons, alcohols, esters, ethers, ketones and amines. Suitable and preferred solvents are, for example, toluene, anisole, o-, m- or p-xylene, methyl benzoate, mesitylene, tetralin, veratrole, THF, methyl-THF, THP, chlorobenzene, dioxane, phenoxytoluene, in particular 3-phenoxytoluene, (-)-fenchone, 1,2,3,5-tetramethylbenzene, 1,2,4,5-tetramethylbenzene, 1-methylnaphthalene, 1-ethylnaphthalene, decylbenzene, phenyl naphthalene, menthyl isovalerate, para tolyl isobutyrate, cyclohexal hexanoate, ethyl para toluate, ethyl ortho toluate, ethyl meta toluate, decahydronaphthalene, ethyl 2-methoxybenzoate, dibutylaniline, dicyclohexylketone, isosorbide dimethyl ether, decahydronaphthalene, 2-methylbiphenyl, ethyl octanoate, octyl octanoate, diethyl sebacate, 3,3-dimethylbiphenyl, 1,4-dimethylnaphthalene, 2,2'-dimethylbiphenyl, 2-methylbenzothiazole, 2-phenoxyethanol, 2-pyrrolidinone, 3-methylanisole, 4-methylanisole, 3,4-dimethylanisole, 3,5-dimethylanisole, acetophenone, $\alpha$-terpineol, benzothiazole, butyl benzoate, cumene, cyclohexanol, cyclohexanone, cyclohexylbenzene, decalin, dodecylbenzene, ethyl benzoate, indane, NMP, p-cymene, phenetole, 1,4-diisopropylbenzene, dibenzyl ether, diethylene glycol butyl methyl ether, triethylene glycol butyl methyl ether, diethylene glycol dibutyl ether, triethylene glycol dimethyl ether, diethylene - glycol monobutyl ether, tripropylene glycol dimethyl ether, tetraethylene glycol dimethyl ether, 2-isopropylnaphthalene, pentylbenzene, hexylbenzene, heptylbenzene, octylbenzene, 1,1-bis(3,4-dimethylphenyl)ethane or mixtures of these solvents.

The present invention therefore furthermore relates to a formulation comprising a compound formula (H1) and a compound of formula (H2) according to the invention and at least one solvent. The solvent may be one of the above-mentioned solvents or a mixture of these solvents.

The proportion of the organic solvent in the formulation according to the invention is preferably at least 60% by weight, preferably at least 70% by weight and more preferably at least 80% by weight, based on the total weight of the formulation.

A formulation in accordance with the present invention can be employed for the production of a layer or multilayered structure in which the organofunctional materials are present in layers, as are required for the production of preferred electronic or opto-electronic components, such as OLEDs.

The formulation of the present invention can preferably be employed for the formation of a functional layer comprising a composition according to the present invention on a substrate or on one of the layers applied to the substrate.

Still further object of the invention is a process for the production of an electronic device, wherein at least one layer is obtained from the application of a formulation of the present invention. Preferably, a formulation according to the invention is applied to a substrate or to another layer and then dried.

The functional layer obtained from the formulation according to the invention can be produced, for example, by flood coating, dip coating, spray coating, spin coating, screen printing, relief printing, gravure printing, rotary printing, roller coating, flexographic printing, offset printing or nozzle printing, preferably ink-jet printing on a substrate or one of the layers applied to the substrate.

After the application of a formulation according to the invention to a substrate or a functional layer already applied, a drying step can be carried out in order to remove the solvent. The drying can preferably be carried out at relatively low temperature and over a relatively long period in order to avoid bubble formation and to obtain a uniform coating. The drying can preferably be carried out at a temperature in the range from 80 to 300°C, particularly preferably 150 to 250°C and especially preferably 180 to 200°C. The drying here can preferably be carried out at a pressure in the range from $10^{-6}$ mbar to 2 bar, particularly preferably in the range from $10^{-2}$ mbar to 1 bar and especially preferably in the range from $10^{-1}$ mbar to 100 mbar. The duration of the drying depends on the degree of drying to be achieved, where small amounts of water can optionally be removed at relatively high temperature and in combination with sintering, which is preferably to be carried out.

Therefore, the present invention relates to a process for the production of an electronic device comprising at least one layer comprising a composition according to the present invention, wherein the process comprises the following steps:

a) Preparation of a formulation according to the invention;
b) Application of the formulation prepared in step a) on a substrate or on another layer in order to form a layer comprising a composition according to the present invention;
c) Drying of the layer in order to remove the solvent.

Preferably, in step b), the formulation is applied by processing from a liquid phase, more preferably via a coating

method or a printing method, very more preferably by a printing method, particularly preferably by an inkjet printing method.

Another object of the invention is an electronic device, which comprises anode, cathode and at least one functional layer in between, where this functional layer comprises a composition according to the invention. Preferably, the at least one functional layer comprising a composition according to the invention is an emitting layer.

The electronic device is preferably selected from organic electroluminescent device (OLEDs), organic integrated circuits, organic field-effect transistors, organic thin-film transistors, organic light-emitting transistors, organic solar cells, dye-sensitised organic solar cells, organic optical detectors, organic photoreceptors, organic field-quench devices, light-emitting electrochemical cells, organic laser diodes and organic plasmon emitting devices. More preferably, the electronic device is an organic electroluminescent device (OLED).

The organic electroluminescent device comprises a cathode, an anode and at least one emitting layer, which comprises a composition according to the invention. Apart from these layers, it may also comprise further layers, for example in each case one or more hole-injection layers, hole-transport layers, hole-blocking layers, electron-transport layers, electron-injection layers, exciton-blocking layers, electron-blocking layers and/or charge-generation layers. It is likewise possible for interlayers, which have, for example, an exciton-blocking function, to be introduced between two emitting layers. However, it should be pointed out that each of these layers does not necessarily have to be present. The organic electroluminescent device here may comprise one emitting layer or a plurality of emitting layers. If a plurality of emission layers are present, these preferably have in total a plurality of emission maxima between 380 nm and 750 nm, resulting overall in white emission, i.e. various emitting compounds which are able to fluoresce or phosphoresce are used in the emitting layers. Particular preference is given to systems having three emitting layers, where the three layers exhibit blue, green and orange or red emission (for the basic structure see, for example, WO 2005/011013). These can be fluorescent or phosphorescent emission layers or hybrid systems, in which fluorescent and phosphorescent emission layers are combined with one another.

The electronic device concerned may comprise a single emitting layer comprising the composition according to the invention or it may comprise two or more emitting layers.

The composition according to the present invention may comprise one or more further matrix materials.

Preferred further matrix materials are selected from the classes of the oligoarylenes (for example 2,2',7,7'-tetraphenylspirobifluorene in accordance with EP 676461 or dinaphthylanthracene), in particular the oligoarylenes containing condensed aromatic groups, the oligoarylenevinylenes (for example DPVBi or spiro-DPVBi in accordance with EP 676461), the polypodal metal complexes (for example in accordance with WO 2004/ 081017), the hole-conducting compounds (for example in accordance with WO 2004/058911), the electron-conducting compounds, in particular ketones, phosphine oxides, sulfoxides, etc. (for example in accordance with WO 2005/084081 and WO 2005/084082), the atropisomers (for example in accordance with WO 2006/048268), the boronic acid derivatives (for example in accordance with WO 2006/117052) or the benzanthracenes (for example in accordance with WO 2008/145239). Particularly preferred matrix materials are selected from the classes of the oligoarylenes, comprising naphthalene, anthracene, benzanthracene and/or pyrene or atropisomers of these compounds, the oligoarylenevinylenes, the ketones, the phosphine oxides and the sulfoxides. Very particularly preferred matrix materials are selected from the classes of the oligoarylenes, comprising anthracene, benzanthracene, benzophenanthrene and/or pyrene or atropisomers of these compounds. An oligoarylene in the sense of this invention is intended to be taken to mean a compound in which at least three aryl or arylene groups are bonded to one another.

Generally preferred classes of material for use as corresponding functional materials in the organic electroluminescent devices according to the invention are indicated below.

Suitable charge-transport materials, as can be used in the hole-injection or hole-transport layer or electron-blocking layer or in the electron-transport layer of the electronic device according to the invention, are, for example, the compounds disclosed in Y. Shirota et al., Chem. Rev. 2007, 107(4), 953-1010, or other materials as are employed in these layers in accordance with the prior art.

Materials which can be used for the electron-transport layer are all materials as are used in accordance with the prior art as electron-transport materials in the electron-transport layer. Particularly suitable are aluminium complexes, for example $Alq_3$, zirconium complexes, for example $Zrq_4$, lithium complexes, for example LiQ, benzimidazole derivatives, triazine derivatives, pyrimidine derivatives, pyridine derivatives, pyrazine derivatives, quinoxaline derivatives, quinoline derivatives, oxadiazole derivatives, aromatic ketones, lactams, boranes, diazaphosphole derivatives and phosphine oxide derivatives. Furthermore, suitable materials are derivatives of the above-mentioned compounds, as disclosed in JP 2000/053957, WO 2003/060956, WO 2004/028217, WO 2004/080975 and WO 2010/072300.

Preferred hole-transport materials which can be used in a hole-transport, hole-injection or electron-blocking layer in the electroluminescent device according to the invention are indenofluorenamine derivatives (for example in accordance with WO 06/122630 or WO 06/100896), the amine derivatives disclosed in EP 1661888, hexaazatriphenylene derivatives (for example in accordance with WO 01/049806), amine derivatives containing condensed aromatic rings (for example in accordance with US 5,061,569), the amine derivatives disclosed in WO 95/09147, monobenzoindenofluorenamines (for example in accordance with WO 08/006449), dibenzoindenofluorenamines (for example in accordance

with WO 07/140847), spirobifluorenamines (for example in accordance with WO 2012/034627 or WO 2013/120577), fluorenamines (for example in accordance with the as applications EP 2875092, EP 2875699 and EP 2875004), spirodibenzopyranamines (for example in accordance with WO 2013/083216) and dihydroacridine derivatives (for example in accordance with WO 2012/150001). The compounds according to the invention can also be used as hole-transport materials.

The cathode of the organic electroluminescent device preferably comprises metals having a low work function, metal alloys or multilayered structures comprising various metals, such as, for example, alkaline-earth metals, alkali metals, main-group metals or lanthanoids (for example Ca, Ba, Mg, Al, In, Mg, Yb, Sm, etc.). Also suitable are alloys comprising an alkali metal or alkaline-earth metal and silver, for example an alloy comprising magnesium and silver. In the case of multilayered structures, further metals which have a relatively high work function, such as, for example, Ag or Al, can also be used in addition to the said metals, in which case combinations of the metals, such as, for example, Ca/Ag, Mg/Ag or Ag/Ag, are generally used. It may also be preferred to introduce a thin interlayer of a material having a high dielectric constant between a metallic cathode and the organic semiconductor. Suitable for this purpose are, for example, alkali metal fluorides or alkaline-earth metal fluorides, but also the corresponding oxides or carbonates (for example LiF, $Li_2O$, $BaF_2$, MgO, NaF, CsF, $Cs_2CO_3$, etc.). Furthermore, lithium quinolinate (LiQ) can be used for this purpose. The layer thickness of this layer is preferably between 0.5 and 5 nm.

The anode preferably comprises materials having a high work function. The anode preferably has a work function of greater than 4.5 eV vs. vacuum. Suitable for this purpose are on the one hand metals having a high redox potential, such as, for example, Ag, Pt or Au. On the other hand, metal/metal oxide electrodes (for example Al/Ni/NiOx, Al/$PtO_x$) may also be preferred. For some applications, at least one of the electrodes must be transparent or partially transparent in order to facilitate either irradiation of the organic material (organic solar cells) or the coupling-out of light (OLEDs, O-lasers). Preferred anode materials here are conductive mixed metal oxides. Particular preference is given to indium tin oxide (ITO) or indium zinc oxide (IZO). Preference is furthermore given to conductive, doped organic materials, in particular conductive doped polymers.

The device is appropriately (depending on the application) structured, provided with contacts and finally sealed, since the lifetime of the devices according to the invention is shortened in the presence of water and/or air.

in a preferred embodiment, the organic electroluminescent device according to the invention is characterised in that one or more layers are coated by means of a sublimation process, in which the materials are applied by vapour deposition in vacuum sublimation units at an initial pressure of less than $10^{-5}$ mbar, preferably less than $10^{-6}$ mbar. However, it is also possible here for the initial pressure to be even lower, for example less than $10^{-7}$ mbar.

Preference is likewise given to an organic electroluminescent device, characterised in that one or more layers are coated by means of the OVPD (organic vapour phase deposition) process or with the aid of carrier-gas sublimation, in which the materials are applied at a pressure of between $10^{-5}$ mbar and 1 bar. A special case of this process is the OVJP (organic vapour jet printing) process, in which the materials are applied directly through a nozzle and are thus structured (for example M. S. Arnold et al., Appl. Phys. Lett. 2008, 92, 053301).

Preference is furthermore given to an organic electroluminescent device, characterised in that one or more layers are produced from solution, such as, for example, by spin coating, or by means of any desired printing process, such as, for example, screen printing, flexographic printing, nozzle printing or offset printing, but particularly preferably LITI (light induced thermal imaging, thermal transfer printing) or ink-jet printing. Soluble compounds of the formula (I) are necessary for this purpose. High solubility can be achieved through suitable substitution of the compounds.

Also possible are hybrid processes, in which, for example, one or more layers are applied from solution and one or more further layers are applied by vapour deposition. Thus, it is possible, for example, to apply the emitting layer from solution and to apply the electron-transport layer by vapour deposition.

These processes are generally known to the person skilled in the art and can be applied by him without inventive step to organic electroluminescent devices comprising the compounds according to the invention.

In accordance with the invention, the electronic devices comprising one or more compounds according to the invention can be employed in displays, as light sources in lighting applications and as light sources in medical and/or cosmetic applications (for example light therapy).

The invention will now be explained in greater detail by the following examples, without wishing to restrict it thereby.

**Working Examples**

**Syntheses examples**

a) Host H1

Synthesis of compound A1:

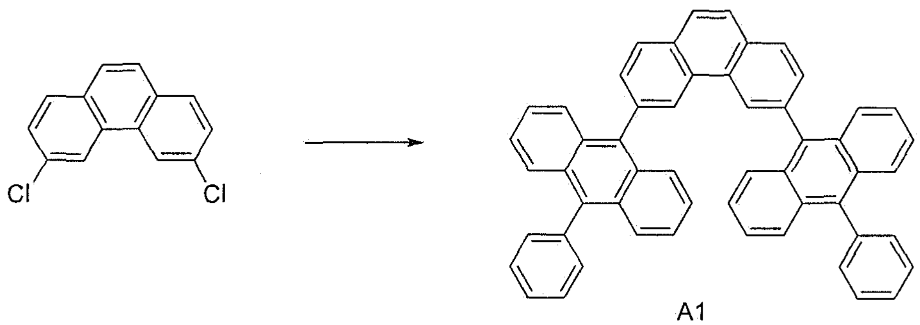

A1

7.9 g (32 mmol) 3,6-Dichloro-phenanthrene (20851-90-5), 30.4 g (80 mmol) 4,4,5,5-tetramethyl-2-(10-phenyl-9-anthracenyl)-1,3,2-dioxaborolane (460347-59-5), 29.5 g (128 mmol) potassium phosphate monohydrate are dissolved in 750 ml THF/water (2:1). 813 mg ( 0.96 mmol) ) XPhos Palladacycle Gen. 3 are added and the mixture is stirred at 65°C. After 16 hours the reaction mixture is allowed to come to room temperature. The reaction mixture is filtered and washed with cold THF. The precipitate is purified by hot extraction over aluminum oxide (toluene) and further purified by crystallization out of toluene/ethanol and toluene/heptane up to a purity of >99.9 by HPLC. The remaining solvents are removed by tempering at 300 °C at $10^{-5}$ bar for 2 hours.
Yield: 4.9 g (7.2 mmol, 23%) of a pale yellow solid

Following compounds can be synthesized in analogous manner:

| Compound | SM1 | SM2 | Product |
|---|---|---|---|
| A2 | CAS 20851-90-5 | 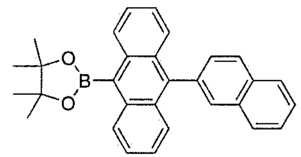 922518-84-1 | 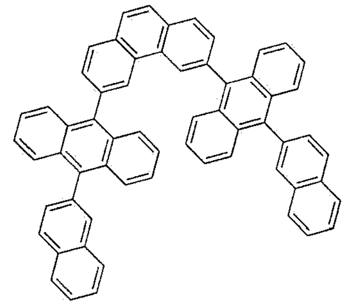 |
| A3 | CAS 20851-90-5 | 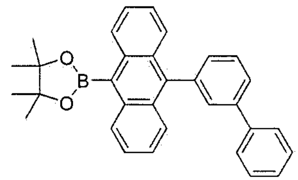 1510788-38-1 | 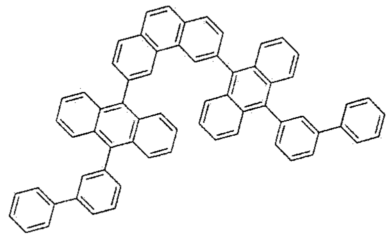 |
| A4 | CAS 20851-90-5 | 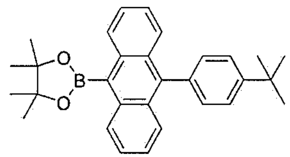 1374785-59-7 | 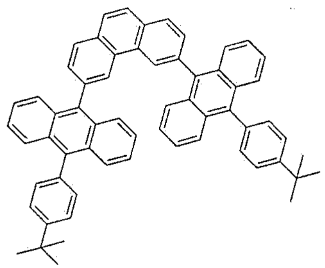 |

(continued)

| Compound | SM1 | SM2 | Product |
| --- | --- | --- | --- |
| A5 | CAS 20851-90-5 | 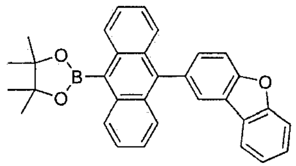 2100281-97-6 | 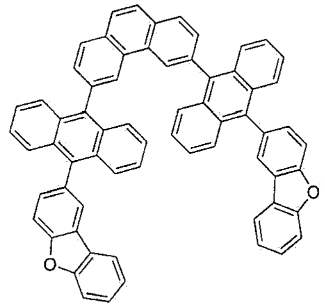 |
| A6 | CAS 20851-90-5 | 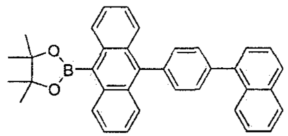 1016653-37-4 | 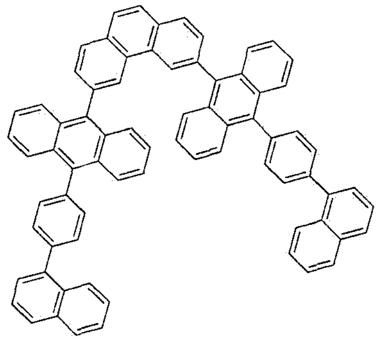 |
| A7 | CAS 20851-90-5 | 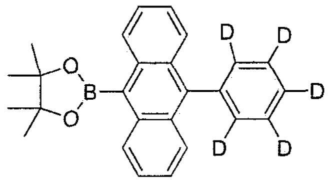 1450898-41-5 | 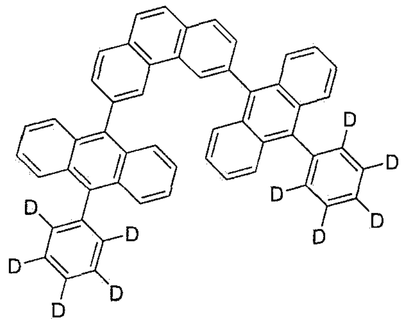 |

(continued)

| Compound | SM1 | SM2 | Product |
|---|---|---|---|
| A8 | CAS 20851-90-5 | 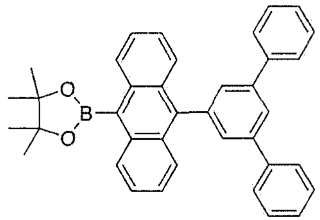 1016653-38-5 | 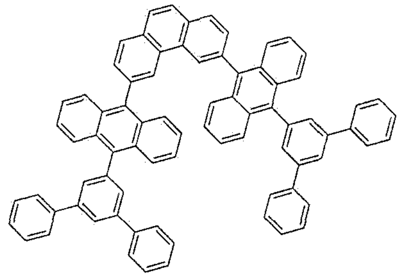 |
| A9 | CAS 20851-90-5 | 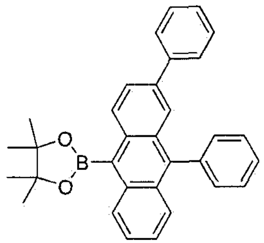 1510788-96-1 | 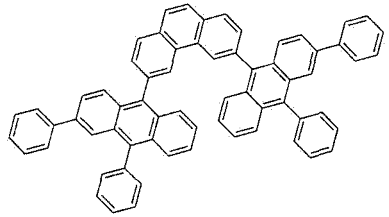 |
| A10 | CAS 20851-90-5 | 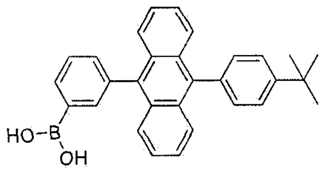 1877377-99-5 | 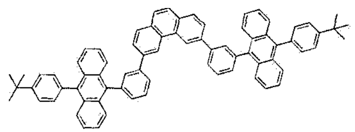 |
| A11 | CAS 20851-90-5 | 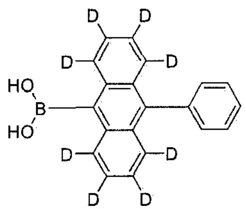 2414494-83-8 | 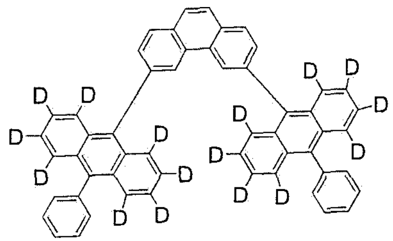 |

Synthesis of compound I1:

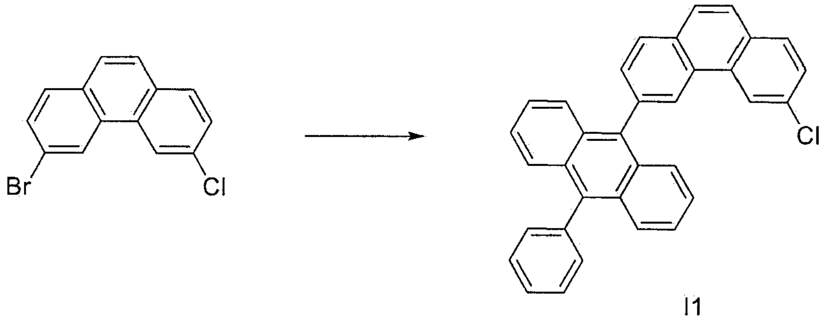

I1

9.3 g (32 mmol) 3-Bromo-6-chloro-phenanthrene (892550-44-6), 13.3 g (35 mmol) 4,4,5,5-tetramethyl-2-(10-phenyl-9-anthracenyl)-1,3,2-dioxaborolane (460347-59-5), 11.5 g (50mmol) potassium phosphate monohydrate are dissolved in 750 ml THF/water (2:1). 813 mg ( 0.96 mmol) ) XPhos Palladacycle Gen. 3 are added and the mixture is stirred at 65°C. After 16 hours the reaction mixture is allowed to come to room temperature. The mixture is diluted with 300 ml toluene. The aqueous phase is extracted with toluene (2x200 ml) and the combined organic phases are washed with water (2x 200 ml) dried over magnesium sulfate, filtered and reduced under reduced pressure. The remaining solid is filtered over silica (toluene) and crystalized out of toluene/ethanol up to a purity of 98% by HPLC.
Yield 10.7 g (23 mmol, 72%)

Following compounds can be synthesized in analogous manner:

| Compound | SM1 | SM2 | Product |
|---|---|---|---|
| I2 | 892550-44-6 | 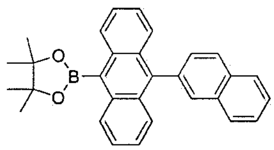 922518-84-1 | 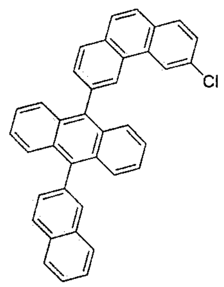 |
| I3 | 892550-44-6 | 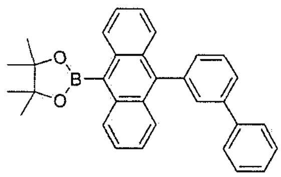 1510788-38-1 | 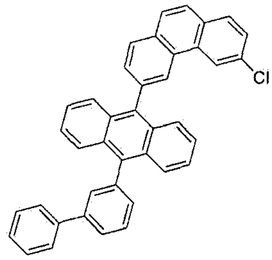 |
| I4 | 892550-44-6 | 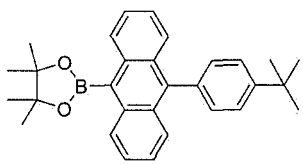 1374785-59-7 | 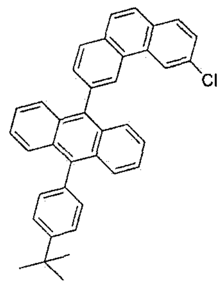 |

(continued)

| | | | |
|---|---|---|---|
| I5 | 892550-44-6 | 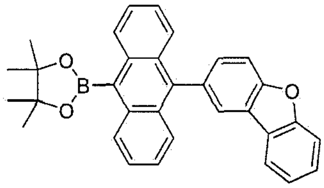 2100281-97-6 | 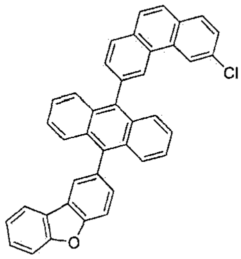 |
| I6 | 892550-44-6 | 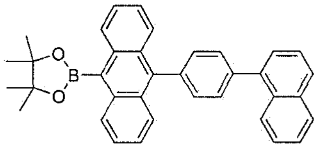 1016653-37-4 | 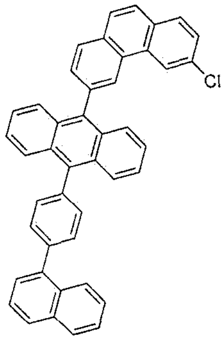 |
| I7 | 892550-44-6 | 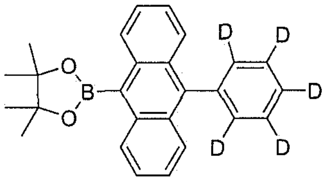 1450898-41-5 | 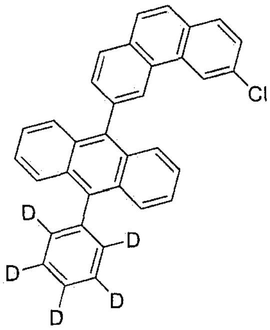 |
| I8 | 892550-44-6 | 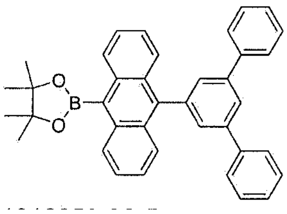 1016653-38-5 | 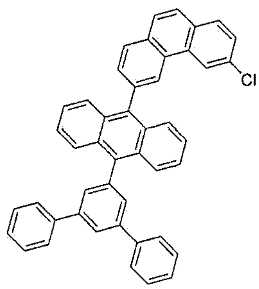 |
| I9 | 892550-44-6 | 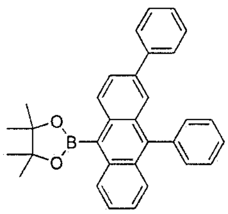 1510788-96-1 | 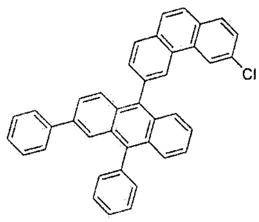 |

Synthesis of compound B1:

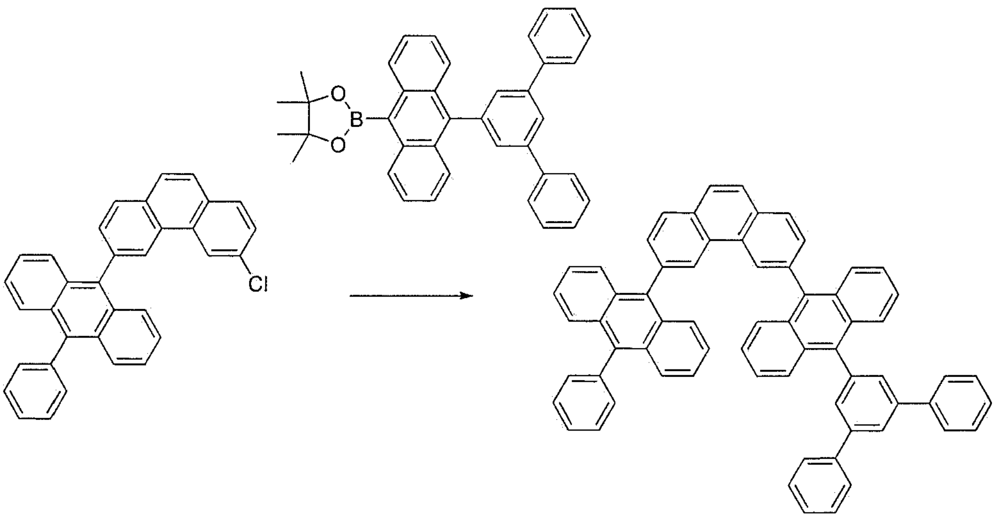

9.3 g (20 mmol) 11, 16.0 g (30 mmol) 4,4,5,5-tetramethyl-2-(10-{5-phenyl-[1,1'-biphenyl]-3-yl}anthracen-9-yl)-1,3,2-dioxaborolane (1016653-38-5), 11.5 g (50mmol) potassium phosphate monohydrate are dissolved in 750 ml THF/water (2:1). 813 mg ( 0.96 mmol) ) XPhos Palladacycle Gen. 3 are added and the mixture is stirred at 65°C. After 16 hours the reaction mixture is allowed to come to room temperature. The mixture is diluted with 300 ml toluene. The aqueous phase is extracted with toluene (2x200 ml) and the combined organic phases are washed with water (2x 200 ml) dried over magnesium sulfate, filtered and reduced under reduced pressure. The remaining solid purified by hot extraction over aluminum oxide (toluene) and crystalized out of toluene/ethanol and toluene/heptane up to a purity of >99.9% by HPLC. The remaining solvents are removed by tempering at 300°C and $10^{-5}$ bar for 2 hours.
Yield: 7.5 g (9 mmol, 45%)

Following compounds can be synthesized in analogous manner:

| Compound | SM1 | SM2 | Product |
|---|---|---|---|
| B1 | I2 | CAS 460347-59-5 | 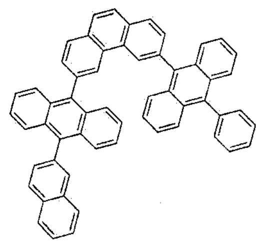 |
| B2 | I3 | CAS 460347-59-5 | 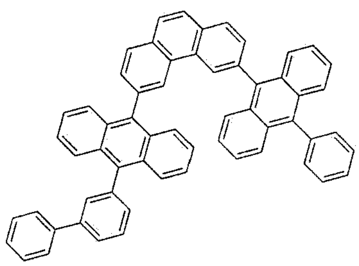 |
| B3 | I4 | CAS 460347-59-5 | 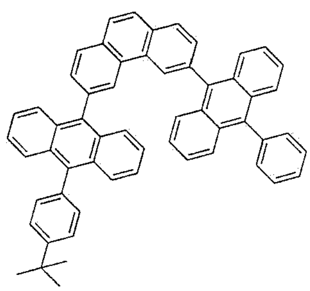 |

(continued)

| Compound | SM1 | SM2 | Product |
|---|---|---|---|
| B4 | I5 | CAS 460347-59-5 | 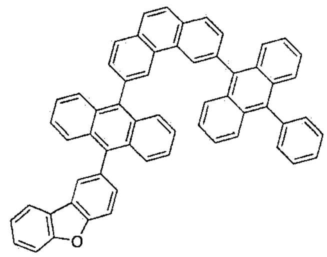 |
| B5 | I6 | CAS 460347-59-5 | 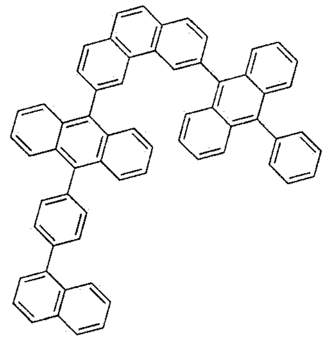 |
| B6 | I7 | CAS 460347-59-5 | 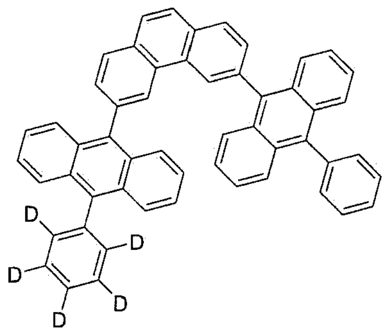 |
| B7 | I9 | CAS 460347-59-5 | 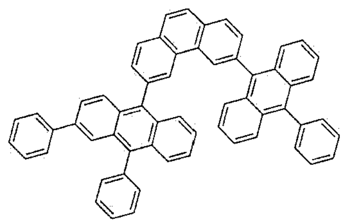 |
| B8 | I1 | 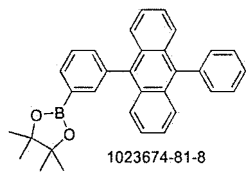 1023674-81-8 | 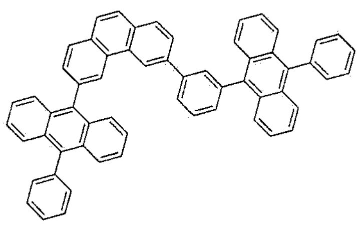 |

b) Host H2

The syntheses of the hosts of formula (H2) are known to the person skilled in the art and are described, for example, in WO 2008/145239 and WO 2015/158409. Further syntheses examples are described below:

Synthesis of C-1

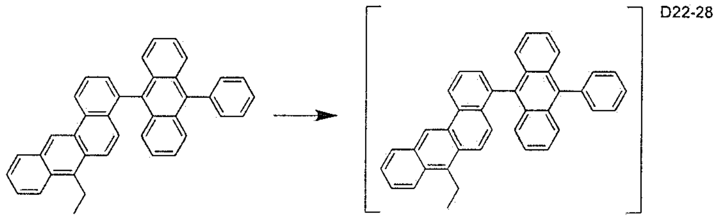


C-I1 C1

In an autoclave C-I1 (1818872-84-2; 10 g, 19.5 mmol) is dissolved in a mixture of 700 ml toluene-d8 and $D_20$ (5:2) and degassed and flushed with nitrogen, 20g Pt-C 5% is added and the mixture is stirred at 165°C. After 11 days the mixture was cooled down to room temperature and the catalyst was filtered of and the two phases are separated. The organic phase is reduced under reduced pressure. The remaining solid is purified by filtration over silica (3 times, toluene as eluent) and several crystallizations out of dichloromethane:cyclohexane and toluene:n-heptane up to a HPLC purity of 99.9%. The remaining solid was dried by tempering at 250°C at $10^{-5}$ bar. The deuterated product (D22-28) is obtained as colorless powder. The grade of deuteration is obtained by ASAP-MS, $^1$H-NMR, $^{13}$C-NMR and 2D-NMR. Yield: 5.4 g (10.5 mmol; 54 %).

Synthesis of D1

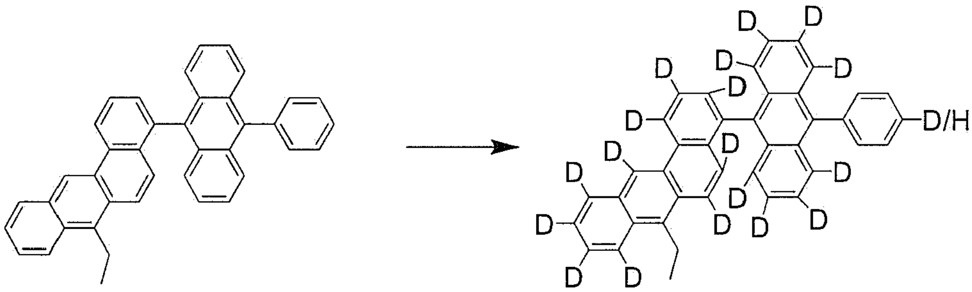

D1

10 g (19.7 mmol) 7-ethyl-4-(10-phenylanthracen-9-yl)tetraphene are dissolved in 230 mL toluene-D8. 10.4 ml (0.12 mol)Trifluoromethanesulfunic acid are added dropwise and the mixture is stirred at room temperature. After two hours 47 ml $D_20$ are added and the mixture is stirred for 10 minutes until it was added to an aqueous potassium phosphate solution. The mixture is extracted with toluene and the combined organic phases are dried over sodium sulfate. The organic phase is reduced under reduced pressure. The remaining solid is purified by column chromatography and several crystallizations out of dichloromethane:cyclohexane and toluene:n-heptane up to a HPLC purity of 99.9%. Yield 5.7g (10.8 mmol, 55%)

**Device examples**

**a) Preparation of films and devices**

Glass substrates covered with pre-structured ITO (50nm) and bank material are cleaned using ultrasonication in de-ionized water. In the following, the substrates are dried using an air-gun and subsequently annealed on a hotplate at 230°C for 2 hours.

All following process steps are carried out in yellow light.

The following layer sequence is shown in Figure 4a and 4b.

A hole-injection layer (HIL) is inkjet-printed onto the substrate with a thickness of 20nm and dried in vacuum. For this the HIL ink has a solid concentration of 6 g/l. The HIL is then annealed at 200°C for 30 minutes. Inkjet-printing

and annealing of the HIL is carried out in air. As the HIL material, a hole-transporting, cross-linkable polymer and a p-doped salt are dissolved in 3-phenoxy toluene. The materials are described i.e. in WO2016/107668, WO2013/081052 and EP2325190.

On top of the HIL, a hole-transport layer is inkjet-printed under ambient conditions, dried in vacuum and annealed at 210°C for 30 minutes in argon atmosphere. The hole-transport layer is either the polymer of the structure shown in Table 1 (HTM1), which is synthesized in accordance with WO2013156130 or the polymer HTM2 (Table 1), which is synthesized in accordance with WO2018/114882.

The polymer is dissolved in 3-phenoxy toluene, so that the solution typically has a solid content of approx. 5 g/l if, as here, the layer thickness of 20nm which is typical for a device, is to be achieved by means of inkjet printing. The layers are applied by inkjet printing in ambient atmosphere, dried in vacuum and annealed by heating at 210°C for 30 min in argon atmosphere.

The emission layer comprises a matrix material (one host compound or two host compounds) and a dopant as described in Table 2 below. The mixture for the emission layer is dissolved in 3-phenoxy toluene. The solid content of such solutions is about 10 mg/ml if, as here, the layer thickness of 30nm which is typical for a device is to be achieved by means of inkjet-printing. The blue emissive layer (B-EML) is also inkjet-printed, then vacuum dried and annealed at 150°C for 10 minutes. Inkjet-printing is done in ambient atmosphere, whereas the annealing is done in argon atmosphere.

The devices, that are prepared according to Figure 4a, are used in order to evaluate the EML film homogeneity.

For the preparation of devices according to Figure 4b, that are used for electro-optical characterization, the samples are then transferred into the vacuum deposition chamber where the deposition of two electron transport layers (ETL1, ETL2), an electron injection layer (EIL) and a cathode (Al) is done using thermal evaporation. Hereby ETL1 consists of ETM1 (10nm film thickness), whereas the ETL2 consists of a 1:1 volume% mixture of ETM1 and ETM2 (40nm film thickness). The electron injection layer consists of ETM2 (3nm) and the cathode is aluminum (100nm). The structures are shown in Table 1.

After evaporation, the devices are encapsulated in a glovebox in argon atmosphere.

**Table 1:** Structures of the materials of the solution processed layers.

| HTM1 | |
|---|---|
| 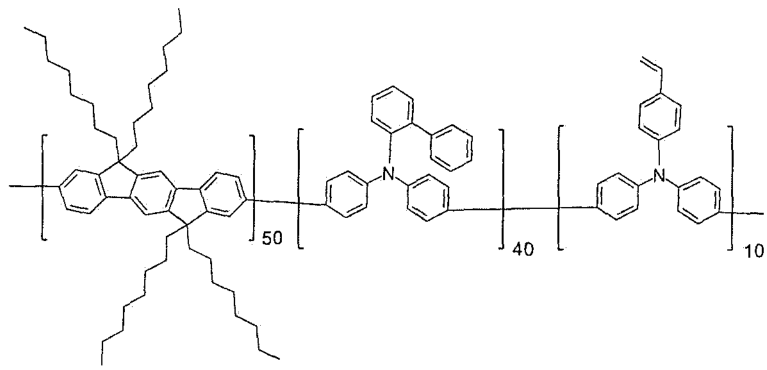 | |
| HTM2 | |
| 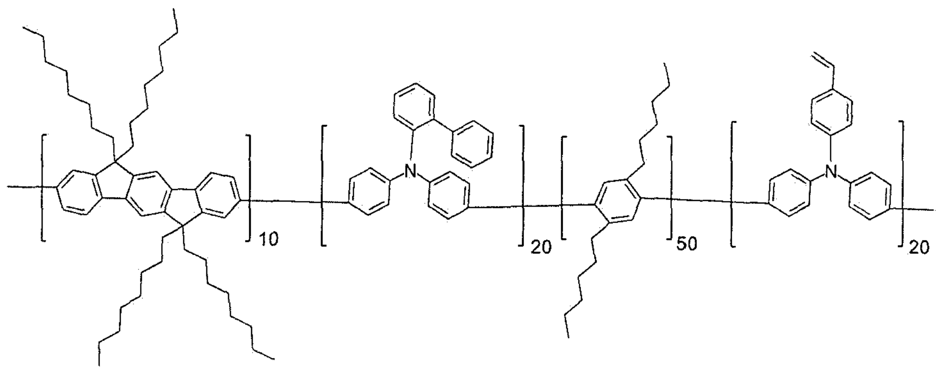 | |
| ETM1 | ETM2 |

(continued)

| | |
|---|---|
| 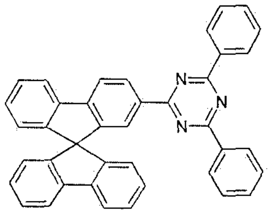 | 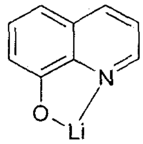 |

**b) Evaluation of emissive film homogeneity**

For the production of displays, it is very important to get a very good pixel homogeneity while having good device performance at the same time. Layer thickness inhomogeneities cause uneven luminance distributions with areas of thinner film thickness showing increased luminance and thicker areas with reduced luminance. Such inhomogeneities vary from pixel to pixel thereby prohibiting a reproduceable appearance among the pixels. In combination, this will lead to a negative perception of such a display's quality. Therefore, the present invention addresses the topic of EML film homogeneity and device performance. The first step for the evaluation is thereby the examination of the film homogeneity. For this the stack shown in Figure 4a is used and the processing is stopped after the EML deposition. The films are prepared as described in part a). The composition of the EML is shown in Table 2-A and Table 2-B.

In order to assess the homogeneity of the printed films, their topography is characterized along an 8μm profile by a profilometer and the Rp-v (peak-to-valley) value as well as the root mean square deviation of the roughness are calculated. A profile-meter Alpha-step D120 from KLA-Tencor Corporation with a 2 μm stylus is used to measure the film profiles. The Rp-v values correspond to the height differences of the measured maximum (Rp) and minimum peaks (Rv) within the measured profiles. For ease of visibility, the baseline of the film profiles is subtracted such that the minimum peak corresponds to a height of 0nm and the axis scales are the same for all diagrams.

The following two Formulas are used to determine the film homogeneity: The peak-to-valley Rp-v, which indicates the maximum height difference within the layer (Formula 1) and the root-mean-squared roughness RMS, which uses the root-mean-squared values of the height differences to the mean line $z_i$ (Formula 2).

$$R(p-v) = Rp - Rv \qquad \text{Formula 1}$$

$$RMS = \sqrt{\frac{1}{n}\sum_{i=1}^{n} z_i^2} \qquad \text{Formula 2}$$

**Table 2-A.** Film profiles and corresponding figures.

| Profile Example | EML composition | | | Rp-v [nm] | RMS [nm] | Figure |
|---|---|---|---|---|---|---|
| | H2 type | H1 type | Emitter | | | |
| PR1 | | A1 (99%) | E4 (1%) | 4.62 | 1.02 | 1 |
| PR2 | H2-4 (99%) | | E4 (1%) | 26.33 | 7.66 | 2 |
| PE1 | H2-4 (69%) | A1 (30%) | E4 (1%) | 4.18 | 1.01 | 3 |

The example PE1, which comprise a host mixture according to the invention, shows a significantly reduced Rp-v and RMS compared to PR2 and corresponds to a much smoother film (Figure 2 and 3).

Furthermore, the example PE1 also shows a similar Rp-v and RMS compared to PR1, while leading to better OLED performance as shown below (see Table 5f, Reference 11 and Example 14).

Further film homogeneities of additional emitting layers (EMLs) are shown in Table 2-B below and the performances of the OLEDs comprising the corresponding EMLs are shown in Tables 5a to 5k.

**Table 2-B.** Further film profiles

| Profile Example | EML composition | | | Rp-v [nm] | RMS [nm] |
|---|---|---|---|---|---|
| | H2 type | H1 type | Emitter | | |
| PR3 | | B2 (99%) | E1 (1%) | 5.01 | 1.12 |
| PR4 | H2-1 (99%) | | E1 (1%) | 21.64 | 7.01 |
| PE2 | H2-1 (79%) | B2 (20%) | E1 (1%) | 3.65 | 1.06 |
| | | | | | |
| PE3 | H2-4 (49%) | A1 (50%) | E4 (1%) | 3.31 | 1.04 |
| | | | | | |
| PR5 | | A10 (97%) | E1 (3%) | 3.02 | 0.99 |
| PR6 | H2-41 (97%) | | E1 (3%) | 12.52 | 5.25 |
| PE4 | H2-41 (67%) | A10 (30%) | E1 (3%) | 4.94 | 1.08 |
| | | | | | |
| PR7-5 | | A1 (95%) | E2 (5%) | 2.83 | 0.67 |
| PR8-6 | H2-49 (95%) | | E2 (5%) | 42.36 | 9.42 |
| PE5-4 | H2-49 (45%) | A1 (50%) | E2 (5%) | 3.95 | 1.26 |
| | | | | | |
| PR9-7 | A4 (99%) | | E3 (1%) | 4.24 | 1.12 |
| PR10-8 | H2-49 (99%) | | E3 (1%) | 30.42 | 7.86 |
| PE6-5 | H2-49 (69%) | A4 (30%) | E3 (1%) | 4.27 | 1.18 |
| | | | | | |
| PR11-9 | | B2 (99%) | E3 (1%) | 4.65 | 1.14 |
| PR12-10 | H2-2 (99%) | | E3 (1%) | 25.98 | 7.54 |
| PE7-6 | H2-2 (89%) | B2 (30%) | E3 (1%) | 3.72 | 0.81 |
| | | | | | |
| PR13-11 | | B8 (97%) | E4 (3%) | 2.99 | 0.89 |
| PR14-12 | H2-4 (97%) | | E4 (3%) | 23.11 | 6.84 |
| PE8-7 | H2-4 (57%) | B8 (40%) | E4 (3%) | 3.71 | 1.21 |
| | | | | | |
| PR15-13 | | A11 (95%) | E2 (5%) | 4.24 | 0.97 |
| PR16-14 | H2-4 (95%) | | E2 (5%) | 24.82 | 8.20 |
| PE9-8 | H2-4 (55%) | A11 (40%) | E2 (5%) | 3.57 | 0.96 |
| | | | | | |
| PR17-15 | | A11 (97%) | E3 (3%) | 2.98 | 1.14 |
| PR18-16 | D1 (97%) | | E3 (3%) | 26.89 | 5.66 |
| PE10-9 | D1 (67%) | A11 (30%) | E3 (3%) | 3.01 | 1.23 |
| | | | | | |
| PR19-17 | | C3 (99%) | E4 (1%) | 4.16 | 0.92 |
| PR20-18 | C1 (99%) | | E4 (1%) | 25.28 | 6.06 |
| PE11-10 | C1 (69%) | C3 (30%) | E4 (1%) | 3.61 | 1.05 |

(continued)

| Profile Example | EML composition | | | Rp-v [nm] | RMS [nm] |
|---|---|---|---|---|---|
| | H2 type | H1 type | Emitter | | |
| | | | | | |
| PR21 | | B8 (97%) | E4 (3%) | 3.25 | 0.69 |
| PR22 | H2-15 (97%) | | E4 (3%) | 24.99 | 7.10 |
| PE12 | H2-15 (67%) | B8 (30%) | E4 (3%) | 3.34 | 0.92 |

For the emitting layers having the profiles PE1, PE2, PE3, PE4, PE5, PE6, PE7, PE8, PE9, PE11 and PE12, the same discussion as for PE1 above is valid.

The performances of devices employing the mixed host EMLs having the profiles PE1 to PE12 can be compared to other devices (see Tables 5a to 5k).

**c) Device results**

The devices like shown in Figure 4b are prepared as described in part a).

The host materials are shown in Table 3 and the emitters in Table 4. The blue EML ink is mixed as shown in Tables 5a-k, in which also the relative external quantum efficiencies (rel. EQE) at 1000 cd/m$^2$ and the relative device lifetimes (rel. LT90 at 1000 cd/m$^2$) are shown for the respective examples.

**Table 3:** Structure hosts

| H1 type | | H2 type | |
|---|---|---|---|
| A1 | 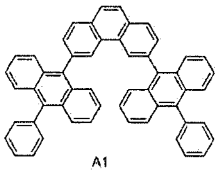 A1 | H2-1 | 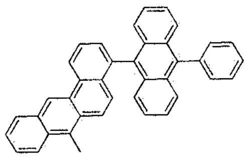 CAS 1818872-74-0 |
| A4 | 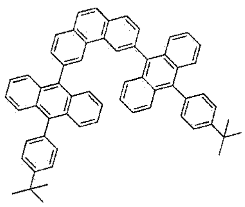 | H2-2 | 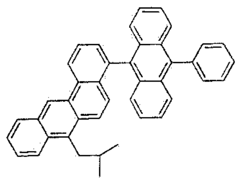 CAS 1818872-85-3 |
| A8 | 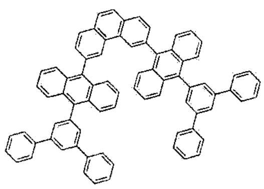 | H2-4 | 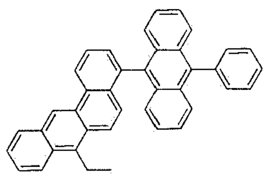 CAS 1818872-84-2 |
| A10 | 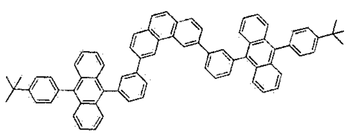 | H2-15 | 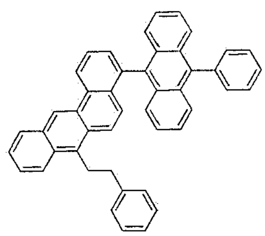 CAS 1818872-89-7 |

(continued)

| H1 type | | H2 type | |
|---|---|---|---|
| B2 | 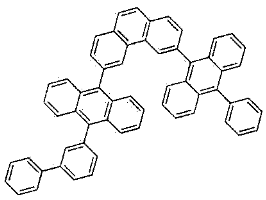 | H2-41 | 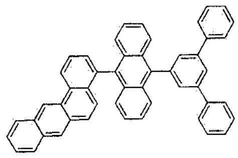 CAS 1273319-88-2 |
| | | | |
| B8 | 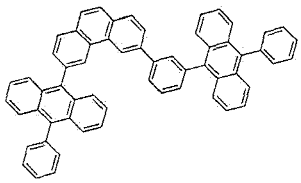 | H2-49 | 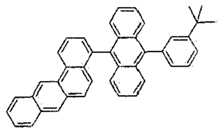 |
| C3 | 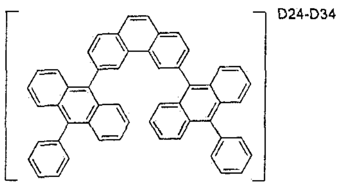 | C1 | 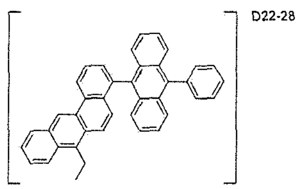 |
| A11 | 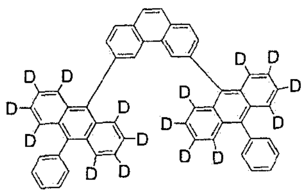 | D1 | 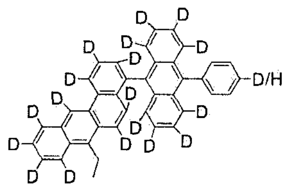 |

**Table 4:** Structure emitters

| Emitter | structure |
|---|---|
| E1 | 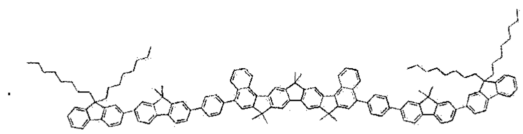 |
| E2 | 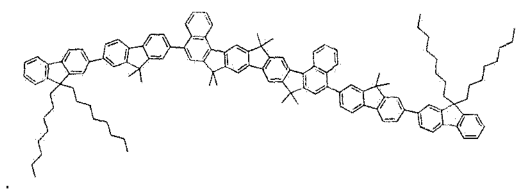 |
| E3 | 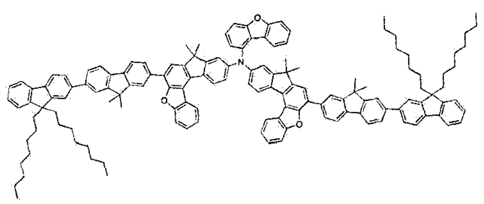 |

(continued)

| Emitter | structure |
|---|---|
| E4 | 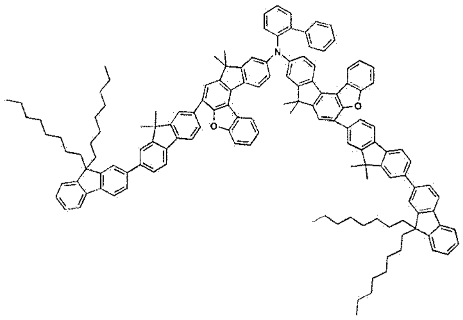 |

After the encapsulation in a glovebox, the OLEDs are characterized by standard methods. For this purpose, the electroluminescence spectra, current/voltage/luminance characteristic curves (IUL characteristic curves) assuming Lambert emission characteristics and the (operating) lifetimes are determined. The IUL characteristic curves are used to determine characteristic figures of merit such as external quantum efficiency (in %) at a certain luminance. The device is driven with constant voltages, at each step of an applied voltage ramp. The device lifetime is measured under a given current with an initial luminance. The luminance is then measured over time by a calibrated photodiode.

**Table 5a:** Blue EML mixtures to use for device examples with 1% E1

| Device Example | HTM | H2 type | % | H1 type | | % D | % | Profil Example | rel. EQE at 1000cd/$m^2$ | rel. LT90 at 1000cd/$m^2$ |
|---|---|---|---|---|---|---|---|---|---|---|
| Reference 1 | HTM1 | | | B2 | 99 | E1 | 1 | PR3 | 0.89 | 0.68 |
| Reference 2 | HTM1 | H2-1 | 99 | | | E1 | 1 | PR4 | 1.00 | 1.00 |
| Example 1 | HTM1 | H2-1 | 79 | B2 | 20 | E1 | 1 | PE2 | 0.98 | 1.44 |
| Example 2 | HTM1 | H2-1 | 69 | B2 | 30 | E1 | 1 | | 0.98 | 1.26 |
| Example 3 | HTM1 | H2-1 | 59 | B2 | 40 | E1 | 1 | | 1.01 | 1.31 |

**Table 5b:** Blue EML mixtures to use for device examples with 3% E1

| Example | Device HTM H2 % H1 | type | | type | % | D | % | Profil Example | rel. EQE at 1000cd/$m^2$ | rel. LT90 at 1000cd/$m^2$ |
|---|---|---|---|---|---|---|---|---|---|---|
| Reference 3 | HTM1 | | | A10 | 97 | E1 | 3 | PR5 | 0.81 | 0.59 |
| Reference 4 | HTM1 | H2-41 | 97 | | | E1 | 3 | PR6 | 1.00 | 1.00 |
| Example 4 | HTM1 | H2-41 | 67 | A10 | 30 | E1 | 3 | PE4 | 0.96 | 1.07 |
| Example 5 | HTM1 | H2-41 | 47 | A10 | 50 | E1 | 3 | | 0.95 | 1.09 |

**Table 5c:** Blue EML mixtures to use for device examples with 5% E2

| Device Example | HTM | H2 type | % | H1 type | % | D | % | Profil Example | rel. EQE at 1000cd/$m^2$ | rel. LT90 at 1000cd/$m^2$ |
|---|---|---|---|---|---|---|---|---|---|---|
| Reference 5 | HTM2 | | | A1 | 95 | E2 | 5 | PR7 | 1.00 | 1.20 |

(continued)

| Device Example | HTM | H2 type | % | H1 type | % | D | % | Profil Example | rel. EQE at 1000cd/m2 | rel. LT90 at 1000cd/m2 |
|---|---|---|---|---|---|---|---|---|---|---|
| Reference 6 | HTM2 | H2-49 | 95 | | | E2 | 5 | PR8 | 1.00 | 1.00 |
| Example 6 | HTM2 | H2-49 | 65 | A1 | 30 | E2 | 5 | | 0.98 | 1.42 |
| Example 7 | HTM2 | H2-49 | 45 | A1 | 50 | E2 | 5 | PE5 | 1.03 | 1.51 |

**Table 5d:** Blue EML mixtures to use for device examples with 1% E3

| Device Example | HTM | H2 type | % | H1 type | | | % D % | Profil Example | rel. EQE at 1000cd/m2 | rel. LT90 at 1000cd/m2 |
|---|---|---|---|---|---|---|---|---|---|---|
| Reference 7 | HTM1 | | | A4 | 99 | E3 | 1 | PR9 | 1.00 | 1.64 |
| Reference 8 | HTM 1 | H2-49 | 99 | | | E3 | 1 | PR10 | 1.00 | 1.00 |
| Example 8 | HTM1 | H2-49 | 69 | A4 | 30 | E3 | 1 | PE6 | 1.15 | 1.79 |
| Example 9 | HTM1 | H2-49 | 49 | A4 | 50 | E3 | 1 | | 1.13 | 2.12 |

**Table 5e:** Blue EML mixtures to use for device examples with 1% E3

| Device Example | HTM | H2 type | % | H1 type | % | D | % | Profile Example | rel. EQE at 1000cd/m2 | rel. LT90 at 1000cd/m2 |
|---|---|---|---|---|---|---|---|---|---|---|
| Reference 9 | HTM2 | | | B2 | 99 | E3 | 1 | PR11 | 0.93 | 0.98 |
| Reference 10 | HTM2 | H2-2 | 99 | | | E3 | 1 | PR12 | 1.00 | 1.00 |
| Example 10 | HTM2 | H2-2 | 89 | B2 | 10 | E3 | 1 | PE7 | 1.03 | 0.98 |
| Example 11 | HTM2 | H2-2 | 69 | B2 | 30 | E3 | 1 | | 0.98 | 1.30 |
| Example 12 | HTM2 | H2-2 | 49 | B2 | 50 | E3 | 1 | | 1.05 | 1.25 |

**Table 5f:** Blue EML mixtures to use for device examples with 1% E4

| Device Example | HTM | H2 type | % | H1 type | % | D | % | Profil Example | rel. EQE at 1000cd/m2 | rel. LT90 at 1000cd/m2 |
|---|---|---|---|---|---|---|---|---|---|---|
| Reference 11 | HTM2 | | | A1 | 99 | E4 | 1 | PR1 | 0.85 | 0.80 |
| Reference 12 | HTM2 | H2-4 | 99 | | | E4 | 1 | PR2 | 1.00 | 1.00 |
| Example 13 | HTM2 | H2-4 | 89 | A1 | 10 | E4 | 1 | | 0.95 | 1.10 |
| Example 14 | HTM2 | H2-4 | 69 | A1 | 30 | E4 | 1 | PE1 | 1.01 | 1.12 |
| Example 15 | HTM2 | H2-4 | 49 | A1 | 50 | E4 | 1 | PE3 | 0.93 | 1.23 |

**Table 5g:** Blue EML mixtures to use for device examples with 3% E4

| Device Example | HTM | H2 type | % | H1 type | % | D | % | Profil Example | rel. EQE at 1000cd/m$^2$ | rel. LT90 at 1000cd/m$^2$ |
|---|---|---|---|---|---|---|---|---|---|---|
| Reference 13 | HTM2 | | | B8 | 97 | E4 | 3 | PR13 | 0.79 | 0.82 |
| Reference 14 | HTM2 | H2-4 | 97 | | | E4 | 3 | PR14 | 1.00 | 1.00 |
| Example 16 | HTM2 | H2-4 | 57 | B8 | 40 | E4 | 3 | PE8 | 1.02 | 1.02 |

**Table 5h:** Blue EML mixtures to use for device examples with 5% E2

| Device Example | HTM | H2 type | % | H1 type | % | D | % | Profil Example | rel. EQE at 1000cd/m$^2$ | rel. LT90 at 1000cd/m$^2$ |
|---|---|---|---|---|---|---|---|---|---|---|
| Reference 15 | HTM1 | | | A11 | 95 | E2 | 5 | PR15 | 0.78 | 0.84 |
| Reference 16 | HTM1 | H2-4 | 95 | | | E2 | 5 | PR16 | 1.00 | 1,00 |
| Example 17 | HTM1 | H2-4 | 55 | A11 | 40 | E2 | 5 | PE9 | 0.98 | 1.01 |

**Table 5i:** Blue EML mixtures to use for device examples with 3% E3

| Device Example | HTM | H2 type | % | H1 type | % | D | % | Profil Example | rel. EQE at 1000cd/m$^2$ | rel. LT90 at 1000cd/m$^2$ |
|---|---|---|---|---|---|---|---|---|---|---|
| Reference 17 | HTM2 | | | A11 | 97 | E3 | 3 | PR17 | 0.92 | 0.79 |
| Reference 18 | HTM2 | D1 | 97 | | | E3 | 3 | PR18 | 1.00 | 1.00 |
| Example 18 | HTM2 | D1 | 67 | A11 | 30 | E3 | 3 | PE10 | 0.98 | 1.02 |

**Table 5j:** Blue EML mixtures to use for device examples with 1% E4

| Device Example | HTM | H2 type | % | H1 type | % | D | % | Profil Example | rel. EQE at 1000cd/m$^2$ | rel. LT90 a 1000cd/m$^2$ |
|---|---|---|---|---|---|---|---|---|---|---|
| Reference 19 | HTM2 | | | C3 | 99 | E4 | 1 | PR19 | 0.94 | 0.84 |
| Reference 20 | HTM2 | C1 | 99 | | | E4 | 1 | PR20 | 1.00 | 1.00 |
| Example 19 | HTM2 | C1 | 69 | C3 | 30 | E4 | 1 | PE11 | 1.00 | 1.01 |
| Example 20 | HTM2 | C1 | 49 | C3 | 50 | E4 | 1 | | 1.05 | 1.02 |

**Table 5k:** Blue EML mixtures to use for device examples with 3% E4

| Device Example | HTM | H2 type | % | H1 type | % | D | % | Profil Example | rel. EQE at 1000cd/m2 | rel. LT90 at 1000cd/m2 |
|---|---|---|---|---|---|---|---|---|---|---|
| Reference 21 | HTM1 | | | B8 | 97 | E4 | 3 | PR21 | 0.78 | 0.72 |
| Reference 22 | HTM1 | H2-15 | 97 | | | E4 | 3 | PR22 | 1.00 | 1.00 |
| Example 20 | HTM1 | H2-15 | 67 | B8 | 30 | E4 | 3 | PE12 | 0.98 | 0.98 |

All shown examples of a mixed host system show an improved device performance compared to single host type H1, whereas similar performances as with the hosts type H2 can be reached.

This is independent of the used emitter and the used emitter concentration.

As discussed above, the profiles are shown for References 11 and 12 and Example 14 and Example 15 from Table 5f (see Figure 1 to 3). Compared to Reference 11, Examples 13, 14 and 15 according to the invention show an improved device performance, visible in an increased efficiency and an increased lifetime. Compared to Reference 12, which shows highly inhomogeneous films, the films according to the invention are very homogeneous while showing similar device performances.

The same discussion is valid for devices according to the present invention represented in Tables 5a to 5k.

With the help of the content of the invention, it is possible to achieve a good OLED device performance while at the same time ensuring homogeneous film quality.

## Claims

1. Composition comprising a compound of formula (H1) and a compound of formula (H2),

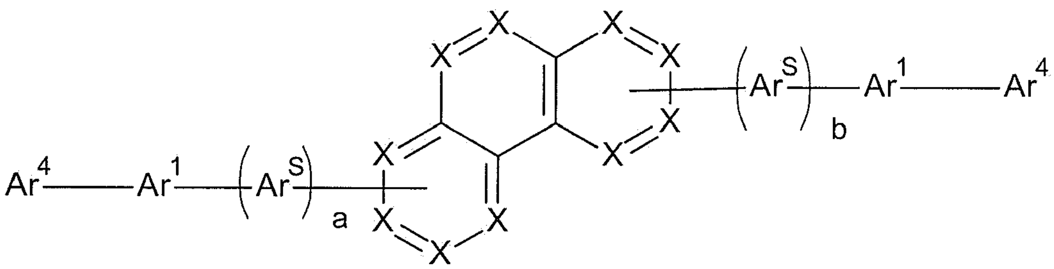

formula (H1)

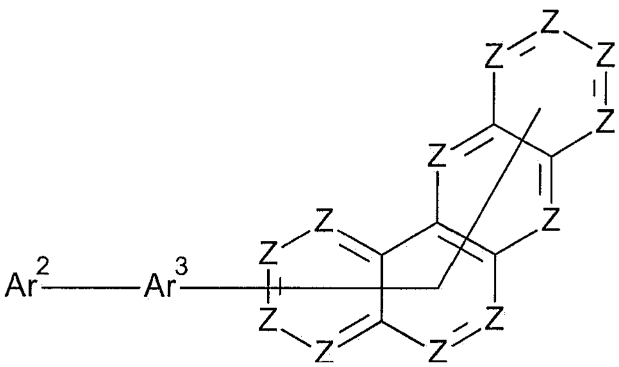

formula (H2)

where the following applies to the symbols and indices used:

X stands on each occurrence, identically or differently, for $CR^X$ or N; and X is C if X is bonded to a group $Ar^1$ or $Ar^5$;
Z stands on each occurrence, identically or differently, for $CR^Z$ or N; and Z is C if Z is bonded to a group $Ar^3$;
$Ar^1$ is, on each occurrence, identically or differently, an aryl or heteroaryl group having 10 to 60 aromatic ring atoms, which may in each case also be substituted by one or more radicals $R^V$;
$Ar^3$ is, on each occurrence, identically or differently, an aryl or heteroaryl group having 10 to 60 aromatic ring atoms, which may in each case also be substituted by one or more radicals $R^Y$;
$Ar^2$, $Ar^4$, $Ar^S$ are, on each occurrence, identically or differently, an aromatic or heteroaromatic ring system having 5 to 60 aromatic ring atoms, which may in each case also be substituted by one or more radicals R;
$R^V$, $R^X$, $R^Y$, $R^Z$ stand on each occurrence, identically or differently, for H, D, F, Cl, Br, I, CHO, CN, C(=O)Ar, $P(=O)(Ar)_2$, S(=O)Ar, $S(=O)_2Ar$, $N(R)_2$, $N(Ar)_2$, $NO_2$, $Si(R)_3$, $B(OR)_2$, $OSO_2R$, a straight-chain alkyl, alkoxy or thioalkyl group having 1 to 40 C atoms or branched or a cyclic alkyl, alkoxy or thioalkyl group having 3 to 40 C atoms, each of which may be substituted by one or more radicals R, where in each case one or more non-adjacent $CH_2$ groups may be replaced by RC=CR, C=C, $Si(R)_2$, $Ge(R)_2$, $Sn(R)_2$, C=O, C=S, C=Se, P(=O)(R), SO, $SO_2$, O, S or CONR and where one or more H atoms may be replaced by D, F, Cl, Br, I, CN or $NO_2$, an aromatic or heteroaromatic ring system having 5 to 60 aromatic ring atoms, which may in each case be substituted by one or more radicals R, or an aryloxy group having 5 to 60 aromatic ring atoms, which may be substituted by one or more radicals R;
where two adjacent radicals $R^V$, two adjacent radicals $R^X$, two adjacent radicals $R^Y$, two adjacent radicals $R^Z$ may form an aliphatic, aromatic or heteroaromatic ring system together, which may be substituted by one or more radicals R;
R stands on each occurrence, identically or differently, for H, D, F, Cl, Br, I, CHO, CN, C(=O)Ar, $P(=O)(Ar)_2$, S(=O)Ar, $S(=O)_2Ar$, $N(R')_2$, $N(Ar)_2$, $NO_2$, $Si(R')_3$, $B(OR')_2$, $OSO_2R'$, a straight-chain alkyl, alkoxy or thioalkyl group having 1 to 40 C atoms or branched or a cyclic alkyl, alkoxy or thioalkyl group having 3 to 40 C atoms, each of which may be substituted by one or more radicals R', where in each case one or more non-adjacent $CH_2$ groups may be replaced by R'C=CR', C=C, $Si(R')_2$, $Ge(R')_2$, $Sn(R')_2$, C=O, C=S, C=Se, P(=O)(R'), SO, $SO_2$, O, S or CONR' and where one or more H atoms may be replaced by D, F, Cl, Br, I, CN or $NO_2$, an aromatic or heteroaromatic ring system having 5 to 60 aromatic ring atoms, which may in each case be substituted by one or more radicals R', or an aryloxy group having 5 to 60 aromatic ring atoms, which may be substituted by one or more radicals R'; where two adjacent substituents R may form an aliphatic or aromatic ring system together, which may be substituted by one or more radicals R';
Ar is, on each occurrence, identically or differently, an aromatic or heteroaromatic ring system having 5 to 60 aromatic ring atoms, which may in each case also be substituted by one or more radicals R';
R' stands on each occurrence, identically or differently, for H, D, F, Cl, Br, I, CN, a straight-chain alkyl, alkoxy or thioalkyl group having 1 to 20 C atoms or branched or cyclic alkyl, alkoxy or thioalkyl group having 3 to 20 C atoms, where in each case one or more non-adjacent $CH_2$ groups may be replaced by SO, $SO_2$, O, S and where one or more H atoms may be replaced by D, F, Cl, Br or I, or an aromatic or heteroaromatic ring system having 5 to 24 aromatic ring atoms; and
a, b are on each occurrence, identically or differently, 0 or 1; wherein when a or b is 0, then the corresponding $Ar^S$ is absent and the group $Ar^1$ is directly bonded to a group X.

2. Composition according to claim 1, **characterized in that** the compound of formula (H2) is selected from the compounds of formula (H2-1),

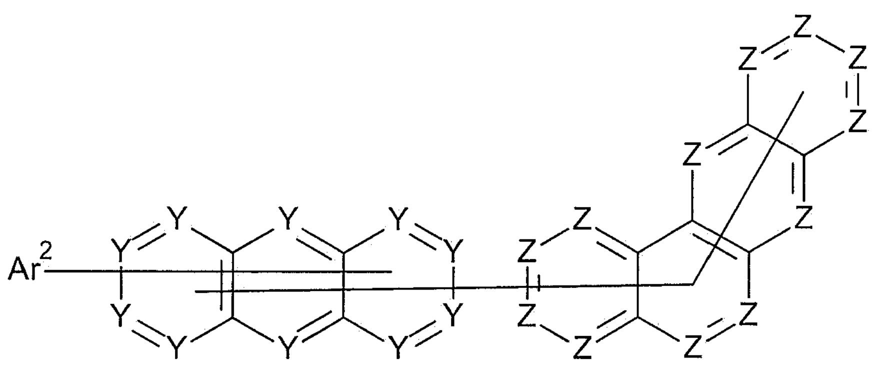

formula (H2-1)

where the symbol $Ar^2$ and Z have the same meaning as in claim 1; and
Y is $CR^Y$ or N; or Y is C if bonded to $Ar^2$ or a group Z; where $R^Y$ has the same meaning as in claim 1.

**3.** Composition according to claim 1 or 2, **characterized in that** the compound of formula (H2) is selected from the compounds of formula (H2-2),

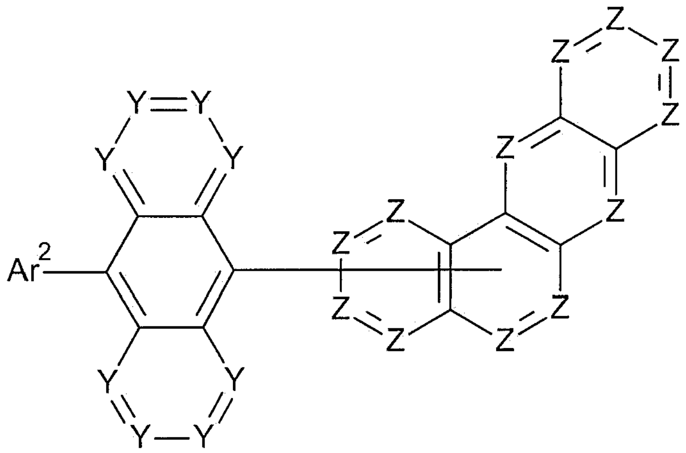

formula (H2-2)

where $Ar^2$ and Z have the same meaning as in claim 1 and Y has the same meaning as in claim 2.

**4.** Composition according to one or more of the preceding claims, **characterized in that** the compound of formula (H2) is selected from the compounds of formula (H2-3),

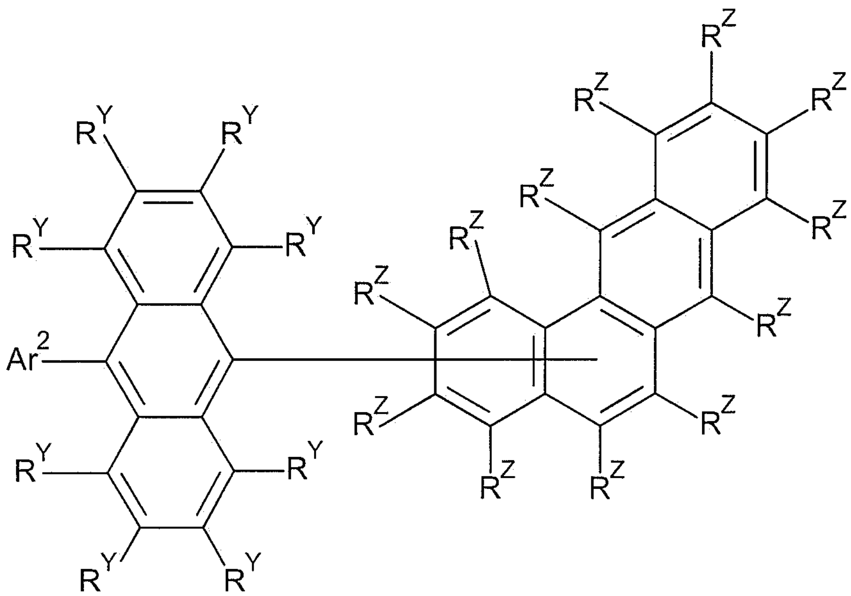

formula (H2-3)

where the symbols have the same meaning as in claim1, with the proviso that the group $CR^Z$ correspond to a group C at the bonding position of the adjacent anthracene.

**5.** Composition according to one or more of the preceding claims, **characterized in that** the compound of formula (H2) is selected from the compounds of formula (H2-4),

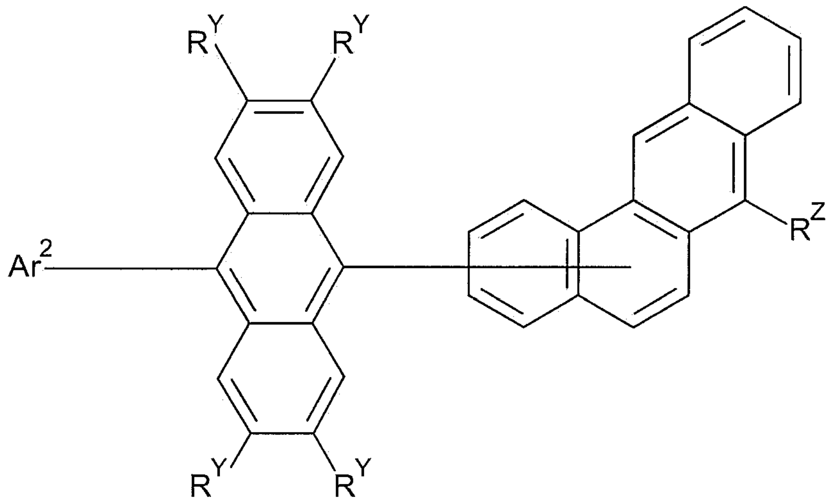

formula (H2-4)

where the symbols have the same meaning as in claim 1.

6. Composition according to one or more of the preceding claims, **characterized in that** the compound of formula (H2) is selected from the compounds of formula (H2-5),

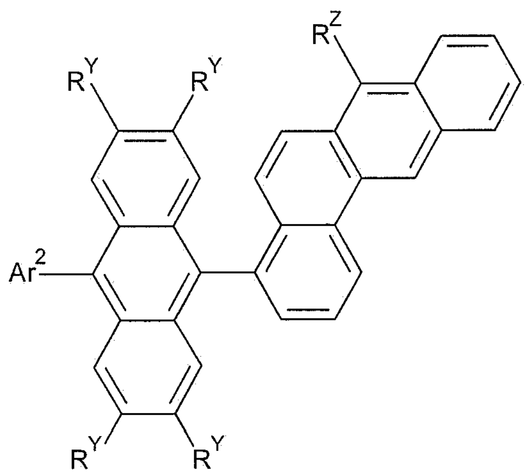

formula (H2-5)

where the symbols have the same meaning as in claim 1.

7. Composition according to one or more of the preceding claims, **characterized in that** the compound of formula (H1) is selected from the compounds of formula (H1-1),

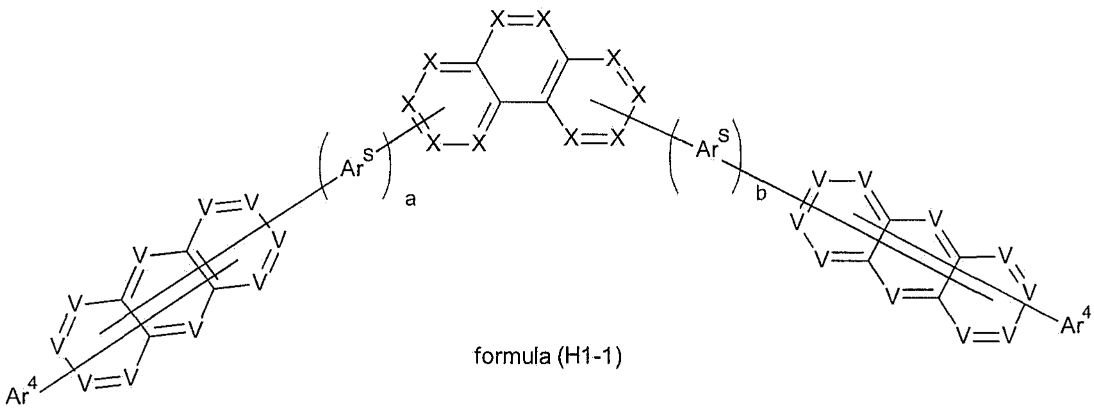

formula (H1-1)

where X, $Ar^S$, $Ar^4$ and the indices a and b have the same meaning as in claim 1; and
V is $CR^V$ or N; or V is C if bonded to $Ar^4$, $Ar^S$ or a group X; where $R^V$ has the same meaning as in claim 1.

**8.** Composition according to one or more of the preceding claims, **characterized in that** the compound of formula (H1) is selected from the compounds of formula (H1-2),

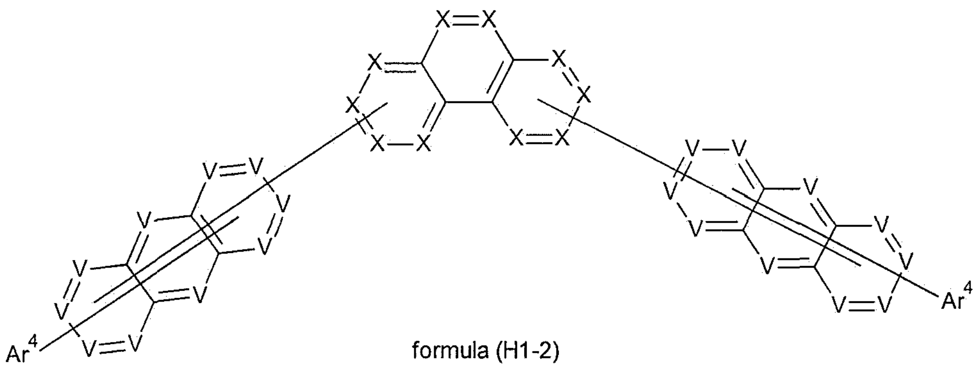

formula (H1-2)

where X and $Ar^4$ have the same meaning as in claim 1; and
V has the same meaning as in claim 7.

**9.** Composition according to one or more of the preceding claims the compound of formula (H1) is selected from the compounds of formula (H1-3),

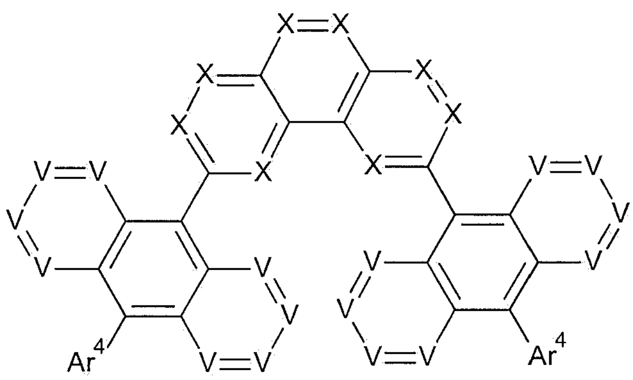

formula (H1-3)

where the symbols have the same meaning as in claims 1 and 7.

**10.** Composition according to one or more of the preceding claims, **characterized in that** the compound of formula (H1) is selected from the compounds of formula (H1-4),

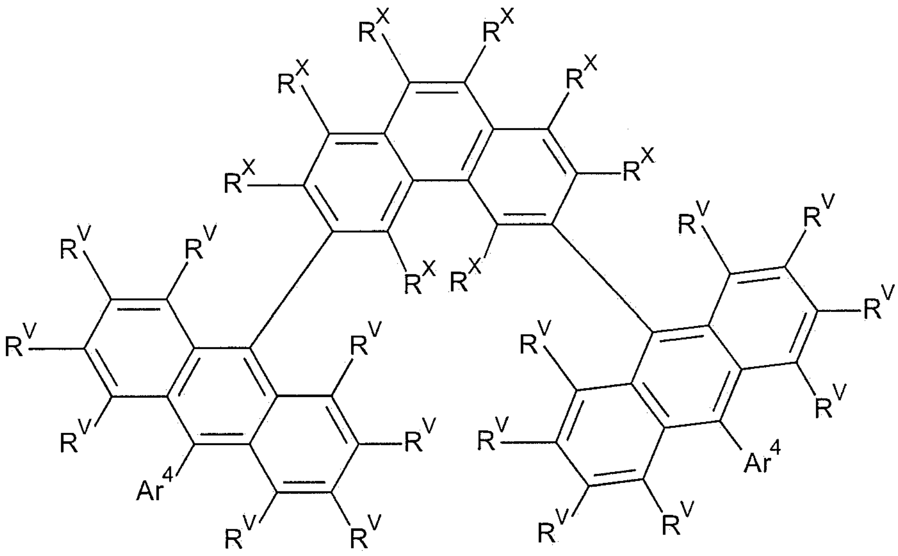

formula (H1-4)

where the symbols have the same meaning as in claim 1.

**11.** Composition according to one or more of the preceding claims, **characterized in that** the compound of formula (H1) is selected from the compound of formula (H1-5),

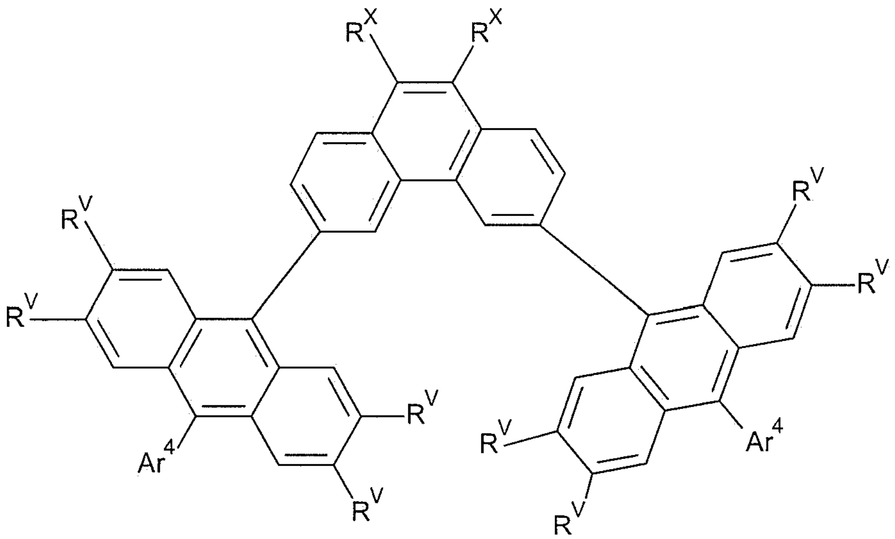

formula (H1-5)

where the symbols have the same meaning as in claim 1.

**12.** Composition according to one or more of the preceding claims, **characterized in that** the groups $Ar^2$, $Ar^4$ are on each occurrence, consisting of phenyl, biphenyl, terphenyl, quaterphenyl, fluorene, spirobifluorene, naphthalene, anthracene, phenanthrene, triphenylene, fluoranthene, tetracene, chrysene, benzanthracene, benzophenanthracene, pyrene or perylene, dibenzofuran, carbazole and dibenzothiophene, each of which may be substituted by one or more radicals R at any free positions; and where $Ar^2$, $Ar^4$ might also be a combination of two or more of the previously cited groups.

**13.** Composition according to one or more of the preceding claims,

**characterized in that** it further comprises a fluorescent emitter.

**14.** Composition according to one or more of the preceding claims,
**characterized in that** it comprises a fluorescent emitter selected from the group consisting of:

- an arylamine containing three substituted or unsubstituted aromatic or heteroaromatic ring systems bonded directly to the nitrogen;
- a condensed aromatic or heteroaromatic ring system having at least 14 aromatic ring atoms;
- an indenofluorene, indenofluorenamine or indenofluorenediamine;
- a benzoindonofluorene, benzoindenofluorenamine or benzoindenofluorenediamine;
- a dibenzoindenofluorene, dibenzoindenofluorenamine or dibenzoindenofluorenediamine;
- an indenofluorene containing a condensed aryl group having at least 10 aromatic ring atoms;
- a bisindenoindenofluorene;
- an indenodibenzofuran; indenofluorenamine or indenofluorenediamine;
- a fluorene dimer;
- a phenoxazine; and
- a boron derivative.

**15.** Composition according to one or more of the preceding claims, **characterized in that** it comprises a fluorescent emitter of formula (E-1), (E-2) or (E-3),

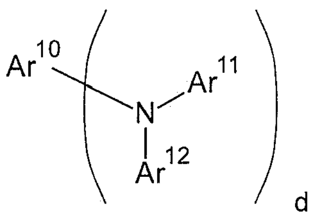

Formula (E-1)

where

$Ar^{10}$, $Ar^{11}$, $Ar^{12}$ are on each occurrence, identically or differently, an aromatic or heteroaromatic ring system having 6 to 60 aromatic ring atoms, which may in each case also be substituted by one or more radicals R; with the proviso that at least one group $Ar^{10}$, $Ar^{11}$, $Ar^{12}$ is an aromatic or heteroaromatic ring system having 10 to 40 aromatic ring atoms, containing at least one condensed aryl or heteroaryl group consisting of 2 to 4 aromatic rings condensed with one another, where the aromatic or heteroaromatic ring system may be substituted by one or more radicals R;
R has the same definition as in claim 1 ; and
d is 1, 2, 3 or 4; more preferably, d is 1;

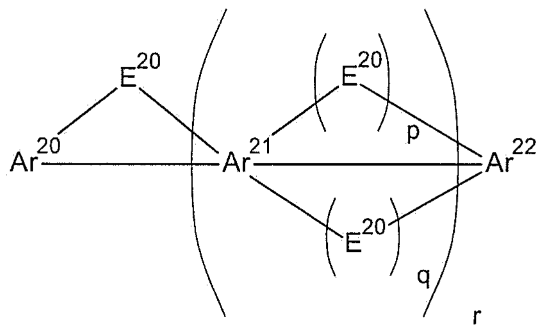

Formula (E-2)

where
$Ar^{20}$, $Ar^{21}$, $Ar^{22}$ are on each occurrence, identically or differently, an aryl or heteroaryl group having 6 to 30

aromatic ring atoms, which may in each case also be substituted by one or more radicals R;
$E^{20}$ is on each occurrence, identically or differently a group selected from BR, $C(R^0)_2$, $Si(R^0)_2$, C=O, $C=NR^0$, $C=C(R^0)_2$, O, S, S=O, $SO_2$, $NR^0$, $PR^0$, $P(=O)R^0$ or $P(=S)R^0$; wherein $Ar^{20}$, $Ar^{21}$ and $E^{20}$ together form a five-membered ring or a six-membered ring, and $Ar^{21}$, $Ar^{23}$ and $E^{20}$ together form a five-membered ring or a six-membered ring;
$R^0$ stands on each occurrence, identically or differently, for H, D, F, a straight-chain alkyl group having 1 to 20 , preferably 1 to 10 C atoms or branched or a cyclic alkyl group having 3 to 20, preferably 3 to 10 C atoms, each of which may be substituted by one or more radicals R, where in each case one or more non-adjacent $CH_2$ groups may be replaced by O or S and where one or more H atoms may be replaced by D or F, or an aromatic or heteroaromatic ring systems having 5 to 40, preferably 5 to 30, more preferably 6 to 18 aromatic ring atoms, which may in each case be substituted by one or more radicals R, where two adjacent radicals $R^0$, may form an aliphatic or aromatic ring system together, which may be substituted by one or more radicals R,
R has the same definition as in claim 1;
p, q are on each occurrence, identically or differently, 0 or 1, with the proviso that $p + q = 1$;
r is 1, 2 oder 3;

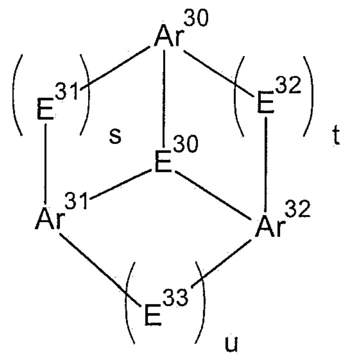

Formula (E-3)

where
$Ar^{30}$, $Ar^{31}$, $Ar^{32}$ stand on each occurrence, identically or differently, for a substituted or unsubstituted aryl or heteroaryl group having 5 to 22, preferably 5 to 18, more preferably 6 to 14 aromatic ring atoms;
$E^{30}$ stands for B or N;

$E^{31}$, $E^{32}$, $E^{33}$ stand on each occurrence, identically or differently, for O, S, $C(R^0)_2$, C=O, C=S, $C=NR^0$, $C=C(R^0)_2$, $Si(R^0)_2$, $BR^0$, $NR^0$, $PR^0$, $SO_2$, $SeO_2$ or a chemical bond, with the proviso that if $E^{30}$ is B, then at least one of the groups $E^{31}$, $E^{32}$, $E^{33}$ stands for $NR^0$ and if $E^{30}$ is N, then at least one of the groups $E^{31}$, $E^{32}$, $E^{33}$ stands for $BR^0$;
$R^0$ has the same definition as above;
s, t, u are on each occurrence, identically or differently, 0 or 1, with the proviso that $s + t + u \geq 1$.

16. Composition according to one or more of the preceding claims, **characterized in that** the compound of formula (H1) is present in the composition in a proportion of 1 to 60 % and the compound of formula (H2) is present in the composition in a proportion of 30 to 99 %.

17. Formulation comprising at least one composition according to one or more of the claims 1 to 16 and at least one solvent.

18. Process for the production of an electronic device comprising at least one layer comprising a composition according to one or more of the claims 1 to 16:

a) Preparation of a formulation comprising at least one composition according to one or more of the claims 1 to 16 and at least one solvent;
b) Application of the formulation prepared in step a) on a substrate or on another layer in order to form a layer;
c) Drying of the layer in order to remove the solvent.

**19.** Process according to claim 18, **characterised in that** the formulation is applied by a coating method or a printing method.

**20.** Process according to claim 18 or 19, **characterized in that** the formulation is applied by flood coating, dip coating, spray coating, spin coating, screen printing, relief printing, gravure printing, roller coating, inkjet printing, rotary printing, flexographic printing, offset printing, slot die coating or nozzle printing.

**21.** Electronic device comprising anode, cathode, and at least one emitting layer, where the emitting layer comprises a composition according to one or more of claims 1 to 16.

**22.** Electronic device according to claim 21, selected from the group consisting of organic electroluminescent devices, organic integrated circuits, organic field-effect transistors, organic thin-film transistors, organic light-emitting transistors, organic solar cells, dye-sensitised organic solar cells, organic optical detectors, organic photoreceptors, organic field-quench devices, light-emitting electrochemical cells, organic laser diodes and organic plasmon emitting devices.

**Patentansprüche**

**1.** Zusammensetzung, umfassend eine Verbindung der Formel (H1) und eine Verbindung der Formel (H2)

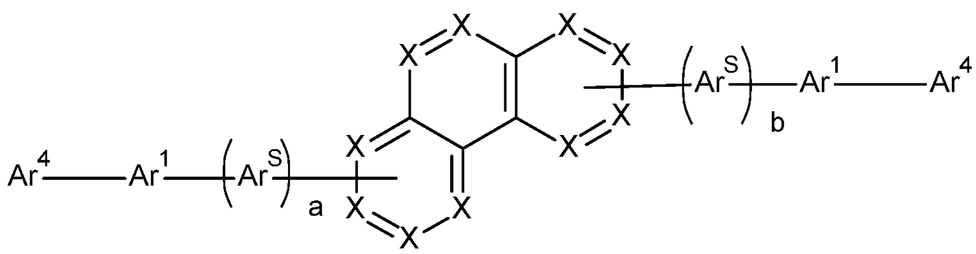

Formel (H1)

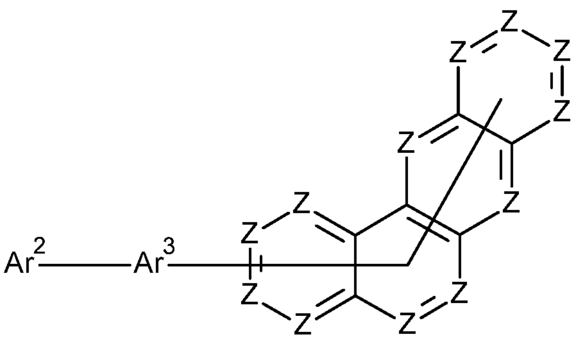

Formel H2

wobei für die verwendeten Symbole und Indizes Folgendes gilt:

X steht bei jedem Auftreten gleich oder verschieden für $CR^X$ oder N; und X steht für C, wenn X an eine Gruppe $Ar^1$ oder $Ar^S$ gebunden ist;

Z steht bei jedem Auftreten gleich oder verschieden für $CR^Z$ oder N; und Z steht für C, wenn Z an eine Gruppe $Ar^3$ gebunden ist;

$Ar^1$ steht bei jedem Auftreten gleich oder verschieden für eine Aryl- oder Heteroarylgruppe mit 10 bis 60 aromatischen Ringatomen, die jeweils auch durch einen oder mehrere Reste $R^V$ substituiert sein kann;

$Ar^3$ steht bei jedem Auftreten gleich oder verschieden für eine Aryl- oder Heteroarylgruppe mit 10 bis 60 aromatischen Ringatomen, die jeweils auch durch einen oder mehrere Reste $R^Y$ substituiert sein kann;

$Ar^2$, $Ar^4$, $Ar^S$ stehen bei jedem Auftreten gleich oder verschieden für ein aromatisches oder heteroaromatisches Ringsystem mit 5 bis 60 aromatischen Ringatomen, das jeweils auch durch einen oder mehrere Reste R substituiert sein kann;

$R^V$, $R^X$, $R^Y$, $R^Z$ stehen bei jedem Auftreten gleich oder verschieden für H, D, F, Cl, Br, I, CHO, CN, C(=O)Ar, $P(=O)(Ar)_2$, S(=O)Ar, $S(=O)_2Ar$, $N(R)_2$, $N(Ar)_2$, $NO_2$, $Si(R)_3$, $B(OR)_2$, $OSO_2R$, eine geradkettige Alkyl-, Alkoxy- oder Thioalkylgruppe mit 1 bis 40 C-Atomen oder eine verzweigte oder cyclische Alkyl-, Alkoxy- oder Thioalkylgruppe mit 3 bis 40 C-Atomen, wobei jede dieser Gruppen durch einen oder mehrere Reste R substituiert sein kann, wobei jeweils eine oder mehrere nicht benachbarte $CH_2$-Gruppen durch RC=CR, C=C, $Si(R)_2$, $Ge(R)_2$, $Sn(R)_2$, C=O, C=S, C=Se, P(=O)(R), SO, $SO_2$, O, S oder CONR ersetzt sein können und wobei ein oder mehrere H-Atome durch D, F, Cl, Br, I, CN oder $NO_2$ ersetzt sein können, ein aromatisches oder heteroaromatisches Ringsystem mit 5 bis 60 aromatischen Ringatomen, das jeweils durch einen oder mehrere Reste R substituiert sein kann, oder eine Aryloxygruppe mit 5 bis 60 aromatischen Ringatomen, die durch einen oder mehrere Reste R substituiert sein kann;

wobei zwei benachbarte Reste $R^V$, zwei benachbarte Reste $R^X$, zwei benachbarte Reste $R^Y$, zwei benachbarte Reste $R^Z$ zusammen ein aliphatisches, aromatisches oder heteroaromatisches Ringsystem, das durch einen oder mehrere Reste R substituiert sein kann, bilden können;

R steht bei jedem Auftreten gleich oder verschieden für H, D, F, Cl, Br, I, CHO, CN, C(=O)Ar, $P(=O)(Ar)_2$, S(=O)Ar, $S(=O)_2Ar$, $N(R')_2$, $N(Ar)_2$, $NO_2$, $Si(R')_3$, $B(OR')_2$, $OSO_2R'$, eine geradkettige Alkyl-, Alkoxy- oder Thioalkylgruppe mit 1 bis 40 C-Atomen oder eine verzweigte oder cyclische Alkyl-, Alkoxy- oder Thioalkylgruppe mit 3 bis 40 C-Atomen, wobei jede dieser Gruppen durch einen oder mehrere Reste R' substituiert sein kann, wobei jeweils eine oder mehrere nicht benachbarte $CH_2$-Gruppen durch R'C=CR', C=C, $Si(R')_2$, $Ge(R')_2$, $Sn(R')_2$, C=O, C=S, C=Se, P(=O)(R'), SO, $SO_2$, O, S oder CONR' ersetzt sein können und wobei ein oder mehrere H-Atome durch D, F, Cl, Br, I, CN oder $NO_2$ ersetzt sein können, ein aromatisches oder heteroaromatisches Ringsystem mit 5 bis 60 aromatischen Ringatomen, das jeweils durch einen oder mehrere Reste R' substituiert sein kann, oder eine Aryloxygruppe mit 5 bis 60 aromatischen Ringatomen, die durch einen oder mehrere Reste R' substituiert sein kann; wobei zwei benachbarte Reste R zusammen ein aliphatisches oder aromatisches Ringsystem, das durch einen oder mehrere Reste R' substituiert sein kann, bilden können;

Ar ist bei jedem Auftreten gleich oder verschieden ein aromatisches oder heteroaromatisches Ringsystem mit 5 bis 60 aromatischen Ringatomen, das jeweils auch durch einen oder mehrere Reste R' substituiert sein kann;

R' steht bei jedem Auftreten gleich oder verschieden für H, D, F, Cl, Br, I, CN, eine geradkettige Alkyl-, Alkoxy- oder Thioalkylgruppe mit 1 bis 20 C-Atomen oder eine verzweigte oder cyclische Alkyl-, Alkoxy- oder Thioalkylgruppe mit 3 bis 20 C-Atomen, wobei jeweils eine oder mehrere nicht benachbarte $CH_2$-Gruppen durch SO, $SO_2$, O, S ersetzt sein können und wobei ein oder mehrere H-Atome durch D, F, Cl, Br oder I ersetzt sein können, oder ein aromatisches oder heteroaromatisches Ringsystem mit 5 bis 24 aromatischen Ringatomen; und

a, b stehen bei jedem Auftreten gleich oder verschieden für 0 oder 1; wobei dann, wenn a oder b gleich 0 ist, das entsprechende $Ar^S$ fehlt und die Gruppe $Ar^1$ direkt an einen Gruppe X gebunden ist.

2. Zusammensetzung nach Anspruch 1, **dadurch gekennzeichnet, dass** die Verbindung der Formel (H2) aus den Verbindungen der Formel (H2-1) ausgewählt ist,

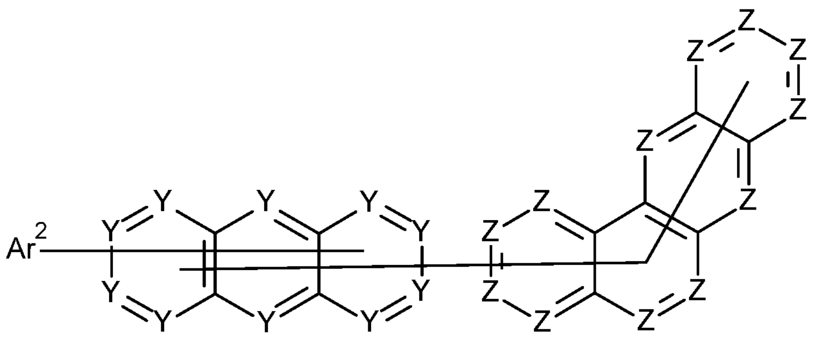

Formel (H2-1)

wobei die Symbole $Ar^2$ und Z die gleiche Bedeutung wie in Anspruch 1 haben und

Y für $CR^Y$ oder N steht oder Y für C steht, wenn es an $Ar^2$ oder eine Gruppe Z gebunden ist; wobei $R^Y$ die gleiche Bedeutung wie in Anspruch 1 hat.

3. Zusammensetzung nach Anspruch 1 oder 2, **dadurch gekennzeichnet, dass** die Verbindung der Formel (H2) aus

den Verbindungen der Formel (H2-2) ausgewählt ist,

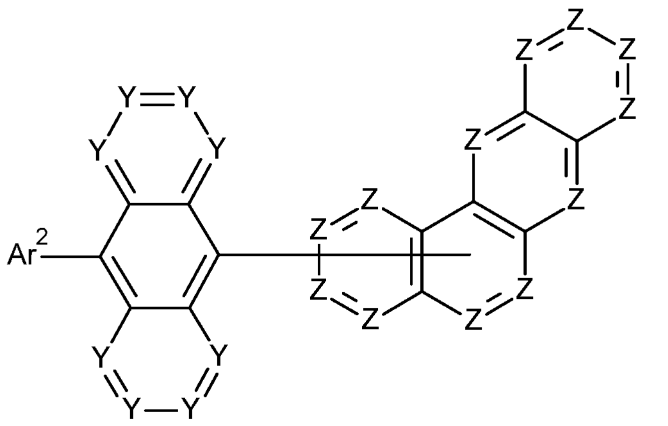

Formel (H2-2)

wobei $Ar^2$ und Z die gleiche Bedeutung wie in Anspruch 1 haben und Y die gleiche Bedeutung wie in Anspruch 2 hat.

**4.** Zusammensetzung nach einem oder mehreren der vorhergehenden Ansprüche, **dadurch gekennzeichnet, dass** die Verbindung der Formel (H2) aus den Verbindungen der Formel (H2-3) ausgewählt ist,

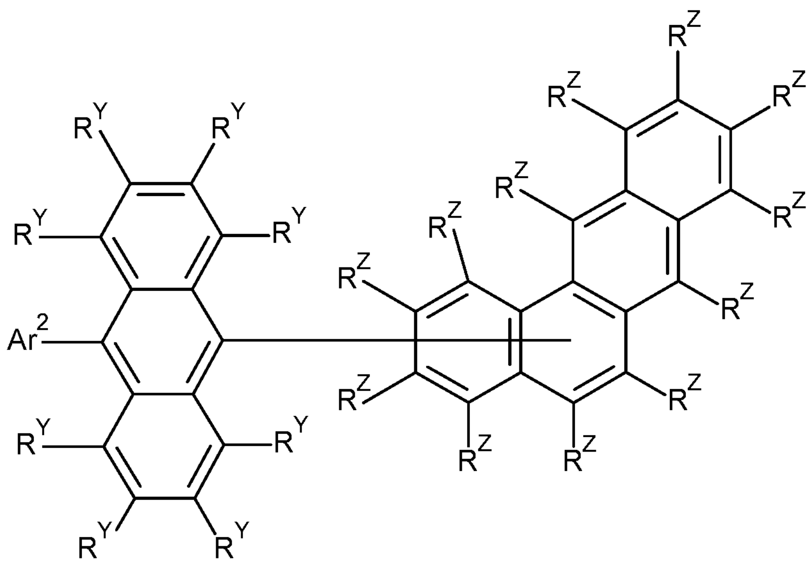

Formel (H2-3)

wobei die Symbole die gleiche Bedeutung wie in Anspruch 1 haben, mit der Maßgabe, dass die Gruppe $CR^Z$ einer Gruppe C an der Bindungsposition des benachbarten Anthracens entspricht.

**5.** Zusammensetzung nach einem oder mehreren der vorhergehenden Ansprüche, **dadurch gekennzeichnet, dass** die Verbindung der Formel (H2) aus den Verbindungen der Formel (H2-4) ausgewählt ist,

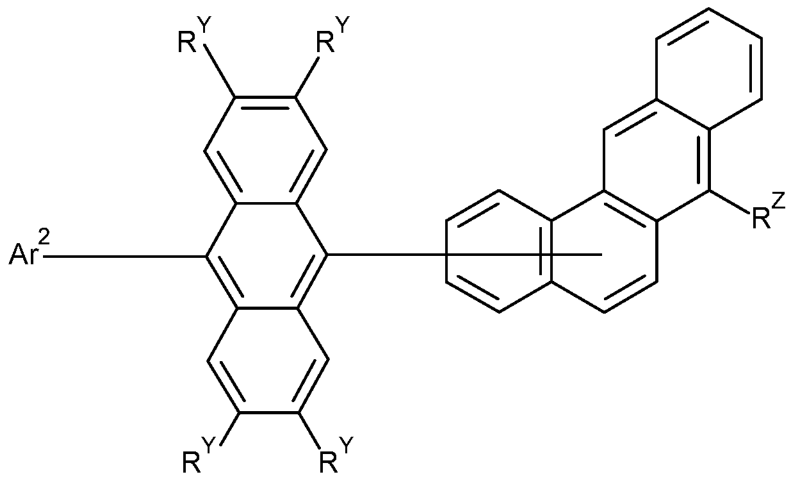

Formel (H2-4)

wobei die Symbole die gleiche Bedeutung wie in Anspruch 1 haben.

6. Zusammensetzung nach einem oder mehreren der vorhergehenden Ansprüche, **dadurch gekennzeichnet, dass** die Verbindung der Formel (H2) aus den Verbindungen der Formel (H2-5) ausgewählt ist,

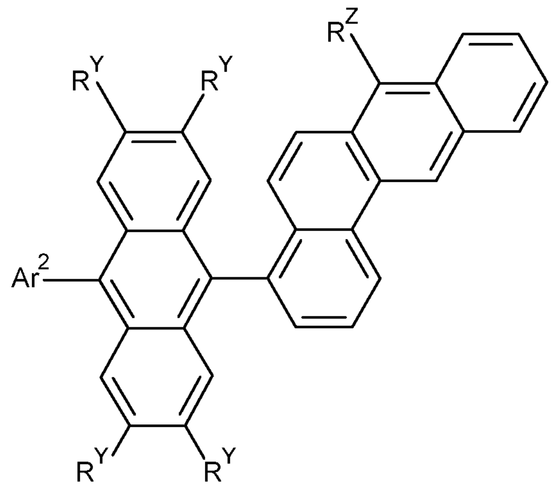

Formel (H2-5)

wobei die Symbole die gleiche Bedeutung wie in Anspruch 1 haben.

7. Zusammensetzung nach einem oder mehreren der vorhergehenden Ansprüche, **dadurch gekennzeichnet, dass** die Verbindung der Formel (H1) aus den Verbindungen der Formel (H1-1) ausgewählt ist,

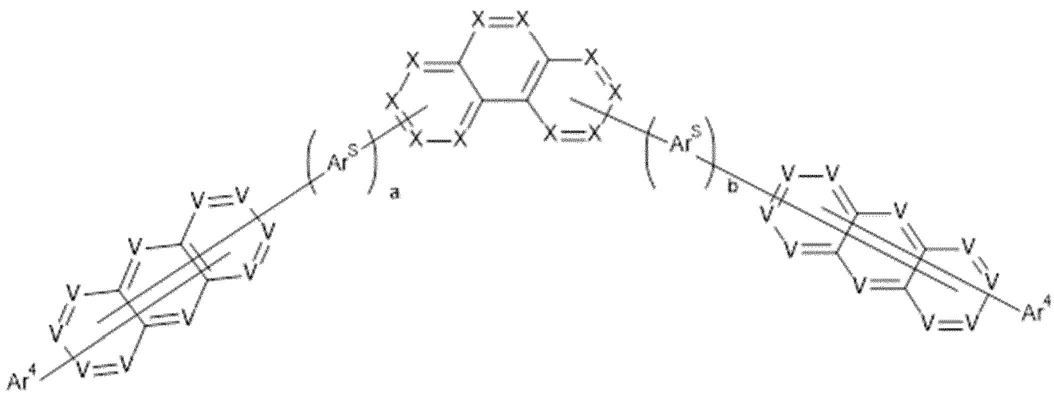

Formel (H1-1)

wobei X, $Ar^S$, $Ar^4$ und die Indizes a und b die gleiche Bedeutung wie in Anspruch 1 haben und V für $CR^V$ oder N steht oder V für C steht, wenn es an $Ar^4$, $Ar^S$ oder eine Gruppe X gebunden ist; wobei $R^V$ die gleiche Bedeutung wie in Anspruch 1 hat.

**8.** Zusammensetzung nach einem oder mehreren der vorhergehenden Ansprüche, **dadurch gekennzeichnet, dass** die Verbindung der Formel (H1) aus den Verbindungen der Formel (H1-2) ausgewählt ist,

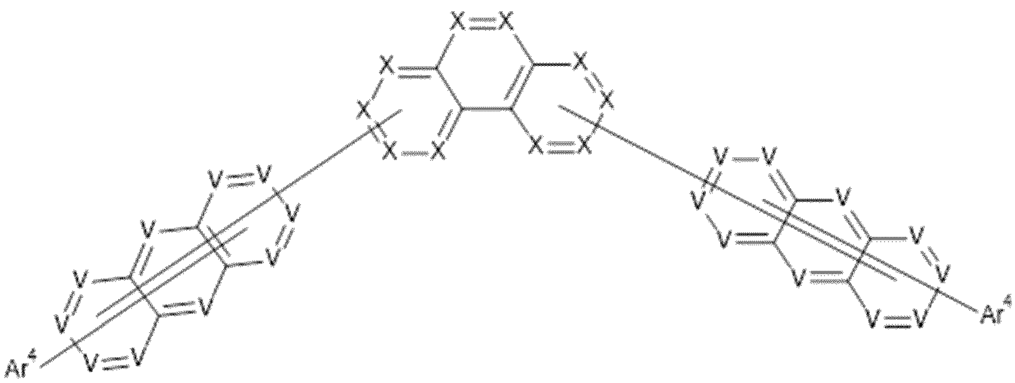

Formel (H1-2)

wobei X und $Ar^4$ die gleiche Bedeutung wie in Anspruch 1 haben und V die gleiche Bedeutung wie in Anspruch 7 hat.

**9.** Zusammensetzung nach einem oder mehreren der vorhergehenden Ansprüche, wobei die Verbindung der Formel (H1) aus den Verbindungen der Formel (H1-3) ausgewählt ist,

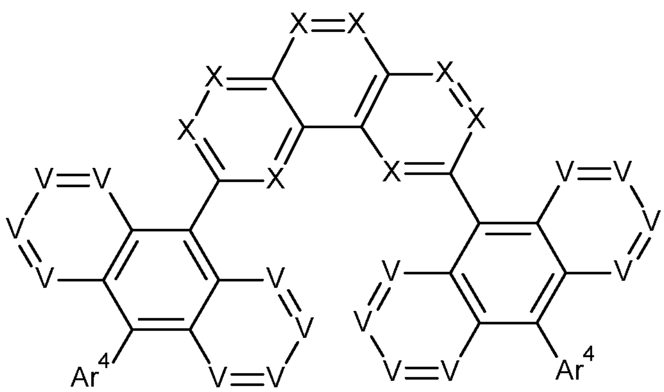

Formel (H1-3)

wobei die Symbole die gleiche Bedeutung wie in den Ansprüchen 1 und 7 haben.

10. Zusammensetzung nach einem oder mehreren der vorhergehenden Ansprüche, **dadurch gekennzeichnet, dass** die Verbindung der Formel (H1) aus den Verbindungen der Formel (H1-4) ausgewählt ist,

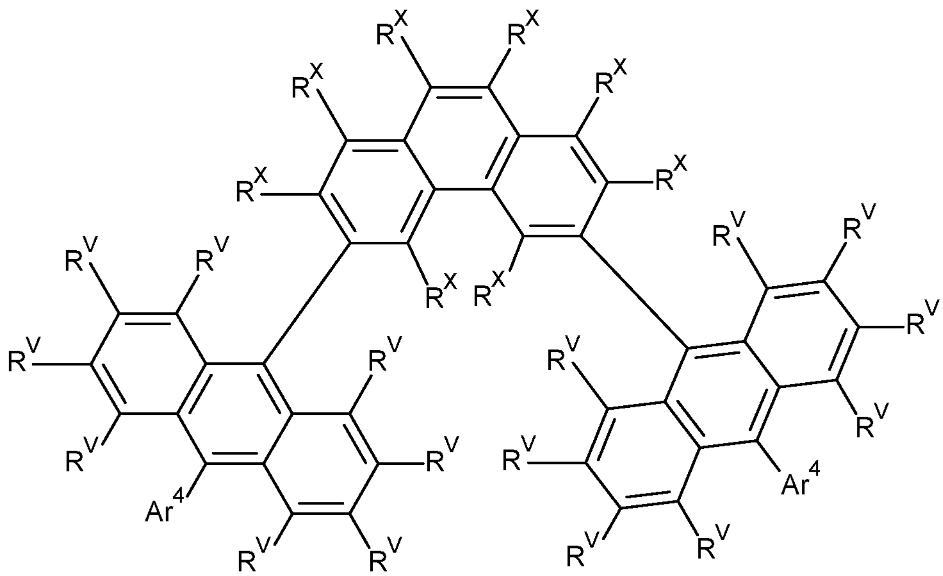

Formel (H1-4)

wobei die Symbole die gleiche Bedeutung wie in Anspruch 1 haben.

11. Zusammensetzung nach einem oder mehreren der vorhergehenden Ansprüche, **dadurch gekennzeichnet, dass** die Verbindung der Formel (H1) aus den Verbindungen der Formel (H1-5) ausgewählt ist,

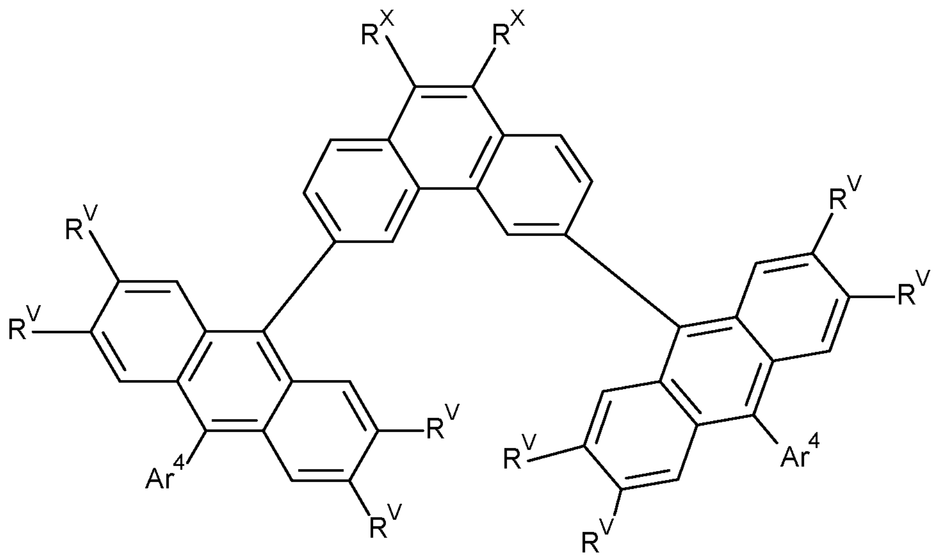

Formel (H1-5)

wobei die Symbole die gleiche Bedeutung wie in Anspruch 1 haben.

12. Zusammensetzung nach einem oder mehreren der vorhergehenden Ansprüche, **dadurch gekennzeichnet, dass** die Gruppen $Ar^2$, $Ar^4$ bei jedem Auftreten aus Phenyl, Biphenyl, Terphenyl, Quaterphenyl, Fluoren, Spirobifluoren, Naphthalin, Anthracen, Phenanthren, Triphenylen, Fluoranthen, Tetracen, Chrysen, Benzanthracen, Benzophenanthracen, Pyren oder Perylen, Dibenzofuran, Carbazol und Dibenzothiophen bestehen, wobei jede dieser Gruppen an beliebigen freien Positionen durch einen oder mehrere Reste R substituiert sein kann; und wobei es sich bei $Ar^2$, $Ar^4$ auch um eine Kombination von zwei oder mehr der oben aufgeführten Gruppen handeln kann.

**13.** Zusammensetzung nach einem oder mehreren der vorhergehenden Ansprüche, **dadurch gekennzeichnet, dass** sie ferner einen fluoreszierenden Emitter umfasst.

**14.** Zusammensetzung nach einem oder mehreren der vorhergehenden Ansprüche, **dadurch gekennzeichnet, dass** sie einen fluoreszierenden Emitter umfasst, der aus der Gruppe bestehend aus:

- einem Arylamin mit drei substituierten oder unsubstituierten aromatischen oder heteroaromatischen Ringsystemen, die direkt an den Stickstoff gebunden sind;
- einem kondensierten aromatischen oder heteroaromatischen Ringsystem mit mindestens 14 aromatischen Ringatomen;
- einem Indenofluoren, Indenofluorenamin oder Indenofluorendiamin;
- einem Benzoindenofluoren, Benzoindenofluorenamin oder Benzoindenofluorendiamin;
- einem Dibenzoindenofluoren, Dibenzoindenofluorenamin oder Dibenzoindenofluorendiamin;
- einem Indenofluoren mit einer kondensierten Arylgruppe mit mindestens zehn aromatischen Ringatomen;
- einem Bisindenoindenofluoren;
- einem Indenodibenzofuran; Indenofluorenamin oder Indenofluorendiamin;
- einem Fluorendimer;
- einem Phenoxazin und
- einem Borderivat

ausgewählt ist.

**15.** Zusammensetzung nach einem oder mehreren der vorhergehenden Ansprüche, **dadurch gekennzeichnet, dass** sie einen fluoreszierenden Emitter der Formel (E-1), (E-2) oder (E-3) umfasst,

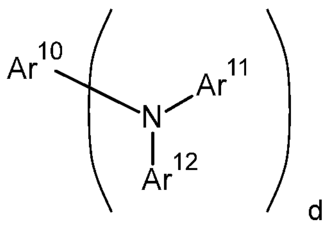

Formel (E-1)

wobei

$Ar^{10}$, $Ar^{11}$, $Ar^{12}$ bei jedem Auftreten gleich oder verschieden für ein aromatisches oder heteroaromatisches Ringsystem mit 6 bis 60 aromatischen Ringatomen stehen, das jeweils auch durch einen oder mehrere Reste R substituiert sein kann; mit der Maßgabe, dass mindestens eine Gruppe $Ar^{10}$, $Ar^{11}$, $Ar^{12}$ für ein aromatisches oder heteroaromatisches Ringsystem mit 10 bis 40 aromatischen Ringatomen steht, das mindestens eine kondensierte Aryl- oder Heteroarylgruppe aus 2 bis 4 miteinander kondensierten aromatischen Ringen enthält, wobei das aromatische oder heteroaromatische Ringsystem durch einen oder mehrere Reste R substituiert sein kann;
R wie in Anspruch 1 definiert ist und
d für 1, 2, 3 oder 4 steht, weiter bevorzugt d für 1 steht;

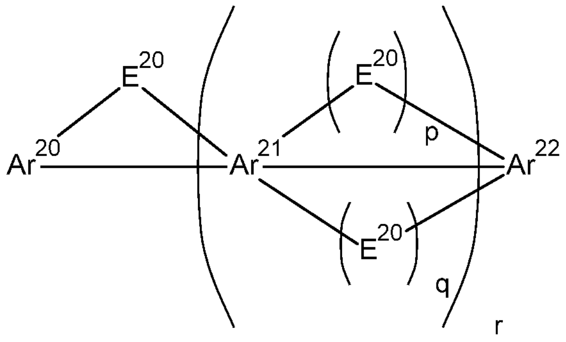

Formel (E-2)

wobei

$Ar^{20}$, $Ar^{21}$, $Ar^{22}$ bei jedem Auftreten gleich oder verschieden für eine Aryl- oder Heteroarylgruppe mit 6 bis 30 aromatischen Ringatomen stehen, die jeweils auch durch einen oder mehrere Reste R substituiert sein kann;

$E^{20}$ bei jedem Auftreten gleich oder verschieden für eine aus BR, $C(R^0)_2$, $Si(R^0)_2$, C=O, $C=NR^0$, $C=C(R^0)_2$, O, S, S=O, $SO_2$, $NR^0$, PR°, $P(=O)R^0$ oder P(=S)R° ausgewählte Gruppe steht, wobei $Ar^{20}$, $Ar^{21}$ and $E^{20}$ zusammen einen fünfgliedrigen Ring oder einen sechsgliedrigen Ring bilden und $Ar^{21}$, $Ar^{23}$ und $E^{20}$ zusammen einen fünfgliedrigen Ring oder einen sechsgliedrigen Ring bilden;

R° bei jedem Auftreten gleich oder verschieden für H, D, F, eine geradkettige Alkylgruppe mit 1 bis 20 und vorzugsweise 1 bis 10 C-Atomen oder eine verzweigte oder cyclische Alkylgruppe mit 3 bis 20 und vorzugsweise 3 bis 10 C-Atomen, wobei jede dieser Gruppen durch einen oder mehrere Reste R substituiert sein kann, wobei jeweils eine oder mehrere nicht benachbarte $CH_2$-Gruppen durch O oder S ersetzt sein können und wobei ein oder mehrere H-Atome durch D oder F ersetzt sein können, oder ein aromatisches oder heteroaromatisches Ringsystem mit 5 bis 40, vorzugsweise 5 bis 30 und weiter bevorzugt 6 bis 18 aromatischen Ringatomen, das jeweils durch einen oder mehrere Reste R substituiert sein kann, steht, wobei zwei benachbarte Reste R° zusammen ein aliphatisches oder aromatisches Ringsystem, das durch einen oder mehrere Reste R substituiert sein kann, bilden können;

R wie in Anspruch 1 definiert ist;

p, q bei jedem Auftreten gleich oder verschieden für 0 oder 1 stehen, mit der Maßgabe, dass p + q = 1;

r für 1, 2 oder 3 steht;

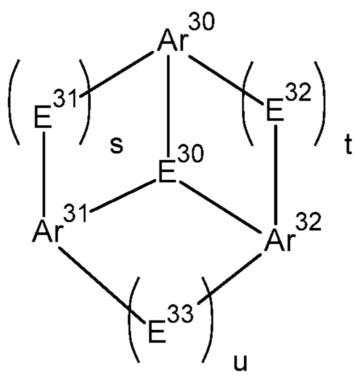

Formel (E-3)

wobei

$Ar^{30}$, $Ar^{31}$, $Ar^{32}$ bei jedem Auftreten gleich oder verschieden für eine substituierte oder unsubstituierte Aryl- oder Heteroarylgruppe mit 5 bis 22, vorzugsweise 5 bis 18 und weiter bevorzugt 6 bis 14 aromatischen Ringatomen stehen;

$E^{30}$ für B oder N steht;

$E^{31}$, $E^{32}$, $E^{33}$ bei jedem Auftreten gleich oder verschieden für O, S, $C(R^0)_2$, C=O, C=S, $C=NR^0$, $C=C(R^0)_2$, $Si(R^0)_2$, $BR^0$, $NR^0$, PR°, $SO_2$, $SeO_2$ oder eine chemische Bindung stehen, mit der Maßgabe, dass dann, wenn $E^{30}$ für B steht, mindestens eine der Gruppen $E^{31}$, $E^{32}$, $E^{33}$ für $NR^0$ steht, und dann, wenn $E^{30}$ für N steht, mindestens eine der Gruppen $E^{31}$, $E^{32}$, $E^{33}$ für $BR^0$ steht;

R° wie oben definiert ist;
s, t, u bei jedem Auftreten gleich oder verschieden für 0 oder 1 stehen, mit der Maßgabe, dass s + t + u ≥ 1.

**16.** Zusammensetzung nach einem oder mehreren der vorhergehenden Ansprüche, **dadurch gekennzeichnet, dass** die Verbindung der Formel (H1) in der Zusammensetzung in einem Anteil von 1 bis 60 % vorliegt und die Verbindung der Formel (H2) in der Zusammensetzung in einem Anteil von 30 bis 99 % vorliegt.

**17.** Formulierung, umfassend mindestens eine Zusammensetzung nach einem oder mehreren der Ansprüche 1 bis 16 und mindestens ein Lösungsmittel.

**18.** Verfahren zur Herstellung einer elektronischen Vorrichtung mit mindestens einer Schicht, die eine Zusammensetzung nach einem oder mehreren der Ansprüche 1 bis 16 umfasst:

a) Herstellen einer Formulierung, die mindestens eine Zusammensetzung nach einem oder mehreren der Ansprüche 1 bis 16 und mindestens ein Lösungsmittel umfasst;
b) Aufbringen der in Schritt a) hergestellten Formulierung auf ein Substrat oder auf eine andere Schicht zur Bildung einer Schicht;
c) Trocknen der Schicht zur Entfernung des Lösungsmittels.

**19.** Verfahren nach Anspruch 18, **dadurch gekennzeichnet, dass** die Formulierung durch ein Beschichtungsverfahren oder ein Druckverfahren aufgebracht wird.

**20.** Verfahren nach Anspruch 18 oder 19, **dadurch gekennzeichnet, dass** die Formulierung durch Flutbeschichten, Tauchbeschichten, Spritzbeschichten, Aufschleudern, Siebdruck, Hochdruck, Tiefdruck, Walzenbeschichten, Tintenstrahldruck, Rotationsdruck, Flexodruck, Offsetdruck, Schlitzdüsenbeschichten oder Düsendruck aufgebracht wird.

**21.** Elektronische Vorrichtung, umfassend Anode, Kathode und mindestens eine emittierende Schicht, wobei die emittierende Schicht eine Zusammensetzung nach einem oder mehreren der Ansprüche 1 bis 16 umfasst.

**22.** Elektronische Vorrichtung nach Anspruch 21, ausgewählt aus der Gruppe bestehend aus organischen Elektrolumineszenzvorrichtungen, organischen integrierten Schaltungen, organischen Feldeffekttransistoren, organischen Dünnfilmtransistoren, organischen lichtemittierenden Transistoren, organischen Solarzellen, farbstoffsensibilisierten organischen Solarzellen, organischen optischen Detektoren, organischen Photorezeptoren, organischen Feld-Quench-Devices, lichtemittierenden elektrochemischen Zellen, organischen Laserdioden und "Organic Plasmon Emitting Devices".

## Revendications

**1.** Composition comprenant un composé de formule (H1) et un composé de formule (H2),

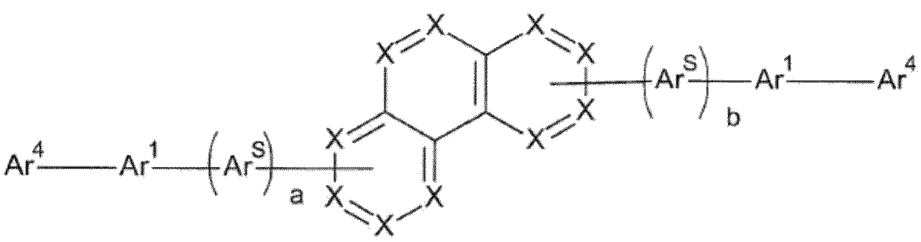

formule (H1)

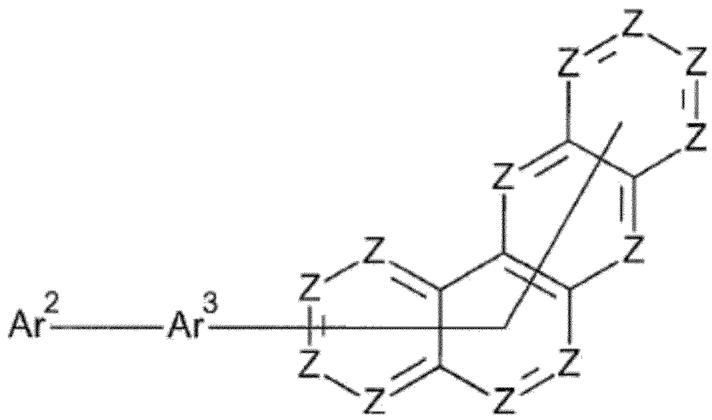

formule (H2)

où ce qui suit s'applique aux symboles et indices utilisés :

X représente en chaque occurrence, de manière identique ou différente, $CR^X$ ou N ; et X étant C si X est lié à un groupe $Ar^1$ ou $Ar^S$ ;
Z représente en chaque occurrence, de manière identique ou différente, $CR^Z$ ou N ; et Z étant C si Z est lié à un groupe $Ar^3$ ;
$Ar^1$ est, en chaque occurrence, de manière identique ou différente, un groupe aryle ou hétéroaryle ayant 10 à 60 atomes de cycle aromatique, qui peut en chaque cas également être substitué par un ou plusieurs radicaux $R^V$ ;
$Ar^3$ est, en chaque occurrence, de manière identique ou différente, un groupe aryle ou hétéroaryle ayant 10 à 60 atomes de cycle aromatique, qui peut en chaque cas également être substitué par un ou plusieurs radicaux $R^Y$ ;
$Ar^2$, $Ar^4$, $Ar^S$ sont, en chaque occurrence, de manière identique ou différente, un système cyclique aromatique ou hétéroaromatique ayant 5 à 60 atomes de cycle aromatique, qui peut en chaque cas également être substitué par un ou plusieurs radicaux R ;
$R^V$, $R^X$, $R^Y$, $R^Z$ représentent en chaque occurrence, de manière identique ou différente, H, D, F, Cl, Br, I, CHO, CN, C(=O)Ar, $P(=O)(Ar)_2$, S(=O)Ar, $S(=O)_2Ar$, $N(R)_2$, $N(Ar)_2$, $NO_2$, $Si(R)_3$, $B(OR)_2$, $OSO_2R$, un groupe alkyle, alcoxy ou thioalkyle linéaire possédant 1 à 40 atomes de C ou un groupe alkyle, alcoxy ou thioalkyle ramifié ou cyclique possédant 3 à 40 atomes de C, chacun desquels pouvant être substitué par un ou plusieurs radicaux R, où en chaque cas un ou plusieurs groupes $CH_2$ non adjacents peuvent être remplacés par RC=CR, C=C, $Si(R)_2$, $Ge(R)_2$, $Sn(R)_2$, C=O, C=S, C=Se, P(=O)(R), SO, $SO_2$, O, S ou CONR et où un ou plusieurs atomes de H peuvent être remplacés par D, F, Cl, Br, I, CN ou $NO_2$, un système cyclique aromatique ou hétéroaromatique possédant 5 à 60 atomes de cycle aromatique, qui peut en chaque cas être substitué par un ou plusieurs radicaux R, ou un groupe aryloxy possédant 5 à 60 atomes de cycle aromatique, qui peut être substitué par un ou plusieurs radicaux R ;
où deux radicaux $R^V$ adjacents, deux radicaux $R^X$ adjacents, deux radicaux $R^Y$ adjacents, deux radicaux $R^Z$ adjacents peuvent former ensemble un système cyclique aliphatique, aromatique ou hétéroaromatique, qui peut être substitué par un ou plusieurs radicaux R ;
R représente en chaque occurrence, de manière identique ou différente, H, D, F, Cl, Br, I, CHO, CN, C(=O)Ar, $P(=O)(Ar)_2$, S(=O)Ar, $S(=O)_2Ar$, $N(R')_2$, $N(Ar)_2$, $NO_2$, $Si(R')_3$, $B(OR')_2$, $OSO_2R'$, un groupe alkyle, alcoxy ou thioalkyle linéaire possédant 1 à 40 atomes de C ou un groupe alkyle, alcoxy ou thioalkyle ramifié ou cyclique possédant 3 à 40 atomes de C, chacun desquels pouvant être substitué par un ou plusieurs radicaux R', où en chaque cas un ou plusieurs groupes $CH_2$ non adjacents peuvent être remplacés par R'C=CR', C=C, $Si(R')_2$, $Ge(R')_2$, $Sn(R')_2$, C=O, C=S, C=Se, P(=O)(R'), SO, $SO_2$, O, S ou CONR' et où un ou plusieurs atomes de H peuvent être remplacés par D, F, Cl, Br, I, CN ou $NO_2$, un système cyclique aromatique ou hétéroaromatique possédant 5 à 60 atomes de cycle aromatique, qui peut en chaque cas être substitué par un ou plusieurs radicaux R', ou un groupe aryloxy possédant 5 à 60 atomes de cycle aromatique, qui peut être substitué par un ou plusieurs radicaux R' ; où deux substituants R adjacents peuvent former ensemble un système cyclique aliphatique ou aromatique qui peut être substitué par un ou plusieurs radicaux R' ;
Ar est, en chaque occurrence, de manière identique ou différente, un système cyclique aromatique ou hétéroaromatique possédant 5 à 60 atomes de cycle aromatique, qui peut en chaque cas également être substitué

par un ou plusieurs radicaux R' ;
R' représente en chaque occurrence, de manière identique ou différente, H, D, F, Cl, Br, I, CN, un groupe alkyle, alcoxy ou thioalkyle linéaire possédant 1 à 20 atomes de C ou un groupe alkyle, alcoxy ou thioalkyle ramifié ou cyclique possédant 3 à 20 atomes de C, où en chaque cas un ou plusieurs groupes $CH_2$ non adjacents peuvent être remplacés par SO, $SO_2$, O, S et où un ou plusieurs atomes de H peuvent être remplacés par D, F, Cl, Br ou I, ou un système cyclique aromatique ou hétéroaromatique possédant 5 à 24 atomes de cycle aromatique ; et
a, b sont en chaque occurrence, de manière identique ou différente, 0 ou 1 ; lorsque a ou b est 0, alors le $Ar^S$ correspondant est absent et le groupe $Ar^1$ est directement lié à un groupe X.

2. Composition selon la revendication 1, **caractérisée en ce que** le composé de formule (H2) est choisi parmi les composés de formule (H2-1),

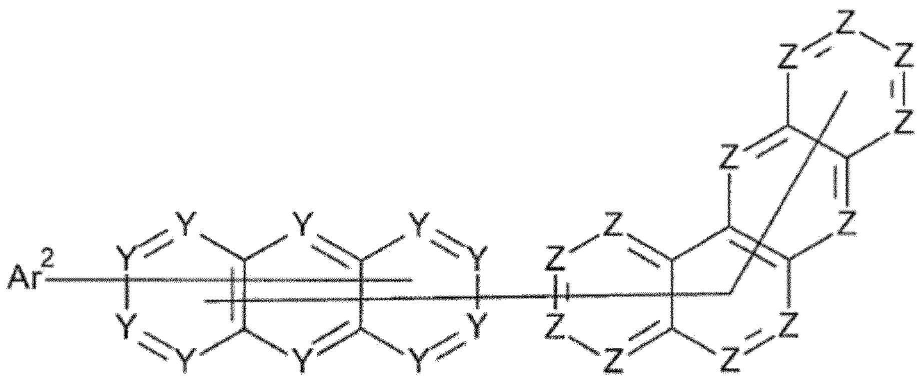

formule (H2-1)

où les symboles $Ar^2$ et Z possèdent la même signification que dans la revendication 1 ; et
Y est $CR^Y$ ou N ; ou Y est C s'il est lié à $Ar^2$ ou un groupe Z ; où $R^Y$ possède la même signification que dans la revendication 1.

3. Composition selon la revendication 1 ou 2, **caractérisée en ce que** le composé de formule (H2) est choisi parmi les composés de formule (H2-2),

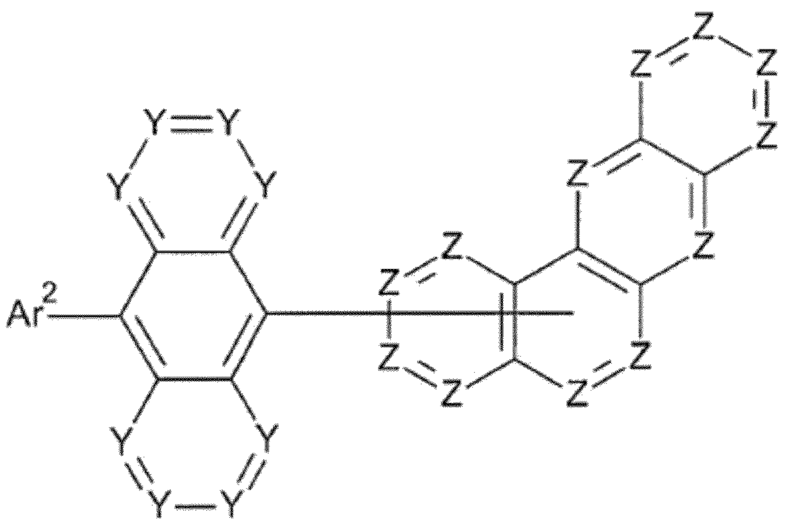

formule (H2-2)

où $Ar^2$ et Z possèdent la même signification que dans la revendication 1 et Y possède la même signification que dans la revendication 2.

4. Composition selon l'une ou plusieurs des revendications précédentes, **caractérisée en ce que** le composé de formule (H2) est choisi parmi les composés de formule (H2-3),

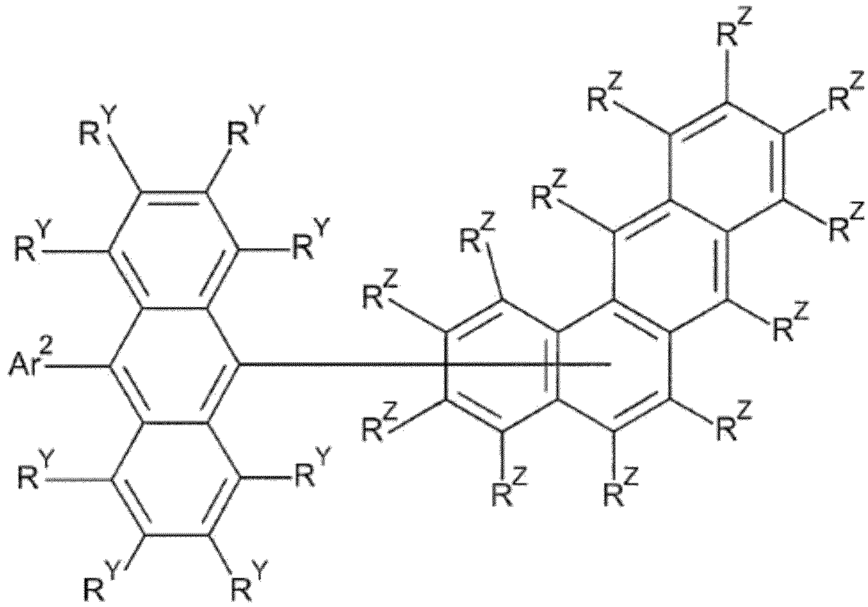

formule (H2-3)

où les symboles possèdent la même signification que dans la revendication 1, à la condition que le groupe $CR^Z$ corresponde à un groupe C au niveau de la position de liaison de l'anthracène adjacent.

5. Composition selon l'une ou plusieurs des revendications précédentes, **caractérisée en ce que** le composé de formule (H2) est choisi parmi les composés de formule (H2-4),

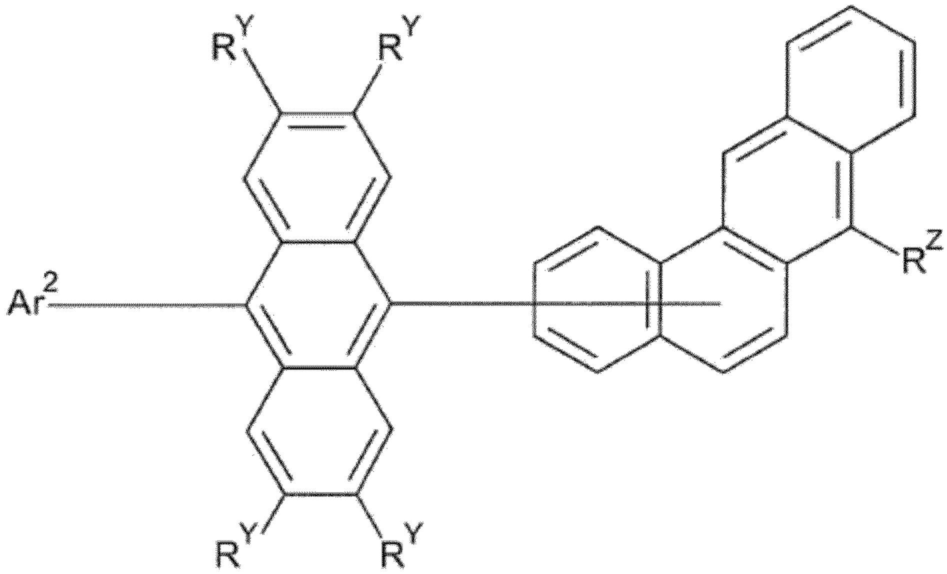

formule (H2-4)

où les symboles possèdent la même signification que dans la revendication 1.

6. Composition selon l'une ou plusieurs des revendications précédentes, **caractérisée en ce que** le composé de formule (H2) est choisi parmi les composés de formule (H2-5),

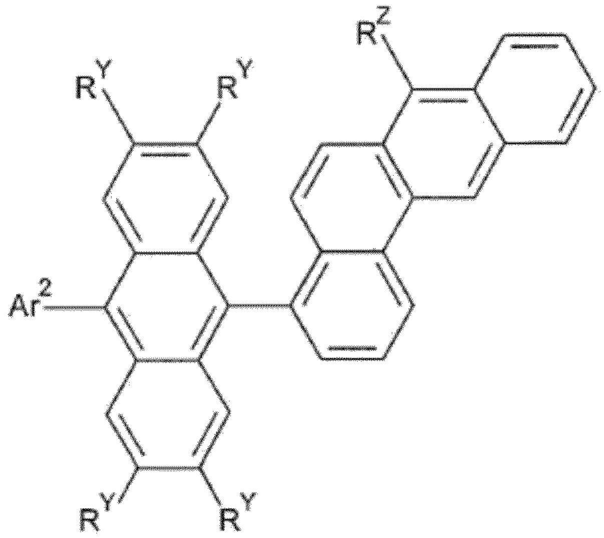

formule (H2-5)

où les symboles possèdent la même signification que dans la revendication 1.

**7.** Composition selon l'une ou plusieurs des revendications précédentes, **caractérisée en ce que** le composé de formule (H1) est choisi parmi les composés de formule (H1-1),

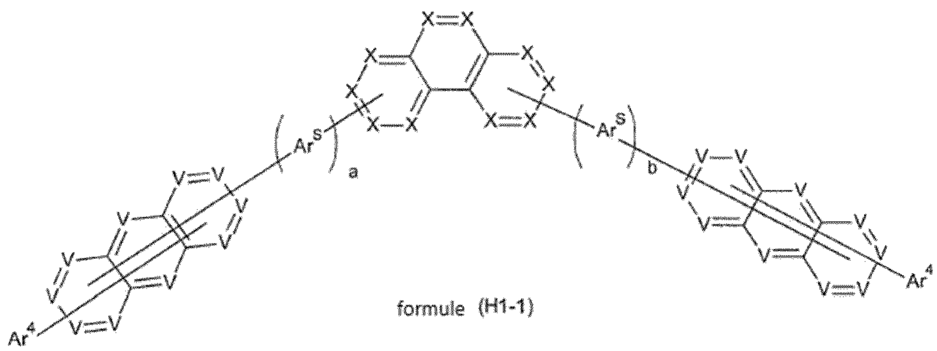

formule (H1-1)

où X, $Ar^S$, $Ar^4$ et les indices a et b possèdent la même signification que dans la revendication 1 ; et V est $CR^V$ ou N ; ou V est C s'il est lié à $Ar^4$, $Ar^S$ ou un groupe X ; où $R^V$ possède la même signification que dans la revendication 1.

**8.** Composition selon l'une ou plusieurs des revendications précédentes, **caractérisée en ce que** le composé de formule (H1) est choisi parmi les composés de formule (H1-2),

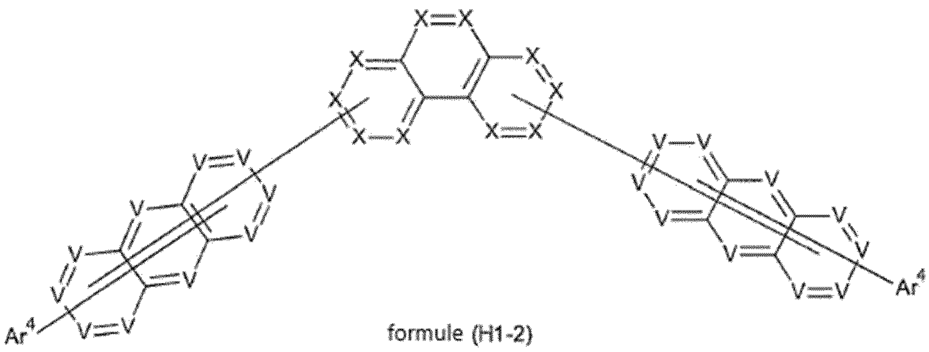

formule (H1-2)

où X et $Ar^4$ possèdent la même signification que dans la revendication 1 ; et

V possède la même signification que dans la revendication 7.

**9.** Composition selon l'une ou plusieurs des revendications précédentes, **caractérisée en ce que** le composé de formule (H1) est choisi parmi les composés de formule (H1-3),

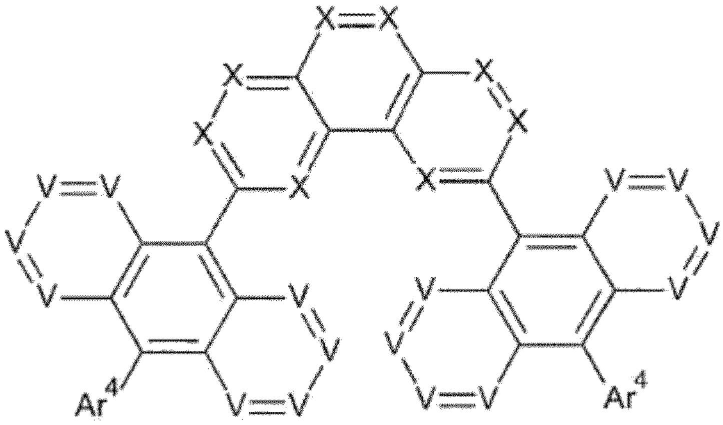

formule (H1-3)

où les symboles possèdent la même signification que dans les revendications 1 et 7.

**10.** Composition selon l'une ou plusieurs des revendications précédentes, **caractérisée en ce que** le composé de formule (H1) est choisi parmi les composés de formule (H1-4),

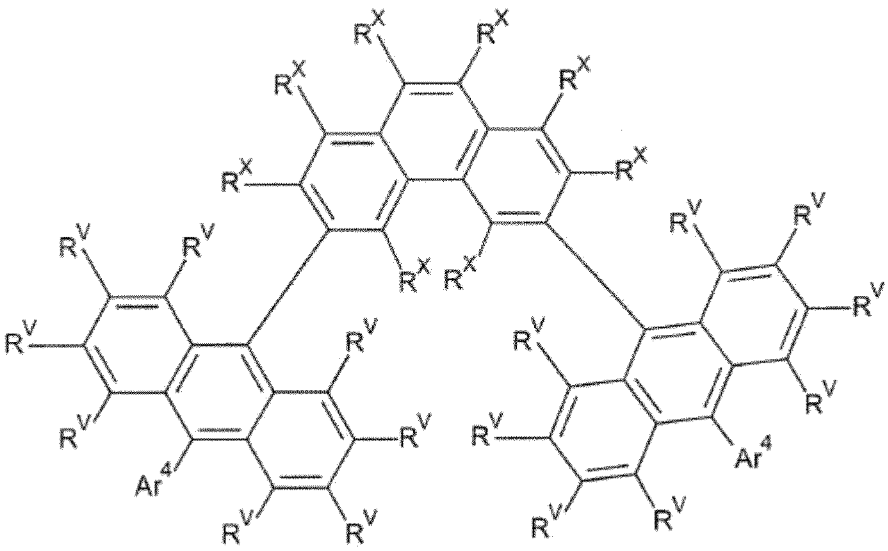

formule (H1-4)

où les symboles possèdent la même signification que dans la revendication 1.

**11.** Composition selon l'une ou plusieurs des revendications précédentes, **caractérisée en ce que** le composé de formule (H1) est choisi parmi les composés de formule (H1-5),

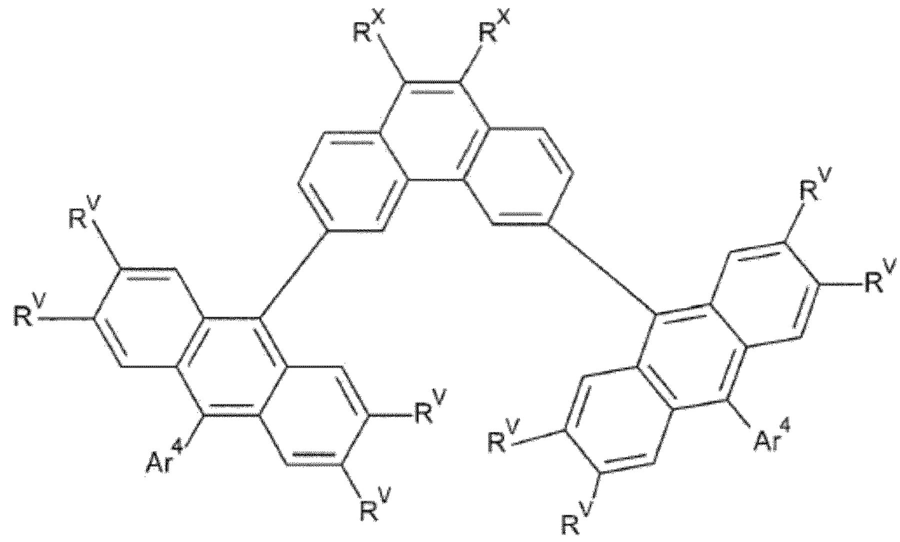

formule (H1-5)

où les symboles possèdent la même signification que dans la revendication 1.

**12.** Composition selon l'une ou plusieurs des revendications précédentes, **caractérisée en ce que** les groupes $Ar^2$, $Ar^4$ sont en chaque occurrence, constitués par phényle, biphényle, terphényle, quaterphényle, fluorène, spirobifluorène, naphtalène, anthracène, phénanthrène, triphénylène, fluoranthène, tétracène, chrysène, benzanthracène, benzophénanthracène, pyrène ou pérylène, dibenzofurane, carbazole et dibenzothiophène, chacun desquels pouvant être substitué par un ou plusieurs radicaux R au niveau de quelconques positions libres ; et où $Ar^2$, $Ar^4$ peuvent également être une combinaison de deux groupes précédemment cités ou plus.

**13.** Composition selon l'une ou plusieurs des revendications précédentes, **caractérisée en ce qu'**elle comprend en outre un émetteur fluorescent.

**14.** Composition selon l'une ou plusieurs des revendications précédentes, **caractérisée en ce qu'**elle comprend un émetteur fluorescent choisi dans le groupe constitué par :

- une arylamine contenant trois systèmes cycliques aromatiques ou hétéroaromatiques substitués ou non substitués liés directement à l'azote ;
- un système cyclique aromatique ou hétéroaromatique condensé ayant au moins 14 atomes de cycle aromatique ;
- un indénofluorène, une indénofluorènamine ou une indénofluorènediamine ;
- un benzoindonofluorène, une benzoindénofluorènamine ou une benzoindénofluorènediamine ;
- un dibenzoindénofluorène, une dibenzoindénofluorènamine ou une dibenzoindénofluorènediamine ;
- un indénofluorène contenant un groupe aryle condensé ayant au moins 10 atomes de cycle aromatique ;
- un bisindénoindénofluorène ;
- un indénodibenzofurane ; une indénofluorènamine ou une indénofluorènediamine ;
- un dimère de fluorène ;
- une phénoxazine ; et
- un dérivé du bore.

**15.** Composition selon l'une ou plusieurs des revendications précédentes, **caractérisée en ce qu'**elle comprend un émetteur fluorescent de formule (E-1), (E-2) ou (E-3),

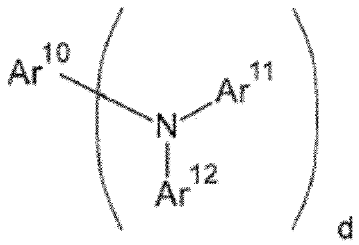

formule (E-1)

où

$Ar^{10}$, $Ar^{11}$, $Ar^{12}$ sont en chaque occurrence, de manière identique ou différente, un système cyclique aromatique ou hétéroaromatique ayant 6 à 60 atomes de cycle aromatique, qui peut en chaque cas également être substitué par un ou plusieurs radicaux R ; à la condition qu'au moins un groupe $Ar^{10}$, $Ar^{11}$, $Ar^{12}$ soit un système cyclique aromatique ou hétéroaromatique ayant 10 à 40 atomes de cycle aromatique, contenant au moins un groupe aryle ou hétéroaryle condensé constitué de 2 à 4 cycles aromatiques condensés l'un à l'autre, où le système cyclique aromatique ou hétéroaromatique peut être substitué par un ou plusieurs radicaux R ;

R possède la même définition que dans la revendication 1 ; et
d est 1, 2, 3 ou 4 ; plus préférablement, d est 1 ;

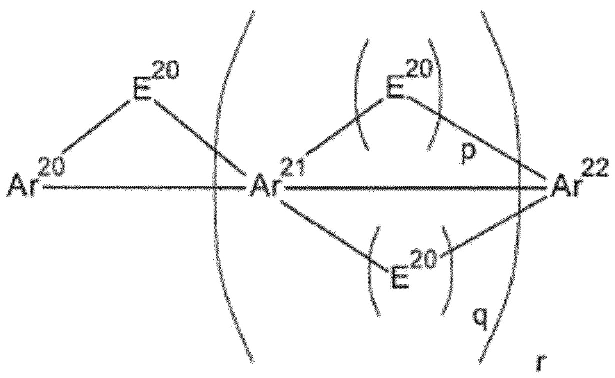

formule (E-2)

où
$Ar^{20}$, $Ar^{21}$, $Ar^{22}$ sont en chaque occurrence, de manière identique ou différente, un groupe aryle ou hétéroaryle ayant 6 à 30 atomes de cycle aromatique, qui peut en chaque cas également être substitué par un ou plusieurs radicaux R ;
$E^{20}$ est en chaque occurrence, de manière identique ou différente, un groupe choisi parmi BR, $C(R^0)_2$, $Si(R^0)_2$, C=O, $C=NR^0$, $C=C(R^0)_2$, O, S, S=O, $SO_2$, $NR^0$, $PR^0$, P(=O)R° ou $P(=S)R^0$; $Ar^{20}$, $Ar^{21}$ et $E^{20}$ formant ensemble un cycle à cinq chaînons ou un cycle à six chaînons, et $Ar^{21}$, $Ar^{23}$ et $E^{20}$ formant ensemble un cycle à cinq chaînons ou un cycle à six chaînons ;
R° représente en chaque occurrence, de manière identique ou différente, H, D, F, un groupe alkyle linéaire ayant 1 à 20, préférablement de 1 à 10 atomes de C ou un groupe alkyle ramifié ou cyclique ayant 3 à 20, préférablement 3 à 10 atomes de C, chacun desquels pouvant être substitué par un ou plusieurs radicaux R, où en chaque cas un ou plusieurs groupes $CH_2$ non adjacents peuvent être remplacés par O ou S et où un ou plusieurs atomes de H peuvent être remplacés par D ou F, ou un système cyclique aromatique ou hétéroaromatique ayant 5 à 40, préférablement 5 à 30, plus préférablement 6 à 18 atomes de cycle aromatique, qui peut en chaque cas être substitué par un ou plusieurs radicaux R, où deux radicaux R° adjacents, peuvent former ensemble un système cyclique aliphatique ou aromatique, qui peut être substitué par un ou plusieurs radicaux R,
R possède la même signification que dans la revendication 1 ;
p, q sont en chaque occurrence, de manière identique ou différente, 0 ou 1, à la condition que p + q = 1 ;
r est 1, 2 ou 3 ;

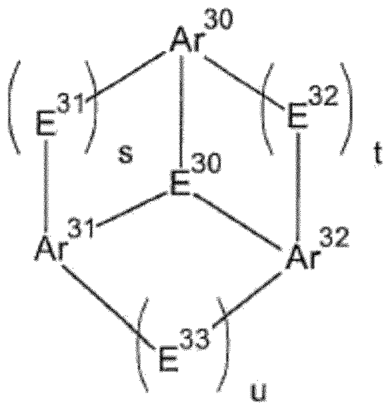

formule (E-3)

où

$Ar^{30}$, $Ar^{31}$, $Ar^{32}$ représentent en chaque occurrence, de manière identique ou différente, un groupe aryle ou hétéroaryle substitué ou non substitué ayant 5 à 22, préférablement 5 à 18, plus préférablement 6 à 14 atomes de cycle aromatique ;
$E^{30}$ représente B ou N ;
$E^{31}$, $E^{32}$, $E^{33}$ représentent en chaque occurrence, de manière identique ou différente, O, S, $C(R^0)_2$, C=O, C=S, $C=NR^0$, $C=C(R^0)_2$, $Si(R^0)_2$, BR°, $NR^0$, PR°, $SO_2$, $SeO_2$ ou une liaison chimique, à la condition que si $E^{30}$ est B, alors au moins l'un des groupes $E^{31}$, $E^{32}$, $E^{33}$ représente NR° et si $E^{30}$ est N, alors au moins l'un des groupes $E^{31}$, $E^{32}$, $E^{33}$ représente BR° ;
R° possède la même définition que ci-dessus ;

s, t, u sont en chaque occurrence, de manière identique ou différente, 0 ou 1, à la condition que $s + t + u \geq 1$.

**16.** Composition selon l'une ou plusieurs des revendications précédentes, **caractérisée en ce que** le composé de formule (H1) est présent dans la composition en une proportion de 1 à 60 % et le composé de formule (H2) est présent dans la composition en une proportion de 30 à 99 %.

**17.** Formulation comprenant au moins une composition selon l'une ou plusieurs des revendications 1 à 16 et au moins un solvant.

**18.** Procédé pour la production d'un dispositif électronique comprenant au moins une couche comprenant une composition selon l'une ou plusieurs des revendications 1 à 16 :

a) préparation d'une formulation comprenant au moins une composition selon l'une ou plusieurs des revendications 1 à 16 et au moins un solvant ;
b) application de la formulation préparée dans l'étape a) sur un substrat ou sur une autre couche afin de former une couche ;
c) séchage de la couche afin d'éliminer le solvant.

**19.** Procédé selon la revendication 18, **caractérisé en ce que** la formulation est appliquée par un procédé de revêtement ou un procédé d'impression.

**20.** Procédé selon la revendication 18 ou 19, **caractérisé en ce que** la formulation est appliquée par revêtement par immersion, revêtement par trempage, revêtement par pulvérisation, revêtement par centrifugation, sérigraphie, impression en relief, héliogravure, impression au rouleau, impression par jet d'encre, impression rotative, impression flexographique, impression offset, revêtement à filière plate ou impression par buse.

**21.** Dispositif électronique comprenant une anode, une cathode et au moins une couche émettrice, où la couche émettrice comprend une composition selon l'une ou plusieurs des revendications 1 à 16.

**22.** Dispositif électronique selon la revendication 21, choisi dans le groupe constitué par des dispositifs électroluminescents organiques, des circuits intégrés organiques, des transistors organiques à effet de champ, des transistors organiques en film mince, des transistors organiques émetteurs de lumière, des cellules solaires organiques, des cellules solaires organiques sensibilisées par un colorant, des détecteurs optiques organiques, des photorécepteurs organiques, des dispositifs organiques d'extinction de champ, des cellules électrochimiques émettrices de lumière, des diodes laser organiques et des dispositifs organiques d'émission de plasmon.

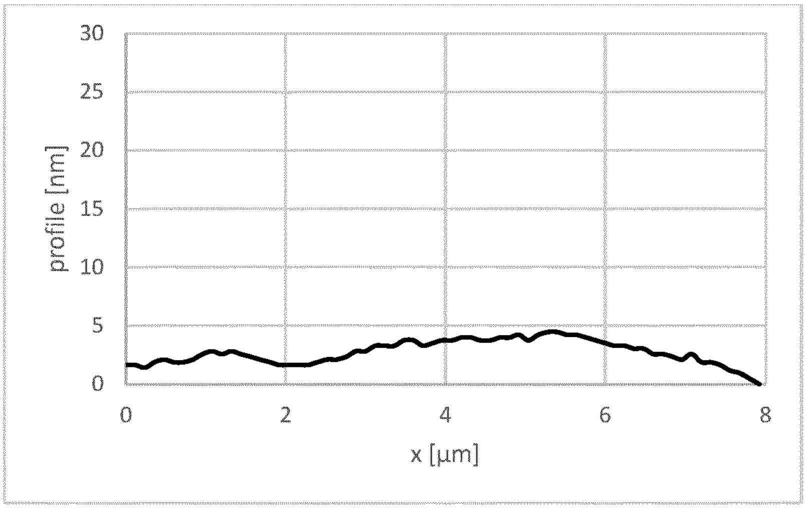


Figure 1

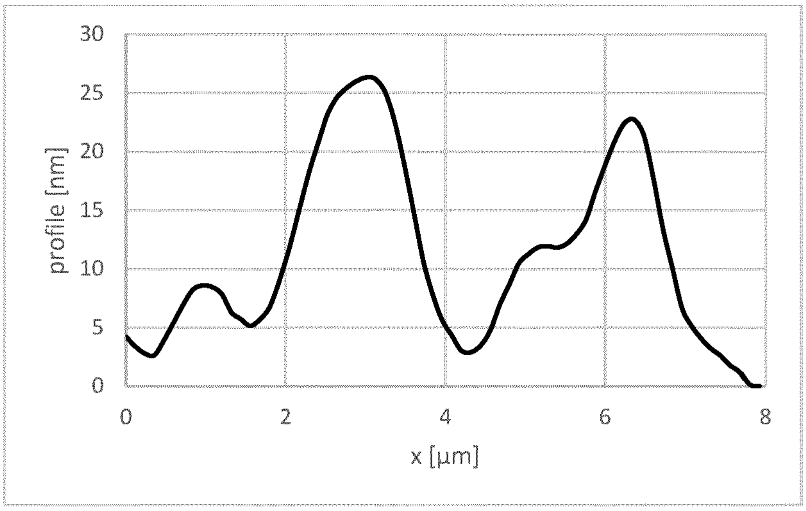


Figure 2

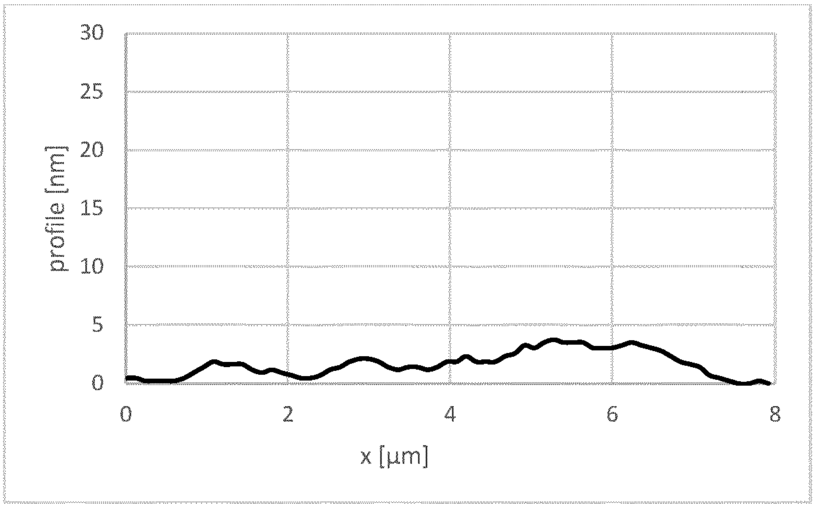


**Figure 3**

| |
|---|
| B-EML |
| HTL |
| HIL |
| ITO Anode |
| Substrate |

**Figure 4a**

| |
|---|
| Al Cathode |
| EIL |
| ETL2 |
| ETL1 |
| B-EML |
| HTL |
| HIL |
| ITO Anode |
| Substrate |

**Figure 4b**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 4539507 A
- US 4769292 A
- WO 2009100925 A
- US 2009026919 A1
- WO 2015158409 A
- WO 2006108497 A
- WO 2006122630 A
- WO 2008006449 A
- WO 2007140847 A
- WO 2010012328 A
- WO 2017036573 A
- WO 2016150544 A
- WO 2017028940 A
- WO 2017028941 A
- WO 2012048780 A
- WO 2013185871 A
- WO 2014037077 A
- WO 2014106522 A
- WO 2014111269 A
- WO 2017036574 A
- WO 2018007421 A
- WO 2018095888 A
- WO 2018095940 A
- WO 2019076789 A
- WO 2019170572 A
- EP 2019072697 W
- EP 2019072670 W
- EP 2019072662 W
- WO 2015102118 A
- CN 108409769
- CN 107266484
- WO 2017195669 A
- US 2018069182 A
- EP 19168728
- EP 19199326
- EP 19208643
- WO 2005011013 A
- EP 676461 A
- WO 2004081017 A
- WO 2004058911 A
- WO 2005084081 A
- WO 2005084082 A
- WO 2006048268 A
- WO 2006117052 A
- WO 2008145239 A
- JP 2000053957 A
- WO 2003060956 A
- WO 2004028217 A
- WO 2004080975 A
- WO 2010072300 A
- WO 06122630 A
- WO 06100896 A
- EP 1661888 A
- WO 01049806 A
- US 5061569 A
- WO 9509147 A
- WO 08006449 A
- WO 07140847 A
- WO 2012034627 A
- WO 2013120577 A
- EP 2875092 A
- EP 2875699 A
- EP 2875004 A
- WO 2013083216 A
- WO 2012150001 A
- WO 2016107668 A
- WO 2013081052 A
- EP 2325190 A
- WO 2013156130 A
- WO 2018114882 A


### Non-patent literature cited in the description


- **Y. SHIROTA et al.** *Chem. Rev.,* 2007, vol. 107 (4), 953-1010
- **M. S. ARNOLD et al.** *Appl. Phys. Lett.,* 2008, vol. 92, 053301
- *CHEMICAL ABSTRACTS,* 20851-90-5
- *CHEMICAL ABSTRACTS,* 460347-59-5
- *CHEMICAL ABSTRACTS,* 1818872-74-0
- *CHEMICAL ABSTRACTS,* 1818872-85-3
- *CHEMICAL ABSTRACTS,* 1818872-84-2
- *CHEMICAL ABSTRACTS,* 1818872-89-7
- *CHEMICAL ABSTRACTS,* 1273319-88-2